# EUROPEAN PATENT APPLICATION

(11) **EP 4 613 865 A1**
(43) Date of publication of application: **10.09.2025**
(21) Application number: 24174982.9
(22) Date of filing: 08.05.2024
(51) Int. Cl.: C12N 15/70, C12N 15/81, C12N 15/82, C12P 21/02, A23L 29/281, C07K 14/78

(54) **ANIMAL COLLAGEN FROM NON-ANIMAL SOURCES**

(30) Priority: 05.03.2024 EP 24161398
(71) Applicant: The Cultivated B. GmbH, 69124 Heidelberg (DE)
(72) Inventor: GALPERIN, Ilya, 69124 Heidelberg (DE); WEBER, Konrad, 69124 Heidelberg (DE); BITTENCOURT MELANI, Natália, 69124 Heidelberg (DE); BLUTHARDT, Nicolai, 69124 Heidelberg (DE); DAKOVIC, Sara, 69124 Heidelberg (DE); MERIÑO-SERRANO, Carolina, 69124 Heidelberg (DE)
(74) Representative: Jacobi, Markus Alexander

(57) **Abstract**

The present invention relates to a non-animal organism expressing a collagen. A non-animal organism expressing a collagen that naturally occurs extracellularly in one or more animal species. Furthermore, the invention refers to methods for preparing such collagen and for preparing a comestible nutrient product comprising such collagen. Furthermore, the invention relates to a comestible nutrient product comprising such collagen obtained from a method of the present invention.

## Description

The present invention relates to a non-animal organism expressing a collagen. A non-animal organism expressing a collagen that naturally occurs extracellularly in one or more animal species. Furthermore, the invention refers to methods for preparing such collagen and for preparing a comestible nutrient product comprising such collagen. Furthermore, the invention relates to a comestible nutrient product comprising such collagen obtained from a method of the present invention.

There is a considerable global demand for animal protein in the fields of nutrition as well as pharmaceutical uses. However, there are increasing ethical and economic constraints and concerns when using such proteins obtained from slaughtered animals. Furthermore, undesirable material such as xenobiotics, hormones and precursors thereof and high caloric ingredients are inevitably present in animal-derived polypeptides, such as collagen, depending on the growth conditions of the animals.

Therefore, there is a considerable interest for obtaining polypeptides of animal origin (animal-like polypeptides such as collagen) obtained from other sources without the need of slaughtering an animal.

It was surprisingly found that a number of collagens that naturally occur extracellularly in one or more animal species could be efficiently obtained from expression in non-animal organism of various species of various living being kingdoms such as such as plants, fungi and bacteria. This is experimentally evidenced as shown below.

An aspect of the present invention relates to a non-animal organism expressing a collagen that naturally occurs extracellularly in one or more animal species.

In other words, the present invention relates to a non-animal organism expressing a collagen that originates from an animal species in the animal's extracellular space. The term "non-animal organism" may be understood in the broadest sense as an organism that is not of animal origin. Thus, the term "non-animal organism" may be understood in the broadest sense as a living being of any biological kingdom, excluding an animal, such as, e.g., an organ or tissue of one or more plants, one or more fungi, one or more protista, one or more eubacteria, and/or one or more archaebacterial. The non-animal organism may be a monocellular organism or may be a multicellular organism, which optionally has different cell types and/or tissues. In one embodiment, the non-animal organism is a protist. In one embodiment, the non-animal organism is a eukaryote. Using a eukaryote may have the advantage that posttranslational modifications may be included.

As used herein, the term "animal" may be understood in the broadest sense as commonly understood in the art. Typically, an animal is a multicellular, eukaryotic organism in the biological kingdom Animalia, typically consuming organic material and breathing oxygen, and having myocytes as typical cell types. Typically, animal cells do not have a cell wall, neither of cellulose, hemicellulose nor of chitin.

The terms "organism", "living being", "creature", and "living creature" may be understood in the broadest sense as a multicellular organism or a single cellular organism. In a preferred embodiment, the non-animal organism is selected from the group consisting of a plant, a fungus, a protist, a bacterium, and an archaebacterium. In a preferred embodiment, the non-animal organism is selected from the group consisting of a plant, a fungus, and a bacterium.

In a preferred embodiment, the non-animal organism is selected from the group consisting of:
(A) a plant, in particular a cultivated plant, in particular tobacco or soy;
(B) a fungus, preferably a yeast cell, in particular *Pichia pastoris;* or
(C) a bacterium, in particular an *Escherichia coli* bacterium.

In a preferred embodiment, the non-animal organism is a plant. In a preferred embodiment, the non-animal organism is a fungi, in particular yeast. In a preferred embodiment, the non-animal organism is a bacterium, in particular *Escherichia coli.*

As used herein, a polypeptide, in particular collagen, that naturally occurs extracellularly in one or more animal species may be understood in the broadest sense as any polypeptide, in particular collagen, that is physiologically located in the extracellular space of at least one animal species. Such extracellular space may be any location outside the cell lumen and cell membrane. An extracellular space may be an extracellular matrix that may be solid.

In the context of the present invention, the terms "polypeptide" and "protein", in particular when used in the context of collagen in the present invention, may be understood interchangeably in the broadest sense as a compound mainly composed of natural amino acid moieties consecutively conjugated with one another via amide bonds. It will be understood that a protein in the sense of the present invention may or may not be subjected to one or more posttranslational modifications and/or be conjugated with one or more non-amino acid moiety/moieties. The termini of the protein may optionally be capped by any means known in the art, such as, e.g., amidation, acetylation, methylation, and/or acylation. Posttranslational modifications are well-known in the art and may be but may not be limited to lipidation, phosphorylation, sulfatation, glycosylation, truncation, oxidation, reduction, decarboxylation, acetylation, amidation, deamidation, disulfide bond formation, hydroxylation, amino acid addition, cofactor addition (e.g., biotinylation, heme addition, eicosanoid addition, steroid addition) and complexation of metal ions, non-metal ions, peptides or small molecules and addition of iron-sulphide clusters. Moreover, optionally, co-factors, in particular cyclic guanidinium monophosphate (cGMP), but optionally also such as, e.g., ATP, ADP, NAD+, NADH+H+, NADP+, NADPH+H+, metal ions, anions, lipids, etc. may be bound to the protein, irrespective on the biological influence of these co-factors. It will be understood that such polypeptide, such as collagen, may also bear one or more non-natural amino acid moiety/moieties and/or one or more posttranscriptional modifications. The one or more optional posttranslational modifications typically depend on the non-animal organism in which the polypeptide, such as collagen, of interest is expressed.

The expression of the polypeptide, such as collagen, may be permanent or may be inducible. This may be adjusted by the choice of a promoter and/or enhancer. In a case where the promoter and/or enhancer is permanently active, the polypeptide, such as collagen, may be permanently expressed. When the promoter and/or enhancer is controllable by a certain component, the non-animal organism may be grown without expression of the protein and triggering expression whenever it is desired.

The collagen may be expressed in any form. For instance, it may be provided in a gel-like or liquid composition, or may be a secret (also: secretion), preferably comprised in a secret, in particular a secret from a gland. As used herein, the terms "secret" and "secretion" may be understood interchangeably.

In a preferred embodiment, the collagen is selected from the list consisting of extracellular matrix of:
(a) skin;
(b) tendon;
(c) bone,
(d) teeth,
(e) cartilage,
(f) ligaments,
(g) blood vessels and
(h) a combination of two or more thereof.

The collagen that is expressed by the non-animal cell may be of any origin. In other words, it may be any animal collagen. The term "... collagen" may be understood in the broadest sense being naturally of this origin or a collagen of high homology (or identity) of at least 80%, of at least 85%, of at least 90%, of at least 95%, of at least 98%, of at least 99%, or 100% being naturally of this origin. This may include native and mutated collagen types. In other words, the respective collagen or a highly homologous collagen thereof is typically found and/or expressed in the respective species. Typically, the collagen or a precursor thereof is genetically encoded in the respective animal organism. Optionally, it may be based on a spliced genetic sequence and/or may be subjected to posttranslational splicing.

Collagens are well-known polypeptides, which typically bear a considerable content of proline. Collagen may be any type of collagen such as, e.g., COL1 (E.f.g, COL1A1 or COL1A2) or COL2. Collagen may form hydroxylated heterodimeric helices. Lysine residues may be hydroxylated (e.g., by lysyl hydroxylase (LH)). In addition, proline may be hydroxylated (e.g., by prolyl-4-hydroxylase (P4H)).

In a preferred embodiment, the collagen that is expressed by the non-animal cell is a vertebrate collagen. In a preferred embodiment, the collagen that is expressed by the non-animal cell is a mammal collagen, including a human or non-human collagen, a bird collagen, a reptile collagen, or a fish collagen. In a preferred embodiment, the collagen that is expressed by the non-animal cell is a mammal collagen, including a human or non-human collagen, or a bird collagen. In a preferred embodiment, the collagen that is expressed by the non-animal cell is a human, a bovine, a porcine, a sheep, a goat, a horse, a donkey, or a chicken collagen.

Alternatively, it may also be an invertebrate collagen such as an arthropod collagen such as, e.g., an insect or crustacean collagen.

In a preferred embodiment, the collagen that is expressed by the non-animal cell has at least 80%, at least 85%, at least 90%, at least 95%, at least 98%, at least 99%, or 100% homology (or identity) of an extracellular matrix collagen such as collagen, more preferably wherein the extracellular matrix collagen is selected from mammal extracellular matrix collagen and bird extracellular matrix collagen.

In a preferred embodiment, the collagen is of comestible grade. In a preferred embodiment, the collagen is of pharmaceutical grade.

In a preferred embodiment, the collagen that is expressed by the non-animal cell has at least 80%, at least 85%, at least 90%, at least 95%, at least 98%, at least 99%, or 100% homology (or identity) of bovine collagen (COL1A1, collagen alpha 1(I)) (>sp|P02453|CO1A1_BOVIN Collagen alpha-1(I) chain OS=Bos taurus OX=9913 GN=COL1A1 PE=1 SV=3) of SEQ ID NO: 1:

MFSFVDLRLLLLLAATALLTHGQEEGQEEGQEEDIPPVTCVQNGLRYHDRDVWK
PVPCQICVCDNGNVLCDDVICDELKDCPNAKVPTDECCPVCPEGQESPTDQETT
GVEGPKGDTGPRGPRGPAGPPGRDGIPGQPGLPGPPGPPGPPGPPGLGGNFA
PQLSYGYDEKSTGISVPGPMGPSGPRGLPGPPGAPGPQGFQGPPGEPGEPGA
SGPMGPRGPPGPPGKNGDDGEAGKPGRPGERGPPGPQGARGLPGTAGLPGM
KGHRGFSGLDGAKGDAGPAGPKGEPGSPGENGAPGQMGPRGLPGERGRPGA
PGPAGARGNDGATGAAGPPGPTGPAGPPGFPGAVGAKGEGGPQGPRGSEGP
QGVRGEPGPPGPAGAAGPAGNPGADGQPGAKGANGAPGIAGAPGFPGARGP
SGPQGPSGPPGPKGNSGEPGAPGSKGDTGAKGEPGPTGIQGPPGPAGEEGKR
GARGEPGPAGLPGPPGERGGPGSRGFPGADGVAGPKGPAGERGAPGPAGPK
GSPGEAGRPGEAGLPGAKGLTGSPGSPGPDGKTGPPGPAGQDGRPGPPGPP
GARGQAGVMGFPGPKGAAGEPGKAGERGVPGPPGAVGPAGKDGEAGAQGPP
GPAGPAGERGEQGPAGSPGFQGLPGPAGPPGEAGKPGEQGVPGDLGAPGPS
GARGERGFPGERGVQGPPGPAGPRGANGAPGNDGAKGDAGAPGAPGSQGAP
GLQGMPGERGAAGLPGPKGDRGDAGPKGADGAPGKDGVRGLTGPIGPPGPAG
APGDKGEAGPSGPAGPTGARGAPGDRGEPGPPGPAGFAGPPGADGQPGAKG
EPGDAGAKGDAGPPGPAGPAGPPGPIGNVGAPGPKGARGSAGPPGATGFPGA

AGRVGPPGPSGNAGPPGPPGPAGKEGSKGPRGETGPAGRPGEVGPPGPPGP
AGEKGAPGADGPAGAPGTPGPQGIAGQRGVVGLPGQRGERGFPGLPGPSGEP
GKQGPSGASGERGPPGPMGPPGLAGPPGESGREGAPGAEGSPGRDGSPGAK
GDRGETGPAGPPGAPGAPGAPGPVGPAGKSGDRGETGPAGPAGPIGPVGARG
PAGPQGPRGDKGETGEQGDRGIKGHRGFSGLQGPPGPPGSPGEQGPSGASG
PAGPRGPPGSAGSPGKDGLNGLPGPIGPPGPRGRTGDAGPAGPPGPPGPPGP
PGPPSGGYDLSFLPQPPQEKAHDGGRYYRADDANVVRDRDLEVDTTLKSLSQQ
IENIRSPEGSRKNPARTCRDLKMCHSDWKSGEYWIDPNQGCNLDAIKVFCNMET
GETCVYPTQPSVAQKNWYISKNPKEKRHVWYGESMTGGFQFEYGGQGSDPAD
VAIQLTFLRLMSTEASQNITYHCKNSVAYMDQQTGNLKKALLLQGSNEIEIRAEGN
SRFTYSVTYDGCTSHTGAWGKTVIEYKTTKTSRLPIIDVAPLDVGAPDQEFGFDV
GPACFL

The sequence without propeptides may be of SEQ ID NO: 2:

QLSYGYDEKSTGISVPGPMGPSGPRGLPGPPGAPGPQGFQGPPGEPGEPGAS
GPMGPRGPPGPPGKNGDDGEAGKPGRPGERGPPGPQGARGLPGTAGLPGMK
GHRGFSGLDGAKGDAGPAGPKGEPGSPGENGAPGQMGPRGLPGERGRPGAP
GPAGARGNDGATGAAGPPGPTGPAGPPGFPGAVGAKGEGGPQGPRGSEGPQ
GVRGEPGPPGPAGAAGPAGNPGADGQPGAKGANGAPGIAGAPGFPGARGPS
GPQGPSGPPGPKGNSGEPGAPGSKGDTGAKGEPGPTGIQGPPGPAGEEGKRG
ARGEPGPAGLPGPPGERGGPGSRGFPGADGVAGPKGPAGERGAPGPAGPKG
SPGEAGRPGEAGLPGAKGLTGSPGSPGPDGKTGPPGPAGQDGRPGPPGPPGA
RGQAGVMGFPGPKGAAGEPGKAGERGVPGPPGAVGPAGKDGEAGAQGPPGP
AGPAGERGEQGPAGSPGFQGLPGPAGPPGEAGKPGEQGVPGDLGAPGPSGA
RGERGFPGERGVQGPPGPAGPRGANGAPGNDGAKGDAGAPGAPGSQGAPGL
QGMPGERGAAGLPGPKGDRGDAGPKGADGAPGKDGVRGLTGPIGPPGPAGAP
GDKGEAGPSGPAGPTGARGAPGDRGEPGPPGPAGFAGPPGADGQPGAKGEP
GDAGAKGDAGPPGPAGPAGPPGPIGNVGAPGPKGARGSAGPPGATGFPGAAG
RVGPPGPSGNAGPPGPPGPAGKEGSKGPRGETGPAGRPGEVGPPGPPGPAG
EKGAPGADGPAGAPGTPGPQGIAGQRGVVGLPGQRGERGFPGLPGPSGEPGK
QGPSGASGERGPPGPMGPPGLAGPPGESGREGAPGAEGSPGRDGSPGAKGD
RGETGPAGPPGAPGAPGAPGPVGPAGKSGDRGETGPAGPAGPIGPVGARGPA
GPQGPRGDKGETGEQGDRGIKGHRGFSGLQGPPGPPGSPGEQGPSGASGPA
GPRGPPGSAGSPGKDGLNGLPGPIGPPGPRGRTGDAGPAGPPGPPGPPGPPG
PPSGGYDLSFLPQPPQEKAHDGGRYYRA

In a preferred embodiment, the collagen that is expressed by the non-animal cell may be based on a DNA sequence having at least 80%, at least 85%, at least 90%, at least 95%, at least 98%, at least 99%, or 100% homology (or identity) of a sequence for the expression in E. *coli* within pET-21 (+) from twist bioscience without propeptides (RBS with ATG from pET151) of SEQ ID NO: 3:

TAAGAAGGAGATATACATATGCATCACCATCACCATCACGAAAACCTGTATTT
TCAGGGC
CAACTGTCATATGGCTATGACGAGAAAAGCACCGGTATATCTGTCCCGGGGC
CAATGGGCCCATCAGGACCACGCGGTTTGCCAGGCCCACCGGGTGCCCCG
GGTCCTCAAGGATTCCAAGGACCACCCGGTGAACCAGGTGAACCGGGTGCT
TCTGGCCCGATGGGTCCTAGAGGTCCGCCGGGGCCCCCAGGCAAGAATGGA
GATGATGGTGAAGCGGGTAAACCAGGCAGACCCGGTGAGAGAGGTCCCCCG
GGACCACAGGGCGCTCGCGGACTTCCAGGTACAGCGGGATTGCCTGGAATG
AAAGGGCATCGTGGCTTTTCTGGCTTGGATGGAGCAAAAGGCGATGCTGGG
CCAGCAGGTCCAAAGGGTGAACCAGGAAGTCCAGGAGAAAATGGTGCTCCT
GGTCAAATGGGACCCCGTGGGTTACCAGGTGAGCGGGGACGTCCAGGAGCT
CCCGGCCCCGCAGGTGCTCGCGGTAACGACGGTGCCACCGGTGCGGCTGG
ACCACCGGGTCCTACAGGGCCGGCAGGGCCCCCTGGATTTCCTGGTGCAGT
AGGTGCGAAGGGAGAAGGTGGACCTCAAGGTCCCCGAGGATCGGAAGGCC
CCCAAGGAGTCCGCGGTGAGCCAGGTCCGCCGGGTCCTGCTGGTGCAGCA
GGGCCGGCTGGTAATCCAGGTGCGGATGGACAGCCTGGTGCCAAAGGTGCA
AATGGTGCTCCGGGGATTGCCGGCGCCCCAGGATTTCCCGGTGCTAGAGGT
CCATCTGGCCCGCAAGGTCCTTCTGGACCGCCGGGCCCTAAAGGCAACAGC
GGTGAACCAGGGGCTCCCGGAAGCAAAGGAGATACCGGAGCCAAGGGAGA
ACCGGGCCCGACCGGGATTCAGGGCCCACCGGGTCCAGCAGGCGAAGAAG
GTAAACGCGGTGCACGTGGGGAACCAGGTCCTGCAGGCTTACCGGGTCCCC
CTGGTGAGAGAGGCGGTCCGGGTTCACGAGGCTTTCCAGGAGCGGATGGAG
TGGCAGGCCCTAAAGGGCCGGCGGGAGAGCGGGGCGCCCCTGGCCCCGC
AGGTCCAAAAGGGTCTCCAGGTGAAGCTGGGCGTCCGGGTGAAGCAGGATT
GCCTGGTGCAAAGGGGTTAACTGGCAGTCCTGGTAGTCCGGGACCGGATGG
TAAAACTGGTCCGCCGGGCCCGGCAGGTCAAGATGGACGTCCAGGACCCCC
GGGACCCCCTGGAGCTCGAGGTCAAGCTGGAGTAATGGGCTTTCCGGGTCC
GAAGGGAGCAGCGGGCGAACCCGGAAAGGCAGGCGAACGGGGAGTTCCAG
GTCCACCCGGTGCAGTAGGCCCAGCAGGGAAAGATGGCGAGGCTGGTGCT
CAAGGGCCACCTGGTCCAGCAGGCCCGGCTGGCGAACGTGGAGAACAAGG
ACCGGCGGGAAGCCCTGGATTTCAGGGATTGCCTGGCCCAGCGGGACCCCC
TGGAGAGGCGGGTAAACCAGGAGAACAAGGTGTACCAGGAGATCTTGGTGC

ACCTGGTCCTAGCGGAGCTCGTGGTGAAAGAGGTTTTCCAGGTGAGCGCGG
TGTTCAAGGTCCCCCGGGGCCAGCAGGACCTCGTGGAGCAAATGGTGCGCC
AGGTAACGATGGCGCAAAAGGTGATGCCGGTGCACCAGGTGCACCAGGGTC
ACAGGGTGCTCCCGGGTTACAAGGAATGCCAGGTGAGAGAGGTGCCGCAGG
GTTACCTGGGCCGAAGGGCGATAGAGGCGACGCTGGACCAAAAGGTGCCGA
TGGAGCTCCGGGTAAGGATGGAGTCAGAGGTTTAACAGGACCAATAGGACC
CCCAGGTCCTGCTGGTGCCCCTGGCGATAAAGGTGAGGCAGGTCCTAGTGG
TCCAGCCGGTCCCACAGGTGCACGCGGAGCTCCAGGTGATCGCGGCGAACC
GGGACCACCGGGGCCTGCTGGGTTTGCGGGTCCACCTGGTGCCGATGGAC
AACCTGGTGCGAAGGGCGAACCCGGTGATGCAGGCGCGAAGGGCGATGCA
GGACCACCAGGACCAGCTGGCCCGGCAGGTCCGCCGGGTCCTATCGGAAA
CGTCGGTGCGCCCGGGCCTAAAGGTGCTAGAGGGTCTGCAGGCCCGCCAG
GTGCTACTGGTTTCCCTGGTGCAGCTGGTCGTGTAGGGCCACCAGGCCCTT
CAGGAAATGCTGGTCCGCCTGGACCACCTGGGCCAGCAGGAAAAGAGGGGA
GTAAAGGACCACGTGGTGAGACAGGTCCGGCTGGCCGGCCTGGTGAAGTCG
GTCCACCTGGTCCGCCCGGACCCGCTGGAGAGAAAGGTGCACCGGGTGCA
GACGGTCCGGCTGGAGCGCCTGGAACTCCAGGCCCTCAAGGAATCGCCGG
CCAAAGAGGCGTAGTTGGTTTACCGGGTCAGCGCGGTGAACGTGGTTTTCCT
GGACTTCCTGGTCCTTCCGGTGAACCGGGTAAGCAAGGACCGTCTGGAGCA
AGCGGGGAACGTGGACCACCGGGCCCGATGGGTCCACCTGGGTTAGCTGG
TCCACCGGGCGAGTCTGGTAGAGAAGGCGCACCCGGGGCAGAAGGTTCAC
CAGGACGAGACGGCTCACCAGGGGCCAAAGGTGATAGAGGCGAAACAGGA
CCAGCGGGCCCGCCGGGAGCACCTGGTGCGCCGGGCGCTCCAGGTCCAGT
TGGACCGGCTGGAAAGTCAGGCGATAGAGGGGAAACGGGCCCGGCGGGAC
CAGCCGGCCCTATTGGACCAGTAGGAGCTCGTGGGCCGGCGGGGCCACAA
GGCCCTCGTGGAGATAAAGGTGAGACCGGTGAACAGGGAGATCGTGGGATT
AAGGGTCATCGAGGATTTTCAGGGCTTCAAGGACCGCCTGGTCCACCGGGC
TCGCCAGGTGAGCAGGGTCCCTCTGGTGCTTCTGGACCAGCTGGTCCACGT
GGCCCGCCAGGCAGTGCAGGCTCGCCAGGTAAAGATGGTCTTAACGGGTTA
CCTGGCCCAATTGGGCCACCAGGTCCTCGTGGTCGTACAGGCGATGCAGGT
CCAGCAGGTCCGCCTGGACCGCCAGGACCGCCTGGTCCCCCTGGTCCCCCA
TCAGGCGGATATGACCTTTCATTTCTCCCTCAGCCACCGCAAGAAAAGGCTC
ATGATGGTGGACGTTATTATCGCGCTTAA

In a preferred embodiment, the collagen that is expressed by the non-animal cell may be based on a DNA sequence having at least 80%, at least 85%, at least 90%, at least 95%, at least 98%, at least 99%, or 100% homology (or identity) of codon-optimized sequence without tags or propeptides of SEQ ID NO: 4:

CAGCTGTCTTACGGGTATGATGAAAAAAGCACCGGCATTTCAGTTCCGGGCC
CGATGGGCCCGAGCGGTCCCCGTGGCCTGCCGGGCCCGCCGGGCGCGCC
GGGTCCGCAGGGCTTCCAAGGCCCGCCGGGCGAACCGGGTGAACCGGGCG
CCAGCGGCCCGATGGGCCCACGTGGCCCGCCGGGCCCGCCGGGCAAAAAT
GGCGATGATGGAGAAGCCGGCAAACCGGGCCGTCCGGGTGAACGTGGCCC
GCCGGGTCCGCAGGGCGCGCGCGGCCTGCCGGGTACCGCCGGCCTGCCG
GGCATGAAAGGTCATCGCGGCTTCAGCGGCCTGGATGGCGCCAAAGGTGAT
GCGGGTCCGGCGGGCCCGAAAGGCGAACCGGGCAGTCCGGGCGAAAATGG
CGCCCCGGGCCAGATGGGTCCGCGCGGTCTGCCGGGTGAACGTGGCCGCC
CGGGCGCGCCGGGTCCGGCAGGCGCGCGCGGCAACGATGGCGCGACCGG
CGCCGCCGGCCCGCCGGGCCCGACCGGCCCGGCGGGCCCGCCGGGATTT
CCGGGTGCTGTGGGCGCGAAAGGCGAAGGTGGCCCGCAGGGCCCGCGTGG
TAGCGAAGGTCCGCAGGGCGTTCGCGGCGAACCGGGCCCGCCGGGCCCGG
CAGGCGCGGCCGGCCCGGCGGGCAATCCGGGTGCCGATGGCCAGCCGGG
GGCCAAAGGCGCCAATGGTGCGCCGGGCATCGCGGGTGCACCGGGTTTTC
CGGGCGCGCGCGGCCCGAGCGGCCCGCAGGGCCCGAGCGGTCCGCCGGG
CCCGAAGGGCAACAGCGGCGAACCGGGCGCCCCGGGCAGCAAAGGCGATA
CCGGCGCGAAAGGCGAACCTGGCCCGACCGGCATTCAGGGCCCGCCGGGC
CCGGCAGGCGAAGAAGGCAAACGTGGCGCCCGCGGCGAACCGGGCCCGG
CGGGTCTGCCGGGCCCGCCGGGCGAACGTGGCGGCCCGGGCAGTCGCGG
CTTTCCGGGCGCCGATGGCGTGGCAGGCCCGAAAGGTCCGGCGGGCGAAC
GTGGCGCCCCGGGCCCGGCGGGCCCGAAAGGCAGCCCGGGGGAAGCGGG
CCGTCCGGGTGAAGCCGGCCTGCCGGGCGCGAAAGGCCTGACCGGCTCGC
CGGGCAGCCCGGGCCCGGATGGCAAAACCGGCCCGCCGGGCCCGGCAGG
CCAGGACGGCCGTCCGGGCCCGCCGGGCCCGCCGGGCGCCCGCGGTCAG
GCCGGCGTCATGGGCTTTCCGGGTCCGAAAGGCGCGGCGGGCGAACCGGG
TAAAGCGGGCGAACGTGGCGTGCCGGGCCCGCCGGGCGCCGTTGGCCCGG
CAGGCAAAGATGGCGAAGCGGGCGCCCAGGGTCCGCCGGGCCCGGCGGG
CCCGGCCGGCGAACGCGGCGAACAGGGCCCGGCGGGCAGCCCGGGTTTTC
AGGGCCTGCCGGGCCCGGCAGGCCCGCCGGGCGAAGCCGGTAAACCGGG
CGAACAGGGCGTGCCTGGCGATCTGGGCGCGCCGGGCCCGTCGGGAGCCC
GTGGTGAACGCGGCTTCCCGGGTGAACGTGGTGTGCAGGGCCCGCCGGGC
CCTGCCGGCCCGCGTGGCGCGAACGGCGCGCCGGGCAACGATGGCGCGAA
AGGCGACGCCGGCGCCCCGGGTGCGCCGGGCAGCCAGGGCGCGCCGGGC
CTGCAGGGTATGCCGGGCGAACGTGGCGCGGCGGGTCTGCCGGGCCCGAA
AGGCGATCGCGGTGATGCTGGCCCGAAAGGCGCGGATGGCGCGCCGGGCA
AGGATGGTGTGCGTGGCCTGACCGGCCCGATTGGCCCGCCGGGCCCGGCA

GGCGCCCCGGGCGATAAAGGAGAAGCGGGCCCGAGCGGCCCGGCCGGCC
CGACCGGTGCCCGTGGTGCGCCGGGCGATCGTGGCGAACCGGGTCCGCCG
GGTCCGGCCGGCTTCGCGGGCCCGCCGGGCGCGGATGGCCAGCCGGGCG
CGAAAGGCGAACCTGGCGATGCCGGTGCAAAGGGAGACGCGGGCCCTCCG
GGCCCGGCGGGCCCGGCCGGACCACCGGGCCCGATTGGCAATGTTGGCGC
GCCGGGCCCGAAAGGCGCACGTGGTAGCGCGGGCCCTCCGGGCGCCACCG
GCTTTCCGGGCGCGGCCGGCCGTGTGGGCCCGCCGGGCCCGAGCGGCAAC
GCGGGACCGCCGGGCCCGCCGGGCCCGGCCGGCAAAGAAGGCAGCAAAG
GCCCGCGTGGCGAAACTGGCCCGGCCGGCCGTCCGGGCGAAGTTGGCCCG
CCGGGCCCGCCGGGCCCGGCCGGTGAAAAAGGTGCCCCGGGTGCCGACG
GCCCGGCGGGCGCACCGGGTACCCCGGGTCCGCAGGGTATTGCGGGCCAG
CGCGGCGTTGTTGGTCTGCCGGGTCAACGTGGCGAACGCGGTTTCCCGGGC
CTGCCGGGTCCGAGCGGCGAACCGGGTAAACAGGGCCCCAGCGGCGCCTC
GGGCGAACGCGGTCCGCCGGGCCCGATGGGCCCGCCGGGCCTGGCGGGC
CCGCCGGGCGAAAGTGGCCGCGAAGGCGCGCCGGGTGCAGAAGGCAGTCC
GGGCCGTGATGGTTCACCGGGTGCGAAAGGTGATCGCGGCGAGACAGGTC
CGGCCGGCCCGCCGGGCGCGCCGGGCGCCCCGGGCGCACCGGGTCCGGT
CGGTCCGGCGGGCAAATCAGGAGATCGCGGCGAAACCGGTCCGGCGGGTC
CGGCGGGCCCGATTGGTCCGGTGGGCGCGCGTGGCCCGGCAGGCCCGCA
GGGTCCGCGCGGTGATAAAGGCGAAACCGGCGAGCAGGGCGATCGCGGTA
TTAAAGGCCATCGTGGTTTCAGCGGTCTGCAAGGCCCGCCGGGCCCGCCGG
GCTCGCCGGGCGAACAGGGCCCGTCGGGTGCGTCCGGCCCTGCCGGTCCG
CGCGGCCCGCCGGGCAGCGCCGGCAGCCCGGGCAAAGATGGTTTAAACGG
CCTGCCGGGCCCGATTGGCCCGCCGGGCCCGCGCGGCCGCACCGGCGATG
CCGGCCCGGCAGGCCCGCCGGGCCCGCCGGGCCCGCCGGGTCCGCCGGG
CCCGCCGAGCGGCGGCTATGATCTGAGCTTTCTGCCGCAGCCGCCGCAGGA
AAAAGCGCATGATGGTGGCCGCTACTATCGAGCGTAA

In a preferred embodiment, the collagen that is expressed by the non-animal cell may be based on a DNA sequence having at least 80%, at least 85%, at least 90%, at least 95%, at least 98%, at least 99%, or 100% homology (or identity) of codon-optimized sequence for the expression in P. pastoris from VB with His Tag and TEV of SEQ ID NO: 5:

ATGAGATTTCCTTCAATTTTTACTGCTGTTTTATTCGCAGCATCCTCCGCATTA
GCTGCTCCAGTCAACACTACAACAGAAGATGAAACGGCACAAATTCCGGCTG
AAGCTGTCATCGGTTACTCAGATTTAGAAGGGGATTTCGATGTTGCTGTTTTG
CCATTTTCCAACAGCACAAATAACGGGTTATTGTTTATAAATACTACTATTGCC

AGCATTGCTGCTAAAGAAGAAGGGGTATCTCTCGAGAAAAGAGAGGCTGAAG
CTCATCACCATCACCATCACGAAAACCTGTATTTTCAGGGCCAATTGTCTTAT
GGTTACGATGAAAAGTCCACTGGTATTTCTGTCCCTGGACCAATGGGTCCAT
CAGGTCCTAGAGGTTTGCCAGGTCCTCCAGGTGCTCCAGGTCCACAAGGTTT
TCAAGGTCCACCAGGTGAACCAGGAGAACCCGGTGCTTCTGGTCCAATGGG
TCCAAGAGGTCCACCTGGACCTCCTGGTAAGAACGGTGATGATGGTGAGGCT
GGTAAGCCAGGAAGACCTGGAGAAAGAGGACCACCTGGTCCACAAGGAGCT
AGAGGTTTACCCGGTACAGCTGGTTTGCCTGGTATGAAGGGTCATAGAGGTT
TCTCCGGTTTGGATGGAGCTAAGGGTGATGCTGGACCTGCTGGTCCTAAGG
GAGAGCCAGGATCTCCAGGTGAGAACGGAGCTCCTGGACAAATGGGACCAA
GAGGTCTGCCTGGTGAAAGAGGTAGACCAGGTGCTCCTGGTCCTGCCGGAG
CCAGAGGTAATGATGGTGCTACTGGTGCTGCTGGTCCTCCAGGTCCAACTGG
TCCTGCTGGTCCTCCAGGTTTTCCAGGTGCTGTTGGAGCCAAAGGTGAAGGT
GGCCCACAAGGTCCTAGAGGATCTGAAGGTCCACAGGGTGTTAGAGGTGAA
CCAGGTCCACCAGGTCCAGCTGGAGCTGCTGGTCCTGCTGGTAACCCAGGT
GCAGATGGTCAACCAGGTGCTAAGGGTGCCAACGGTGCTCCAGGCATTGCT
GGAGCTCCAGGTTTTCCAGGAGCTAGAGGTCCTTCTGGACCTCAAGGACCAT
CTGGTCCACCTGGACCAAAAGGTAACTCTGGAGAACCAGGTGCTCCTGGTTC
CAAAGGTGATACCGGTGCTAAAGGAGAGCCAGGTCCTACTGGTATTCAAGGT
CCTCCTGGTCCTGCTGGTGAAGAGGGTAAAAGAGGAGCCAGAGGTGAACCT
GGTCCAGCCGGTTTGCCAGGTCCACCAGGTGAAAGAGGAGGTCCAGGATCT
AGAGGTTTTCCAGGTGCTGACGGTGTTGCTGGTCCAAAGGGTCCTGCTGGTG
AGAGAGGAGCCCCAGGTCCAGCTGGACCTAAAGGATCTCCAGGAGAAGCTG
GTCGTCCTGGTGAAGCTGGTCTTCCAGGTGCTAAGGGTTTGACAGGCTCCCC
AGGATCCCCTGGTCCAGATGGTAAGACTGGACCACCTGGACCAGCTGGTCA
AGATGGAAGACCAGGTCCTCCAGGACCACCTGGAGCCAGAGGCCAAGCTGG
TGTTATGGGTTTCCCTGGTCCAAAAGGTGCTGCTGGAGAACCGGGTAAAGCC
GGTGAAAGAGGTGTTCCAGGTCCTCCAGGTGCTGTTGGTCCTGCTGGTAAAG
ATGGAGAAGCCGGTGCTCAAGGACCACCAGGTCCAGCTGGTCCAGCTGGAG
AGAGAGGTGAACAAGGTCCTGCTGGATCCCCAGGTTTCCAAGGTTTGCCTGG
TCCTGCTGGACCACCAGGTGAAGCTGGTAAGCCAGGTGAACAAGGAGTTCC
AGGTGATTTGGGTGCTCCTGGTCCATCCGGTGCTAGAGGTGAAAGAGGATTT
CCAGGAGAGAGAGGTGTTCAAGGACCACCTGGTCCTGCTGGACCAAGAGGA
GCAAACGGTGCTCCAGGAAACGATGGTGCTAAAGGTGATGCTGGTGCTCCA
GGAGCTCCAGGTTCACAAGGTGCTCCAGGTTTGCAAGGAATGCCTGGAGAA
AGAGGTGCTGCTGGATTACCAGGTCCAAAAGGAGACAGAGGTGATGCCGGT
CCAAAGGGTGCTGATGGTGCTCCAGGTAAGGACGGTGTGCGAGGTTTGACT
GGACCAATTGGACCTCCAGGTCCAGCTGGAGCCCCAGGAGATAAGGGTGAA

GCTGGTCCATCGGGTCCTGCTGGTCCTACTGGTGCTAGAGGAGCTCCAGGA
GATAGAGGTGAACCTGGTCCACCTGGACCCGCTGGATTTGCTGGTCCACCA
GGTGCTGATGGTCAACCAGGGGCTAAAGGTGAACCAGGTGACGCTGGAGCT
AAAGGAGATGCCGGTCCACCAGGTCCAGCCGGTCCAGCTGGTCCTCCTGGT
CCAATTGGTAATGTTGGCGCTCCTGGTCCTAAGGGTGCCAGAGGTTCAGCTG
GACCACCAGGTGCTACTGGTTTCCCAGGTGCTGCCGGTAGAGTTGGTCCAC
CAGGTCCATCTGGTAACGCTGGTCCTCCAGGTCCACCAGGTCCTGCTGGAAA
GGAGGGATCTAAGGGACCAAGAGGTGAAACTGGTCCAGCTGGAAGACCTGG
TGAAGTTGGTCCTCCAGGACCACCAGGTCCTGCTGGAGAAAAAGGTGCTCCA
GGTGCTGATGGACCAGCTGGTGCTCCTGGTACCCCAGGTCCACAAGGTATT
GCTGGTCAAAGAGGTGTGGTTGGTCTTCCTGGACAAAGAGGTGAGAGAGGTT
TTCCTGGTTTGCCAGGTCCTTCTGGAGAACCTGGAAAACAGGGTCCCTCTGG
TGCTTCTGGTGAGAGAGGTCCTCCAGGACCAATGGGACCTCCAGGTTTGGCT
GGTCCACCAGGTGAGTCTGGTAGAGAGGGTGCTCCAGGTGCTGAGGGTTCT
CCAGGTAGAGATGGTAGTCCAGGTGCTAAAGGAGACAGAGGAGAGACTGGT
CCAGCTGGTCCTCCAGGTGCTCCTGGTGCTCCCGGTGCTCCTGGTCCTGTC
GGTCCTGCTGGAAAGTCTGGTGATAGAGGTGAGACTGGTCCAGCTGGACCA
GCTGGTCCAATCGGACCAGTTGGAGCTAGAGGTCCTGCTGGTCCTCAAGGT
CCAAGAGGTGACAAGGGTGAAACAGGTGAGCAAGGTGACAGAGGTATTAAA
GGACATAGAGGTTTCTCTGGTCTACAGGGACCTCCTGGTCCTCCAGGTTCCC
CAGGTGAGCAAGGTCCTTCTGGTGCTTCAGGTCCTGCTGGTCCAAGAGGTCC
ACCAGGTTCTGCTGGTTCTCCAGGAAAGGATGGTTTGAATGGTTTGCCTGGA
CCTATTGGTCCTCCTGGACCTAGAGGTAGAACAGGTGATGCTGGTCCTGCTG
GTCCACCTGGTCCTCCTGGTCCTCCTGGTCCCCCTGGACCTCCATCTGGAGG
TTACGATTTGTCTTTTTTGCCACAACCTCCACAGGAAAAAGCCCATGACGGTG
GAAGATATTATAGAGCTTAA

A corresponding collagen sequence may be as follow of SEQ ID NO: 42 as depicted below. In a preferred embodiment, the collagen that is expressed by the non-animal cell has at least 80%, at least 85%, at least 90%, at least 95%, at least 98%, at least 99%, or 100% homology (or identity) of a collagen of SEQ ID NO: 6:

MRFPSIFTAVLFAASSALAAPVNTTTEDETAQIPAEAVIGYSDLEGDFDVAVLPFS
NSTNNGLLFINTTIASIAAKEEGVSLEKREAEAHHHHHHENLYFQGQLSYGYDEK
STGISVPGPMGPSGPRGLPGPPGAPGPQGFQGPPGEPGEPGASGPMGPRGPP
GPPGKNGDDGEAGKPGRPGERGPPGPQGARGLPGTAGLPGMKGHRGFSGLD
GAKGDAGPAGPKGEPGSPGENGAPGQMGPRGLPGERGRPGAPGPAGARGND
GATGAAGPPGPTGPAGPPGFPGAVGAKGEGGPQGPRGSEGPQGVRGEPGPP

GPAGAAGPAGNPGADGQPGAKGANGAPGIAGAPGFPGARGPSGPQGPSGPP
GPKGNSGEPGAPGSKGDTGAKGEPGPTGIQGPPGPAGEEGKRGARGEPGPAG
LPGPPGERGGPGSRGFPGADGVAGPKGPAGERGAPGPAGPKGSPGEAGRPG
EAGLPGAKGLTGSPGSPGPDGKTGPPGPAGQDGRPGPPGPPGARGQAGVMG
FPGPKGAAGEPGKAGERGVPGPPGAVGPAGKDGEAGAQGPPGPAGPAGERG
EQGPAGSPGFQGLPGPAGPPGEAGKPGEQGVPGDLGAPGPSGARGERGFPG
ERGVQGPPGPAGPRGANGAPGNDGAKGDAGAPGAPGSQGAPGLQGMPGER
GAAGLPGPKGDRGDAGPKGADGAPGKDGVRGLTGPIGPPGPAGAPGDKGEAG
PSGPAGPTGARGAPGDRGEPGPPGPAGFAGPPGADGQPGAKGEPGDAGAKG
DAGPPGPAGPAGPPGPIGNVGAPGPKGARGSAGPPGATGFPGAAGRVGPPGP
SGNAGPPGPPGPAGKEGSKGPRGETGPAGRPGEVGPPGPPGPAGEKGAPGA
DGPAGAPGTPGPQGIAGQRGVVGLPGQRGERGFPGLPGPSGEPGKQGPSGAS
GERGPPGPMGPPGLAGPPGESGREGAPGAEGSPGRDGSPGAKGDRGETGPA
GPPGAPGAPGAPGPVGPAGKSGDRGETGPAGPAGPIGPVGARGPAGPQGPRG
DKGETGEQGDRGIKGHRGFSGLQGPPGPPGSPGEQGPSGASGPAGPRGPPG
SAGSPGKDGLNGLPGPIGPPGPRGRTGDAGPAGPPGPPGPPGPPGPPSGGYD
LSFLPQPPQEKAHDGGRYYRA

In a preferred embodiment, a further polypeptide that is expressed by the non-animal cell (preferably in addition to one or more collagens) has at least 80%, at least 85%, at least 90%, at least 95%, at least 98%, at least 99%, or 100% homology (or identity) of prolyl 4-hydroxylase subunit alpha-1 (Q1 RMU3) of SEQ ID NO: 7:

HPGFFTSIGQMTDLIHTEKDLVTSLKDYIKAEEDKLEQIKKWAEKLDRLTSTATKD
PEGFVGHPVNAFKLMKRLNTEWSELENLVLKDMSDGFISNLTIQRQYFPNDEDQ
VGAAKALLRLQDTYNLDTDTISKGDLPGVKHKSFLTVEDCFELGKVAYTEADYYH
TELWMEQALRQLDEGEVSTVDKVSVLDYLSYAVYQQGDLDKALLLTKKLLELDP
EHQRANGNLKYFEYIMAKEKDANKSSSDDQSDQKTTLKKKGAAVDYLPERQKYE
MLCRGEGIKMTPRRQKKLFCRYHDGNRNPKFILAPAKQEDEWDKPRIIRFHDIIS
DAEIEVVKDLAKPRLRRATISNPITGDLETVHYRISKSAWLSGYENPVVSRINMRIQ
DLTGLDVSTAEELQVANYGVGGQYEPHFDFARKDEPDAFKELGTGNRIATWLFY
MSDVLAGGATVFPEVGASVWPKKGTAVFWYNLFASGEGDYSTRHAACPVLVGN
KWVSNKWLHERGQEFRRPCTLSELE

In a preferred embodiment, the optional further polypeptide that is expressed by the non-animal cell (preferably in addition to one or more collagens) may be based on a DNA sequence having at least 80%, at least 85%, at least 90%, at least 95%, at least 98%, at least 99%, or 100% homology (or identity) of codon-optimized polypeptide of SEQ ID NO: 8:

CATCCTGGTTTCTTTACTTCTATTGGACAAATGACTGACTTGATCCATACCGAA
AAGGATTTGGTTACTTCCTTGAAAGATTACATTAAGGCTGAAGAGGATAAATT
GGAACAGATTAAAAAATGGGCTGAAAAGTTGGATAGATTGACTTCCACAGCTA
CTAAAGATCCAGAAGGTTTCGTTGGTCATCCAGTTAACGCTTTTAAGTTGATG
AAGAGATTGAACACTGAATGGTCTGAGTTGGAAAATTTGGTCCTAAAGGATAT
GTCTGACGGTTTTATCTCTAACTTGACTATCCAAAGACAATACTTCCCAAATGA
TGAAGACCAAGTTGGTGCTGCTAAAGCTCTTTTGAGACTGCAAGACACCTAC
AACTTGGATACCGATACAATTTCTAAGGGTGATTTGCCAGGTGTTAAGCACAA
GTCTTTTTTAACCGTTGAAGATTGTTTCGAGTTGGGAAAGGTTGCATATACTG
AGGCTGACTACTACCATACTGAATTGTGGATGGAACAGGCTTTGAGACAATTA
GATGAAGGTGAGGTTTCTACAGTTGATAAGGTTTCTGTTTTGGATTACTTGTC
TTATGCAGTTTACCAGCAAGGTGACTTGGATAAAGCATTGTTGTTGACTAAGA
AGTTGTTGGAATTGGACCCTGAGCATCAAAGAGCAAATGGAAACTTGAAATAT
TTTGAGTACATTATGGCTAAAGAAAAAGATGCAAACAAGTCTTCTTCTGATGAT
CAATCCGACCAAAAGACAACTTTGAAGAAGAAGGGTGCTGCTGTTGATTACC
TTCCAGAAAGACAGAAGTACGAGATGTTATGTAGAGGTGAAGGAATTAAGAT
GACTCCAAGAAGACAGAAAAAGTTGTTTTGTAGATACCATGACGGTAACAGAA
ACCCAAAGTTTATTTTGGCTCCTGCTAAGCAGGAGGATGAGTGGGATAAGCC
TAGAATTATCAGATTCCATGACATTATCTCTGACGCTGAGATCGAGGTTGTTA
AGGATTTGGCTAAGCCAAGATTGAGAAGAGCTACTATTTCTAATCCTATCACT
GGTGATTTGGAGACTGTTCATTATAGAATTTCTAAATCCGCTTGGTTGTCTGG
TTATGAAAACCCTGTTGTGTCTAGAATTAACATGAGAATTCAAGACCTCACTG
GTTTGGATGTTTCTACAGCCGAAGAATTACAAGTCGCTAACTACGGTGTTGGT
GGACAATACGAGCCACATTTCGACTTTGCTCGTAAGGATGAACCAGACGCTT
TTAAGGAATTAGGTACCGGTAACAGAATCGCTACATGGCTTTTCTACATGTCT
GATGTTCTGGCTGGAGGAGCTACTGTTTTTCCAGAAGTTGGTGCTTCTGTCTG
GCCAAAGAAAGGTACCGCTGTGTTTTGGTATAATTTGTTCGCATCTGGTGAAG
GCGATTACTCTACCAGACATGCTGCTTGTCCAGTCTTGGTGGGTAATAAGTG
GGTTTCCAACAAATGGTTGCATGAAAGAGGTCAGGAGTTCAGAAGGCCATGT
ACCTTGTCTGAATTGGAATAA

In a preferred embodiment, a further polypeptide that is expressed by the non-animal cell (preferably in addition to one or more collagens) has at least 80%, at least 85%, at least 90%, at least 95%, at least 98%, at least 99%, or 100% homology (or identity) of protein disulfide-isomerase or prolyl 4-hydroxylase subunit beta (P05307, PDIA1_BOVIN) of SEQ ID NO: 9: 
APDEEDHVLVLHKGNFDEALAAHKYLLVEFYAPWCGHCKALAPEYAKAAGKLKA
EGSEIRLAKVDATEESDLAQQYGVRGYPTIKFFKNGDTASPKEYTAGREADDIVN
WLKKRTGPAASTLSDGAAAEALVESSEVAVIGFFKDMESDSAKQFFLAAEVIDDI
PFGITSNSDVFSKYQLDKDGVVLFKKFDEGRNNFEGEVTKEKLLDFIKHNQLPLVI
EFTEQTAPKIFGGEIKTHILLFLPKSVSDYEGKLSNFKKAAESFKGKILFIFIDSDHT
DNQRILEFFGLKKEECPAVRLITLEEEMTKYKPESDELTAEKITEFCHRFLEGKIKP
HLMSQELPDDWDKQPVKVLVGKNFEEVAFDEKKNVFVEFYAPWCGHCKQLAPI
WDKLGETYKDHENIVIAKMDSTANEVEAVKVHSFPTLKFFPASADRTVIDYNGER
TLDGFKKFLESGGQDGAGDDDDLEDLEEAEEPDLEEDDDQKAVKDEL
Bovine Collagen alpha-1 (1) Expression vector 1
   SEQ ID NO: 10: 
CAACTTTGTATAGAAAAGTTGGCCAACATGGTGGAGCACGACACTCTCGTCT
ACTCCAAGAATATCAAAGATACAGTCTCAGAAGACCAAAGGGCTATTGAGACT
TTTCAACAAAGGGTAATATCGGGAAACCTCCTCGGATTCCATTGCCCAGCTAT
CTGTCACTTCATCAAAAGGACAGTAGAAAAGGAAGGTGGCACCTACAAATGC
CATCATTGCGATAAAGGAAAGGCTATCGTTCAAGATGCCTCTGCCGACAGTG
GTCCCAAAGATGGACCCCCACCCACGAGGAGCATCGTGGAAAAAGAAGACG
TTCCAACCACGTCTTCAAAGCAAGTGGATTGATGTGAACATGGTGGAGCACG
ACACTCTCGTCTACTCCAAGAATATCAAAGATACAGTCTCAGAAGACCAAAGG
GCTATTGAGACTTTTCAACAAAGGGTAATATCGGGAAACCTCCTCGGATTCCA
TTGCCCAGCTATCTGTCACTTCATCAAAAGGACAGTAGAAAAGGAAGGTGGC
ACCTACAAATGCCATCATTGCGATAAAGGAAAGGCTATCGTTCAAGATGCCTC
TGCCGACAGTGGTCCCAAAGATGGACCCCCACCCACGAGGAGCATCGTGGA
AAAAGAAGACGTTCCAACCACGTCTTCAAAGCAAGTGGATTGATGTGATATCT
CCACTGACGTAAGGGATGACGCACAATCCCACTATCCTTCGCAAGACCCTTC
CTCTATATAAGGAAGTTCATTTCATTTGGAGAGGACACGCTGACAAGTTTGTA
CAAAAAAGCAGGCTGCCACCATGCATCACCATCATCATCATGGATCTGAGAA
TTTATATTTTCAAGGTAGCATGTTTTCTTTCGTTGATTTGAGGTTGCTCCTTCTT
TTAGCAGCTACTGCATTGCTTACTCATGGTCAAGAAGAGGGTCAAGAGGAAG
GCCAAGAGGAGGATATTCCACCAGTCACTTGCGTGCAAAATGGTCTTAGGTA
TCATGATAGAGATGTCTGGAAACCTGTTCCATGTCAAATTTGTGTGTGCGATA
ATGGAAACGTGCTATGCGATGATGTTATTTGCGATGAATTGAAAGATTGCCCA
AATGCTAAGGTGCCAACTGATGAATGTTGTCCAGTTTGCCCAGAGGGACAAG
AAAGTCCAACAGATCAAGAAACTACAGGTGTTGAAGGTCCAAAGGGGGATAC
 
TGGCCCTAGAGGTCCACGGGGTCCAGCTGGACCACCAGGAAGAGATGGAAT
TCCAGGTCAACCTGGACTTCCAGGTCCACCAGGACCACCAGGCCCACCAGG
TCCTCCTGGTTTGGGAGGTAATTTTGCACCACAATTGAGTTATGGATACGACG
AAAAATCAACTGGAATATCAGTGCCAGGACCAATGGGTCCATCAGGACCTAG
GGGTCTTCCAGGGCCACCTGGCGCTCCTGGTCCTCAGGGATTTCAAGGTCC
TCCAGGTGAACCTGGAGAGCCTGGTGCTTCTGGTCCAATGGGACCAAGAGG
CCCTCCTGGTCCACCTGGTAAAAATGGTGATGATGGTGAGGCTGGAAAGCCT
GGTCGTCCTGGAGAAAGAGGACCACCAGGACCACAGGGAGCTAGAGGTTTG
CCAGGTACAGCCGGTCTTCCAGGAATGAAAGGACACAGGGGTTTTTCTGGTC
TTGATGGTGCTAAGGGAGACGCAGGACCTGCTGGACCAAAGGGTGAACCTG
GATCTCCTGGTGAAAATGGTGCTCCTGGACAAATGGGACCTAGAGGATTGCC
TGGCGAAAGAGGAAGACCTGGTGCTCCTGGACCAGCTGGAGCTAGAGGAAA
TGATGGAGCTACTGGTGCTGCCGGACCACCAGGACCAACTGGACCAGCTGG
ACCACCTGGTTTTCCAGGAGCAGTTGGGGCTAAGGGAGAAGGTGGACCACA
AGGACCTAGAGGATCAGAAGGACCTCAAGGAGTTAGGGGAGAACCAGGTCC
TCCTGGTCCTGCTGGAGCTGCTGGACCCGCTGGTAACCCAGGAGCAGATGG
ACAACCTGGAGCAAAAGGGGCTAATGGTGCTCCTGGTATTGCAGGTGCTCCT
GGATTCCCTGGTGCTAGAGGACCTTCAGGACCTCAGGGACCATCCGGTCCT
CCAGGACCAAAGGGAAACTCTGGTGAACCAGGGGCTCCTGGATCTAAGGGA
GATACTGGAGCTAAGGGTGAGCCAGGACCTACAGGAATTCAAGGACCACCA
GGACCTGCAGGAGAGGAAGGTAAGAGGGGTGCTAGAGGTGAACCTGGACCA
GCTGGATTGCCAGGCCCACCTGGAGAGAGGGGTGGTCCAGGTTCTAGAGGT
TTTCCAGGAGCTGATGGAGTTGCTGGTCCAAAGGGTCCTGCTGGTGAGAGA
GGTGCACCTGGACCAGCAGGACCTAAAGGATCACCTGGAGAAGCTGGTCGT
CCTGGTGAGGCTGGTCTTCCAGGTGCTAAGGGCCTTACAGGATCTCCAGGAT
CACCAGGTCCAGATGGAAAGACTGGACCACCAGGTCCTGCAGGACAAGATG
GAAGGCCTGGTCCACCTGGACCACCTGGTGCTAGGGGACAAGCTGGTGTTA
TGGGATTCCCAGGTCCAAAGGGTGCAGCTGGTGAGCCTGGTAAAGCTGGTG
AAAGAGGAGTTCCAGGACCTCCTGGTGCTGTTGGACCTGCTGGTAAAGATGG
TGAAGCAGGTGCTCAGGGTCCACCAGGTCCAGCCGGTCCAGCTGGTGAGAG
GGGAGAGCAAGGTCCTGCTGGATCTCCTGGATTTCAAGGTTTGCCTGGACCT
GCTGGACCTCCAGGAGAGGCTGGTAAACCTGGTGAGCAGGGTGTGCCTGGT
GATCTTGGTGCTCCTGGACCTAGTGGCGCAAGAGGCGAAAGGGGATTTCCA
GGTGAAAGAGGAGTTCAGGGACCACCAGGTCCAGCTGGTCCAAGAGGCGCT
AATGGAGCTCCAGGAAATGATGGAGCAAAGGGTGATGCTGGCGCTCCAGGA
GCTCCTGGTTCTCAAGGTGCACCTGGATTGCAAGGAATGCCAGGAGAGAGA
GGTGCAGCTGGTTTGCCGGGACCAAAGGGTGATAGAGGTGATGCTGGTCCT
AAGGGAGCTGATGGAGCTCCAGGAAAGGATGGTGTGAGAGGATTGACTGGT
 
CCAATTGGACCACCAGGTCCTGCTGGAGCACCAGGAGATAAAGGAGAAGCT
GGTCCTTCAGGACCAGCCGGTCCAACTGGAGCTAGAGGAGCTCCAGGAGAT
AGAGGTGAACCAGGACCACCAGGACCTGCTGGGTTTGCAGGTCCACCTGGT
GCTGATGGACAACCTGGTGCTAAAGGAGAACCAGGCGATGCTGGTGCTAAG
GGAGATGCTGGCCCACCTGGTCCAGCTGGTCCTGCTGGGCCACCAGGTCCT
ATCGGAAATGTTGGTGCTCCAGGGCCTAAAGGAGCTAGAGGATCAGCAGGT
CCCCCAGGTGCTACAGGTTTTCCAGGGGCTGCTGGAAGAGTGGGTCCCCCA
GGTCCTTCTGGGAATGCAGGACCACCAGGTCCTCCAGGACCAGCTGGAAAA
GAAGGAAGTAAGGGACCAAGGGGAGAAACTGGACCTGCAGGACGTCCAGGT
GAAGTTGGTCCACCTGGACCTCCAGGTCCAGCTGGAGAGAAAGGAGCTCCA
GGAGCTGATGGTCCAGCAGGTGCTCCTGGTACTCCAGGTCCACAAGGTATTG
CTGGTCAAAGGGGAGTGGTTGGTCTCCCAGGTCAGAGAGGTGAAAGAGGTT
TCCCAGGTTTGCCTGGACCATCTGGAGAACCAGGTAAGCAGGGCCCATCTG
GAGCTTCTGGTGAGAGAGGGCCACCTGGACCTATGGGACCTCCTGGGCTTG
CTGGACCTCCAGGGGAATCTGGTAGGGAGGGTGCTCCAGGTGCTGAAGGAT
CACCTGGTAGAGATGGATCACCTGGAGCTAAAGGTGATAGAGGGGAAACAG
GTCCAGCAGGTCCACCTGGCGCTCCTGGTGCTCCAGGAGCACCAGGGCCAG
TTGGACCTGCTGGAAAGTCTGGAGATAGGGGAGAGACTGGACCAGCTGGAC
CTGCTGGACCAATCGGTCCTGTTGGCGCTAGAGGTCCTGCTGGACCACAAG
GTCCTAGGGGAGATAAAGGTGAAACAGGTGAACAAGGAGATAGAGGAATTAA
AGGTCATAGAGGGTTTTCTGGTTTGCAAGGACCTCCAGGACCTCCTGGATCT
CCAGGAGAGCAAGGACCATCTGGTGCAAGCGGACCTGCTGGTCCTAGAGGA
CCTCCAGGTTCAGCTGGCTCACCTGGTAAGGATGGATTAAATGGTTTGCCTG
GACCAATAGGTCCACCAGGACCTAGAGGAAGGACAGGTGATGCAGGACCAG
CTGGTCCACCTGGTCCACCAGGACCACCAGGACCACCAGGTCCACCTTCTG
GTGGATATGATCTTAGTTTTCTTCCTCAACCTCCCCAGGAGAAAGCTCATGAT
GGTGGTAGATATTATAGGGCTGATGATGCAAATGTTGTTAGAGATAGAGATCT
TGAGGTTGATACTACTCTTAAATCTCTTAGCCAACAAATTGAAAACATTCGTTC
TCCTGAGGGAAGCAGAAAAAATCCTGCAAGGACTTGTAGAGATCTTAAGATG
TGCCATTCAGATTGGAAGTCTGGTGAGTACTGGATTGATCCAAATCAAGGTTG
TAATTTGGATGCTATCAAAGTTTTTTGTAATATGGAAACTGGAGAGACATGTGT
GTACCCTACACAACCTTCTGTTGCTCAAAAAAATTGGTATATTTCAAAAAATCC
TAAAGAAAAGAGACATGTTTGGTATGGAGAATCTATGACTGGAGGATTTCAAT
TTGAGTATGGTGGACAAGGATCAGATCCAGCTGATGTGGCTATTCAACTTACT
TTTCTTAGATTGATGTCCACTGAAGCTTCTCAAAATATTACATATCATTGTAAG
AATTCTGTTGCTTATATGGATCAACAGACTGGAAATCTTAAGAAGGCATTGTT
GTTGCAAGGCTCAAACGAAATTGAGATTAGGGCTGAAGGTAATAGTCGATTTA
CTTATTCTGTTACATATGATGGATGTACATCTCACACAGGTGCTTGGGGAAAA
 
ACAGTTATTGAGTATAAAACTACAAAAACTAGTAGACTTCCAATTATTGATGTT
GCACCATTGGATGTTGGTGCTCCTGATCAAGAATTTGGGTTTGATGTTGGACC
TGCTTGTTTCCTTGCATTTGTTTATTAAACCCAGCTTTCTTGTACAAAGTGGTA
GCTCGAATTTCCCCGATCGTTCAAACATTTGGCAATAAAGTTTCTTAAGATTGA
ATCCTGTTGCCGGTCTTGCGATGATTATCATATAATTTCTGTTGAATTACGTTA
AGCATGTAATAATTAACATGTAATGCATGACGTTATTTATGAGATGGGTTTTTA
TGATTAGAGTCCCGCAATTATACATTTAATACGCGATAGAAAACAAAATATAG
CGCGCAAACTAGGATAAATTATCGCGCGCGGTGTCATCTATGTTACTAGATC
GGGAAAGCTTTTAAACTATCAGTGTTTGACAGGATATATTGGCGGGTAAACCT
AAGAGAAAAGAGCGTTTATTAGAATAATCGGATATTTAAAAGGGCGTGAAAAG
GTTTATCCGTTCGTCCATTTGTATGTGCATGCCAACCACAGGGTTCCCCTCGG
GATCAAAGTACTTTGATCCAACCCCTCCGCTGCTATAGTGCAGTCGGCTTCTG
ACGTTCAGTGCAGCCGTCTTCTGAAAACGACATGTCGCACAAGTCCTAAGTTA
CGCGACAGGCTGCCGCCCTGCCCTTTTCCTGGCGTTTTCTTGTCGCGTGTTT
TAGTCGCATAAAGTAGAATACTTGCGACTAGAACCGGAGACATTACGCCATG
AACAAGAGCGCCGCCGCTGGCCTGCTGGGCTATGCCCGCGTCAGCACCGAC
GACCAGGACTTGACCAACCAACGGGCCGAACTGCACGCGGCCGGCTGCACC
AAGCTGTTTTCCGAGAAGATCACCGGCACCAGGCGCGACCGCCCGGAGCTG
GCCAGGATGCTTGACCACCTACGCCCTGGCGACGTTGTGACAGTGACCAGG
CTAGACCGCCTGGCCCGCAGCACCCGCGACCTACTGGACATTGCCGAGCGC
ATCCAGGAGGCCGGCGCGGGCCTGCGTAGCCTGGCAGAGCCGTGGGCCGA
CACCACCACGCCGGCCGGCCGCATGGTGTTGACCGTGTTCGCCGGCATTGC
CGAGTTCGAGCGTTCCCTAATCATCGACCGCACCCGGAGCGGGCGCGAGGC
CGCCAAGGCCCGAGGCGTGAAGTTTGGCCCCCGCCCTACCCTCACCCCGGC
ACAGATCGCGCACGCCCGCGAGCTGATCGACCAGGAAGGCCGCACCGTGAA
AGAGGCGGCTGCACTGCTTGGCGTGCATCGCTCGACCCTGTACCGCGCACT
TGAGCGCAGCGAGGAAGTGACGCCCACCGAGGCCAGGCGGCGCGGTGCCT
TCCGTGAGGACGCATTGACCGAGGCCGACGCCCTGGCGGCCGCCGAGAAT
GAACGCCAAGAGGAACAAGCATGAAACCGCACCAGGACGGCCAGGACGAAC
CGTTTTTCATTACCGAAGAGATCGAGGCGGAGATGATCGCGGCCGGGTACGT
GTTCGAGCCGCCCGCGCACGTCTCAACCGTGCGGCTGCATGAAATCCTGGC
CGGTTTGTCTGATGCCAAGCTGGCGGCCTGGCCGGCCAGCTTGGCCGCTGA
AGAAACCGAGCGCCGCCGTCTAAAAAGGTGATGTGTATTTGAGTAAAACAGC
TTGCGTCATGCGGTCGCTGCGTATATGATGCGATGAGTAAATAAACAAATAC
GCAAGGGGAACGCATGAAGGTTATCGCTGTACTTAACCAGAAAGGCGGGTC
AGGCAAGACGACCATCGCAACCCATCTAGCCCGCGCCCTGCAACTCGCCGG
GGCCGATGTTCTGTTAGTCGATTCCGATCCCCAGGGCAGTGCCCGCGATTG
GGCGGCCGTGCGGGAAGATCAACCGCTAACCGTTGTCGGCATCGACCGCCC
 
GACGATTGACCGCGACGTGAAGGCCATCGGCCGGCGCGACTTCGTAGTGAT
CGACGGAGCGCCCCAGGCGGCGGACTTGGCTGTGTCCGCGATCAAGGCAG
CCGACTTCGTGCTGATTCCGGTGCAGCCAAGCCCTTACGACATATGGGCCAC
CGCCGACCTGGTGGAGCTGGTTAAGCAGCGCATTGAGGTCACGGATGGAAG
GCTACAAGCGGCCTTTGTCGTGTCGCGGGCGATCAAAGGCACGCGCATCGG
CGGTGAGGTTGCCGAGGCGCTGGCCGGGTACGAGCTGCCCATTCTTGAGTC
CCGTATCACGCAGCGCGTGAGCTACCCAGGCACTGCCGCCGCCGGCACAAC
CGTTCTTGAATCAGAACCCGAGGGCGACGCTGCCCGCGAGGTCCAGGCGCT
GGCCGCTGAAATTAAATCAAAACTCATTTGAGTTAATGAGGTAAAGAGAAAAT
GAGCAAAAGCACAAACACGCTAAGTGCCGGCCGTCCGAGCGCACGCAGCAG
CAAGGCTGCAACGTTGGCCAGCCTGGCAGACACGCCAGCCATGAAGCGGGT
CAACTTTCAGTTGCCGGCGGAGGATCACACCAAGCTGAAGATGTACGCGGTA
CGCCAAGGCAAGACCATTACCGAGCTGCTATCTGAATACATCGCGCAGCTAC
CAGAGTAAATGAGCAAATGAATAAATGAGTAGATGAATTTTAGCGGCTAAAGG
AGGCGGCATGGAAAATCAAGAACAACCAGGCACCGACGCCGTGGAATGCCC
CATGTGTGGAGGAACGGGCGGTTGGCCAGGCGTAAGCGGCTGGGTTGCCT
GCCGGCCCTGCAATGGCACTGGAACCCCCAAGCCCGAGGAATCGGCGTGA
GCGGTCGCAAACCATCCGGCCCGGTACAAATCGGCGCGGCGCTGGGTGATG
ACCTGGTGGAGAAGTTGAAGGCCGCGCAGGCCGCCCAGCGGCAACGCATC
GAGGCAGAAGCACGCCCCGGTGAATCGTGGCAAGCGGCCGCTGATCGAATC
CGCAAAGAATCCCGGCAACCGCCGGCAGCCGGTGCGCCGTCGATTAGGAAG
CCGCCCAAGGGCGACGAGCAACCAGATTTTTTCGTTCCGATGCTCTATGACG
TGGGCACCCGCGATAGTCGCAGCATCATGGACGTGGCCGTTTTCCGTCTGTC
GAAGCGTGACCGACGAGCTGGCGAGGTGATCCGCTACGAGCTTCCAGACGG
GCACGTAGAGGTTTCCGCAGGGCCGGCCGGCATGGCCAGTGTGTGGGATTA
CGACCTGGTACTGATGGCGGTTTCCCATCTAACCGAATCCATGAACCGATAC
CGGGAAGGGAAGGGAGACAAGCCCGGCCGCGTGTTCCGTCCACACGTTGC
GGACGTACTCAAGTTCTGCCGGCGAGCCGATGGCGGAAAGCAGAAAGACGA
CCTGGTAGAAACCTGCATTCGGTTAAACACCACGCACGTTGCCATGCAGCGT
ACGAAGAAGGCCAAGAACGGCCGCCTGGTGACGGTATCCGAGGGTGAAGCC
TTGATTAGCCGCTACAAGATCGTAAAGAGCGAAACCGGGCGGCCGGAGTAC
ATCGAGATCGAGCTAGCTGATTGGATGTACCGCGAGATCACAGAAGGCAAGA
ACCCGGACGTGCTGACGGTTCACCCCGATTACTTTTTGATCGATCCCGGCAT
CGGCCGTTTTCTCTACCGCCTGGCACGCCGCGCCGCAGGCAAGGCAGAAGC
CAGATGGTTGTTCAAGACGATCTACGAACGCAGTGGCAGCGCCGGAGAGTT
CAAGAAGTTCTGTTTCACCGTGCGCAAGCTGATCGGGTCAAATGACCTGCCG
GAGTACGATTTGAAGGAGGAGGCGGGGCAGGCTGGCCCGATCCTAGTCATG
CGCTACCGCAACCTGATCGAGGGCGAAGCATCCGCCGGTTCCTAATGTACG
 
GAGCAGATGCTAGGGCAAATTGCCCTAGCAGGGGAAAAAGGTCGAAAAGGT
CTCTTTCCTGTGGATAGCACGTACATTGGGAACCCAAAGCCGTACATTGGGA
ACCGGAACCCGTACATTGGGAACCCAAAGCCGTACATTGGGAACCGGTCAC
ACATGTAAGTGACTGATATAAAAGAGAAAAAAGGCGATTTTTCCGCCTAAAAC
TCTTTAAAACTTATTAAAACTCTTAAAACCCGCCTGGCCTGTGCATAACTGTCT
GGCCAGCGCACAGCCGAAGAGCTGCAAAAAGCGCCTACCCTTCGGTCGCTG
CGCTCCCTACGCCCCGCCGCTTCGCGTCGGCCTATCGCGGCCGCTGGCCG
CTCAAAAATGGCTGGCCTACGGCCAGGCAATCTACCAGGGCGCGGACAAGC
CGCGCCGTCGCCACTCGACCGCCGGCGCCCACATCAAGGCACCCTGCCTCG
CGCGTTTCGGTGATGACGGTGAAAACCTCTGACACATGCAGCTCCCGGAGAC
GGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCAC
GTAGCGATAGCGGAGTGTATACTGGCTTAACTATGCGGCATCAGAGCAGATT
GTACTGAGAGTGCACCATATGCGGTGTGAAATACCGCACAGATGCGTAAGGA
GAAAATACCGCATCAGGCGCTCTTCCGCTTCCTCGCTCACTGACTCGCTGCG
CTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAA
GGCCAGCAAAAGGCCAGGAACCGTAAAAAGGCCGCGTTGCTGGCGTTTTTC
CATAGGCTCCGCCCCCCTGACGAGCATCACAAAAATCGACGCTCAAGTCAGA
GGTGGCGAAACCCGACAGGACTATAAAGATACCAGGCGTTTCCCCCTGGAA
GCTCCCTCGTGCGCTCTCCTGTTCCGACCCTGCCGCTTACCGGATACCTGTC
CGCCTTTCTCCCTTCGGGAAGCGTGGCGCTTTCTCATAGCTCACGCTGTAGG
TATCTCAGTTCGGTGTAGGTCGTTCGCTCCAAGCTGGGCTGTGTGCACGAAC
CCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAG
GATTAGCAGAGCGAGGTATGTAGGCGGTGCTACAGAGTTCTTGAAGTGGTGG
CCTAACTACGGCTACACTAGAAGGACAGTATTTGGTATCTGCGCTCTGCTGAA
GCCAGTTACCTTCGGAAAAAGAGTTGGTAGCTCTTGATCCGGCAAACAAACC
ACCGCTGGTAGCGGTGGTTTTTTTGTTTGCAAGCAGCAGATTACGCGCAGAA
AAAAAGGATCTCAAGAAGATCCTTTGATCTTTTCTACGGGGTCTGACGCTCAG
TGGAACGAAAACTCACGTTAAGGGATTTTGGTCATGCATTCTAGGTACTAAAA
CAATTCATCCAGTAAAATATAATATTTTATTTTCTCCCAATCAGGCTTGATCCC
CAGTAAGTCAAAAAATAGCTCGACATACTGTTCTTCCCCGATATCCTCCCTGA
TCGACCGGACGCAGAAGGCAATGTCATACCACTTGTCCGCCCTGCCGCTTCT
CCCAAGATCAATAAAGCCACTTACTTTGCCATCTTTCACAAAGATGTTGCTGT
CTCCCAGGTCGCCGTGGGAAAAGACAAGTTCCTCTTCGGGCTTTTCCGTCTT
TAAAAAATCATACAGCTCGCGCGGATCTTTAAATGGAGTGTCTTCTTCCCAGT
TTTCGCAATCCACATCGGCCAGATCGTTATTCAGTAAGTAATCCAATTCGGCT
 
AAGCGGCTGTCTAAGCTATTCGTATAGGGACAATCCGATATGTCGATGGAGT
GAAAGAGCCTGATGCACTCCGCATACAGCTCGATAATCTTTTCAGGGCTTTGT
TCATCTTCATACTCTTCCGAGCAAAGGACGCCATCGGCCTCACTCATGAGCA
GATTGCTCCAGCCATCATGCCGTTCAAAGTGCAGGACCTTTGGAACAGGCAG
CTTTCCTTCCAGCCATAGCATCATGTCCTTTTCCCGTTCCACATCATAGGTGG
TCCCTTTATACCGGCTGTCCGTCATTTTTAAATATAGGTTTTCATTTTCTCCCA
CCAGCTTATATACCTTAGCAGGAGACATTCCTTCCGTATCTTTTACGCAGCGG
TATTTTTCGATCAGTTTTTTCAATTCCGGTGATATTCTCATTTTAGCCATTTATT
ATTTCCTTCCTCTTTTCTACAGTATTTAAAGATACCCCAAGAAGCTAATTATAA
CAAGACGAACTCCAATTCACTGTTCCTTGCATTCTAAAACCTTAAATACCAGA
AAACAGCTTTTTCAAAGTTGTTTTCAAAGTTGGCGTATAACATAGTATCGACG
GAGCCGATTTTGAAACCGCGGTGATCACAGGCAGCAACGCTCTGTCATCGTT
ACAATCAACATGCTACCCTCCGCGAGATCATCCGTGTTTCAAACCCGGCAGC
TTAGTTGCCGTTCTTCCGAATAGCATCGGTAACATGAGCAAAGTCTGCCGCCT
TACAACGGCTCTCCCGCTGACGCCGTCCCGGACTGATGGGCTGCCTGTATC
GAGTGGTGATTTTGTGCCGAGCTGCCGGTCGGGGAGCTGTTGGCTGGCTGG
TGGCAGGATATATTGTGGTGTAAACAAATTGACGCTTAGACAACTTAATAACA
CATTGCGGACGTTTTTAATGTACTGAATTAACGCCGAATTGAATTCGAGCTCG
GTACCGGGGGATCTGGATTTTAGTACTGGATTTTGGTTTTAGGAATTAGAAAT
TTTATTGATAGAAGTATTTTACAAATACAAATACATACTAAGGGTTTCTTATATG
CTCAACACATGAGCGAAACCCTATAGGAACCCTAATTCCCTTATCTGGGAACT
ACTCACACATTATTATGGAGAAACTCGAGCTTGTCGATCGACTCAAATCTCGG
TGACGGGCAGGACCGGACGGGGCGGTACCGGCAGGCTGAAGTCCAGCTGC
CAGAAACCCACGTCATGCCAGTTCCCGTGCTTGAAGCCGGCCGCCCGCAGC
ATGCCGCGGGGGGCATATCCGAGCGCCTCGTGCATGCGCACGCTCGGGTC
GTTGGGCAGCCCGATGACAGCGACCACGCTCTTGAAGCCCTGTGCCTCCAG
GGACTTCAGCAGGTGGGTGTAGAGCGTGGAGCCCAGTCCCGTCCGCTGGTG
GCGGGGGGAGACGTACACGGTCGACTCGGCCGTCCAGTCGTAGGCGTTGC
GTGCCTTCCAGGGGCCCGCGTAGGCGATGCCGGCGACCTCGCCGTCCACCT
CGGCGACGAGCCAGGGATAGCGCTCCCGCAGACGGACGAGGTCGTCCGTC
CACTCCTGCGGTTCCTGCGGCTCGGTACGGAAGTTGACCGTGCTTGTCTCGA
TGTAGTGGTTGACGATGGTGCAGACCGCCGGCATGTCCGCCTCGGTGGCAC
GGCGGATGTCGGCCGGGCGTCGTTCTGGGCTCATGGTAGACGATCTGCGAA
AGCTCGAGAGAGATAGATTTGTAGAGAGAGACTGGTGATTTCAGCGTGTCCT
CTCCAAATGAAATGAACTTCCTTATATAGAGGAAGGGTCTTGCGAAGGATAGT
GGGATTGTGCGTCATCCCTTACGTCAGTGGAGATATCACATCAATCCACTTGC
TTTGAAGACGTGGTTGGAACGTCTTCTTTTTCCACGATGCTCCTCGTGGGTG
GGGGTCCATCTTTGGGACCACTGTCGGCAGAGGCATCTTGAACGATAGCCTT
 
TCCTTTATCGCAATGATGGCATTTGTAGGTGCCACCTTCCTTTTCTACTGTCCT
TTTGATGAAGTGACAGATAGCTGGGCAATGGAATCCGAGGAGGTTTCCCGAT
ATTACCCTTTGTTGAAAAGTCTCAATAGCCCTTTGGTCTTCTGAGACTGTATCT
TTGATATTCTTGGAGTAGACGAGAGTGTCGTGCTCCACCATGTTCACATCAAT
CCACTTGCTTTGAAGACGTGGTTGGAACGTCTTCTTTTTCCACGATGCTCCTC
GTGGGTGGGGGTCCATCTTTGGGACCACTGTCGGCAGAGGCATCTTGAACG
ATAGCCTTTCCTTTATCGCAATGATGGCATTTGTAGGTGCCACCTTCCTTTTCT
ACTGTCCTTTTGATGAAGTGACAGATAGCTGGGCAATGGAATCCGAGGAGGT
TTCCCGATATTACCCTTTGTTGAAAAGTCTCAATAGCCCTTTGGTCTTCTGAGA
CTGTATCTTTGATATTCTTGGAGTAGACGAGAGTGTCGTGCTCCACCATGTTG
GCGTACCCGGGGATCCTCTAGAGTCGACCTGCAGGCATGCAAGCT
Bovine Collagen alpha-1 (1) Expression vector 2
   SEQ ID NO: 11: 
CAACTTTGTATAGAAAAGTTGGCCAACATGGTGGAGCACGACACTCTCGTCT
ACTCCAAGAATATCAAAGATACAGTCTCAGAAGACCAAAGGGCTATTGAGACT
TTTCAACAAAGGGTAATATCGGGAAACCTCCTCGGATTCCATTGCCCAGCTAT
CTGTCACTTCATCAAAAGGACAGTAGAAAAGGAAGGTGGCACCTACAAATGC
CATCATTGCGATAAAGGAAAGGCTATCGTTCAAGATGCCTCTGCCGACAGTG
GTCCCAAAGATGGACCCCCACCCACGAGGAGCATCGTGGAAAAAGAAGACG
TTCCAACCACGTCTTCAAAGCAAGTGGATTGATGTGAACATGGTGGAGCACG
ACACTCTCGTCTACTCCAAGAATATCAAAGATACAGTCTCAGAAGACCAAAGG
GCTATTGAGACTTTTCAACAAAGGGTAATATCGGGAAACCTCCTCGGATTCCA
TTGCCCAGCTATCTGTCACTTCATCAAAAGGACAGTAGAAAAGGAAGGTGGC
ACCTACAAATGCCATCATTGCGATAAAGGAAAGGCTATCGTTCAAGATGCCTC
TGCCGACAGTGGTCCCAAAGATGGACCCCCACCCACGAGGAGCATCGTGGA
AAAAGAAGACGTTCCAACCACGTCTTCAAAGCAAGTGGATTGATGTGATATCT
CCACTGACGTAAGGGATGACGCACAATCCCACTATCCTTCGCAAGACCCTTC
CTCTATATAAGGAAGTTCATTTCATTTGGAGAGGACACGCTGACAAGTTTGTA
CAAAAAAGCAGGCTGCCACCATGTTTTCTTTTGTTGATCTTAGATTGCTCCTTC
TTCTTGCTGCTACAGCACTTCTTACTCACGGGCAAGAAGAAGGTCAGGAAGA
AGGTCAAGAAGAAGATATTCCACCAGTTACTTGTGTTCAAAACGGTCTTAGAT
ACCATGATAGGGATGTTTGGAAGCCTGTGCCATGTCAAATTTGTGTTTGTGAT
AATGGAAATGTTTTATGTGATGATGTAATTTGTGATGAACTTAAGGATTGCCCT
AATGCTAAGGTACCAACTGATGAATGTTGTCCTGTGTGTCCAGAGGGTCAAG
AATCTCCTACAGATCAAGAAACTACTGGAGTTGAAGGACCAAAAGGTGATACT
GGACCCAGGGGCCCTAGGGGTCCAGCAGGTCCTCCAGGAAGGGATGGAATT
CCTGGCCAGCCTGGTCTACCAGGACCACCGGGACCACCTGGCCCTCCTGGT
 
CCACCTGGACTTGGAGGTAATTTTGCACCTCAACTTTCTTACGGATACGATGA
GAAGTCTACAGGTATTTCTGTTCCTGGACCTATGGGTCCTTCAGGTCCAAGA
GGTCTTCCAGGCCCTCCAGGAGCTCCAGGCCCTCAAGGATTTCAAGGTCCTC
CTGGTGAACCAGGAGAACCAGGAGCATCAGGACCAATGGGACCCAGAGGAC
CTCCAGGTCCTCCTGGAAAAAATGGTGATGATGGCGAGGCTGGTAAGCCAG
GTAGACCTGGTGAAAGAGGGCCTCCAGGTCCACAAGGTGCTAGAGGACTAC
CAGGAACAGCTGGTCTTCCAGGAATGAAGGGTCATAGGGGATTTTCTGGTTT
GGATGGAGCAAAAGGTGATGCTGGACCAGCTGGTCCAAAAGGAGAACCAGG
ATCTCCTGGAGAGAATGGAGCTCCAGGACAAATGGGACCTAGAGGACTTCCA
GGTGAAAGAGGAAGACCAGGAGCACCAGGACCTGCTGGTGCTAGAGGAAAT
GATGGGGCTACTGGTGCTGCTGGACCACCTGGACCAACAGGACCTGCAGGA
CCACCTGGTTTTCCAGGTGCTGTTGGAGCTAAGGGAGAGGGAGGACCACAA
GGACCTCGTGGCTCAGAAGGTCCACAAGGTGTCAGAGGTGAGCCAGGTCCA
CCTGGACCTGCTGGAGCAGCTGGTCCTGCTGGAAATCCCGGTGCTGATGGA
CAGCCTGGAGCCAAAGGAGCAAACGGAGCTCCTGGAATTGCTGGTGCACCT
GGTTTCCCTGGTGCCAGAGGACCTTCAGGTCCTCAGGGCCTTCTGGTCCA
CCAGGTCCTAAAGGAAACTCTGGAGAGCCTGGAGCTCCTGGTTCTAAAGGAG
ATACAGGCGCTAAGGGCGAACCTGGACCTACAGGAATCCAAGGACCTCCTG
GTCCAGCAGGTGAGGAAGGAAGAGAGGAGCTAGAGGAGAGCCTGGACCT
GCTGGTCTTCCTGGCCCACCAGGAGAAAGAGGAGGTCCTGGATCTAGAGGA
TTCCCAGGAGCTGATGGAGTTGCCGGACCTAAAGGACCTGCTGGTGAAAGA
GGAGCTCCAGGTCCAGCAGGTCCTAAAGGATCACCAGGAGAAGCTGGTAGG
CCAGGTGAAGCTGGTTTGCCAGGAGCTAAAGGTCTTACAGGATCTCCTGGCT
CTCCAGGTCCTGATGGAAGACTGGACCACCAGGTCCTGCTGGACAAGATG
GAAGGCCAGGTCCTCCAGGACCACCTGGAGCAAGAGGTCAAGCTGGTGTGA
TGGGTTTTCCAGGACCAAAGGGAGCTGCAGGAGAACCAGGTAAGGCTGGAG
AAAGGGGAGTACCTGGTCCTCCTGGTGCTGTTGGTCCAGCTGGTAAAGATGG
AGAAGCAGGAGCACAAGGTCCTCCAGGTCCTGCAGGACCTGCTGGAGAAAG
AGGTGAACAAGGACCTGCAGGATCACCAGGATTTCAAGGATTGCCTGGCCCT
GCAGGACCTCCAGGTGAAGCTGGTAAGCCAGGAGAGCAAGGTGTCCCAGGT
GATCTCGGGGCTCCTGGTCCATCAGGAGCAAGAGGAGAGAGAGGATTTCCA
GGAGAAAGAGGAGTTCAGGGACCACCAGGACCAGCAGGACCAAGAGGTGCT
AACGGCGCACCAGGAAATGACGGTGCAAAGGGGGATGCTGGTGCTCCAGGA
GCTCCTGGATCACAAGGAGCTCCAGGTTTGCAAGGCATGCCAGGAGAAAGA
GGAGCAGCAGGATTGCCTGGACCTAAGGGTGATAGAGGCGATGCAGGACCA
AAAGGAGCTGATGGTGCTCCAGGTAAGGATGGAGTTCGTGGATTGACAGGT
CCAATTGGTCCACCAGGACCAGCTGGAGCTCCTGGAGATAAAGGCGAAGCT
GGACCATCAGGACCAGCTGGACCAACTGGTGCTAGAGGAGCTCCTGGAGAC
 
AGGGGAGAGCCTGGACCTCCTGGTCCTGCTGGTTTCGCTGGACCTCCTGGA
GCAGATGGACAGCCAGGTGCAAAGGGAGAGCCAGGTGATGCCGGAGCTAAA
GGGGATGCTGGTCCACCTGGTCCAGCAGGTCCAGCTGGTCCTCCTGGCCCT
ATAGGTAATGTTGGTGCTCCTGGTCCAAAAGGAGCTAGAGGGTCTGCTGGTC
CTCCAGGAGCTACAGGTTTTCCTGGAGCAGCTGGAAGAGTTGGTCCTCCAGG
TCCTTCAGGAAATGCTGGACCTCCTGGTCCACCAGGACCTGCTGGAAAGGAA
GGATCAAAAGGTCCAAGAGGTGAAACTGGACCTGCTGGTAGGCCAGGAGAA
GTGGGTCCACCAGGACCACCAGGACCTGCAGGAGAAAAGGGTGCTCCAGGA
GCTGATGGTCCTGCTGGTGCTCCAGGAACACCTGGACCACAAGGAATTGCTG
GTCAAAGAGGTGTTGTGGGTCTTCCAGGTCAAAGAGGTGAAAGAGGTTTCCC
TGGACTTCCTGGTCCATCAGGTGAACCTGGAAAGCAAGGTCCAAGTGGAGCT
TCTGGAGAAAGAGGTCCTCCAGGACCTATGGGTCCTCCTGGTTTGGCAGGTC
CTCCTGGAGAATCTGGTAGAGAAGGTGCACCTGGAGCAGAAGGTTCTCCAG
GTAGGGATGGATCTCCTGGAGCTAAAGGAGATAGGGGTGAAACAGGACCTG
CAGGACCACCTGGAGCACCTGGAGCTCCTGGTGCTCCAGGACCAGTTGGAC
CAGCAGGTAAGTCTGGAGATCGTGGTGAAACTGGACCAGCTGGACCAGCTG
GACCTATTGGTCCTGTTGGTGCTAGGGGACCAGCAGGCCCACAAGGACCTA
GAGGTGATAAAGGAGAAACTGGTGAACAGGGTGATAGAGGAATAAAAGGCC
ATAGAGGGTTCAGTGGACTCCAAGGACCTCCTGGTCCACCAGGATCTCCTGG
AGAGCAAGGACCTTCTGGTGCTTCTGGCCCAGCTGGTCCTAGAGGACCTCCA
GGTTCTGCAGGATCTCCTGGTAAAGATGGATTGAACGGTCTTCCAGGACCTA
TTGGTCCTCCTGGACCTAGGGGAAGGACTGGTGATGCTGGTCCAGCTGGAC
CACCAGGTCCACCTGGTCCTCCAGGACCACCAGGTCCACCAAGTGGTGGTT
ATGATCTTAGCTTTCTTCCTCAACCTCCTCAAGAGAAGGCCCACGATGGAGG
ACGTTATTATAGAGCTGATGATGCTAACGTTGTTAGAGATAGAGATTTGGAAG
TTGATACTACTTTGAAATCTCTTTCACAACAAATTGAAAATATTAGATCACCTG
AAGGATCAAGGAAGAACCCTGCTAGGACTTGTAGAGATCTTAAAATGTGCCA
TAGCGATTGGAAGTCTGGAGAGTATTGGATTGATCCAAATCAAGGATGCAAC
TTGGATGCTATTAAAGTTTTCTGTAACATGGAGACTGGAGAAACATGTGTTTA
CCCAACTCAACCAAGCGTTGCTCAAAAAAACTGGTACATCTCAAAAAATCCAA
AAGAAAAGAGGCATGTGTGGTATGGAGAATCTATGACTGGAGGATTCCAGTT
TGAATATGGAGGTCAAGGTTCTGATCCTGCTGATGTTGCTATTCAATTGACTT
TCTTGAGACTTATGTCTACAGAGGCATCACAAAACATTACTTATCATTGCAAG
AACTCAGTTGCTTATATGGATCAACAAACTGGAAATCTTAAAAAGGCTCTTCT
CTTGCAAGGATCTAATGAAATTGAGATAAGAGCTGAAGGAAACTCTAGATTTA
CTTATTCTGTTACATACGATGGATGCACTTCACATACTGGTGCATGGGGTAAA
ACTGTAATTGAGTACAAGACTACTAAGACATCTCGTCTTCCTATTATTGATGTT
GCTCCACTTGATGTTGGTGCTCCAGACCAAGAATTTGGTTTTGATGTGGGTCC
 
TGCATGTTTTCTTGCTTTTGTTTATTAAACCCAGCTTTCTTGTACAAAGTGGTA
GCTCGAATTTCCCCGATCGTTCAAACATTTGGCAATAAAGTTTCTTAAGATTGA
ATCCTGTTGCCGGTCTTGCGATGATTATCATATAATTTCTGTTGAATTACGTTA
AGCATGTAATAATTAACATGTAATGCATGACGTTATTTATGAGATGGGTTTTTA
TGATTAGAGTCCCGCAATTATACATTTAATACGCGATAGAAAACAAAATATAG
CGCGCAAACTAGGATAAATTATCGCGCGCGGTGTCATCTATGTTACTAGATC
GGGAAAGCTTTTAAACTATCAGTGTTTGACAGGATATATTGGCGGGTAAACCT
AAGAGAAAAGAGCGTTTATTAGAATAATCGGATATTTAAAAGGGCGTGAAAAG
GTTTATCCGTTCGTCCATTTGTATGTGCATGCCAACCACAGGGTTCCCCTCGG
GATCAAAGTACTTTGATCCAACCCCTCCGCTGCTATAGTGCAGTCGGCTTCTG
ACGTTCAGTGCAGCCGTCTTCTGAAAACGACATGTCGCACAAGTCCTAAGTTA
CGCGACAGGCTGCCGCCCTGCCCTTTTCCTGGCGTTTTCTTGTCGCGTGTTT
TAGTCGCATAAAGTAGAATACTTGCGACTAGAACCGGAGACATTACGCCATG
AACAAGAGCGCCGCCGCTGGCCTGCTGGGCTATGCCCGCGTCAGCACCGAC
GACCAGGACTTGACCAACCAACGGGCCGAACTGCACGCGGCCGGCTGCACC
AAGCTGTTTTCCGAGAAGATCACCGGCACCAGGCGCGACCGCCCGGAGCTG
GCCAGGATGCTTGACCACCTACGCCCTGGCGACGTTGTGACAGTGACCAGG
CTAGACCGCCTGGCCCGCAGCACCCGCGACCTACTGGACATTGCCGAGCGC
ATCCAGGAGGCCGGCGCGGGCCTGCGTAGCCTGGCAGAGCCGTGGGCCGA
CACCACCACGCCGGCCGGCCGCATGGTGTTGACCGTGTTCGCCGGCATTGC
CGAGTTCGAGCGTTCCCTAATCATCGACCGCACCCGGAGCGGGCGCGAGGC
CGCCAAGGCCCGAGGCGTGAAGTTTGGCCCCCGCCCTACCCTCACCCCGGC
ACAGATCGCGCACGCCCGCGAGCTGATCGACCAGGAAGGCCGCACCGTGAA
AGAGGCGGCTGCACTGCTTGGCGTGCATCGCTCGACCCTGTACCGCGCACT
TGAGCGCAGCGAGGAAGTGACGCCCACCGAGGCCAGGCGGCGCGGTGCCT
TCCGTGAGGACGCATTGACCGAGGCCGACGCCCTGGCGGCCGCCGAGAAT
GAACGCCAAGAGGAACAAGCATGAAACCGCACCAGGACGGCCAGGACGAAC
CGTTTTTCATTACCGAAGAGATCGAGGCGGAGATGATCGCGGCCGGGTACGT
GTTCGAGCCGCCCGCGCACGTCTCAACCGTGCGGCTGCATGAAATCCTGGC
CGGTTTGTCTGATGCCAAGCTGGCGGCCTGGCCGGCCAGCTTGGCCGCTGA
AGAAACCGAGCGCCGCCGTCTAAAAAGGTGATGTGTATTTGAGTAAAACAGC
TTGCGTCATGCGGTCGCTGCGTATATGATGCGATGAGTAAATAAACAAATAC
GCAAGGGGAACGCATGAAGGTTATCGCTGTACTTAACCAGAAAGGCGGGTC
AGGCAAGACGACCATCGCAACCCATCTAGCCCGCGCCCTGCAACTCGCCGG
GGCCGATGTTCTGTTAGTCGATTCCGATCCCCAGGGCAGTGCCCGCGATTG
GGCGGCCGTGCGGGAAGATCAACCGCTAACCGTTGTCGGCATCGACCGCCC
GACGATTGACCGCGACGTGAAGGCCATCGGCCGGCGCGACTTCGTAGTGAT
CGACGGAGCGCCCCAGGCGGCGGACTTGGCTGTGTCCGCGATCAAGGCAG
 
CCGACTTCGTGCTGATTCCGGTGCAGCCAAGCCCTTACGACATATGGGCCAC
CGCCGACCTGGTGGAGCTGGTTAAGCAGCGCATTGAGGTCACGGATGGAAG
GCTACAAGCGGCCTTTGTCGTGTCGCGGGCGATCAAAGGCACGCGCATCGG
CGGTGAGGTTGCCGAGGCGCTGGCCGGGTACGAGCTGCCCATTCTTGAGTC
CCGTATCACGCAGCGCGTGAGCTACCCAGGCACTGCCGCCGGCACAAC
CGTTCTTGAATCAGAACCCGAGGGCGACGCTGCCCGCGAGGTCCAGGCGCT
GGCCGCTGAAATTAAATCAAAACTCATTTGAGTTAATGAGGTAAAGAGAAAAT
GAGCAAAAGCACAAACACGCTAAGTGCCGGCCGTCCGAGCGCACGCAGCAG
CAAGGCTGCAACGTTGGCCAGCCTGGCAGACACGCCAGCCATGAAGCGGGT
CAACTTTCAGTTGCCGGCGGAGGATCACACCAAGCTGAAGATGTACGCGGTA
CGCCAAGGCAAGACCATTACCGAGCTGCTATCTGAATACATCGCGCAGCTAC
CAGAGTAAATGAGCAAATGAATAAATGAGTAGATGAATTTTAGCGGCTAAAGG
AGGCGGCATGGAAAATCAAGAACAACCAGGCACCGACGCCGTGGAATGCCC
CATGTGTGGAGGAACGGGCGGTTGGCCAGGCGTAAGCGGCTGGGTTGCCT
GCCGGCCCTGCAATGGCACTGGAACCCCCAAGCCCGAGGAATCGGCGTGA
GCGGTCGCAAACCATCCGGCCCGGTACAAATCGGCGCGGCGCTGGGTGATG
ACCTGGTGGAGAAGTTGAAGGCCGCGCAGGCCGCCCAGCGGCAACGCATC
GAGGCAGAAGCACGCCCCGGTGAATCGTGGCAAGCGGCCGCTGATCGAATC
CGCAAAGAATCCCGGCAACCGCCGGCAGCCGGTGCGCCGTCGATTAGGAAG
CCGCCCAAGGGCGACGAGCAACCAGATTTTTTCGTTCCGATGCTCTATGACG
TGGGCACCCGCGATAGTCGCAGCATCATGGACGTGGCCGTTTTCCGTCTGTC
GAAGCGTGACCGACGAGCTGGCGAGGTGATCCGCTACGAGCTTCCAGACGG
GCACGTAGAGGTTTCCGCAGGGCCGGCCGGCATGGCCAGTGTGTGGGATTA
CGACCTGGTACTGATGGCGGTTTCCCATCTAACCGAATCCATGAACCGATAC
CGGGAAGGGAAGGGAGACAAGCCCGGCCGCGTGTTCCGTCCACACGTTGC
GGACGTACTCAAGTTCTGCCGGCGAGCCGATGGCGGAAAGCAGAAAGACGA
CCTGGTAGAAACCTGCATTCGGTTAAACACCACGCACGTTGCCATGCAGCGT
ACGAAGAAGGCCAAGAACGGCCGCCTGGTGACGGTATCCGAGGGTGAAGCC
TTGATTAGCCGCTACAAGATCGTAAAGAGCGAAACCGGGCGGCCGGAGTAC
ATCGAGATCGAGCTAGCTGATTGGATGTACCGCGAGATCACAGAAGGCAAGA
ACCCGGACGTGCTGACGGTTCACCCCGATTACTTTTTGATCGATCCCGGCAT
CGGCCGTTTTCTCTACCGCCTGGCACGCCGCGCCGCAGGCAAGGCAGAAGC
CAGATGGTTGTTCAAGACGATCTACGAACGCAGTGGCAGCGCCGGAGAGTT
CAAGAAGTTCTGTTTCACCGTGCGCAAGCTGATCGGGTCAAATGACCTGCCG
GAGTACGATTTGAAGGAGGAGGCGGGGCAGGCTGGCCCGATCCTAGTCATG
CGCTACCGCAACCTGATCGAGGGCGAAGCATCCGCCGGTTCCTAATGTACG
GAGCAGATGCTAGGGCAAATTGCCCTAGCAGGGGAAAAAGGTCGAAAAGGT
CTCTTTCCTGTGGATAGCACGTACATTGGGAACCCAAAGCCGTACATTGGGA
 
ACCGGAACCCGTACATTGGGAACCCAAAGCCGTACATTGGGAACCGGTCAC
ACATGTAAGTGACTGATATAAAAGAGAAAAAAGGCGATTTTTCCGCCTAAAAC
TCTTTAAAACTTATTAAAACTCTTAAAACCCGCCTGGCCTGTGCATAACTGTCT
GGCCAGCGCACAGCCGAAGAGCTGCAAAAAGCGCCTACCCTTCGGTCGCTG
CGCTCCCTACGCCCCGCCGCTTCGCGTCGGCCTATCGCGGCCGCTGGCCG
CTCAAAAATGGCTGGCCTACGGCCAGGCAATCTACCAGGGCGCGGACAAGC
CGCGCCGTCGCCACTCGACCGCCGGCGCCCACATCAAGGCACCCTGCCTCG
CGCGTTTCGGTGATGACGGTGAAAACCTCTGACACATGCAGCTCCCGGAGAC
GGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCAC
GTAGCGATAGCGGAGTGTATACTGGCTTAACTATGCGGCATCAGAGCAGATT
GTACTGAGAGTGCACCATATGCGGTGTGAAATACCGCACAGATGCGTAAGGA
GAAAATACCGCATCAGGCGCTCTTCCGCTTCCTCGCTCACTGACTCGCTGCG
CTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAA
GGCCAGCAAAAGGCCAGGAACCGTAAAAAGGCCGCGTTGCTGGCGTTTTTC
CATAGGCTCCGCCCCCCTGACGAGCATCACAAAAATCGACGCTCAAGTCAGA
GGTGGCGAAACCCGACAGGACTATAAAGATACCAGGCGTTTCCCCCTGGAA
GCTCCCTCGTGCGCTCTCCTGTTCCGACCCTGCCGCTTACCGGATACCTGTC
CGCCTTTCTCCCTTCGGGAAGCGTGGCGCTTTCTCATAGCTCACGCTGTAGG
TATCTCAGTTCGGTGTAGGTCGTTCGCTCCAAGCTGGGCTGTGTGCACGAAC
CCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAG
GATTAGCAGAGCGAGGTATGTAGGCGGTGCTACAGAGTTCTTGAAGTGGTGG
CCTAACTACGGCTACACTAGAAGGACAGTATTTGGTATCTGCGCTCTGCTGAA
GCCAGTTACCTTCGGAAAAAGAGTTGGTAGCTCTTGATCCGGCAAACAAACC
ACCGCTGGTAGCGGTGGTTTTTTTGTTTGCAAGCAGCAGATTACGCGCAGAA
AAAAAGGATCTCAAGAAGATCCTTTGATCTTTTCTACGGGGTCTGACGCTCAG
TGGAACGAAAACTCACGTTAAGGGATTTTGGTCATGCATTCTAGGTACTAAAA
CAATTCATCCAGTAAAATATAATATTTTATTTTCTCCCAATCAGGCTTGATCCC
CAGTAAGTCAAAAAATAGCTCGACATACTGTTCTTCCCCGATATCCTCCCTGA
TCGACCGGACGCAGAAGGCAATGTCATACCACTTGTCCGCCCTGCCGCTTCT
CCCAAGATCAATAAAGCCACTTACTTTGCCATCTTTCACAAAGATGTTGCTGT
CTCCCAGGTCGCCGTGGGAAAAGACAAGTTCCTCTTCGGGCTTTTCCGTCTT
TAAAAAATCATACAGCTCGCGCGGATCTTTAAATGGAGTGTCTTCTTCCCAGT
TTTCGCAATCCACATCGGCCAGATCGTTATTCAGTAAGTAATCCAATTCGGCT
AAGCGGCTGTCTAAGCTATTCGTATAGGGACAATCCGATATGTCGATGGAGT
GAAAGAGCCTGATGCACTCCGCATACAGCTCGATAATCTTTTCAGGGCTTTGT
 
TCATCTTCATACTCTTCCGAGCAAAGGACGCCATCGGCCTCACTCATGAGCA
GATTGCTCCAGCCATCATGCCGTTCAAAGTGCAGGACCTTTGGAACAGGCAG
CTTTCCTTCCAGCCATAGCATCATGTCCTTTTCCCGTTCCACATCATAGGTGG
TCCCTTTATACCGGCTGTCCGTCATTTTTAAATATAGGTTTTCATTTTCTCCCA
CCAGCTTATATACCTTAGCAGGAGACATTCCTTCCGTATCTTTTACGCAGCGG
TATTTTTCGATCAGTTTTTTCAATTCCGGTGATATTCTCATTTTAGCCATTTATT
ATTTCCTTCCTCTTTTCTACAGTATTTAAAGATACCCCAAGAAGCTAATTATAA
CAAGACGAACTCCAATTCACTGTTCCTTGCATTCTAAAACCTTAAATACCAGA
AAACAGCTTTTTCAAAGTTGTTTTCAAAGTTGGCGTATAACATAGTATCGACG
GAGCCGATTTTGAAACCGCGGTGATCACAGGCAGCAACGCTCTGTCATCGTT
ACAATCAACATGCTACCCTCCGCGAGATCATCCGTGTTTCAAACCCGGCAGC
TTAGTTGCCGTTCTTCCGAATAGCATCGGTAACATGAGCAAAGTCTGCCGCCT
TACAACGGCTCTCCCGCTGACGCCGTCCCGGACTGATGGGCTGCCTGTATC
GAGTGGTGATTTTGTGCCGAGCTGCCGGTCGGGGAGCTGTTGGCTGGCTGG
TGGCAGGATATATTGTGGTGTAAACAAATTGACGCTTAGACAACTTAATAACA
CATTGCGGACGTTTTTAATGTACTGAATTAACGCCGAATTGAATTCGAGCTCG
GTACCGGGGGATCTGGATTTTAGTACTGGATTTTGGTTTTAGGAATTAGAAAT
TTTATTGATAGAAGTATTTTACAAATACAAATACATACTAAGGGTTTCTTATATG
CTCAACACATGAGCGAAACCCTATAGGAACCCTAATTCCCTTATCTGGGAACT
ACTCACACATTATTATGGAGAAACTCGAGCTTGTCGATCGACTCAAATCTCGG
TGACGGGCAGGACCGGACGGGGCGGTACCGGCAGGCTGAAGTCCAGCTGC
CAGAAACCCACGTCATGCCAGTTCCCGTGCTTGAAGCCGGCCGCCCGCAGC
ATGCCGCGGGGGGCATATCCGAGCGCCTCGTGCATGCGCACGCTCGGGTC
GTTGGGCAGCCCGATGACAGCGACCACGCTCTTGAAGCCCTGTGCCTCCAG
GGACTTCAGCAGGTGGGTGTAGAGCGTGGAGCCCAGTCCCGTCCGCTGGTG
GCGGGGGGAGACGTACACGGTCGACTCGGCCGTCCAGTCGTAGGCGTTGC
GTGCCTTCCAGGGGCCCGCGTAGGCGATGCCGGCGACCTCGCCGTCCACCT
CGGCGACGAGCCAGGGATAGCGCTCCCGCAGACGGACGAGGTCGTCCGTC
CACTCCTGCGGTTCCTGCGGCTCGGTACGGAAGTTGACCGTGCTTGTCTCGA
TGTAGTGGTTGACGATGGTGCAGACCGCCGGCATGTCCGCCTCGGTGGCAC
GGCGGATGTCGGCCGGGCGTCGTTCTGGGCTCATGGTAGACGATCTGCGAA
AGCTCGAGAGAGATAGATTTGTAGAGAGAGACTGGTGATTTCAGCGTGTCCT
CTCCAAATGAAATGAACTTCCTTATATAGAGGAAGGGTCTTGCGAAGGATAGT
GGGATTGTGCGTCATCCCTTACGTCAGTGGAGATATCACATCAATCCACTTGC
TTTGAAGACGTGGTTGGAACGTCTTCTTTTTCCACGATGCTCCTCGTGGGTG
GGGGTCCATCTTTGGGACCACTGTCGGCAGAGGCATCTTGAACGATAGCCTT
TCCTTTATCGCAATGATGGCATTTGTAGGTGCCACCTTCCTTTTCTACTGTCCT
TTTGATGAAGTGACAGATAGCTGGGCAATGGAATCCGAGGAGGTTTCCCGAT
 
ATTACCCTTTGTTGAAAAGTCTCAATAGCCCTTTGGTCTTCTGAGACTGTATCT
TTGATATTCTTGGAGTAGACGAGAGTGTCGTGCTCCACCATGTTCACATCAAT
CCACTTGCTTTGAAGACGTGGTTGGAACGTCTTCTTTTTCCACGATGCTCCTC
GTGGGTGGGGGTCCATCTTTGGGACCACTGTCGGCAGAGGCATCTTGAACG
ATAGCCTTTCCTTTATCGCAATGATGGCATTTGTAGGTGCCACCTTCCTTTTCT
ACTGTCCTTTTGATGAAGTGACAGATAGCTGGGCAATGGAATCCGAGGAGGT
TTCCCGATATTACCCTTTGTTGAAAAGTCTCAATAGCCCTTTGGTCTTCTGAGA
CTGTATCTTTGATATTCTTGGAGTAGACGAGAGTGTCGTGCTCCACCATGTTG
GCGTACCCGGGGATCCTCTAGAGTCGACCTGCAGGCATGCAAGCT
Bovine Collagen alpha-1 (1) Expression vector 8
   SEQ ID NO: 12: 
AGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCC
CTTCGGGAAGCGTGGCGCTTTCTCATAGCTCACGCTGTAGGTATCTCAGTTC
GGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGG
CTACACTAGAAGGACAGTATTTGGTATCTGCGCTCTGCTGAAGCCAGTTACCT
TCGGAAAAAGAGTTGGTAGCTCTTGATCCGGCAAACAAACCACCGCTGGTAG
CGGTGGTTTTTTTGTTTGCAAGCAGCAGATTACGCGCAGAAAAAAAGGATCTC
AAGAAGATCCTTTGATCTTTTCTACGGGGTCTGACGCTCAGTGGAACGAAAAC
TCACGTTAAGGGATTTTGGTCATGCATTCTAGGTACTAAAACAATTCATCCAG
TAAAATATAATATTTTATTTTCTCCCAATCAGGCTTGATCCCCAGTAAGTCAAA
AAATAGCTCGACATACTGTTCTTCCCCGATATCCTCCCTGATCGACCGGACG
CAGAAGGCAATGTCATACCACTTGTCCGCCCTGCCGCTTCTCCCAAGATCAA
TAAAGCCACTTACTTTGCCATCTTTCACAAAGATGTTGCTGTCTCCCAGGTCG
CCGTGGGAAAAGACAAGTTCCTCTTCGGGCTTTTCCGTCTTTAAAAAATCATA
CAGCTCGCGCGGATCTTTAAATGGAGTGTCTTCTTCCCAGTTTTCGCAATCCA
CATCGGCCAGATCGTTATTCAGTAAGTAATCCAATTCGGCTAAGCGGCTGTCT
AAGCTATTCGTATAGGGACAATCCGATATGTCGATGGAGTGAAAGAGCCTGA
TGCACTCCGCATACAGCTCGATAATCTTTTCAGGGCTTTGTTCATCTTCATACT
CTTCCGAGCAAAGGACGCCATCGGCCTCACTCATGAGCAGATTGCTCCAGCC
ATCATGCCGTTCAAAGTGCAGGACCTTTGGAACAGGCAGCTTTCCTTCCAGC
CATAGCATCATGTCCTTTTCCCGTTCCACATCATAGGTGGTCCCTTTATACCG
 
GCTGTCCGTCATTTTTAAATATAGGTTTTCATTTTCTCCCACCAGCTTATATAC
CTTAGCAGGAGACATTCCTTCCGTATCTTTTACGCAGCGGTATTTTTCGATCA
GTTTTTTCAATTCCGGTGATATTCTCATTTTAGCCATTTATTATTTCCTTCCTCT
TTTCTACAGTATTTAAAGATACCCCAAGAAGCTAATTATAACAAGACGAACTCC
AATTCACTGTTCCTTGCATTCTAAAACCTTAAATACCAGAAAACAGCTTTTTCA
AAGTTGTTTTCAAAGTTGGCGTATAACATAGTATCGACGGAGCCGATTTTGAA
ACCGCGGTGATCACAGGCAGCAACGCTCTGTCATCGTTACAATCAACATGCT
ACCCTCCGCGAGATCATCCGTGTTTCAAACCCGGCAGCTTAGTTGCCGTTCT
TCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTT
GTGCCGAGCTGCCGGTCGGGGAGCTGTTGGCTGGCTGGTGGCAGGATATAT
TGTGGTGTAAACAAATTGACGCTTAGACAACTTAATAACACATTGCGGACGTT
TTTAATGTACTGAATTAACGCCGAATTGAATTCGAGCTCGGTGGAGGATCTGG
ATTTTAGTACTGGATTTTGGTTTTAGGAATTAGAAATTTTATTGATAGAAGTATT
TTACAAATACAAATACATACTAAGGGTTTCTTATATGCTCAACACATGAGCGAA
ACCCTATAGGAACCCTAATTCCCTTATCTGGGAACTACTCACACATTATTATG
GAGAAACTATTCACTCTCCTTCTCAAACGTTTCTGTTCCCTCTGGACAGCCCC
GACTGACGTTGGACTCAACCTCAATGGGCGCTAATTGTAATAGCTCCTGTTTG
CTTCTTATGGCCATTTCGCAAAGGTGTTGCAGTGTTCTGCTACCACCACTCAC
TGTAATTGACACACCTCTGAAACGGATGGAGTAAGTGCAACCAATTTGATCAA
ATCCAAAGAAACGACTAGCAGCGTAATTTACTGAGTCACCAGTCCAGGAACC
TAGAACTCGATGGAGAGAGGCCTCTGTCCGATCATAGCGTAGGTAGCGTTTG
AAAACTACCTTGCCAAATCCCCAGGACTCTTTAAATCCCAGTGGGTTGTCCTG
GTTGGAGTTTGTTTCGTCATTGTGTAAACGCCATTCTGTCCAAGATGGGCTCT
CATCGGGTAACTTAAATGGGCTGGTAGTGCCGCCAGAACCACCGTCCCATCT
CTCTGAATTGGCCTGCTCTCTTGCATCATTACCCTGGATTGCTCTCTCCATTG
TCCTCTCCAAATGAAATGAACTTCCTTATATAGAGGAAGGGTCTTGCGAAGGA
TAGTGGGATTGTGCGTCATCCCTTACGTCAGTGGAGATGTCACATCAATCCA
CTTGCTTTGTAGACGTGGTTGGAACCTCTTCTTTTTCCACGATGCTCCTCGTG
GGTGGGGGTCCATCTTTGGGACCACTGTCGGCAGAGAGATCTTGAATGATAG
CCTTTCCTTTATCGCAATGATGGCATTTGTAGGAGCCACCTTCCTTTTCTACT
GTCCTTTCGATGAAGTGACAGATAGCTGGGCAATGGAATCCGAGGAGGTTTC
CCGAAATTATCCTTTGTTGAAAAGTCTCAAACAATAGGCAACCGTGGACTTCT
TCACTAGCTCATCGAGATAGCAATGCCACTAGCTAATTTCTTACGTTGTATCTT
ATTTGTTTTACTTTGGGGCATGACATGGTTAAACCCCATCAAAGAGAAAGTGT
TTCATCCACTAGTCAATATACGAATTCATTCGAATTTATCCCTGTAAATCCTAG
TTCTTAGGATGAACTGGTGTAATAAACAGCAAAAAAAAATAAAAAAAAATAAAT
CTGGAATCATTCGACCACCTCAATAAACTAAAGCTACCAATTACCACAATATA
 
GTCATCCATATCCACTTAGATATAAAAGATAAAAGTAAACAAATATTAAATTTC
ATATGCACGCATAGGAAACTCATGATCTTATCTTTTAAATAGACATCTAGTTTT
CTTAGGTTATAAATAGACATTTTGTCCTAGAACTTCTTCACTACTAAAACCTAG
CCTCAAAAAGCAGGCTGCCACCATGCATCACCATCATCATCATGGATCTGAG
AATTTATATTTTCAAGGTCAATTGAGTTATGGATACGACGAAAAATCAACTGGA
ATATCAGTGCCAGGACCAATGGGTCCATCAGGACCTAGGGGTCTTCCAGGG
CCACCTGGCGCTCCTGGTCCTCAGGGATTTCAAGGTCCTCCAGGTGAACCTG
GAGAGCCTGGTGCTTCTGGTCCAATGGGACCAAGAGGCCCTCCTGGTCCAC
CTGGTAAAAATGGTGATGATGGTGAGGCTGGAAAGCCTGGTCGTCCTGGAG
AAAGAGGACCACCAGGACCACAGGGAGCTAGAGGTTTGCCAGGTACAGCCG
GTCTTCCAGGAATGAAAGGACACAGGGGTTTTTCTGGTCTTGATGGTGCTAA
GGGAGACGCAGGACCTGCTGGACCAAAGGGTGAACCTGGATCTCCTGGTGA
AAATGGTGCTCCTGGACAAATGGGACCTAGAGGATTGCCTGGCGAAAGAGG
AAGACCTGGTGCTCCTGGACCAGCTGGAGCTAGAGGAAATGATGGAGCTAC
TGGTGCTGCCGGACCACCAGGACCAACTGGACCAGCTGGACCACCTGGTTT
TCCAGGAGCAGTTGGGGCTAAGGGAGAAGGTGGACCACAAGGACCTAGAGG
ATCAGAAGGACCTCAAGGAGTTAGGGGAGAACCAGGTCCTCCTGGTCCTGC
TGGAGCTGCTGGACCCGCTGGTAACCCAGGAGCAGATGGACAACCTGGAGC
AAAAGGGGCTAATGGTGCTCCTGGTATTGCAGGTGCTCCTGGATTCCCTGGT
GCTAGAGGACCTTCAGGACCTCAGGGACCATCCGGTCCTCCAGGACCAAAG
GGAAACTCTGGTGAACCAGGGGCTCCTGGATCTAAGGGAGATACTGGAGCT
AAGGGTGAGCCAGGACCTACAGGAATTCAAGGACCACCAGGACCTGCAGGA
GAGGAAGGTAAGAGGGGTGCTAGAGGTGAACCTGGACCAGCTGGATTGCCA
GGCCCACCTGGAGAGAGGGGTGGTCCAGGTTCTAGAGGTTTTCCAGGAGCT
GATGGAGTTGCTGGTCCAAAGGGTCCTGCTGGTGAGAGAGGTGCACCTGGA
CCAGCAGGACCTAAAGGATCACCTGGAGAAGCTGGTCGTCCTGGTGAGGCT
GGTCTTCCAGGTGCTAAGGGCCTTACAGGATCTCCAGGATCACCAGGTCCAG
ATGGAAAGACTGGACCACCAGGTCCTGCAGGACAAGATGGAAGGCCTGGTC
CACCTGGACCACCTGGTGCTAGGGGACAAGCTGGTGTTATGGGATTCCCAG
GTCCAAAGGGTGCAGCTGGTGAGCCTGGTAAAGCTGGTGAAAGAGGAGTTC
CAGGACCTCCTGGTGCTGTTGGACCTGCTGGTAAAGATGGTGAAGCAGGTG
CTCAGGGTCCACCAGGTCCAGCCGGTCCAGCTGGTGAGAGGGGAGAGCAA
GGTCCTGCTGGATCTCCTGGATTTCAAGGTTTGCCTGGACCTGCTGGACCTC
CAGGAGAGGCTGGTAAACCTGGTGAGCAGGGTGTGCCTGGTGATCTTGGTG
CTCCTGGACCTAGTGGCGCAAGAGGCGAAAGGGGATTTCCAGGTGAAAGAG
GAGTTCAGGGACCACCAGGTCCAGCTGGTCCAAGAGGCGCTAATGGAGCTC
CAGGAAATGATGGAGCAAAGGGTGATGCTGGCGCTCCAGGAGCTCCTGGTT
CTCAAGGTGCACCTGGATTGCAAGGAATGCCAGGAGAGAGAGGTGCAGCTG
 
GTTTGCCGGGACCAAAGGGTGATAGAGGTGATGCTGGTCCTAAGGGAGCTG
ATGGAGCTCCAGGAAAGGATGGTGTGAGAGGATTGACTGGTCCAATTGGAC
CACCAGGTCCTGCTGGAGCACCAGGAGATAAAGGAGAAGCTGGTCCTTCAG
GACCAGCCGGTCCAACTGGAGCTAGAGGAGCTCCAGGAGATAGAGGTGAAC
CAGGACCACCAGGACCTGCTGGGTTTGCAGGTCCACCTGGTGCTGATGGAC
AACCTGGTGCTAAAGGAGAACCAGGCGATGCTGGTGCTAAGGGAGATGCTG
GCCCACCTGGTCCAGCTGGTCCTGCTGGGCCACCAGGTCCTATCGGAAATG
TTGGTGCTCCAGGGCCTAAAGGAGCTAGAGGATCAGCAGGTCCCCCAGGTG
CTACAGGTTTTCCAGGGGCTGCTGGAAGAGTGGGTCCCCCAGGTCCTTCTG
GGAATGCAGGACCACCAGGTCCTCCAGGACCAGCTGGAAAAGAAGGAAGTA
AGGGACCAAGGGGAGAAACTGGACCTGCAGGACGTCCAGGTGAAGTTGGTC
CACCTGGACCTCCAGGTCCAGCTGGAGAGAAAGGAGCTCCAGGAGCTGATG
GTCCAGCAGGTGCTCCTGGTACTCCAGGTCCACAAGGTATTGCTGGTCAAAG
GGGAGTGGTTGGTCTCCCAGGTCAGAGAGGTGAAAGAGGTTTCCCAGGTTT
GCCTGGACCATCTGGAGAACCAGGTAAGCAGGGCCCATCTGGAGCTTCTGG
TGAGAGAGGGCCACCTGGACCTATGGGACCTCCTGGGCTTGCTGGACCTCC
AGGGGAATCTGGTAGGGAGGGTGCTCCAGGTGCTGAAGGATCACCTGGTAG
AGATGGATCACCTGGAGCTAAAGGTGATAGAGGGGAAACAGGTCCAGCAGG
TCCACCTGGCGCTCCTGGTGCTCCAGGAGCACCAGGGCCAGTTGGACCTGC
TGGAAAGTCTGGAGATAGGGGAGAGACTGGACCAGCTGGACCTGCTGGACC
AATCGGTCCTGTTGGCGCTAGAGGTCCTGCTGGACCACAAGGTCCTAGGGG
AGATAAAGGTGAAACAGGTGAACAAGGAGATAGAGGAATTAAAGGTCATAGA
GGGTTTTCTGGTTTGCAAGGACCTCCAGGACCTCCTGGATCTCCAGGAGAGC
AAGGACCATCTGGTGCAAGCGGACCTGCTGGTCCTAGAGGACCTCCAGGTT
CAGCTGGCTCACCTGGTAAGGATGGATTAAATGGTTTGCCTGGACCAATAGG
TCCACCAGGACCTAGAGGAAGGACAGGTGATGCAGGACCAGCTGGTCCACC
TGGTCCACCAGGACCACCAGGACCACCAGGTCCACCTTCTGGTGGATATGAT
CTTAGTTTTCTTCCTCAACCTCCCCAGGAGAAAGCTCATGATGGTGGTAGATA
TTATAGGGCTTAAACCCAGCTTTCTTGTACAAAGTGGTAGCTCGAATTTCCCC
GATCGTTCAAACATTTGGCAATAAAGTTTCTTAAGATTGAATCCTGTTGCCGG
TCTTGCGATGATTATCATATAATTTCTGTTGAATTACGTTAAGCATGTAATAATT
AACATGTAATGCATGACGTTATTTATGAGATGGGTTTTTATGATTAGAGTCCC
GCAATTATACATTTAATACGCGATAGAAAACAAAATATAGCGCGCAAACTAGG
ATAAATTATCGCGCGCGGTGTCATCTATGTTACTAGATCGGGAAAGCTTTTAA
ACTATCAGTGTTTGACAGGATATATTGGCGGGTAAACCTAAGAGAAAAGAGC
GTTTATTAGAATAATCGGATATTTAAAAGGGCGTGAAAAGGTTTATCCGTTCG
TCCATTTGTATGTGCATGCCAACCACAGGGTTCCCCTCGGGATCAAAGTACTT
TGATCCAACCCCTCCGCTGCTATAGTGCAGTCGGCTTCTGACGTTCAGTGCA
 
GCCGTCTTCTGAAAACGACATGTCGCACAAGTCCTAAGTTACGCGACAGGCT
GCCGCCCTGCCCTTTTCCTGGCGTTTTCTTGTCGCGTGTTTTAGTCGCATAAA
GTAGAATACTTGCGACTAGAACCGGAGACATTACGCCATGAACAAGAGCGCC
GCCGCTGGCCTGCTGGGCTATGCCCGCGTCAGCACCGACGACCAGGACTTG
ACCAACCAACGGGCCGAACTGCACGCGGCCGGCTGCACCAAGCTGTTTTCC
GAGAAGATCACCGGCACCAGGCGCGACCGCCCGGAGCTGGCCAGGATGCT
TGACCACCTACGCCCTGGCGACGTTGTGACAGTGACCAGGCTAGACCGCCT
GGCCCGCAGCACCCGCGACCTACTGGACATTGCCGAGCGCATCCAGGAGG
CCGGCGCGGGCCTGCGTAGCCTGGCAGAGCCGTGGGCCGACACCACCACG
CCGGCCGGCCGCATGGTGTTGACCGTGTTCGCCGGCATTGCCGAGTTCGAG
CGTTCCCTAATCATCGACCGCACCCGGAGCGGGCGCGAGGCCGCCAAGGC
CCGAGGCGTGAAGTTTGGCCCCCGCCCTACCCTCACCCCGGCACAGATCGC
GCACGCCCGCGAGCTGATCGACCAGGAAGGCCGCACCGTGAAAGAGGCGG
CTGCACTGCTTGGCGTGCATCGCTCGACCCTGTACCGCGCACTTGAGCGCA
GCGAGGAAGTGACGCCCACCGAGGCCAGGCGGCGCGGTGCCTTCCGTGAG
GACGCATTGACCGAGGCCGACGCCCTGGCGGCCGCCGAGAATGAACGCCA
AGAGGAACAAGCATGAAACCGCACCAGGACGGCCAGGACGAACCGTTTTTC
ATTACCGAAGAGATCGAGGCGGAGATGATCGCGGCCGGGTACGTGTTCGAG
CCGCCCGCGCACGTCTCAACCGTGCGGCTGCATGAAATCCTGGCCGGTTTG
TCTGATGCCAAGCTGGCGGCCTGGCCGGCCAGCTTGGCCGCTGAAGAAACC
GAGCGCCGCCGTCTAAAAAGGTGATGTGTATTTGAGTAAAACAGCTTGCGTC
ATGCGGTCGCTGCGTATATGATGCGATGAGTAAATAAACAAATACGCAAGGG
GAACGCATGAAGGTTATCGCTGTACTTAACCAGAAAGGCGGGTCAGGCAAGA
CGACCATCGCAACCCATCTAGCCCGCGCCCTGCAACTCGCCGGGGCCGATG
TTCTGTTAGTCGATTCCGATCCCCAGGGCAGTGCCCGCGATTGGGCGGCCG
TGCGGGAAGATCAACCGCTAACCGTTGTCGGCATCGACCGCCCGACGATTG
ACCGCGACGTGAAGGCCATCGGCCGGCGCGACTTCGTAGTGATCGACGGAG
CGCCCCAGGCGGCGGACTTGGCTGTGTCCGCGATCAAGGCAGCCGACTTCG
TGCTGATTCCGGTGCAGCCAAGCCCTTACGACATATGGGCCACCGCCGACCT
GGTGGAGCTGGTTAAGCAGCGCATTGAGGTCACGGATGGAAGGCTACAAGC
GGCCTTTGTCGTGTCGCGGGCGATCAAAGGCACGCGCATCGGCGGTGAGGT
TGCCGAGGCGCTGGCCGGGTACGAGCTGCCCATTCTTGAGTCCCGTATCAC
GCAGCGCGTGAGCTACCCAGGCACTGCCGCCGCCGGCACAACCGTTCTTGA
ATCAGAACCCGAGGGCGACGCTGCCCGCGAGGTCCAGGCGCTGGCCGCTG
AAATTAAATCAAAACTCATTTGAGTTAATGAGGTAAAGAGAAAATGAGCAAAA
GCACAAACACGCTAAGTGCCGGCCGTCCGAGCGCACGCAGCAGCAAGGCTG
CAACGTTGGCCAGCCTGGCAGACACGCCAGCCATGAAGCGGGTCAACTTTC
AGTTGCCGGCGGAGGATCACACCAAGCTGAAGATGTACGCGGTACGCCAAG
 
GCAAGACCATTACCGAGCTGCTATCTGAATACATCGCGCAGCTACCAGAGTA
AATGAGCAAATGAATAAATGAGTAGATGAATTTTAGCGGCTAAAGGAGGCGG
CATGGAAAATCAAGAACAACCAGGCACCGACGCCGTGGAATGCCCCATGTGT
GGAGGAACGGGCGGTTGGCCAGGCGTAAGCGGCTGGGTTGCCTGCCGGCC
CTGCAATGGCACTGGAACCCCCAAGCCCGAGGAATCGGCGTGAGCGGTCGC
AAACCATCCGGCCCGGTACAAATCGGCGCGGCGCTGGGTGATGACCTGGTG
GAGAAGTTGAAGGCCGCGCAGGCCGCCCAGCGGCAACGCATCGAGGCAGA
AGCACGCCCCGGTGAATCGTGGCAAGCGGCCGCTGATCGAATCCGCAAAGA
ATCCCGGCAACCGCCGGCAGCCGGTGCGCCGTCGATTAGGAAGCCGCCCA
AGGGCGACGAGCAACCAGATTTTTTCGTTCCGATGCTCTATGACGTGGGCAC
CCGCGATAGTCGCAGCATCATGGACGTGGCCGTTTTCCGTCTGTCGAAGCGT
GACCGACGAGCTGGCGAGGTGATCCGCTACGAGCTTCCAGACGGGCACGTA
GAGGTTTCCGCAGGGCCGGCCGGCATGGCCAGTGTGTGGGATTACGACCTG
GTACTGATGGCGGTTTCCCATCTAACCGAATCCATGAACCGATACCGGGAAG
GGAAGGGAGACAAGCCCGGCCGCGTGTTCCGTCCACACGTTGCGGACGTAC
TCAAGTTCTGCCGGCGAGCCGATGGCGGAAAGCAGAAAGACGACCTGGTAG
AAACCTGCATTCGGTTAAACACCACGCACGTTGCCATGCAGCGTACGAAGAA
GGCCAAGAACGGCCGCCTGGTGACGGTATCCGAGGGTGAAGCCTTGATTAG
CCGCTACAAGATCGTAAAGAGCGAAACCGGGCGGCCGGAGTACATCGAGAT
CGAGCTAGCTGATTGGATGTACCGCGAGATCACAGAAGGCAAGAACCCGGA
CGTGCTGACGGTTCACCCCGATTACTTTTTGATCGATCCCGGCATCGGCCGT
TTTCTCTACCGCCTGGCACGCCGCGCCGCAGGCAAGGCAGAAGCCAGATGG
TTGTTCAAGACGATCTACGAACGCAGTGGCAGCGCCGGAGAGTTCAAGAAGT
TCTGTTTCACCGTGCGCAAGCTGATCGGGTCAAATGACCTGCCGGAGTACGA
TTTGAAGGAGGAGGCGGGGCAGGCTGGCCCGATCCTAGTCATGCGCTACCG
CAACCTGATCGAGGGCGAAGCATCCGCCGGTTCCTAATGTACGGAGCAGAT
GCTAGGGCAAATTGCCCTAGCAGGGGAAAAAGGTCGAAAAGGTCTCTTTCCT
GTGGATAGCACGTACATTGGGAACCCAAAGCCGTACATTGGGAACCGGAAC
CCGTACATTGGGAACCCAAAGCCGTACATTGGGAACCGGTCACACATGTAAG
TGACTGATATAAAAGAGAAAAAAGGCGATTTTTCCGCCTAAAACTCTTTAAAA
CTTATTAAAACTCTTAAAACCCGCCTGGCCTGTGCATAACTGTCTGGCCAGCG
CACAGCCGAAGAGCTGCAAAAAGCGCCTACCCTTCGGTCGCTGCGCTCCCT
ACGCCCCGCCGCTTCGCGTCGGCCTATCGCGGCCGCTGGCCGCTCAAAAAT
GGCTGGCCTACGGCCAGGCAATCTACCAGGGCGCGGACAAGCCGCGCCGT
CGCCACTCGACCGCCGGCGCCCACATCAAGGCACCCTGCCTCGCGCGTTTC
GGTGATGACGGTGAAAACCTCTGACACATGCAGCTCCCGGAGACGGTCACA
GCTTGTCTGTAAGCGGATGCCGGGAGCAGACAAGCCCGTCAGGGCGCGTCA
GCGGGTGTTGGCGGGTGTCGGGGCGCAGCCATGACCCAGTCACGTAGCGA
 
TAGCGGAGTGTATACTGGCTTAACTATGCGGCATCAGAGCAGATTGTACTGA
GAGTGCACCATATGCGGTGTGAAATACCGCACAGATGCGTAAGGAGAAAATA
CCGCATCAGGCGCTCTTCCGCTTCCTCGCTCACTGACTCGCTGCGCTCGGTC
GTTCGGCTGCGGCGAGCGGTATCAGCTCACTCAAAGGCGGTAATACGGTTAT
CCACAGAATCAGGGGATAACGCAGGAAAGAACATGTGAGCAAAAGGCCAGC
AAA
Bovine Collagen alpha-1 (1) Expression vector 9
   SEQ ID NO: 15: 
AGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCC
CTTCGGGAAGCGTGGCGCTTTCTCATAGCTCACGCTGTAGGTATCTCAGTTC
GGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGG
CTACACTAGAAGGACAGTATTTGGTATCTGCGCTCTGCTGAAGCCAGTTACCT
TCGGAAAAAGAGTTGGTAGCTCTTGATCCGGCAAACAAACCACCGCTGGTAG
CGGTGGTTTTTTTGTTTGCAAGCAGCAGATTACGCGCAGAAAAAAAGGATCTC
AAGAAGATCCTTTGATCTTTTCTACGGGGTCTGACGCTCAGTGGAACGAAAAC
TCACGTTAAGGGATTTTGGTCATGCATTCTAGGTACTAAAACAATTCATCCAG
TAAAATATAATATTTTATTTTCTCCCAATCAGGCTTGATCCCCAGTAAGTCAAA
AAATAGCTCGACATACTGTTCTTCCCCGATATCCTCCCTGATCGACCGGACG
CAGAAGGCAATGTCATACCACTTGTCCGCCCTGCCGCTTCTCCCAAGATCAA
TAAAGCCACTTACTTTGCCATCTTTCACAAAGATGTTGCTGTCTCCCAGGTCG
CCGTGGGAAAAGACAAGTTCCTCTTCGGGCTTTTCCGTCTTTAAAAAATCATA
CAGCTCGCGCGGATCTTTAAATGGAGTGTCTTCTTCCCAGTTTTCGCAATCCA
CATCGGCCAGATCGTTATTCAGTAAGTAATCCAATTCGGCTAAGCGGCTGTCT
AAGCTATTCGTATAGGGACAATCCGATATGTCGATGGAGTGAAAGAGCCTGA
TGCACTCCGCATACAGCTCGATAATCTTTTCAGGGCTTTGTTCATCTTCATACT
CTTCCGAGCAAAGGACGCCATCGGCCTCACTCATGAGCAGATTGCTCCAGCC
ATCATGCCGTTCAAAGTGCAGGACCTTTGGAACAGGCAGCTTTCCTTCCAGC
CATAGCATCATGTCCTTTTCCCGTTCCACATCATAGGTGGTCCCTTTATACCG
GCTGTCCGTCATTTTTAAATATAGGTTTTCATTTTCTCCCACCAGCTTATATAC
CTTAGCAGGAGACATTCCTTCCGTATCTTTTACGCAGCGGTATTTTTCGATCA
GTTTTTTCAATTCCGGTGATATTCTCATTTTAGCCATTTATTATTTCCTTCCTCT
 
TTTCTACAGTATTTAAAGATACCCCAAGAAGCTAATTATAACAAGACGAACTCC
AATTCACTGTTCCTTGCATTCTAAAACCTTAAATACCAGAAAACAGCTTTTTCA
AAGTTGTTTTCAAAGTTGGCGTATAACATAGTATCGACGGAGCCGATTTTGAA
ACCGCGGTGATCACAGGCAGCAACGCTCTGTCATCGTTACAATCAACATGCT
ACCCTCCGCGAGATCATCCGTGTTTCAAACCCGGCAGCTTAGTTGCCGTTCT
TCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTT
GTGCCGAGCTGCCGGTCGGGGAGCTGTTGGCTGGCTGGTGGCAGGATATAT
TGTGGTGTAAACAAATTGACGCTTAGACAACTTAATAACACATTGCGGACGTT
TTTAATGTACTGAATTAACGCCGAATTGAATTCGAGCTCGGTGGAGGATCTGG
ATTTTAGTACTGGATTTTGGTTTTAGGAATTAGAAATTTTATTGATAGAAGTATT
TTACAAATACAAATACATACTAAGGGTTTCTTATATGCTCAACACATGAGCGAA
ACCCTATAGGAACCCTAATTCCCTTATCTGGGAACTACTCACACATTATTATG
GAGAAACTATTCACTCTCCTTCTCAAACGTTTCTGTTCCCTCTGGACAGCCCC
GACTGACGTTGGACTCAACCTCAATGGGCGCTAATTGTAATAGCTCCTGTTTG
CTTCTTATGGCCATTTCGCAAAGGTGTTGCAGTGTTCTGCTACCACCACTCAC
TGTAATTGACACACCTCTGAAACGGATGGAGTAAGTGCAACCAATTTGATCAA
ATCCAAAGAAACGACTAGCAGCGTAATTTACTGAGTCACCAGTCCAGGAACC
TAGAACTCGATGGAGAGAGGCCTCTGTCCGATCATAGCGTAGGTAGCGTTTG
AAAACTACCTTGCCAAATCCCCAGGACTCTTTAAATCCCAGTGGGTTGTCCTG
GTTGGAGTTTGTTTCGTCATTGTGTAAACGCCATTCTGTCCAAGATGGGCTCT
CATCGGGTAACTTAAATGGGCTGGTAGTGCCGCCAGAACCACCGTCCCATCT
CTCTGAATTGGCCTGCTCTCTTGCATCATTACCCTGGATTGCTCTCTCCATTG
TCCTCTCCAAATGAAATGAACTTCCTTATATAGAGGAAGGGTCTTGCGAAGGA
TAGTGGGATTGTGCGTCATCCCTTACGTCAGTGGAGATGTCACATCAATCCA
CTTGCTTTGTAGACGTGGTTGGAACCTCTTCTTTTTCCACGATGCTCCTCGTG
GGTGGGGGTCCATCTTTGGGACCACTGTCGGCAGAGAGATCTTGAATGATAG
CCTTTCCTTTATCGCAATGATGGCATTTGTAGGAGCCACCTTCCTTTTCTACT
GTCCTTTCGATGAAGTGACAGATAGCTGGGCAATGGAATCCGAGGAGGTTTC
CCGAAATTATCCTTTGTTGAAAAGTCTCAAACAATAGGCAACCGTGGACTTCT
TCACTAGCTCATCGAGATAGCAATGCCACTAGCTAATTTCTTACGTTGTATCTT
ATTTGTTTTACTTTGGGGCATGACATGGTTAAACCCCATCAAAGAGAAAGTGT
TTCATCCACTAGTCAATATACGAATTCATTCGAATTTATCCCTGTAAATCCTAG
TTCTTAGGATGAACTGGTGTAATAAACAGCAAAAAAAAATAAAAAAAAATAAAT
CTGGAATCATTCGACCACCTCAATAAACTAAAGCTACCAATTACCACAATATA
GTCATCCATATCCACTTAGATATAAAAGATAAAAGTAAACAAATATTAAATTTC
ATATGCACGCATAGGAAACTCATGATCTTATCTTTTAAATAGACATCTAGTTTT
CTTAGGTTATAAATAGACATTTTGTCCTAGAACTTCTTCACTACTAAAACCTAG
 
CCTCAAAAAGCAGGCTGCCACCATGGTGAGCAGTGGTGAGGACATCTTTTCA
GGCCTAGTCCCGATACTTATTGAACTTGAAGGAGACGTCAATGGGCACAGAT
TCTCAGTACGAGGCGAGGGATACGGGGATGCTTCCAATGGAAAGCTTGAGAT
AAAATTTATATGTACAACTGGAAGGCTTCCTGTCCCATGGCCAACTTTAGTTA
CCACTCTTAGCTACGGTGTACAATGCTTCGCTAAATACCCAGAACATATGCGT
CAGAATGACTTCTTCAAGTCAGCAATGCCTGATGGATATGTTCAAGAAAGAAC
AATCTCTTTTAAAGAGGACGGAACTTATAAGACCAGAGCTGAAGTCAAATTCG
AGGGAGAGGCCTTGGTGAACAGAATTGACTTAAAAGGACTGGAATTCAAGGA
AGATGGCAATATCCTCGGTCACAAGCTTGAATACAGTTTTAATTCACACTACG
TGTATATAACCGCGGATAAGAACAGGAATGGTTTAGAAGCTCAGTTTAGAATA
AGACATAACGTGGACGATGGATCCGTGCAGCTAGCAGATCACTATCAGCAAA
ATACGCCGATAGGGGAAGGCCCTGTTCTTTTGCCAGAGCAGCATTACCTAAC
TACTAACTCTGTTTTGTCTAAGGACCCACAAGAGCGGAGAGATCATATGGTTT
TGGTTGAGTTCGTTACGGCAGCTGGACTCTCCCTAGGCATGGATGAATTATA
CAAATCAGGATCTGAGAATTTATATTTTCAAGGTCAATTGAGTTATGGATACGA
CGAAAAATCAACTGGAATATCAGTGCCAGGACCAATGGGTCCATCAGGACCT
AGGGGTCTTCCAGGGCCACCTGGCGCTCCTGGTCCTCAGGGATTTCAAGGT
CCTCCAGGTGAACCTGGAGAGCCTGGTGCTTCTGGTCCAATGGGACCAAGA
GGCCCTCCTGGTCCACCTGGTAAAAATGGTGATGATGGTGAGGCTGGAAAG
CCTGGTCGTCCTGGAGAAAGAGGACCACCAGGACCACAGGGAGCTAGAGGT
TTGCCAGGTACAGCCGGTCTTCCAGGAATGAAAGGACACAGGGGTTTTTCTG
GTCTTGATGGTGCTAAGGGAGACGCAGGACCTGCTGGACCAAAGGGTGAAC
CTGGATCTCCTGGTGAAAATGGTGCTCCTGGACAAATGGGACCTAGAGGATT
GCCTGGCGAAAGAGGAAGACCTGGTGCTCCTGGACCAGCTGGAGCTAGAGG
AAATGATGGAGCTACTGGTGCTGCCGGACCACCAGGACCAACTGGACCAGC
TGGACCACCTGGTTTTCCAGGAGCAGTTGGGGCTAAGGGAGAAGGTGGACC
ACAAGGACCTAGAGGATCAGAAGGACCTCAAGGAGTTAGGGGAGAACCAGG
TCCTCCTGGTCCTGCTGGAGCTGCTGGACCCGCTGGTAACCCAGGAGCAGA
TGGACAACCTGGAGCAAAAGGGGCTAATGGTGCTCCTGGTATTGCAGGTGCT
CCTGGATTCCCTGGTGCTAGAGGACCTTCAGGACCTCAGGGACCATCCGGT
CCTCCAGGACCAAAGGGAAACTCTGGTGAACCAGGGGCTCCTGGATCTAAG
GGAGATACTGGAGCTAAGGGTGAGCCAGGACCTACAGGAATTCAAGGACCA
CCAGGACCTGCAGGAGAGGAAGGTAAGAGGGGTGCTAGAGGTGAACCTGGA
CCAGCTGGATTGCCAGGCCCACCTGGAGAGAGGGGTGGTCCAGGTTCTAGA
GGTTTTCCAGGAGCTGATGGAGTTGCTGGTCCAAAGGGTCCTGCTGGTGAGA
GAGGTGCACCTGGACCAGCAGGACCTAAAGGATCACCTGGAGAAGCTGGTC
GTCCTGGTGAGGCTGGTCTTCCAGGTGCTAAGGGCCTTACAGGATCTCCAG
GATCACCAGGTCCAGATGGAAAGACTGGACCACCAGGTCCTGCAGGACAAG
 
ATGGAAGGCCTGGTCCACCTGGACCACCTGGTGCTAGGGGACAAGCTGGTG
TTATGGGATTCCCAGGTCCAAAGGGTGCAGCTGGTGAGCCTGGTAAAGCTG
GTGAAAGAGGAGTTCCAGGACCTCCTGGTGCTGTTGGACCTGCTGGTAAAGA
TGGTGAAGCAGGTGCTCAGGGTCCACCAGGTCCAGCCGGTCCAGCTGGTGA
GAGGGGAGAGCAAGGTCCTGCTGGATCTCCTGGATTTCAAGGTTTGCCTGGA
CCTGCTGGACCTCCAGGAGAGGCTGGTAAACCTGGTGAGCAGGGTGTGCCT
GGTGATCTTGGTGCTCCTGGACCTAGTGGCGCAAGAGGCGAAAGGGGATTT
CCAGGTGAAAGAGGAGTTCAGGGACCACCAGGTCCAGCTGGTCCAAGAGGC
GCTAATGGAGCTCCAGGAAATGATGGAGCAAAGGGTGATGCTGGCGCTCCA
GGAGCTCCTGGTTCTCAAGGTGCACCTGGATTGCAAGGAATGCCAGGAGAG
AGAGGTGCAGCTGGTTTGCCGGGACCAAAGGGTGATAGAGGTGATGCTGGT
CCTAAGGGAGCTGATGGAGCTCCAGGAAAGGATGGTGTGAGAGGATTGACT
GGTCCAATTGGACCACCAGGTCCTGCTGGAGCACCAGGAGATAAAGGAGAA
GCTGGTCCTTCAGGACCAGCCGGTCCAACTGGAGCTAGAGGAGCTCCAGGA
GATAGAGGTGAACCAGGACCACCAGGACCTGCTGGGTTTGCAGGTCCACCT
GGTGCTGATGGACAACCTGGTGCTAAAGGAGAACCAGGCGATGCTGGTGCT
AAGGGAGATGCTGGCCCACCTGGTCCAGCTGGTCCTGCTGGGCCACCAGGT
CCTATCGGAAATGTTGGTGCTCCAGGGCCTAAAGGAGCTAGAGGATCAGCA
GGTCCCCCAGGTGCTACAGGTTTTCCAGGGGCTGCTGGAAGAGTGGGTCCC
CCAGGTCCTTCTGGGAATGCAGGACCACCAGGTCCTCCAGGACCAGCTGGA
AAAGAAGGAAGTAAGGGACCAAGGGGAGAAACTGGACCTGCAGGACGTCCA
GGTGAAGTTGGTCCACCTGGACCTCCAGGTCCAGCTGGAGAGAAAGGAGCT
CCAGGAGCTGATGGTCCAGCAGGTGCTCCTGGTACTCCAGGTCCACAAGGT
ATTGCTGGTCAAAGGGGAGTGGTTGGTCTCCCAGGTCAGAGAGGTGAAAGA
GGTTTCCCAGGTTTGCCTGGACCATCTGGAGAACCAGGTAAGCAGGGCCCAT
CTGGAGCTTCTGGTGAGAGAGGGCCACCTGGACCTATGGGACCTCCTGGGC
TTGCTGGACCTCCAGGGGAATCTGGTAGGGAGGGTGCTCCAGGTGCTGAAG
GATCACCTGGTAGAGATGGATCACCTGGAGCTAAAGGTGATAGAGGGGAAA
CAGGTCCAGCAGGTCCACCTGGCGCTCCTGGTGCTCCAGGAGCACCAGGGC
CAGTTGGACCTGCTGGAAAGTCTGGAGATAGGGGAGAGACTGGACCAGCTG
GACCTGCTGGACCAATCGGTCCTGTTGGCGCTAGAGGTCCTGCTGGACCAC
AAGGTCCTAGGGGAGATAAAGGTGAAACAGGTGAACAAGGAGATAGAGGAA
TTAAAGGTCATAGAGGGTTTTCTGGTTTGCAAGGACCTCCAGGACCTCCTGG
ATCTCCAGGAGAGCAAGGACCATCTGGTGCAAGCGGACCTGCTGGTCCTAG
AGGACCTCCAGGTTCAGCTGGCTCACCTGGTAAGGATGGATTAAATGGTTTG
CCTGGACCAATAGGTCCACCAGGACCTAGAGGAAGGACAGGTGATGCAGGA
CCAGCTGGTCCACCTGGTCCACCAGGACCACCAGGACCACCAGGTCCACCT
TCTGGTGGATATGATCTTAGTTTTCTTCCTCAACCTCCCCAGGAGAAAGCTCA
 
TGATGGTGGTAGATATTATAGGGCTGCATTTGTTTATTAAACCCAGCTTTCTTG
TACAAAGTGGTAGCTCGAATTTCCCCGATCGTTCAAACATTTGGCAATAAAGT
TTCTTAAGATTGAATCCTGTTGCCGGTCTTGCGATGATTATCATATAATTTCTG
TTGAATTACGTTAAGCATGTAATAATTAACATGTAATGCATGACGTTATTTATG
AGATGGGTTTTTATGATTAGAGTCCCGCAATTATACATTTAATACGCGATAGAA
AACAAAATATAGCGCGCAAACTAGGATAAATTATCGCGCGCGGTGTCATCTAT
GTTACTAGATCGGGAAAGCTTTTAAACTATCAGTGTTTGACAGGATATATTGG
CGGGTAAACCTAAGAGAAAAGAGCGTTTATTAGAATAATCGGATATTTAAAAG
GGCGTGAAAAGGTTTATCCGTTCGTCCATTTGTATGTGCATGCCAACCACAG
GGTTCCCCTCGGGATCAAAGTACTTTGATCCAACCCCTCCGCTGCTATAGTG
CAGTCGGCTTCTGACGTTCAGTGCAGCCGTCTTCTGAAAACGACATGTCGCA
CAAGTCCTAAGTTACGCGACAGGCTGCCGCCCTGCCCTTTTCCTGGCGTTTT
CTTGTCGCGTGTTTTAGTCGCATAAAGTAGAATACTTGCGACTAGAACCGGAG
ACATTACGCCATGAACAAGAGCGCCGCCGCTGGCCTGCTGGGCTATGCCCG
CGTCAGCACCGACGACCAGGACTTGACCAACCAACGGGCCGAACTGCACGC
GGCCGGCTGCACCAAGCTGTTTTCCGAGAAGATCACCGGCACCAGGCGCGA
CCGCCCGGAGCTGGCCAGGATGCTTGACCACCTACGCCCTGGCGACGTTGT
GACAGTGACCAGGCTAGACCGCCTGGCCCGCAGCACCCGCGACCTACTGGA
CATTGCCGAGCGCATCCAGGAGGCCGGCGCGGGCCTGCGTAGCCTGGCAG
AGCCGTGGGCCGACACCACCACGCCGGCCGGCCGCATGGTGTTGACCGTG
TTCGCCGGCATTGCCGAGTTCGAGCGTTCCCTAATCATCGACCGCACCCGGA
GCGGGCGCGAGGCCGCCAAGGCCCGAGGCGTGAAGTTTGGCCCCCGCCCT
ACCCTCACCCCGGCACAGATCGCGCACGCCCGCGAGCTGATCGACCAGGAA
GGCCGCACCGTGAAAGAGGCGGCTGCACTGCTTGGCGTGCATCGCTCGACC
CTGTACCGCGCACTTGAGCGCAGCGAGGAAGTGACGCCCACCGAGGCCAG
GCGGCGCGGTGCCTTCCGTGAGGACGCATTGACCGAGGCCGACGCCCTGG
CGGCCGCCGAGAATGAACGCCAAGAGGAACAAGCATGAAACCGCACCAGGA
CGGCCAGGACGAACCGTTTTTCATTACCGAAGAGATCGAGGCGGAGATGATC
GCGGCCGGGTACGTGTTCGAGCCGCCCGCGCACGTCTCAACCGTGCGGCT
GCATGAAATCCTGGCCGGTTTGTCTGATGCCAAGCTGGCGGCCTGGCCGGC
CAGCTTGGCCGCTGAAGAAACCGAGCGCCGCCGTCTAAAAAGGTGATGTGT
ATTTGAGTAAAACAGCTTGCGTCATGCGGTCGCTGCGTATATGATGCGATGA
GTAAATAAACAAATACGCAAGGGGAACGCATGAAGGTTATCGCTGTACTTAAC
CAGAAAGGCGGGTCAGGCAAGACGACCATCGCAACCCATCTAGCCCGCGCC
CTGCAACTCGCCGGGGCCGATGTTCTGTTAGTCGATTCCGATCCCCAGGGCA
GTGCCCGCGATTGGGCGGCCGTGCGGGAAGATCAACCGCTAACCGTTGTCG
GCATCGACCGCCCGACGATTGACCGCGACGTGAAGGCCATCGGCCGGCGC
GACTTCGTAGTGATCGACGGAGCGCCCCAGGCGGCGGACTTGGCTGTGTCC
 
GCGATCAAGGCAGCCGACTTCGTGCTGATTCCGGTGCAGCCAAGCCCTTAC
GACATATGGGCCACCGCCGACCTGGTGGAGCTGGTTAAGCAGCGCATTGAG
GTCACGGATGGAAGGCTACAAGCGGCCTTTGTCGTGTCGCGGGCGATCAAA
GGCACGCGCATCGGCGGTGAGGTTGCCGAGGCGCTGGCCGGGTACGAGCT
GCCCATTCTTGAGTCCCGTATCACGCAGCGCGTGAGCTACCCAGGCACTGC
CGCCGCCGGCACAACCGTTCTTGAATCAGAACCCGAGGGCGACGCTGCCCG
CGAGGTCCAGGCGCTGGCCGCTGAAATTAAATCAAAACTCATTTGAGTTAAT
GAGGTAAAGAGAAAATGAGCAAAAGCACAAACACGCTAAGTGCCGGCCGTC
CGAGCGCACGCAGCAGCAAGGCTGCAACGTTGGCCAGCCTGGCAGACACG
CCAGCCATGAAGCGGGTCAACTTTCAGTTGCCGGCGGAGGATCACACCAAG
CTGAAGATGTACGCGGTACGCCAAGGCAAGACCATTACCGAGCTGCTATCTG
AATACATCGCGCAGCTACCAGAGTAAATGAGCAAATGAATAAATGAGTAGATG
AATTTTAGCGGCTAAAGGAGGCGGCATGGAAAATCAAGAACAACCAGGCACC
GACGCCGTGGAATGCCCCATGTGTGGAGGAACGGGCGGTTGGCCAGGCGT
AAGCGGCTGGGTTGCCTGCCGGCCCTGCAATGGCACTGGAACCCCCAAGCC
CGAGGAATCGGCGTGAGCGGTCGCAAACCATCCGGCCCGGTACAAATCGGC
GCGGCGCTGGGTGATGACCTGGTGGAGAAGTTGAAGGCCGCGCAGGCCGC
CCAGCGGCAACGCATCGAGGCAGAAGCACGCCCCGGTGAATCGTGGCAAG
CGGCCGCTGATCGAATCCGCAAAGAATCCCGGCAACCGCCGGCAGCCGGTG
CGCCGTCGATTAGGAAGCCGCCCAAGGGCGACGAGCAACCAGATTTTTTCGT
TCCGATGCTCTATGACGTGGGCACCCGCGATAGTCGCAGCATCATGGACGT
GGCCGTTTTCCGTCTGTCGAAGCGTGACCGACGAGCTGGCGAGGTGATCCG
CTACGAGCTTCCAGACGGGCACGTAGAGGTTTCCGCAGGGCCGGCCGGCAT
GGCCAGTGTGTGGGATTACGACCTGGTACTGATGGCGGTTTCCCATCTAACC
GAATCCATGAACCGATACCGGGAAGGGAAGGGAGACAAGCCCGGCCGCGT
GTTCCGTCCACACGTTGCGGACGTACTCAAGTTCTGCCGGCGAGCCGATGG
CGGAAAGCAGAAAGACGACCTGGTAGAAACCTGCATTCGGTTAAACACCACG
CACGTTGCCATGCAGCGTACGAAGAAGGCCAAGAACGGCCGCCTGGTGACG
GTATCCGAGGGTGAAGCCTTGATTAGCCGCTACAAGATCGTAAAGAGCGAAA
CCGGGCGGCCGGAGTACATCGAGATCGAGCTAGCTGATTGGATGTACCGCG
AGATCACAGAAGGCAAGAACCCGGACGTGCTGACGGTTCACCCCGATTACTT
TTTGATCGATCCCGGCATCGGCCGTTTTCTCTACCGCCTGGCACGCCGCGCC
GCAGGCAAGGCAGAAGCCAGATGGTTGTTCAAGACGATCTACGAACGCAGT
GGCAGCGCCGGAGAGTTCAAGAAGTTCTGTTTCACCGTGCGCAAGCTGATC
GGGTCAAATGACCTGCCGGAGTACGATTTGAAGGAGGAGGCGGGGCAGGCT
GGCCCGATCCTAGTCATGCGCTACCGCAACCTGATCGAGGGCGAAGCATCC
GCCGGTTCCTAATGTACGGAGCAGATGCTAGGGCAAATTGCCCTAGCAGGG
GAAAAAGGTCGAAAAGGTCTCTTTCCTGTGGATAGCACGTACATTGGGAACC
 
CAAAGCCGTACATTGGGAACCGGAACCCGTACATTGGGAACCCAAAGCCGTA
CATTGGGAACCGGTCACACATGTAAGTGACTGATATAAAAGAGAAAAAAGGC
GATTTTTCCGCCTAAAACTCTTTAAAACTTATTAAAACTCTTAAAACCCGCCTG
GCCTGTGCATAACTGTCTGGCCAGCGCACAGCCGAAGAGCTGCAAAAAGCG
CCTACCCTTCGGTCGCTGCGCTCCCTACGCCCCGCCGCTTCGCGTCGGCCT
ATCGCGGCCGCTGGCCGCTCAAAAATGGCTGGCCTACGGCCAGGCAATCTA
CCAGGGCGCGGACAAGCCGCGCCGTCGCCACTCGACCGCCGGCGCCCACA
TCAAGGCACCCTGCCTCGCGCGTTTCGGTGATGACGGTGAAAACCTCTGACA
CATGCAGCTCCCGGAGACGGTCACAGCTTGTCTGTAAGCGGATGCCGGGAG
CAGACAAGCCCGTCAGGGCGCGTCAGCGGGTGTTGGCGGGTGTCGGGGCG
CAGCCATGACCCAGTCACGTAGCGATAGCGGAGTGTATACTGGCTTAACTAT
GCGGCATCAGAGCAGATTGTACTGAGAGTGCACCATATGCGGTGTGAAATAC
CGCACAGATGCGTAAGGAGAAAATACCGCATCAGGCGCTCTTCCGCTTCCTC
GCTCACTGACTCGCTGCGCTCGGTCGTTCGGCTGCGGCGAGCGGTATCAGC
TCACTCAAAGGCGGTAATACGGTTATCCACAGAATCAGGGGATAACGCAGGA
AAGAACATGTGAGCAAAAGGCCAGCAAA
Bovine Collagen alpha-1 (1) Expression vector 10
   SEQ ID NO: 21: 
AGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCC
CTTCGGGAAGCGTGGCGCTTTCTCATAGCTCACGCTGTAGGTATCTCAGTTC
GGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGG
CTACACTAGAAGGACAGTATTTGGTATCTGCGCTCTGCTGAAGCCAGTTACCT
TCGGAAAAAGAGTTGGTAGCTCTTGATCCGGCAAACAAACCACCGCTGGTAG
CGGTGGTTTTTTTGTTTGCAAGCAGCAGATTACGCGCAGAAAAAAAGGATCTC
AAGAAGATCCTTTGATCTTTTCTACGGGGTCTGACGCTCAGTGGAACGAAAAC
TCACGTTAAGGGATTTTGGTCATGCATTCTAGGTACTAAAACAATTCATCCAG
TAAAATATAATATTTTATTTTCTCCCAATCAGGCTTGATCCCCAGTAAGTCAAA
AAATAGCTCGACATACTGTTCTTCCCCGATATCCTCCCTGATCGACCGGACG
CAGAAGGCAATGTCATACCACTTGTCCGCCCTGCCGCTTCTCCCAAGATCAA
TAAAGCCACTTACTTTGCCATCTTTCACAAAGATGTTGCTGTCTCCCAGGTCG
CCGTGGGAAAAGACAAGTTCCTCTTCGGGCTTTTCCGTCTTTAAAAAATCATA
 
CAGCTCGCGCGGATCTTTAAATGGAGTGTCTTCTTCCCAGTTTTCGCAATCCA
CATCGGCCAGATCGTTATTCAGTAAGTAATCCAATTCGGCTAAGCGGCTGTCT
AAGCTATTCGTATAGGGACAATCCGATATGTCGATGGAGTGAAAGAGCCTGA
TGCACTCCGCATACAGCTCGATAATCTTTTCAGGGCTTTGTTCATCTTCATACT
CTTCCGAGCAAAGGACGCCATCGGCCTCACTCATGAGCAGATTGCTCCAGCC
ATCATGCCGTTCAAAGTGCAGGACCTTTGGAACAGGCAGCTTTCCTTCCAGC
CATAGCATCATGTCCTTTTCCCGTTCCACATCATAGGTGGTCCCTTTATACCG
GCTGTCCGTCATTTTTAAATATAGGTTTTCATTTTCTCCCACCAGCTTATATAC
CTTAGCAGGAGACATTCCTTCCGTATCTTTTACGCAGCGGTATTTTTCGATCA
GTTTTTTCAATTCCGGTGATATTCTCATTTTAGCCATTTATTATTTCCTTCCTCT
TTTCTACAGTATTTAAAGATACCCCAAGAAGCTAATTATAACAAGACGAACTCC
AATTCACTGTTCCTTGCATTCTAAAACCTTAAATACCAGAAAACAGCTTTTTCA
AAGTTGTTTTCAAAGTTGGCGTATAACATAGTATCGACGGAGCCGATTTTGAA
ACCGCGGTGATCACAGGCAGCAACGCTCTGTCATCGTTACAATCAACATGCT
ACCCTCCGCGAGATCATCCGTGTTTCAAACCCGGCAGCTTAGTTGCCGTTCT
TCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTT
GTGCCGAGCTGCCGGTCGGGGAGCTGTTGGCTGGCTGGTGGCAGGATATAT
TGTGGTGTAAACAAATTGACGCTTAGACAACTTAATAACACATTGCGGACGTT
TTTAATGTACTGAATTAACGCCGAATTGAATTCGAGCTCGGTGGAGGATCTGG
ATTTTAGTACTGGATTTTGGTTTTAGGAATTAGAAATTTTATTGATAGAAGTATT
TTACAAATACAAATACATACTAAGGGTTTCTTATATGCTCAACACATGAGCGAA
ACCCTATAGGAACCCTAATTCCCTTATCTGGGAACTACTCACACATTATTATG
GAGAAACTATTCACTCTCCTTCTCAAACGTTTCTGTTCCCTCTGGACAGCCCC
GACTGACGTTGGACTCAACCTCAATGGGCGCTAATTGTAATAGCTCCTGTTTG
CTTCTTATGGCCATTTCGCAAAGGTGTTGCAGTGTTCTGCTACCACCACTCAC
TGTAATTGACACACCTCTGAAACGGATGGAGTAAGTGCAACCAATTTGATCAA
ATCCAAAGAAACGACTAGCAGCGTAATTTACTGAGTCACCAGTCCAGGAACC
TAGAACTCGATGGAGAGAGGCCTCTGTCCGATCATAGCGTAGGTAGCGTTTG
AAAACTACCTTGCCAAATCCCCAGGACTCTTTAAATCCCAGTGGGTTGTCCTG
GTTGGAGTTTGTTTCGTCATTGTGTAAACGCCATTCTGTCCAAGATGGGCTCT
CATCGGGTAACTTAAATGGGCTGGTAGTGCCGCCAGAACCACCGTCCCATCT
CTCTGAATTGGCCTGCTCTCTTGCATCATTACCCTGGATTGCTCTCTCCATTG
TCCTCTCCAAATGAAATGAACTTCCTTATATAGAGGAAGGGTCTTGCGAAGGA
TAGTGGGATTGTGCGTCATCCCTTACGTCAGTGGAGATGTCACATCAATCCA
CTTGCTTTGTAGACGTGGTTGGAACCTCTTCTTTTTCCACGATGCTCCTCGTG
GGTGGGGGTCCATCTTTGGGACCACTGTCGGCAGAGAGATCTTGAATGATAG
CCTTTCCTTTATCGCAATGATGGCATTTGTAGGAGCCACCTTCCTTTTCTACT
 
GTCCTTTCGATGAAGTGACAGATAGCTGGGCAATGGAATCCGAGGAGGTTTC
CCGAAATTATCCTTTGTTGAAAAGTCTCAAACAATAGGCAACCGTGGACTTCT
TCACTAGCTCATCGAGATAGCAATGCCACTAGCTAATTTCTTACGTTGTATCTT
ATTTGTTTTACTTTGGGGCATGACATGGTTAAACCCCATCAAAGAGAAAGTGT
TTCATCCACTAGTCAATATACGAATTCATTCGAATTTATCCCTGTAAATCCTAG
TTCTTAGGATGAACTGGTGTAATAAACAGCAAAAAAAAATAAAAAAAAATAAAT
CTGGAATCATTCGACCACCTCAATAAACTAAAGCTACCAATTACCACAATATA
GTCATCCATATCCACTTAGATATAAAAGATAAAAGTAAACAAATATTAAATTTC
ATATGCACGCATAGGAAACTCATGATCTTATCTTTTAAATAGACATCTAGTTTT
CTTAGGTTATAAATAGACATTTTGTCCTAGAACTTCTTCACTACTAAAACCTAG
CCTCAAAAAGCAGGCTGCCACCATGGTGAGCAGTGGTGAGGACATCTTTTCA
GGCCTAGTCCCGATACTTATTGAACTTGAAGGAGACGTCAATGGGCACAGAT
TCTCAGTACGAGGCGAGGGATACGGGGATGCTTCCAATGGAAAGCTTGAGAT
AAAATTTATATGTACAACTGGAAGGCTTCCTGTCCCATGGCCAACTTTAGTTA
CCACTCTTAGCTACGGTGTACAATGCTTCGCTAAATACCCAGAACATATGCGT
CAGAATGACTTCTTCAAGTCAGCAATGCCTGATGGATATGTTCAAGAAAGAAC
AATCTCTTTTAAAGAGGACGGAACTTATAAGACCAGAGCTGAAGTCAAATTCG
AGGGAGAGGCCTTGGTGAACAGAATTGACTTAAAAGGACTGGAATTCAAGGA
AGATGGCAATATCCTCGGTCACAAGCTTGAATACAGTTTTAATTCACACTACG
TGTATATAACCGCGGATAAGAACAGGAATGGTTTAGAAGCTCAGTTTAGAATA
AGACATAACGTGGACGATGGATCCGTGCAGCTAGCAGATCACTATCAGCAAA
ATACGCCGATAGGGGAAGGCCCTGTTCTTTTGCCAGAGCAGCATTACCTAAC
TACTAACTCTGTTTTGTCTAAGGACCCACAAGAGCGGAGAGATCATATGGTTT
TGGTTGAGTTCGTTACGGCAGCTGGACTCTCCCTAGGCATGGATGAATTATA
CAAATCAGGATCTGAGAATTTATATTTTCAAGGTCAATTGAGTTATGGATACGA
CGAAAAATCAACTGGAATATCAGTGCCAGGACCAATGGGTCCATCAGGACCT
AGGGGTCTTCCAGGGCCACCTGGCGCTCCTGGTCCTCAGGGATTTCAAGGT
CCTCCAGGTGAACCTGGAGAGCCTGGTGCTTCTGGTCCAATGGGACCAAGA
GGCCCTCCTGGTCCACCTGGTAAAAATGGTGATGATGGTGAGGCTGGAAAG
CCTGGTCGTCCTGGAGAAAGAGGACCACCAGGACCACAGGGAGCTAGAGGT
TTGCCAGGTACAGCCGGTCTTCCAGGAATGAAAGGACACAGGGGTTTTTCTG
GTCTTGATGGTGCTAAGGGAGACGCAGGACCTGCTGGACCAAAGGGTGAAC
CTGGATCTCCTGGTGAAAATGGTGCTCCTGGACAAATGGGACCTAGAGGATT
GCCTGGCGAAAGAGGAAGACCTGGTGCTCCTGGACCAGCTGGAGCTAGAGG
AAATGATGGAGCTACTGGTGCTGCCGGACCACCAGGACCAACTGGACCAGC
TGGACCACCTGGTTTTCCAGGAGCAGTTGGGGCTAAGGGAGAAGGTGGACC
ACAAGGACCTAGAGGATCAGAAGGACCTCAAGGAGTTAGGGGAGAACCAGG
TCCTCCTGGTCCTGCTGGAGCTGCTGGACCCGCTGGTAACCCAGGAGCAGA
 
TGGACAACCTGGAGCAAAAGGGGCTAATGGTGCTCCTGGTATTGCAGGTGCT
CCTGGATTCCCTGGTGCTAGAGGACCTTCAGGACCTCAGGGACCATCCGGT
CCTCCAGGACCAAAGGGAAACTCTGGTGAACCAGGGGCTCCTGGATCTAAG
GGAGATACTGGAGCTAAGGGTGAGCCAGGACCTACAGGAATTCAAGGACCA
CCAGGACCTGCAGGAGAGGAAGGTAAGAGGGGTGCTAGAGGTGAACCTGGA
CCAGCTGGATTGCCAGGCCCACCTGGAGAGAGGGGTGGTCCAGGTTCTAGA
GGTTTTCCAGGAGCTGATGGAGTTGCTGGTCCAAAGGGTCCTGCTGGTGAGA
GAGGTGCACCTGGACCAGCAGGACCTAAAGGATCACCTGGAGAAGCTGGTC
GTCCTGGTGAGGCTGGTCTTCCAGGTGCTAAGGGCCTTACAGGATCTCCAG
GATCACCAGGTCCAGATGGAAAGACTGGACCACCAGGTCCTGCAGGACAAG
ATGGAAGGCCTGGTCCACCTGGACCACCTGGTGCTAGGGGACAAGCTGGTG
TTATGGGATTCCCAGGTCCAAAGGGTGCAGCTGGTGAGCCTGGTAAAGCTG
GTGAAAGAGGAGTTCCAGGACCTCCTGGTGCTGTTGGACCTGCTGGTAAAGA
TGGTGAAGCAGGTGCTCAGGGTCCACCAGGTCCAGCCGGTCCAGCTGGTGA
GAGGGGAGAGCAAGGTCCTGCTGGATCTCCTGGATTTCAAGGTTTGCCTGGA
CCTGCTGGACCTCCAGGAGAGGCTGGTAAACCTGGTGAGCAGGGTGTGCCT
GGTGATCTTGGTGCTCCTGGACCTAGTGGCGCAAGAGGCGAAAGGGGATTT
CCAGGTGAAAGAGGAGTTCAGGGACCACCAGGTCCAGCTGGTCCAAGAGGC
GCTAATGGAGCTCCAGGAAATGATGGAGCAAAGGGTGATGCTGGCGCTCCA
GGAGCTCCTGGTTCTCAAGGTGCACCTGGATTGCAAGGAATGCCAGGAGAG
AGAGGTGCAGCTGGTTTGCCGGGACCAAAGGGTGATAGAGGTGATGCTGGT
CCTAAGGGAGCTGATGGAGCTCCAGGAAAGGATGGTGTGAGAGGATTGACT
GGTCCAATTGGACCACCAGGTCCTGCTGGAGCACCAGGAGATAAAGGAGAA
GCTGGTCCTTCAGGACCAGCCGGTCCAACTGGAGCTAGAGGAGCTCCAGGA
GATAGAGGTGAACCAGGACCACCAGGACCTGCTGGGTTTGCAGGTCCACCT
GGTGCTGATGGACAACCTGGTGCTAAAGGAGAACCAGGCGATGCTGGTGCT
AAGGGAGATGCTGGCCCACCTGGTCCAGCTGGTCCTGCTGGGCCACCAGGT
CCTATCGGAAATGTTGGTGCTCCAGGGCCTAAAGGAGCTAGAGGATCAGCA
GGTCCCCCAGGTGCTACAGGTTTTCCAGGGGCTGCTGGAAGAGTGGGTCCC
CCAGGTCCTTCTGGGAATGCAGGACCACCAGGTCCTCCAGGACCAGCTGGA
AAAGAAGGAAGTAAGGGACCAAGGGGAGAAACTGGACCTGCAGGACGTCCA
GGTGAAGTTGGTCCACCTGGACCTCCAGGTCCAGCTGGAGAGAAAGGAGCT
CCAGGAGCTGATGGTCCAGCAGGTGCTCCTGGTACTCCAGGTCCACAAGGT
ATTGCTGGTCAAAGGGGAGTGGTTGGTCTCCCAGGTCAGAGAGGTGAAAGA
GGTTTCCCAGGTTTGCCTGGACCATCTGGAGAACCAGGTAAGCAGGGCCCAT
CTGGAGCTTCTGGTGAGAGAGGGCCACCTGGACCTATGGGACCTCCTGGGC
TTGCTGGACCTCCAGGGGAATCTGGTAGGGAGGGTGCTCCAGGTGCTGAAG
GATCACCTGGTAGAGATGGATCACCTGGAGCTAAAGGTGATAGAGGGGAAA
 
CAGGTCCAGCAGGTCCACCTGGCGCTCCTGGTGCTCCAGGAGCACCAGGGC
CAGTTGGACCTGCTGGAAAGTCTGGAGATAGGGGAGAGACTGGACCAGCTG
GACCTGCTGGACCAATCGGTCCTGTTGGCGCTAGAGGTCCTGCTGGACCAC
AAGGTCCTAGGGGAGATAAAGGTGAAACAGGTGAACAAGGAGATAGAGGAA
TTAAAGGTCATAGAGGGTTTTCTGGTTTGCAAGGACCTCCAGGACCTCCTGG
ATCTCCAGGAGAGCAAGGACCATCTGGTGCAAGCGGACCTGCTGGTCCTAG
AGGACCTCCAGGTTCAGCTGGCTCACCTGGTAAGGATGGATTAAATGGTTTG
CCTGGACCAATAGGTCCACCAGGACCTAGAGGAAGGACAGGTGATGCAGGA
CCAGCTGGTCCACCTGGTCCACCAGGACCACCAGGACCACCAGGTCCACCT
TCTGGTGGATATGATCTTAGTTTTCTTCCTCAACCTCCCCAGGAGAAAGCTCA
TGATGGTGGTAGATATTATAGGGCTTAAACCCAGCTTTCTTGTACAAAGTGGT
AGCTCGAATTTCCCCGATCGTTCAAACATTTGGCAATAAAGTTTCTTAAGATTG
AATCCTGTTGCCGGTCTTGCGATGATTATCATATAATTTCTGTTGAATTACGTT
AAGCATGTAATAATTAACATGTAATGCATGACGTTATTTATGAGATGGGTTTTT
ATGATTAGAGTCCCGCAATTATACATTTAATACGCGATAGAAAACAAAATATAG
CGCGCAAACTAGGATAAATTATCGCGCGCGGTGTCATCTATGTTACTAGATC
GGGAAAGCTTTTAAACTATCAGTGTTTGACAGGATATATTGGCGGGTAAACCT
AAGAGAAAAGAGCGTTTATTAGAATAATCGGATATTTAAAAGGGCGTGAAAAG
GTTTATCCGTTCGTCCATTTGTATGTGCATGCCAACCACAGGGTTCCCCTCGG
GATCAAAGTACTTTGATCCAACCCCTCCGCTGCTATAGTGCAGTCGGCTTCTG
ACGTTCAGTGCAGCCGTCTTCTGAAAACGACATGTCGCACAAGTCCTAAGTTA
CGCGACAGGCTGCCGCCCTGCCCTTTTCCTGGCGTTTTCTTGTCGCGTGTTT
TAGTCGCATAAAGTAGAATACTTGCGACTAGAACCGGAGACATTACGCCATG
AACAAGAGCGCCGCCGCTGGCCTGCTGGGCTATGCCCGCGTCAGCACCGAC
GACCAGGACTTGACCAACCAACGGGCCGAACTGCACGCGGCCGGCTGCACC
AAGCTGTTTTCCGAGAAGATCACCGGCACCAGGCGCGACCGCCCGGAGCTG
GCCAGGATGCTTGACCACCTACGCCCTGGCGACGTTGTGACAGTGACCAGG
CTAGACCGCCTGGCCCGCAGCACCCGCGACCTACTGGACATTGCCGAGCGC
ATCCAGGAGGCCGGCGCGGGCCTGCGTAGCCTGGCAGAGCCGTGGGCCGA
CACCACCACGCCGGCCGGCCGCATGGTGTTGACCGTGTTCGCCGGCATTGC
CGAGTTCGAGCGTTCCCTAATCATCGACCGCACCCGGAGCGGGCGCGAGGC
CGCCAAGGCCCGAGGCGTGAAGTTTGGCCCCCGCCCTACCCTCACCCCGGC
ACAGATCGCGCACGCCCGCGAGCTGATCGACCAGGAAGGCCGCACCGTGAA
AGAGGCGGCTGCACTGCTTGGCGTGCATCGCTCGACCCTGTACCGCGCACT
TGAGCGCAGCGAGGAAGTGACGCCCACCGAGGCCAGGCGGCGCGGTGCCT
TCCGTGAGGACGCATTGACCGAGGCCGACGCCCTGGCGGCCGCCGAGAAT
GAACGCCAAGAGGAACAAGCATGAAACCGCACCAGGACGGCCAGGACGAAC
CGTTTTTCATTACCGAAGAGATCGAGGCGGAGATGATCGCGGCCGGGTACGT
 
GTTCGAGCCGCCCGCGCACGTCTCAACCGTGCGGCTGCATGAAATCCTGGC
CGGTTTGTCTGATGCCAAGCTGGCGGCCTGGCCGGCCAGCTTGGCCGCTGA
AGAAACCGAGCGCCGCCGTCTAAAAAGGTGATGTGTATTTGAGTAAAACAGC
TTGCGTCATGCGGTCGCTGCGTATATGATGCGATGAGTAAATAAACAAATAC
GCAAGGGGAACGCATGAAGGTTATCGCTGTACTTAACCAGAAAGGCGGGTC
AGGCAAGACGACCATCGCAACCCATCTAGCCCGCGCCCTGCAACTCGCCGG
GGCCGATGTTCTGTTAGTCGATTCCGATCCCCAGGGCAGTGCCCGCGATTG
GGCGGCCGTGCGGGAAGATCAACCGCTAACCGTTGTCGGCATCGACCGCCC
GACGATTGACCGCGACGTGAAGGCCATCGGCCGGCGCGACTTCGTAGTGAT
CGACGGAGCGCCCCAGGCGGCGGACTTGGCTGTGTCCGCGATCAAGGCAG
CCGACTTCGTGCTGATTCCGGTGCAGCCAAGCCCTTACGACATATGGGCCAC
CGCCGACCTGGTGGAGCTGGTTAAGCAGCGCATTGAGGTCACGGATGGAAG
GCTACAAGCGGCCTTTGTCGTGTCGCGGGCGATCAAAGGCACGCGCATCGG
CGGTGAGGTTGCCGAGGCGCTGGCCGGGTACGAGCTGCCCATTCTTGAGTC
CCGTATCACGCAGCGCGTGAGCTACCCAGGCACTGCCGCCGCCGGCACAAC
CGTTCTTGAATCAGAACCCGAGGGCGACGCTGCCCGCGAGGTCCAGGCGCT
GGCCGCTGAAATTAAATCAAAACTCATTTGAGTTAATGAGGTAAAGAGAAAAT
GAGCAAAAGCACAAACACGCTAAGTGCCGGCCGTCCGAGCGCACGCAGCAG
CAAGGCTGCAACGTTGGCCAGCCTGGCAGACACGCCAGCCATGAAGCGGGT
CAACTTTCAGTTGCCGGCGGAGGATCACACCAAGCTGAAGATGTACGCGGTA
CGCCAAGGCAAGACCATTACCGAGCTGCTATCTGAATACATCGCGCAGCTAC
CAGAGTAAATGAGCAAATGAATAAATGAGTAGATGAATTTTAGCGGCTAAAGG
AGGCGGCATGGAAAATCAAGAACAACCAGGCACCGACGCCGTGGAATGCCC
CATGTGTGGAGGAACGGGCGGTTGGCCAGGCGTAAGCGGCTGGGTTGCCT
GCCGGCCCTGCAATGGCACTGGAACCCCCAAGCCCGAGGAATCGGCGTGA
GCGGTCGCAAACCATCCGGCCCGGTACAAATCGGCGCGGCGCTGGGTGATG
ACCTGGTGGAGAAGTTGAAGGCCGCGCAGGCCGCCCAGCGGCAACGCATC
GAGGCAGAAGCACGCCCCGGTGAATCGTGGCAAGCGGCCGCTGATCGAATC
CGCAAAGAATCCCGGCAACCGCCGGCAGCCGGTGCGCCGTCGATTAGGAAG
CCGCCCAAGGGCGACGAGCAACCAGATTTTTTCGTTCCGATGCTCTATGACG
TGGGCACCCGCGATAGTCGCAGCATCATGGACGTGGCCGTTTTCCGTCTGTC
GAAGCGTGACCGACGAGCTGGCGAGGTGATCCGCTACGAGCTTCCAGACGG
GCACGTAGAGGTTTCCGCAGGGCCGGCCGGCATGGCCAGTGTGTGGGATTA
CGACCTGGTACTGATGGCGGTTTCCCATCTAACCGAATCCATGAACCGATAC
CGGGAAGGGAAGGGAGACAAGCCCGGCCGCGTGTTCCGTCCACACGTTGC
GGACGTACTCAAGTTCTGCCGGCGAGCCGATGGCGGAAAGCAGAAAGACGA
CCTGGTAGAAACCTGCATTCGGTTAAACACCACGCACGTTGCCATGCAGCGT
ACGAAGAAGGCCAAGAACGGCCGCCTGGTGACGGTATCCGAGGGTGAAGCC
 
TTGATTAGCCGCTACAAGATCGTAAAGAGCGAAACCGGGCGGCCGGAGTAC
ATCGAGATCGAGCTAGCTGATTGGATGTACCGCGAGATCACAGAAGGCAAGA
ACCCGGACGTGCTGACGGTTCACCCCGATTACTTTTTGATCGATCCCGGCAT
CGGCCGTTTTCTCTACCGCCTGGCACGCCGCGCCGCAGGCAAGGCAGAAGC
CAGATGGTTGTTCAAGACGATCTACGAACGCAGTGGCAGCGCCGGAGAGTT
CAAGAAGTTCTGTTTCACCGTGCGCAAGCTGATCGGGTCAAATGACCTGCCG
GAGTACGATTTGAAGGAGGAGGCGGGGCAGGCTGGCCCGATCCTAGTCATG
CGCTACCGCAACCTGATCGAGGGCGAAGCATCCGCCGGTTCCTAATGTACG
GAGCAGATGCTAGGGCAAATTGCCCTAGCAGGGGAAAAAGGTCGAAAAGGT
CTCTTTCCTGTGGATAGCACGTACATTGGGAACCCAAAGCCGTACATTGGGA
ACCGGAACCCGTACATTGGGAACCCAAAGCCGTACATTGGGAACCGGTCAC
ACATGTAAGTGACTGATATAAAAGAGAAAAAAGGCGATTTTTCCGCCTAAAAC
TCTTTAAAACTTATTAAAACTCTTAAAACCCGCCTGGCCTGTGCATAACTGTCT
GGCCAGCGCACAGCCGAAGAGCTGCAAAAAGCGCCTACCCTTCGGTCGCTG
CGCTCCCTACGCCCCGCCGCTTCGCGTCGGCCTATCGCGGCCGCTGGCCG
CTCAAAAATGGCTGGCCTACGGCCAGGCAATCTACCAGGGCGCGGACAAGC
CGCGCCGTCGCCACTCGACCGCCGGCGCCCACATCAAGGCACCCTGCCTCG
CGCGTTTCGGTGATGACGGTGAAAACCTCTGACACATGCAGCTCCCGGAGAC
GGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCAC
GTAGCGATAGCGGAGTGTATACTGGCTTAACTATGCGGCATCAGAGCAGATT
GTACTGAGAGTGCACCATATGCGGTGTGAAATACCGCACAGATGCGTAAGGA
GAAAATACCGCATCAGGCGCTCTTCCGCTTCCTCGCTCACTGACTCGCTGCG
CTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAA
GGCCAGCAAA
Bovine Collagen alpha-1 (1) Expression vector 11
   SEQ ID NO: 25: 
AGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCC
CTTCGGGAAGCGTGGCGCTTTCTCATAGCTCACGCTGTAGGTATCTCAGTTC
GGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGG
 
CTACACTAGAAGGACAGTATTTGGTATCTGCGCTCTGCTGAAGCCAGTTACCT
TCGGAAAAAGAGTTGGTAGCTCTTGATCCGGCAAACAAACCACCGCTGGTAG
CGGTGGTTTTTTTGTTTGCAAGCAGCAGATTACGCGCAGAAAAAAAGGATCTC
AAGAAGATCCTTTGATCTTTTCTACGGGGTCTGACGCTCAGTGGAACGAAAAC
TCACGTTAAGGGATTTTGGTCATGCATTCTAGGTACTAAAACAATTCATCCAG
TAAAATATAATATTTTATTTTCTCCCAATCAGGCTTGATCCCCAGTAAGTCAAA
AAATAGCTCGACATACTGTTCTTCCCCGATATCCTCCCTGATCGACCGGACG
CAGAAGGCAATGTCATACCACTTGTCCGCCCTGCCGCTTCTCCCAAGATCAA
TAAAGCCACTTACTTTGCCATCTTTCACAAAGATGTTGCTGTCTCCCAGGTCG
CCGTGGGAAAAGACAAGTTCCTCTTCGGGCTTTTCCGTCTTTAAAAAATCATA
CAGCTCGCGCGGATCTTTAAATGGAGTGTCTTCTTCCCAGTTTTCGCAATCCA
CATCGGCCAGATCGTTATTCAGTAAGTAATCCAATTCGGCTAAGCGGCTGTCT
AAGCTATTCGTATAGGGACAATCCGATATGTCGATGGAGTGAAAGAGCCTGA
TGCACTCCGCATACAGCTCGATAATCTTTTCAGGGCTTTGTTCATCTTCATACT
CTTCCGAGCAAAGGACGCCATCGGCCTCACTCATGAGCAGATTGCTCCAGCC
ATCATGCCGTTCAAAGTGCAGGACCTTTGGAACAGGCAGCTTTCCTTCCAGC
CATAGCATCATGTCCTTTTCCCGTTCCACATCATAGGTGGTCCCTTTATACCG
GCTGTCCGTCATTTTTAAATATAGGTTTTCATTTTCTCCCACCAGCTTATATAC
CTTAGCAGGAGACATTCCTTCCGTATCTTTTACGCAGCGGTATTTTTCGATCA
GTTTTTTCAATTCCGGTGATATTCTCATTTTAGCCATTTATTATTTCCTTCCTCT
TTTCTACAGTATTTAAAGATACCCCAAGAAGCTAATTATAACAAGACGAACTCC
AATTCACTGTTCCTTGCATTCTAAAACCTTAAATACCAGAAAACAGCTTTTTCA
AAGTTGTTTTCAAAGTTGGCGTATAACATAGTATCGACGGAGCCGATTTTGAA
ACCGCGGTGATCACAGGCAGCAACGCTCTGTCATCGTTACAATCAACATGCT
ACCCTCCGCGAGATCATCCGTGTTTCAAACCCGGCAGCTTAGTTGCCGTTCT
TCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTT
GTGCCGAGCTGCCGGTCGGGGAGCTGTTGGCTGGCTGGTGGCAGGATATAT
TGTGGTGTAAACAAATTGACGCTTAGACAACTTAATAACACATTGCGGACGTT
TTTAATGTACTGAATTAACGCCGAATTGAATTCGAGCTCGGTGGAGGATCTGG
ATTTTAGTACTGGATTTTGGTTTTAGGAATTAGAAATTTTATTGATAGAAGTATT
TTACAAATACAAATACATACTAAGGGTTTCTTATATGCTCAACACATGAGCGAA
ACCCTATAGGAACCCTAATTCCCTTATCTGGGAACTACTCACACATTATTATG
GAGAAACTATTCACTCTCCTTCTCAAACGTTTCTGTTCCCTCTGGACAGCCCC
GACTGACGTTGGACTCAACCTCAATGGGCGCTAATTGTAATAGCTCCTGTTTG
CTTCTTATGGCCATTTCGCAAAGGTGTTGCAGTGTTCTGCTACCACCACTCAC
TGTAATTGACACACCTCTGAAACGGATGGAGTAAGTGCAACCAATTTGATCAA
ATCCAAAGAAACGACTAGCAGCGTAATTTACTGAGTCACCAGTCCAGGAACC
 
TAGAACTCGATGGAGAGAGGCCTCTGTCCGATCATAGCGTAGGTAGCGTTTG
AAAACTACCTTGCCAAATCCCCAGGACTCTTTAAATCCCAGTGGGTTGTCCTG
GTTGGAGTTTGTTTCGTCATTGTGTAAACGCCATTCTGTCCAAGATGGGCTCT
CATCGGGTAACTTAAATGGGCTGGTAGTGCCGCCAGAACCACCGTCCCATCT
CTCTGAATTGGCCTGCTCTCTTGCATCATTACCCTGGATTGCTCTCTCCATTG
TCCTCTCCAAATGAAATGAACTTCCTTATATAGAGGAAGGGTCTTGCGAAGGA
TAGTGGGATTGTGCGTCATCCCTTACGTCAGTGGAGATGTCACATCAATCCA
CTTGCTTTGTAGACGTGGTTGGAACCTCTTCTTTTTCCACGATGCTCCTCGTG
GGTGGGGGTCCATCTTTGGGACCACTGTCGGCAGAGAGATCTTGAATGATAG
CCTTTCCTTTATCGCAATGATGGCATTTGTAGGAGCCACCTTCCTTTTCTACT
GTCCTTTCGATGAAGTGACAGATAGCTGGGCAATGGAATCCGAGGAGGTTTC
CCGAAATTATCCTTTGTTGAAAAGTCTCAAACAATAGGCAACCGTGGACTTCT
TCACTAGCTCATCGAGATAGCAATGCCACTAGCTAATTTCTTACGTTGTATCTT
ATTTGTTTTACTTTGGGGCATGACATGGTTAAACCCCATCAAAGAGAAAGTGT
TTCATCCACTAGTCAATATACGAATTCATTCGAATTTATCCCTGTAAATCCTAG
TTCTTAGGATGAACTGGTGTAATAAACAGCAAAAAAAAATAAAAAAAAATAAAT
CTGGAATCATTCGACCACCTCAATAAACTAAAGCTACCAATTACCACAATATA
GTCATCCATATCCACTTAGATATAAAAGATAAAAGTAAACAAATATTAAATTTC
ATATGCACGCATAGGAAACTCATGATCTTATCTTTTAAATAGACATCTAGTTTT
CTTAGGTTATAAATAGACATTTTGTCCTAGAACTTCTTCACTACTAAAACCTAG
CCTCAAAAAGCAGGCTGCCACCATGGTGAGCAGTGGTGAGGACATCTTTTCA
GGCCTAGTCCCGATACTTATTGAACTTGAAGGAGACGTCAATGGGCACAGAT
TCTCAGTACGAGGCGAGGGATACGGGGATGCTTCCAATGGAAAGCTTGAGAT
AAAATTTATATGTACAACTGGAAGGCTTCCTGTCCCATGGCCAACTTTAGTTA
CCACTCTTAGCTACGGTGTACAATGCTTCGCTAAATACCCAGAACATATGCGT
CAGAATGACTTCTTCAAGTCAGCAATGCCTGATGGATATGTTCAAGAAAGAAC
AATCTCTTTTAAAGAGGACGGAACTTATAAGACCAGAGCTGAAGTCAAATTCG
AGGGAGAGGCCTTGGTGAACAGAATTGACTTAAAAGGACTGGAATTCAAGGA
AGATGGCAATATCCTCGGTCACAAGCTTGAATACAGTTTTAATTCACACTACG
TGTATATAACCGCGGATAAGAACAGGAATGGTTTAGAAGCTCAGTTTAGAATA
AGACATAACGTGGACGATGGATCCGTGCAGCTAGCAGATCACTATCAGCAAA
ATACGCCGATAGGGGAAGGCCCTGTTCTTTTGCCAGAGCAGCATTACCTAAC
TACTAACTCTGTTTTGTCTAAGGACCCACAAGAGCGGAGAGATCATATGGTTT
TGGTTGAGTTCGTTACGGCAGCTGGACTCTCCCTAGGCATGGATGAATTATA
CAAATCAGGATCTGAGAATTTATATTTTCAAGGTCAATTGAGTTATGGATACGA
CGAAAAATCAACTGGAATATCAGTGCCAGGACCAATGGGTCCATCAGGACCT
AGGGGTCTTCCAGGGCCACCTGGCGCTCCTGGTCCTCAGGGATTTCAAGGT
CCTCCAGGTGAACCTGGAGAGCCTGGTGCTTCTGGTCCAATGGGACCAAGA
 
GGCCCTCCTGGTCCACCTGGTAAAAATGGTGATGATGGTGAGGCTGGAAAG
CCTGGTCGTCCTGGAGAAAGAGGACCACCAGGACCACAGGGAGCTAGAGGT
TTGCCAGGTACAGCCGGTCTTCCAGGAATGAAAGGACACAGGGGTTTTTCTG
GTCTTGATGGTGCTAAGGGAGACGCAGGACCTGCTGGACCAAAGGGTGAAC
CTGGATCTCCTGGTGAAAATGGTGCTCCTGGACAAATGGGACCTAGAGGATT
GCCTGGCGAAAGAGGAAGACCTGGTGCTCCTGGACCAGCTGGAGCTAGAGG
AAATGATGGAGCTACTGGTGCTGCCGGACCACCAGGACCAACTGGACCAGC
TGGACCACCTGGTTTTCCAGGAGCAGTTGGGGCTAAGGGAGAAGGTGGACC
ACAAGGACCTAGAGGATCAGAAGGACCTCAAGGAGTTAGGGGAGAACCAGG
TCCTCCTGGTCCTGCTGGAGCTGCTGGACCCGCTGGTAACCCAGGAGCAGA
TGGACAACCTGGAGCAAAAGGGGCTAATGGTGCTCCTGGTATTGCAGGTGCT
CCTGGATTCCCTGGTGCTAGAGGACCTTCAGGACCTCAGGGACCATCCGGT
CCTCCAGGACCAAAGGGAAACTCTGGTGAACCAGGGGCTCCTGGATCTAAG
GGAGATACTGGAGCTAAGGGTGAGCCAGGACCTACAGGAATTCAAGGACCA
CCAGGACCTGCAGGAGAGGAAGGTAAGAGGGGTGCTAGAGGTGAACCTGGA
CCAGCTGGATTGCCAGGCCCACCTGGAGAGAGGGGTGGTCCAGGTTCTAGA
GGTTTTCCAGGAGCTGATGGAGTTGCTGGTCCAAAGGGTCCTGCTGGTGAGA
GAGGTGCACCTGGACCAGCAGGACCTAAAGGATCACCTGGAGAAGCTGGTC
GTCCTGGTGAGGCTGGTCTTCCAGGTGCTAAGGGCCTTACAGGATCTCCAG
GATCACCAGGTCCAGATGGAAAGACTGGACCACCAGGTCCTGCAGGACAAG
ATGGAAGGCCTGGTCCACCTGGACCACCTGGTGCTAGGGGACAAGCTGGTG
TTATGGGATTCCCAGGTCCAAAGGGTGCAGCTGGTGAGCCTGGTAAAGCTG
GTGAAAGAGGAGTTCCAGGACCTCCTGGTGCTGTTGGACCTGCTGGTAAAGA
TGGTGAAGCAGGTGCTCAGGGTCCACCAGGTCCAGCCGGTCCAGCTGGTGA
GAGGGGAGAGCAAGGTCCTGCTGGATCTCCTGGATTTCAAGGTTTGCCTGGA
CCTGCTGGACCTCCAGGAGAGGCTGGTAAACCTGGTGAGCAGGGTGTGCCT
GGTGATCTTGGTGCTCCTGGACCTAGTGGCGCAAGAGGCGAAAGGGGATTT
CCAGGTGAAAGAGGAGTTCAGGGACCACCAGGTCCAGCTGGTCCAAGAGGC
GCTAATGGAGCTCCAGGAAATGATGGAGCAAAGGGTGATGCTGGCGCTCCA
GGAGCTCCTGGTTCTCAAGGTGCACCTGGATTGCAAGGAATGCCAGGAGAG
AGAGGTGCAGCTGGTTTGCCGGGACCAAAGGGTGATAGAGGTGATGCTGGT
CCTAAGGGAGCTGATGGAGCTCCAGGAAAGGATGGTGTGAGAGGATTGACT
GGTCCAATTGGACCACCAGGTCCTGCTGGAGCACCAGGAGATAAAGGAGAA
GCTGGTCCTTCAGGACCAGCCGGTCCAACTGGAGCTAGAGGAGCTCCAGGA
GATAGAGGTGAACCAGGACCACCAGGACCTGCTGGGTTTGCAGGTCCACCT
GGTGCTGATGGACAACCTGGTGCTAAAGGAGAACCAGGCGATGCTGGTGCT
AAGGGAGATGCTGGCCCACCTGGTCCAGCTGGTCCTGCTGGGCCACCAGGT
CCTATCGGAAATGTTGGTGCTCCAGGGCCTAAAGGAGCTAGAGGATCAGCA
 
GGTCCCCCAGGTGCTACAGGTTTTCCAGGGGCTGCTGGAAGAGTGGGTCCC
CCAGGTCCTTCTGGGAATGCAGGACCACCAGGTCCTCCAGGACCAGCTGGA
AAAGAAGGAAGTAAGGGACCAAGGGGAGAAACTGGACCTGCAGGACGTCCA
GGTGAAGTTGGTCCACCTGGACCTCCAGGTCCAGCTGGAGAGAAAGGAGCT
CCAGGAGCTGATGGTCCAGCAGGTGCTCCTGGTACTCCAGGTCCACAAGGT
ATTGCTGGTCAAAGGGGAGTGGTTGGTCTCCCAGGTCAGAGAGGTGAAAGA
GGTTTCCCAGGTTTGCCTGGACCATCTGGAGAACCAGGTAAGCAGGGCCCAT
CTGGAGCTTCTGGTGAGAGAGGGCCACCTGGACCTATGGGACCTCCTGGGC
TTGCTGGACCTCCAGGGGAATCTGGTAGGGAGGGTGCTCCAGGTGCTGAAG
GATCACCTGGTAGAGATGGATCACCTGGAGCTAAAGGTGATAGAGGGGAAA
CAGGTCCAGCAGGTCCACCTGGCGCTCCTGGTGCTCCAGGAGCACCAGGGC
CAGTTGGACCTGCTGGAAAGTCTGGAGATAGGGGAGAGACTGGACCAGCTG
GACCTGCTGGACCAATCGGTCCTGTTGGCGCTAGAGGTCCTGCTGGACCAC
AAGGTCCTAGGGGAGATAAAGGTGAAACAGGTGAACAAGGAGATAGAGGAA
TTAAAGGTCATAGAGGGTTTTCTGGTTTGCAAGGACCTCCAGGACCTCCTGG
ATCTCCAGGAGAGCAAGGACCATCTGGTGCAAGCGGACCTGCTGGTCCTAG
AGGACCTCCAGGTTCAGCTGGCTCACCTGGTAAGGATGGATTAAATGGTTTG
CCTGGACCAATAGGTCCACCAGGACCTAGAGGAAGGACAGGTGATGCAGGA
CCAGCTGGTCCACCTGGTCCACCAGGACCACCAGGACCACCAGGTCCACCT
TCTGGTGGATATGATCTTAGTTTTCTTCCTCAACCTCCCCAGGAGAAAGCTCA
TGATGGTGGTAGATATTATAGGGCTAAGGATGAGCTTTAAACCCAGCTTTCTT
GTACAAAGTGGTAGCTCGAATTTCCCCGATCGTTCAAACATTTGGCAATAAAG
TTTCTTAAGATTGAATCCTGTTGCCGGTCTTGCGATGATTATCATATAATTTCT
GTTGAATTACGTTAAGCATGTAATAATTAACATGTAATGCATGACGTTATTTAT
GAGATGGGTTTTTATGATTAGAGTCCCGCAATTATACATTTAATACGCGATAG
AAAACAAAATATAGCGCGCAAACTAGGATAAATTATCGCGCGCGGTGTCATCT
ATGTTACTAGATCGGGAAAGCTTTTAAACTATCAGTGTTTGACAGGATATATTG
GCGGGTAAACCTAAGAGAAAAGAGCGTTTATTAGAATAATCGGATATTTAAAA
GGGCGTGAAAAGGTTTATCCGTTCGTCCATTTGTATGTGCATGCCAACCACA
GGGTTCCCCTCGGGATCAAAGTACTTTGATCCAACCCCTCCGCTGCTATAGT
GCAGTCGGCTTCTGACGTTCAGTGCAGCCGTCTTCTGAAAACGACATGTCGC
ACAAGTCCTAAGTTACGCGACAGGCTGCCGCCCTGCCCTTTTCCTGGCGTTT
TCTTGTCGCGTGTTTTAGTCGCATAAAGTAGAATACTTGCGACTAGAACCGGA
GACATTACGCCATGAACAAGAGCGCCGCCGCTGGCCTGCTGGGCTATGCCC
GCGTCAGCACCGACGACCAGGACTTGACCAACCAACGGGCCGAACTGCACG
CGGCCGGCTGCACCAAGCTGTTTTCCGAGAAGATCACCGGCACCAGGCGCG
ACCGCCCGGAGCTGGCCAGGATGCTTGACCACCTACGCCCTGGCGACGTTG
TGACAGTGACCAGGCTAGACCGCCTGGCCCGCAGCACCCGCGACCTACTGG
 
ACATTGCCGAGCGCATCCAGGAGGCCGGCGCGGGCCTGCGTAGCCTGGCA
GAGCCGTGGGCCGACACCACCACGCCGGCCGGCCGCATGGTGTTGACCGT
GTTCGCCGGCATTGCCGAGTTCGAGCGTTCCCTAATCATCGACCGCACCCG
GAGCGGGCGCGAGGCCGCCAAGGCCCGAGGCGTGAAGTTTGGCCCCCGCC
CTACCCTCACCCCGGCACAGATCGCGCACGCCCGCGAGCTGATCGACCAGG
AAGGCCGCACCGTGAAAGAGGCGGCTGCACTGCTTGGCGTGCATCGCTCGA
CCCTGTACCGCGCACTTGAGCGCAGCGAGGAAGTGACGCCCACCGAGGCCA
GGCGGCGCGGTGCCTTCCGTGAGGACGCATTGACCGAGGCCGACGCCCTG
GCGGCCGCCGAGAATGAACGCCAAGAGGAACAAGCATGAAACCGCACCAGG
ACGGCCAGGACGAACCGTTTTTCATTACCGAAGAGATCGAGGCGGAGATGAT
CGCGGCCGGGTACGTGTTCGAGCCGCCCGCGCACGTCTCAACCGTGCGGC
TGCATGAAATCCTGGCCGGTTTGTCTGATGCCAAGCTGGCGGCCTGGCCGG
CCAGCTTGGCCGCTGAAGAAACCGAGCGCCGCCGTCTAAAAAGGTGATGTG
TATTTGAGTAAAACAGCTTGCGTCATGCGGTCGCTGCGTATATGATGCGATGA
GTAAATAAACAAATACGCAAGGGGAACGCATGAAGGTTATCGCTGTACTTAAC
CAGAAAGGCGGGTCAGGCAAGACGACCATCGCAACCCATCTAGCCCGCGCC
CTGCAACTCGCCGGGGCCGATGTTCTGTTAGTCGATTCCGATCCCCAGGGCA
GTGCCCGCGATTGGGCGGCCGTGCGGGAAGATCAACCGCTAACCGTTGTCG
GCATCGACCGCCCGACGATTGACCGCGACGTGAAGGCCATCGGCCGGCGC
GACTTCGTAGTGATCGACGGAGCGCCCCAGGCGGCGGACTTGGCTGTGTCC
GCGATCAAGGCAGCCGACTTCGTGCTGATTCCGGTGCAGCCAAGCCCTTAC
GACATATGGGCCACCGCCGACCTGGTGGAGCTGGTTAAGCAGCGCATTGAG
GTCACGGATGGAAGGCTACAAGCGGCCTTTGTCGTGTCGCGGGCGATCAAA
GGCACGCGCATCGGCGGTGAGGTTGCCGAGGCGCTGGCCGGGTACGAGCT
GCCCATTCTTGAGTCCCGTATCACGCAGCGCGTGAGCTACCCAGGCACTGC
CGCCGCCGGCACAACCGTTCTTGAATCAGAACCCGAGGGCGACGCTGCCCG
CGAGGTCCAGGCGCTGGCCGCTGAAATTAAATCAAAACTCATTTGAGTTAAT
GAGGTAAAGAGAAAATGAGCAAAAGCACAAACACGCTAAGTGCCGGCCGTC
CGAGCGCACGCAGCAGCAAGGCTGCAACGTTGGCCAGCCTGGCAGACACG
CCAGCCATGAAGCGGGTCAACTTTCAGTTGCCGGCGGAGGATCACACCAAG
CTGAAGATGTACGCGGTACGCCAAGGCAAGACCATTACCGAGCTGCTATCTG
AATACATCGCGCAGCTACCAGAGTAAATGAGCAAATGAATAAATGAGTAGATG
AATTTTAGCGGCTAAAGGAGGCGGCATGGAAAATCAAGAACAACCAGGCACC
GACGCCGTGGAATGCCCCATGTGTGGAGGAACGGGCGGTTGGCCAGGCGT
AAGCGGCTGGGTTGCCTGCCGGCCCTGCAATGGCACTGGAACCCCCAAGCC
CGAGGAATCGGCGTGAGCGGTCGCAAACCATCCGGCCCGGTACAAATCGGC
GCGGCGCTGGGTGATGACCTGGTGGAGAAGTTGAAGGCCGCGCAGGCCGC
CCAGCGGCAACGCATCGAGGCAGAAGCACGCCCCGGTGAATCGTGGCAAG
 
CGGCCGCTGATCGAATCCGCAAAGAATCCCGGCAACCGCCGGCAGCCGGTG
CGCCGTCGATTAGGAAGCCGCCCAAGGGCGACGAGCAACCAGATTTTTTCGT
TCCGATGCTCTATGACGTGGGCACCCGCGATAGTCGCAGCATCATGGACGT
GGCCGTTTTCCGTCTGTCGAAGCGTGACCGACGAGCTGGCGAGGTGATCCG
CTACGAGCTTCCAGACGGGCACGTAGAGGTTTCCGCAGGGCCGGCCGGCAT
GGCCAGTGTGTGGGATTACGACCTGGTACTGATGGCGGTTTCCCATCTAACC
GAATCCATGAACCGATACCGGGAAGGGAAGGGAGACAAGCCCGGCCGCGT
GTTCCGTCCACACGTTGCGGACGTACTCAAGTTCTGCCGGCGAGCCGATGG
CGGAAAGCAGAAAGACGACCTGGTAGAAACCTGCATTCGGTTAAACACCACG
CACGTTGCCATGCAGCGTACGAAGAAGGCCAAGAACGGCCGCCTGGTGACG
GTATCCGAGGGTGAAGCCTTGATTAGCCGCTACAAGATCGTAAAGAGCGAAA
CCGGGCGGCCGGAGTACATCGAGATCGAGCTAGCTGATTGGATGTACCGCG
AGATCACAGAAGGCAAGAACCCGGACGTGCTGACGGTTCACCCCGATTACTT
TTTGATCGATCCCGGCATCGGCCGTTTTCTCTACCGCCTGGCACGCCGCGCC
GCAGGCAAGGCAGAAGCCAGATGGTTGTTCAAGACGATCTACGAACGCAGT
GGCAGCGCCGGAGAGTTCAAGAAGTTCTGTTTCACCGTGCGCAAGCTGATC
GGGTCAAATGACCTGCCGGAGTACGATTTGAAGGAGGAGGCGGGGCAGGCT
GGCCCGATCCTAGTCATGCGCTACCGCAACCTGATCGAGGGCGAAGCATCC
GCCGGTTCCTAATGTACGGAGCAGATGCTAGGGCAAATTGCCCTAGCAGGG
GAAAAAGGTCGAAAAGGTCTCTTTCCTGTGGATAGCACGTACATTGGGAACC
CAAAGCCGTACATTGGGAACCGGAACCCGTACATTGGGAACCCAAAGCCGTA
CATTGGGAACCGGTCACACATGTAAGTGACTGATATAAAAGAGAAAAAAGGC
GATTTTTCCGCCTAAAACTCTTTAAAACTTATTAAAACTCTTAAAACCCGCCTG
GCCTGTGCATAACTGTCTGGCCAGCGCACAGCCGAAGAGCTGCAAAAAGCG
CCTACCCTTCGGTCGCTGCGCTCCCTACGCCCCGCCGCTTCGCGTCGGCCT
ATCGCGGCCGCTGGCCGCTCAAAAATGGCTGGCCTACGGCCAGGCAATCTA
CCAGGGCGCGGACAAGCCGCGCCGTCGCCACTCGACCGCCGGCGCCCACA
TCAAGGCACCCTGCCTCGCGCGTTTCGGTGATGACGGTGAAAACCTCTGACA
CATGCAGCTCCCGGAGACGGTCACAGCTTGTCTGTAAGCGGATGCCGGGAG
CAGACAAGCCCGTCAGGGCGCGTCAGCGGGTGTTGGCGGGTGTCGGGGCG
CAGCCATGACCCAGTCACGTAGCGATAGCGGAGTGTATACTGGCTTAACTAT
GCGGCATCAGAGCAGATTGTACTGAGAGTGCACCATATGCGGTGTGAAATAC
CGCACAGATGCGTAAGGAGAAAATACCGCATCAGGCGCTCTTCCGCTTCCTC
GCTCACTGACTCGCTGCGCTCGGTCGTTCGGCTGCGGCGAGCGGTATCAGC
TCACTCAAAGGCGGTAATACGGTTATCCACAGAATCAGGGGATAACGCAGGA
AAGAACATGTGAGCAAAAGGCCAGCAAA
Prolyl 4-hydroxylase expression vector P4HA_1
   SEQ ID NO: 29: 
CAACTTTGTATAGAAAAGTTGGCCAACATGGTGGAGCACGACACTCTCGTCT
ACTCCAAGAATATCAAAGATACAGTCTCAGAAGACCAAAGGGCTATTGAGACT
TTTCAACAAAGGGTAATATCGGGAAACCTCCTCGGATTCCATTGCCCAGCTAT
CTGTCACTTCATCAAAAGGACAGTAGAAAAGGAAGGTGGCACCTACAAATGC
CATCATTGCGATAAAGGAAAGGCTATCGTTCAAGATGCCTCTGCCGACAGTG
GTCCCAAAGATGGACCCCCACCCACGAGGAGCATCGTGGAAAAAGAAGACG
TTCCAACCACGTCTTCAAAGCAAGTGGATTGATGTGAACATGGTGGAGCACG
ACACTCTCGTCTACTCCAAGAATATCAAAGATACAGTCTCAGAAGACCAAAGG
GCTATTGAGACTTTTCAACAAAGGGTAATATCGGGAAACCTCCTCGGATTCCA
TTGCCCAGCTATCTGTCACTTCATCAAAAGGACAGTAGAAAAGGAAGGTGGC
ACCTACAAATGCCATCATTGCGATAAAGGAAAGGCTATCGTTCAAGATGCCTC
TGCCGACAGTGGTCCCAAAGATGGACCCCCACCCACGAGGAGCATCGTGGA
AAAAGAAGACGTTCCAACCACGTCTTCAAAGCAAGTGGATTGATGTGATATCT
CCACTGACGTAAGGGATGACGCACAATCCCACTATCCTTCGCAAGACCCTTC
CTCTATATAAGGAAGTTCATTTCATTTGGAGAGGACACGCTGACAAGTTTGTA
CAAAAAAGCAGGCTGCCACCATGATTTGGTATATTTTGGTGGTTGGAATTTTA
TTGCCTCAATCTCTTGCACACCCCGGATTTTTTACCTCTATTGGACAAATGAC
GGATCTTATTCATACTGAAAAGGATTTGGTTACTAGCCTTAAAGATTACATTAA
AGCTGAGGAGGATAAACTTGAACAGATTAAGAAGTGGGCAGAAAAGTTGGAT
AGACTTACCTCTACTGCTACTAAGGATCCAGAGGGCTTCGTCGGACACCCTG
TTAACGCATTCAAGTTAATGAAGAGATTGAACACTGAATGGTCTGAACTTGAA
AATCTTGTGTTGAAGGACATGTCAGATGGTTTCATTTCTAATCTTACAATACAG
CGACAATATTTTCCAAACGATGAAGATCAAGTTGGCGCAGCTAAGGCACTTTT
GAGACTCCAAGATACTTATAACCTCGATACTGATACAATTTCTAAAGGTGATTT
GCCAGGTGTTAAGCATAAGAGTTTTTTGACTGTTGAAGATTGTTTCGAGCTTG
GTAAGGTTGCATATACTGAGGCTGATTACTATCATACTGAGTTGTGGATGGAA
CAAGCACTGAGGCAGCTAGATGAGGGTGAAGTTTCTACAGTTGACAAGGTTT
CTGTTCTTGATTACCTTTCTTATGCTGTTTATCAACAAGGAGATTTGGATAAAG
CTTTGCTTCTTACAAAAAAGCTTTTGGAACTTGATCCAGAACACCAGAGAGCT
AACGGTAATCTCAAATACTTTGAATATATTATGGCAAAAGAAAAAGATGCTAAC
AAGTCTTCATCTGATGATCAATCTGATCAAAAGACTACTCTTAAGAAGAAGGG
TGCTGCAGTTGATTATCTCCCTGAGAGACAGAAGTATGAAATGCTTTGTAGGG
GAGAAGGAATTAAAATGACACCTAGAAGACAGAAGAAACTTTTTTGTAGGTAT
CATGATGGTAACAGGAATCCAAAATTTATTCTCGCTCCTGCTAAACAAGAGGA
TGAGTGGGACAAGCCAAGGATCATTCGTTTTCACGACATTATATCTGACGCTG
AGATCGAAGTTGTGAAGGATTTAGCTAAGCCTAGGCTTTCAAGAGCTACAGTT
CATGATCCAGAAACTGGAAAATTGACTACTGCTCAATACAGAGTTTCAAAGTC
 
TGCTTGGCTTTCAGGATATGAGAACCCTGTTGTGTCTAGGATTAATATGAGAA
TCCAAGATTTGACTGGACTTGATGTTTCAACTGCAGAAGAATTGCAGGTTGCT
AACTATGGTGTGGGAGGACAATATGAGCCTCATTTCGATTTTGCTCGGAAGG
ATGAACCAGATGCTTTTAAAGAACTCGGGACTGGTAACAGAATTGCCACTTG
GCTTTTTTATATGTCAGATGTGCTTGCTGGTGGTGCTACTGTTTTTCCTGAAGT
GGGAGCTTCTGTTTGGCCAAAGAAAGGTACCGCCGTCTTTTGGTATAATCTTT
TTGCAAGTGGTGAAGGTGACTATTCAACAAGGCATGCTGCATGCCCTGTGTT
GGTTGGTAATAAGTGGGTGTCTAATAAATGGCTTCATGAGCGTGGCCAGGAA
TTTAGAAGACCATGCACTCTGTCAGAGCTTGAAGCTTTTGTTTATTAGACCCA
GCTTTCTTGTACAAAGTGGTAGCTCGAATTTCCCCGATCGTTCAAACATTTGG
CAATAAAGTTTCTTAAGATTGAATCCTGTTGCCGGTCTTGCGATGATTATCATA
TAATTTCTGTTGAATTACGTTAAGCATGTAATAATTAACATGTAATGCATGACG
TTATTTATGAGATGGGTTTTTATGATTAGAGTCCCGCAATTATACATTTAATAC
GCGATAGAAAACAAAATATAGCGCGCAAACTAGGATAAATTATCGCGCGCGG
TGTCATCTATGTTACTAGATCGGGAAAGCTTTTAAACTATCAGTGTTTGACAG
GATATATTGGCGGGTAAACCTAAGAGAAAAGAGCGTTTATTAGAATAATCGGA
TATTTAAAAGGGCGTGAAAAGGTTTATCCGTTCGTCCATTTGTATGTGCATGC
CAACCACAGGGTTCCCCTCGGGATCAAAGTACTTTGATCCAACCCCTCCGCT
GCTATAGTGCAGTCGGCTTCTGACGTTCAGTGCAGCCGTCTTCTGAAAACGA
CATGTCGCACAAGTCCTAAGTTACGCGACAGGCTGCCGCCCTGCCCTTTTCC
TGGCGTTTTCTTGTCGCGTGTTTTAGTCGCATAAAGTAGAATACTTGCGACTA
GAACCGGAGACATTACGCCATGAACAAGAGCGCCGCCGCTGGCCTGCTGGG
CTATGCCCGCGTCAGCACCGACGACCAGGACTTGACCAACCAACGGGCCGA
ACTGCACGCGGCCGGCTGCACCAAGCTGTTTTCCGAGAAGATCACCGGCAC
CAGGCGCGACCGCCCGGAGCTGGCCAGGATGCTTGACCACCTACGCCCTG
GCGACGTTGTGACAGTGACCAGGCTAGACCGCCTGGCCCGCAGCACCCGCG
ACCTACTGGACATTGCCGAGCGCATCCAGGAGGCCGGCGCGGGCCTGCGTA
GCCTGGCAGAGCCGTGGGCCGACACCACCACGCCGGCCGGCCGCATGGTG
TTGACCGTGTTCGCCGGCATTGCCGAGTTCGAGCGTTCCCTAATCATCGACC
GCACCCGGAGCGGGCGCGAGGCCGCCAAGGCCCGAGGCGTGAAGTTTGGC
CCCCGCCCTACCCTCACCCCGGCACAGATCGCGCACGCCCGCGAGCTGATC
GACCAGGAAGGCCGCACCGTGAAAGAGGCGGCTGCACTGCTTGGCGTGCAT
CGCTCGACCCTGTACCGCGCACTTGAGCGCAGCGAGGAAGTGACGCCCACC
GAGGCCAGGCGGCGCGGTGCCTTCCGTGAGGACGCATTGACCGAGGCCGA
CGCCCTGGCGGCCGCCGAGAATGAACGCCAAGAGGAACAAGCATGAAACCG
CACCAGGACGGCCAGGACGAACCGTTTTTCATTACCGAAGAGATCGAGGCG
GAGATGATCGCGGCCGGGTACGTGTTCGAGCCGCCCGCGCACGTCTCAACC
GTGCGGCTGCATGAAATCCTGGCCGGTTTGTCTGATGCCAAGCTGGCGGCC
 
TGGCCGGCCAGCTTGGCCGCTGAAGAAACCGAGCGCCGCCGTCTAAAAAGG
TGATGTGTATTTGAGTAAAACAGCTTGCGTCATGCGGTCGCTGCGTATATGAT
GCGATGAGTAAATAAACAAATACGCAAGGGGAACGCATGAAGGTTATCGCTG
TACTTAACCAGAAAGGCGGGTCAGGCAAGACGACCATCGCAACCCATCTAGC
CCGCGCCCTGCAACTCGCCGGGGCCGATGTTCTGTTAGTCGATTCCGATCC
CCAGGGCAGTGCCCGCGATTGGGCGGCCGTGCGGGAAGATCAACCGCTAA
CCGTTGTCGGCATCGACCGCCCGACGATTGACCGCGACGTGAAGGCCATCG
GCCGGCGCGACTTCGTAGTGATCGACGGAGCGCCCCAGGCGGCGGACTTG
GCTGTGTCCGCGATCAAGGCAGCCGACTTCGTGCTGATTCCGGTGCAGCCA
AGCCCTTACGACATATGGGCCACCGCCGACCTGGTGGAGCTGGTTAAGCAG
CGCATTGAGGTCACGGATGGAAGGCTACAAGCGGCCTTTGTCGTGTCGCGG
GCGATCAAAGGCACGCGCATCGGCGGTGAGGTTGCCGAGGCGCTGGCCGG
GTACGAGCTGCCCATTCTTGAGTCCCGTATCACGCAGCGCGTGAGCTACCCA
GGCACTGCCGCCGCCGGCACAACCGTTCTTGAATCAGAACCCGAGGGCGAC
GCTGCCCGCGAGGTCCAGGCGCTGGCCGCTGAAATTAAATCAAAACTCATTT
GAGTTAATGAGGTAAAGAGAAAATGAGCAAAAGCACAAACACGCTAAGTGCC
GGCCGTCCGAGCGCACGCAGCAGCAAGGCTGCAACGTTGGCCAGCCTGGC
AGACACGCCAGCCATGAAGCGGGTCAACTTTCAGTTGCCGGCGGAGGATCA
CACCAAGCTGAAGATGTACGCGGTACGCCAAGGCAAGACCATTACCGAGCT
GCTATCTGAATACATCGCGCAGCTACCAGAGTAAATGAGCAAATGAATAAATG
AGTAGATGAATTTTAGCGGCTAAAGGAGGCGGCATGGAAAATCAAGAACAAC
CAGGCACCGACGCCGTGGAATGCCCCATGTGTGGAGGAACGGGCGGTTGG
CCAGGCGTAAGCGGCTGGGTTGCCTGCCGGCCCTGCAATGGCACTGGAACC
CCCAAGCCCGAGGAATCGGCGTGAGCGGTCGCAAACCATCCGGCCCGGTAC
AAATCGGCGCGGCGCTGGGTGATGACCTGGTGGAGAAGTTGAAGGCCGCG
CAGGCCGCCCAGCGGCAACGCATCGAGGCAGAAGCACGCCCCGGTGAATC
GTGGCAAGCGGCCGCTGATCGAATCCGCAAAGAATCCCGGCAACCGCCGGC
AGCCGGTGCGCCGTCGATTAGGAAGCCGCCCAAGGGCGACGAGCAACCAG
ATTTTTTCGTTCCGATGCTCTATGACGTGGGCACCCGCGATAGTCGCAGCAT
CATGGACGTGGCCGTTTTCCGTCTGTCGAAGCGTGACCGACGAGCTGGCGA
GGTGATCCGCTACGAGCTTCCAGACGGGCACGTAGAGGTTTCCGCAGGGCC
GGCCGGCATGGCCAGTGTGTGGGATTACGACCTGGTACTGATGGCGGTTTC
CCATCTAACCGAATCCATGAACCGATACCGGGAAGGGAAGGGAGACAAGCC
CGGCCGCGTGTTCCGTCCACACGTTGCGGACGTACTCAAGTTCTGCCGGCG
AGCCGATGGCGGAAAGCAGAAAGACGACCTGGTAGAAACCTGCATTCGGTT
AAACACCACGCACGTTGCCATGCAGCGTACGAAGAAGGCCAAGAACGGCCG
CCTGGTGACGGTATCCGAGGGTGAAGCCTTGATTAGCCGCTACAAGATCGTA
AAGAGCGAAACCGGGCGGCCGGAGTACATCGAGATCGAGCTAGCTGATTGG
 
ATGTACCGCGAGATCACAGAAGGCAAGAACCCGGACGTGCTGACGGTTCAC
CCCGATTACTTTTTGATCGATCCCGGCATCGGCCGTTTTCTCTACCGCCTGG
CACGCCGCGCCGCAGGCAAGGCAGAAGCCAGATGGTTGTTCAAGACGATCT
ACGAACGCAGTGGCAGCGCCGGAGAGTTCAAGAAGTTCTGTTTCACCGTGC
GCAAGCTGATCGGGTCAAATGACCTGCCGGAGTACGATTTGAAGGAGGAGG
CGGGGCAGGCTGGCCCGATCCTAGTCATGCGCTACCGCAACCTGATCGAGG
GCGAAGCATCCGCCGGTTCCTAATGTACGGAGCAGATGCTAGGGCAAATTG
CCCTAGCAGGGGAAAAAGGTCGAAAAGGTCTCTTTCCTGTGGATAGCACGTA
CATTGGGAACCCAAAGCCGTACATTGGGAACCGGAACCCGTACATTGGGAAC
CCAAAGCCGTACATTGGGAACCGGTCACACATGTAAGTGACTGATATAAAAG
AGAAAAAAGGCGATTTTTCCGCCTAAAACTCTTTAAAACTTATTAAAACTCTTA
AAACCCGCCTGGCCTGTGCATAACTGTCTGGCCAGCGCACAGCCGAAGAGC
TGCAAAAAGCGCCTACCCTTCGGTCGCTGCGCTCCCTACGCCCCGCCGCTT
CGCGTCGGCCTATCGCGGCCGCTGGCCGCTCAAAAATGGCTGGCCTACGGC
CAGGCAATCTACCAGGGCGCGGACAAGCCGCGCCGTCGCCACTCGACCGC
CGGCGCCCACATCAAGGCACCCTGCCTCGCGCGTTTCGGTGATGACGGTGA
AAACCTCTGACACATGCAGCTCCCGGAGACGGTCACAGCTTGTCTGTAAGCG
GATGCCGGGAGCAGACAAGCCCGTCAGGGCGCGTCAGCGGGTGTTGGCGG
GTGTCGGGGCGCAGCCATGACCCAGTCACGTAGCGATAGCGGAGTGTATAC
TGGCTTAACTATGCGGCATCAGAGCAGATTGTACTGAGAGTGCACCATATGC
GGTGTGAAATACCGCACAGATGCGTAAGGAGAAAATACCGCATCAGGCGCTC
TTCCGCTTCCTCGCTCACTGACTCGCTGCGCTCGGTCGTTCGGCTGCGGCG
AGCGGTATCAGCTCACTCAAAGGCGGTAATACGGTTATCCACAGAATCAGGG
GATAACGCAGGAAAGAACATGTGAGCAAAAGGCCAGCAAAAGGCCAGGAAC
CGTAAAAAGGCCGCGTTGCTGGCGTTTTTCCATAGGCTCCGCCCCCCTGACG
AGCATCACAAAAATCGACGCTCAAGTCAGAGGTGGCGAAACCCGACAGGACT
ATAAAGATACCAGGCGTTTCCCCCTGGAAGCTCCCTCGTGCGCTCTCCTGTT
CCGACCCTGCCGCTTACCGGATACCTGTCCGCCTTTCTCCCTTCGGGAAGCG
TGGCGCTTTCTCATAGCTCACGCTGTAGGTATCTCAGTTCGGTGTAGGTCGTT
CGCTCCAAGCTGGGCTGTGTGCACGAACCCCCCGTTCAGCCCGACCGCTGC
GCCTTATCCGGTAACTATCGTCTTGAGTCCAACCCGGTAAGACACGACTTATC
GCCACTGGCAGCAGCCACTGGTAACAGGATTAGCAGAGCGAGGTATGTAGG
CGGTGCTACAGAGTTCTTGAAGTGGTGGCCTAACTACGGCTACACTAGAAGG
ACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTT
GTTTGCAAGCAGCAGATTACGCGCAGAAAAAAAGGATCTCAAGAAGATCCTT
TGATCTTTTCTACGGGGTCTGACGCTCAGTGGAACGAAAACTCACGTTAAGG
GATTTTGGTCATGCATTCTAGGTACTAAAACAATTCATCCAGTAAAATATAATA
 
TTTTATTTTCTCCCAATCAGGCTTGATCCCCAGTAAGTCAAAAAATAGCTCGAC
ATACTGTTCTTCCCCGATATCCTCCCTGATCGACCGGACGCAGAAGGCAATG
TCATACCACTTGTCCGCCCTGCCGCTTCTCCCAAGATCAATAAAGCCACTTAC
TTTGCCATCTTTCACAAAGATGTTGCTGTCTCCCAGGTCGCCGTGGGAAAAG
ACAAGTTCCTCTTCGGGCTTTTCCGTCTTTAAAAAATCATACAGCTCGCGCGG
ATCTTTAAATGGAGTGTCTTCTTCCCAGTTTTCGCAATCCACATCGGCCAGAT
CGTTATTCAGTAAGTAATCCAATTCGGCTAAGCGGCTGTCTAAGCTATTCGTA
TAGGGACAATCCGATATGTCGATGGAGTGAAAGAGCCTGATGCACTCCGCAT
ACAGCTCGATAATCTTTTCAGGGCTTTGTTCATCTTCATACTCTTCCGAGCAAA
GGACGCCATCGGCCTCACTCATGAGCAGATTGCTCCAGCCATCATGCCGTTC
AAAGTGCAGGACCTTTGGAACAGGCAGCTTTCCTTCCAGCCATAGCATCATG
TCCTTTTCCCGTTCCACATCATAGGTGGTCCCTTTATACCGGCTGTCCGTCAT
TTTTAAATATAGGTTTTCATTTTCTCCCACCAGCTTATATACCTTAGCAGGAGA
CATTCCTTCCGTATCTTTTACGCAGCGGTATTTTTCGATCAGTTTTTTCAATTC
CGGTGATATTCTCATTTTAGCCATTTATTATTTCCTTCCTCTTTTCTACAGTATT
TAAAGATACCCCAAGAAGCTAATTATAACAAGACGAACTCCAATTCACTGTTC
CTTGCATTCTAAAACCTTAAATACCAGAAAACAGCTTTTTCAAAGTTGTTTTCA
AAGTTGGCGTATAACATAGTATCGACGGAGCCGATTTTGAAACCGCGGTGAT
CACAGGCAGCAACGCTCTGTCATCGTTACAATCAACATGCTACCCTCCGCGA
GATCATCCGTGTTTCAAACCCGGCAGCTTAGTTGCCGTTCTTCCGAATAGCAT
CGGTAACATGAGCAAAGTCTGCCGCCTTACAACGGCTCTCCCGCTGACGCC
GTCCCGGACTGATGGGCTGCCTGTATCGAGTGGTGATTTTGTGCCGAGCTGC
CGGTCGGGGAGCTGTTGGCTGGCTGGTGGCAGGATATATTGTGGTGTAAAC
AAATTGACGCTTAGACAACTTAATAACACATTGCGGACGTTTTTAATGTACTGA
ATTAACGCCGAATTGAATTCGAGCTCGGTACCGGGGGATCTGGATTTTAGTA
CTGGATTTTGGTTTTAGGAATTAGAAATTTTATTGATAGAAGTATTTTACAAATA
CAAATACATACTAAGGGTTTCTTATATGCTCAACACATGAGCGAAACCCTATA
GGAACCCTAATTCCCTTATCTGGGAACTACTCACACATTATTATGGAGAAACT
CGAGCTTGTCGATCGACTCAAATCTCGGTGACGGGCAGGACCGGACGGGGC
GGTACCGGCAGGCTGAAGTCCAGCTGCCAGAAACCCACGTCATGCCAGTTC
CCGTGCTTGAAGCCGGCCGCCCGCAGCATGCCGCGGGGGGCATATCCGAG
CGCCTCGTGCATGCGCACGCTCGGGTCGTTGGGCAGCCCGATGACAGCGAC
CACGCTCTTGAAGCCCTGTGCCTCCAGGGACTTCAGCAGGTGGGTGTAGAG
CGTGGAGCCCAGTCCCGTCCGCTGGTGGCGGGGGGAGACGTACACGGTCG
ACTCGGCCGTCCAGTCGTAGGCGTTGCGTGCCTTCCAGGGGCCCGCGTAGG
CGATGCCGGCGACCTCGCCGTCCACCTCGGCGACGAGCCAGGGATAGCGC
TCCCGCAGACGGACGAGGTCGTCCGTCCACTCCTGCGGTTCCTGCGGCTCG
GTACGGAAGTTGACCGTGCTTGTCTCGATGTAGTGGTTGACGATGGTGCAGA
 
CCGCCGGCATGTCCGCCTCGGTGGCACGGCGGATGTCGGCCGGGCGTCGT
TCTGGGCTCATGGTAGACGATCTGCGAAAGCTCGAGAGAGATAGATTTGTAG
AGAGAGACTGGTGATTTCAGCGTGTCCTCTCCAAATGAAATGAACTTCCTTAT
ATAGAGGAAGGGTCTTGCGAAGGATAGTGGGATTGTGCGTCATCCCTTACGT
CAGTGGAGATATCACATCAATCCACTTGCTTTGAAGACGTGGTTGGAACGTCT
TCTTTTTCCACGATGCTCCTCGTGGGTGGGGGTCCATCTTTGGGACCACTGT
CGGCAGAGGCATCTTGAACGATAGCCTTTCCTTTATCGCAATGATGGCATTTG
TAGGTGCCACCTTCCTTTTCTACTGTCCTTTTGATGAAGTGACAGATAGCTGG
GCAATGGAATCCGAGGAGGTTTCCCGATATTACCCTTTGTTGAAAAGTCTCAA
TAGCCCTTTGGTCTTCTGAGACTGTATCTTTGATATTCTTGGAGTAGACGAGA
GTGTCGTGCTCCACCATGTTCACATCAATCCACTTGCTTTGAAGACGTGGTTG
GAACGTCTTCTTTTTCCACGATGCTCCTCGTGGGTGGGGGTCCATCTTTGGG
ACCACTGTCGGCAGAGGCATCTTGAACGATAGCCTTTCCTTTATCGCAATGAT
GGCATTTGTAGGTGCCACCTTCCTTTTCTACTGTCCTTTTGATGAAGTGACAG
ATAGCTGGGCAATGGAATCCGAGGAGGTTTCCCGATATTACCCTTTGTTGAAA
AGTCTCAATAGCCCTTTGGTCTTCTGAGACTGTATCTTTGATATTCTTGGAGTA
GACGAGAGTGTCGTGCTCCACCATGTTGGCGTACCCGGGGATCCTCTAGAG
TCGACCTGCAGGCATGCAAGCT
Prolyl 4-hydroxylase expression vector P4Hb_1
   SEQ ID NO: 30: 
CAACTTTGTATAGAAAAGTTGGCCAACATGGTGGAGCACGACACTCTCGTCT
ACTCCAAGAATATCAAAGATACAGTCTCAGAAGACCAAAGGGCTATTGAGACT
TTTCAACAAAGGGTAATATCGGGAAACCTCCTCGGATTCCATTGCCCAGCTAT
CTGTCACTTCATCAAAAGGACAGTAGAAAAGGAAGGTGGCACCTACAAATGC
CATCATTGCGATAAAGGAAAGGCTATCGTTCAAGATGCCTCTGCCGACAGTG
GTCCCAAAGATGGACCCCCACCCACGAGGAGCATCGTGGAAAAAGAAGACG
TTCCAACCACGTCTTCAAAGCAAGTGGATTGATGTGAACATGGTGGAGCACG
ACACTCTCGTCTACTCCAAGAATATCAAAGATACAGTCTCAGAAGACCAAAGG
GCTATTGAGACTTTTCAACAAAGGGTAATATCGGGAAACCTCCTCGGATTCCA
TTGCCCAGCTATCTGTCACTTCATCAAAAGGACAGTAGAAAAGGAAGGTGGC
ACCTACAAATGCCATCATTGCGATAAAGGAAAGGCTATCGTTCAAGATGCCTC
TGCCGACAGTGGTCCCAAAGATGGACCCCCACCCACGAGGAGCATCGTGGA
AAAAGAAGACGTTCCAACCACGTCTTCAAAGCAAGTGGATTGATGTGATATCT
CCACTGACGTAAGGGATGACGCACAATCCCACTATCCTTCGCAAGACCCTTC
CTCTATATAAGGAAGTTCATTTCATTTGGAGAGGACACGCTGACAAGTTTGTA
CAAAAAAGCAGGCTGCCACCATGAGAGTACAAAGAAGGAGGGCTCCAGATC
CTCCAGGTCAGTCTGAGGTTGGGACAGGAGGAAGGGCTAGAGGAGGAAGAA
 
CTTATAAATCTAGGACCGGAGGTGCTAGGTCAAGAGGACTTCTTAGCTGGAG
GAGACAGCTTAAGAGGAGGGTTTGCGCTGATATGCTTAGAAGGGCACTTCTT
TGCTTGGCTCTTACTGCTCTTTTTCGTGCTGGAGCTGGAGCTCCAGATGAGG
AGGATCATGTTTTGGTTCTTCATAAGGGTAATTTCGATGAAGCTCTTGCAGCT
CATAAGTATTTGCTTGTTGAGTTTTATGCTCCATGGTGTGGGCACTGTAAGGC
ACTTGCTCCAGAGTACGCTAAAGCTGCTGGCAAACTTAAGGCCGAGGGTTCA
GAAATTAGACTTGCAAAGGTGGATGCTACAGAGGAATCAGATTTGGCTCAAC
AGTATGGTGTTAGGGGTTATCCTACTATCAAGTTTTTTAAAAATGGTGATACAG
CTTCACCAAAGGAATATACAGCTGGAAGGGAAGCTGATGATATTGTTAATTGG
TTGAAGAAAAGAACTGGTCCAGCTGCTTCTACACTTTCAGATGGAGCTGCTG
CTGAAGCTCTTGTTGAAAGTTCCGAGGTTGCTGTTATTGGCTTTTTTAAGGAT
ATGGAATCCGATTCAGCTAAGCAGTTTTTGTTGGCAGCTGAAGCTATTGATGA
TATTCCTTTTGGAATTACTTCTAATTCTGACGTTTTCTCTAAATACCAATTGGAT
AAAGACGGTGTGGTTCTTTTCAAGAAGTTTGACGAAGGAAGAAACAATTTTGA
GGGTGAGGTTACCAAGGAGAAGCTTCTTGATTTTATCAAACATAACCAACTTC
CACTTGTTATTGAATTTACTGAACAAACTGCACCTAAGATTTTTGGTGGAGAAA
TTAAAACTCATATTCTCCTTTTCCTCCCTAAGAGCGTTAGCGATTATGAGGGTA
AGCTGTCAAATTTTAAGAAGGCTGCAGAATCATTTAAGGGTAAGATTTTGTTC
ATCTTTATCGATTCTGATCATACTGATAATCAAAGGATTTTGGAGTTTTTCGGT
TTGAAGAAGGAAGAGTGTCCTGCTGTTAGACTTATTACTTTGGAAGAGGAGAT
GACAAAATATAAACCTGAGTCTGATGAACTTACAGCAGAGAAAATTACTGAAT
TTTGCCACAGGTTTCTTGAAGGAAAGATTAAGCCTCATTTGATGAGTCAAGAA
TTGCCTGACGATTGGGATAAACAACCTGTTAAGGTTTTGGTTGGTAAAAACTT
CGAGGAGGTTGCTTTTGATGAGAAGAAGAACGTGTTTGTTGAATTTTATGCTC
CTTGGTGCGGACATTGTAAGCAGCTTGCTCCAATCTGGGATAAGTTGGGAGA
GACCTATAAAGATCATGAGAATATTGTTATTGCTAAAATGGATTCTACTGCAAA
CGAGGTTGAGGCAGTTAAGGTTCATAGTTTCCCTACTCTCAAGTTTTTTCCTG
CTTCTGCTGATAGAACTGTTATTGATTATAATGGTGAAAGAACACTCGATGGT
TTTAAGAAATTCCTTGAATCAGGAGGACAAGATGGTGCTGGTGACGATGATG
ATCTTGAAGATCTCGAAGAAGCTGAAGAGCCAGATTTGGAAGAAGATGATGA
TCAGAAGGCTGTTAAGGATGAGTTGGCATTTGTGTATTAAACCCAGCTTTCTT
GTACAAAGTGGTAGCTCGAATTTCCCCGATCGTTCAAACATTTGGCAATAAAG
TTTCTTAAGATTGAATCCTGTTGCCGGTCTTGCGATGATTATCATATAATTTCT
GTTGAATTACGTTAAGCATGTAATAATTAACATGTAATGCATGACGTTATTTAT
GAGATGGGTTTTTATGATTAGAGTCCCGCAATTATACATTTAATACGCGATAG
AAAACAAAATATAGCGCGCAAACTAGGATAAATTATCGCGCGCGGTGTCATCT
ATGTTACTAGATCGGGAAAGCTTTTAAACTATCAGTGTTTGACAGGATATATTG
GCGGGTAAACCTAAGAGAAAAGAGCGTTTATTAGAATAATCGGATATTTAAAA
 
GGGCGTGAAAAGGTTTATCCGTTCGTCCATTTGTATGTGCATGCCAACCACA
GGGTTCCCCTCGGGATCAAAGTACTTTGATCCAACCCCTCCGCTGCTATAGT
GCAGTCGGCTTCTGACGTTCAGTGCAGCCGTCTTCTGAAAACGACATGTCGC
ACAAGTCCTAAGTTACGCGACAGGCTGCCGCCCTGCCCTTTTCCTGGCGTTT
TCTTGTCGCGTGTTTTAGTCGCATAAAGTAGAATACTTGCGACTAGAACCGGA
GACATTACGCCATGAACAAGAGCGCCGCCGCTGGCCTGCTGGGCTATGCCC
GCGTCAGCACCGACGACCAGGACTTGACCAACCAACGGGCCGAACTGCACG
CGGCCGGCTGCACCAAGCTGTTTTCCGAGAAGATCACCGGCACCAGGCGCG
ACCGCCCGGAGCTGGCCAGGATGCTTGACCACCTACGCCCTGGCGACGTTG
TGACAGTGACCAGGCTAGACCGCCTGGCCCGCAGCACCCGCGACCTACTGG
ACATTGCCGAGCGCATCCAGGAGGCCGGCGCGGGCCTGCGTAGCCTGGCA
GAGCCGTGGGCCGACACCACCACGCCGGCCGGCCGCATGGTGTTGACCGT
GTTCGCCGGCATTGCCGAGTTCGAGCGTTCCCTAATCATCGACCGCACCCG
GAGCGGGCGCGAGGCCGCCAAGGCCCGAGGCGTGAAGTTTGGCCCCCGCC
CTACCCTCACCCCGGCACAGATCGCGCACGCCCGCGAGCTGATCGACCAGG
AAGGCCGCACCGTGAAAGAGGCGGCTGCACTGCTTGGCGTGCATCGCTCGA
CCCTGTACCGCGCACTTGAGCGCAGCGAGGAAGTGACGCCCACCGAGGCCA
GGCGGCGCGGTGCCTTCCGTGAGGACGCATTGACCGAGGCCGACGCCCTG
GCGGCCGCCGAGAATGAACGCCAAGAGGAACAAGCATGAAACCGCACCAGG
ACGGCCAGGACGAACCGTTTTTCATTACCGAAGAGATCGAGGCGGAGATGAT
CGCGGCCGGGTACGTGTTCGAGCCGCCCGCGCACGTCTCAACCGTGCGGC
TGCATGAAATCCTGGCCGGTTTGTCTGATGCCAAGCTGGCGGCCTGGCCGG
CCAGCTTGGCCGCTGAAGAAACCGAGCGCCGCCGTCTAAAAAGGTGATGTG
TATTTGAGTAAAACAGCTTGCGTCATGCGGTCGCTGCGTATATGATGCGATGA
GTAAATAAACAAATACGCAAGGGGAACGCATGAAGGTTATCGCTGTACTTAAC
CAGAAAGGCGGGTCAGGCAAGACGACCATCGCAACCCATCTAGCCCGCGCC
CTGCAACTCGCCGGGGCCGATGTTCTGTTAGTCGATTCCGATCCCCAGGGCA
GTGCCCGCGATTGGGCGGCCGTGCGGGAAGATCAACCGCTAACCGTTGTCG
GCATCGACCGCCCGACGATTGACCGCGACGTGAAGGCCATCGGCCGGCGC
GACTTCGTAGTGATCGACGGAGCGCCCCAGGCGGCGGACTTGGCTGTGTCC
GCGATCAAGGCAGCCGACTTCGTGCTGATTCCGGTGCAGCCAAGCCCTTAC
GACATATGGGCCACCGCCGACCTGGTGGAGCTGGTTAAGCAGCGCATTGAG
GTCACGGATGGAAGGCTACAAGCGGCCTTTGTCGTGTCGCGGGCGATCAAA
GGCACGCGCATCGGCGGTGAGGTTGCCGAGGCGCTGGCCGGGTACGAGCT
GCCCATTCTTGAGTCCCGTATCACGCAGCGCGTGAGCTACCCAGGCACTGC
CGCCGCCGGCACAACCGTTCTTGAATCAGAACCCGAGGGCGACGCTGCCCG
CGAGGTCCAGGCGCTGGCCGCTGAAATTAAATCAAAACTCATTTGAGTTAAT
GAGGTAAAGAGAAAATGAGCAAAAGCACAAACACGCTAAGTGCCGGCCGTC
 
CGAGCGCACGCAGCAGCAAGGCTGCAACGTTGGCCAGCCTGGCAGACACG
CCAGCCATGAAGCGGGTCAACTTTCAGTTGCCGGCGGAGGATCACACCAAG
CTGAAGATGTACGCGGTACGCCAAGGCAAGACCATTACCGAGCTGCTATCTG
AATACATCGCGCAGCTACCAGAGTAAATGAGCAAATGAATAAATGAGTAGATG
AATTTTAGCGGCTAAAGGAGGCGGCATGGAAAATCAAGAACAACCAGGCACC
GACGCCGTGGAATGCCCCATGTGTGGAGGAACGGGCGGTTGGCCAGGCGT
AAGCGGCTGGGTTGCCTGCCGGCCCTGCAATGGCACTGGAACCCCCAAGCC
CGAGGAATCGGCGTGAGCGGTCGCAAACCATCCGGCCCGGTACAAATCGGC
GCGGCGCTGGGTGATGACCTGGTGGAGAAGTTGAAGGCCGCGCAGGCCGC
CCAGCGGCAACGCATCGAGGCAGAAGCACGCCCCGGTGAATCGTGGCAAG
CGGCCGCTGATCGAATCCGCAAAGAATCCCGGCAACCGCCGGCAGCCGGTG
CGCCGTCGATTAGGAAGCCGCCCAAGGGCGACGAGCAACCAGATTTTTTCGT
TCCGATGCTCTATGACGTGGGCACCCGCGATAGTCGCAGCATCATGGACGT
GGCCGTTTTCCGTCTGTCGAAGCGTGACCGACGAGCTGGCGAGGTGATCCG
CTACGAGCTTCCAGACGGGCACGTAGAGGTTTCCGCAGGGCCGGCCGGCAT
GGCCAGTGTGTGGGATTACGACCTGGTACTGATGGCGGTTTCCCATCTAACC
GAATCCATGAACCGATACCGGGAAGGGAAGGGAGACAAGCCCGGCCGCGT
GTTCCGTCCACACGTTGCGGACGTACTCAAGTTCTGCCGGCGAGCCGATGG
CGGAAAGCAGAAAGACGACCTGGTAGAAACCTGCATTCGGTTAAACACCACG
CACGTTGCCATGCAGCGTACGAAGAAGGCCAAGAACGGCCGCCTGGTGACG
GTATCCGAGGGTGAAGCCTTGATTAGCCGCTACAAGATCGTAAAGAGCGAAA
CCGGGCGGCCGGAGTACATCGAGATCGAGCTAGCTGATTGGATGTACCGCG
AGATCACAGAAGGCAAGAACCCGGACGTGCTGACGGTTCACCCCGATTACTT
TTTGATCGATCCCGGCATCGGCCGTTTTCTCTACCGCCTGGCACGCCGCGCC
GCAGGCAAGGCAGAAGCCAGATGGTTGTTCAAGACGATCTACGAACGCAGT
GGCAGCGCCGGAGAGTTCAAGAAGTTCTGTTTCACCGTGCGCAAGCTGATC
GGGTCAAATGACCTGCCGGAGTACGATTTGAAGGAGGAGGCGGGGCAGGCT
GGCCCGATCCTAGTCATGCGCTACCGCAACCTGATCGAGGGCGAAGCATCC
GCCGGTTCCTAATGTACGGAGCAGATGCTAGGGCAAATTGCCCTAGCAGGG
GAAAAAGGTCGAAAAGGTCTCTTTCCTGTGGATAGCACGTACATTGGGAACC
CAAAGCCGTACATTGGGAACCGGAACCCGTACATTGGGAACCCAAAGCCGTA
CATTGGGAACCGGTCACACATGTAAGTGACTGATATAAAAGAGAAAAAAGGC
GATTTTTCCGCCTAAAACTCTTTAAAACTTATTAAAACTCTTAAAACCCGCCTG
GCCTGTGCATAACTGTCTGGCCAGCGCACAGCCGAAGAGCTGCAAAAAGCG
CCTACCCTTCGGTCGCTGCGCTCCCTACGCCCCGCCGCTTCGCGTCGGCCT
ATCGCGGCCGCTGGCCGCTCAAAAATGGCTGGCCTACGGCCAGGCAATCTA
CCAGGGCGCGGACAAGCCGCGCCGTCGCCACTCGACCGCCGGCGCCCACA
TCAAGGCACCCTGCCTCGCGCGTTTCGGTGATGACGGTGAAAACCTCTGACA
 
CATGCAGCTCCCGGAGACGGTCACAGCTTGTCTGTAAGCGGATGCCGGGAG
CAGACAAGCCCGTCAGGGCGCGTCAGCGGGTGTTGGCGGGTGTCGGGGCG
CAGCCATGACCCAGTCACGTAGCGATAGCGGAGTGTATACTGGCTTAACTAT
GCGGCATCAGAGCAGATTGTACTGAGAGTGCACCATATGCGGTGTGAAATAC
CGCACAGATGCGTAAGGAGAAAATACCGCATCAGGCGCTCTTCCGCTTCCTC
GCTCACTGACTCGCTGCGCTCGGTCGTTCGGCTGCGGCGAGCGGTATCAGC
TCACTCAAAGGCGGTAATACGGTTATCCACAGAATCAGGGGATAACGCAGGA
AAGAACATGTGAGCAAAAGGCCAGCAAAAGGCCAGGAACCGTAAAAAGGCC
GCGTTGCTGGCGTTTTTCCATAGGCTCCGCCCCCCTGACGAGCATCACAAAA
ATCGACGCTCAAGTCAGAGGTGGCGAAACCCGACAGGACTATAAAGATACCA
GGCGTTTCCCCCTGGAAGCTCCCTCGTGCGCTCTCCTGTTCCGACCCTGCC
GCTTACCGGATACCTGTCCGCCTTTCTCCCTTCGGGAAGCGTGGCGCTTTCT
CATAGCTCACGCTGTAGGTATCTCAGTTCGGTGTAGGTCGTTCGCTCCAAGC
TGGGCTGTGTGCACGAACCCCCCGTTCAGCCCGACCGCTGCGCCTTATCCG
GTAACTATCGTCTTGAGTCCAACCCGGTAAGACACGACTTATCGCCACTGGC
AGCAGCCACTGGTAACAGGATTAGCAGAGCGAGGTATGTAGGCGGTGCTAC
AGAGTTCTTGAAGTGGTGGCCTAACTACGGCTACACTAGAAGGACAGTATTT
GGTATCTGCGCTCTGCTGAAGCCAGTTACCTTCGGAAAAAGAGTTGGTAGCT
CTTGATCCGGCAAACAAACCACCGCTGGTAGCGGTGGTTTTTTTGTTTGCAA
GCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGT
CATGCATTCTAGGTACTAAAACAATTCATCCAGTAAAATATAATATTTTATTTTC
TCCCAATCAGGCTTGATCCCCAGTAAGTCAAAAAATAGCTCGACATACTGTTC
TTCCCCGATATCCTCCCTGATCGACCGGACGCAGAAGGCAATGTCATACCAC
TTGTCCGCCCTGCCGCTTCTCCCAAGATCAATAAAGCCACTTACTTTGCCATC
TTTCACAAAGATGTTGCTGTCTCCCAGGTCGCCGTGGGAAAAGACAAGTTCC
TCTTCGGGCTTTTCCGTCTTTAAAAAATCATACAGCTCGCGCGGATCTTTAAA
TGGAGTGTCTTCTTCCCAGTTTTCGCAATCCACATCGGCCAGATCGTTATTCA
GTAAGTAATCCAATTCGGCTAAGCGGCTGTCTAAGCTATTCGTATAGGGACAA
TCCGATATGTCGATGGAGTGAAAGAGCCTGATGCACTCCGCATACAGCTCGA
TAATCTTTTCAGGGCTTTGTTCATCTTCATACTCTTCCGAGCAAAGGACGCCA
TCGGCCTCACTCATGAGCAGATTGCTCCAGCCATCATGCCGTTCAAAGTGCA
GGACCTTTGGAACAGGCAGCTTTCCTTCCAGCCATAGCATCATGTCCTTTTCC
CGTTCCACATCATAGGTGGTCCCTTTATACCGGCTGTCCGTCATTTTTAAATA
TAGGTTTTCATTTTCTCCCACCAGCTTATATACCTTAGCAGGAGACATTCCTTC
CGTATCTTTTACGCAGCGGTATTTTTCGATCAGTTTTTTCAATTCCGGTGATAT
TCTCATTTTAGCCATTTATTATTTCCTTCCTCTTTTCTACAGTATTTAAAGATAC
CCCAAGAAGCTAATTATAACAAGACGAACTCCAATTCACTGTTCCTTGCATTC
 
TAAAACCTTAAATACCAGAAAACAGCTTTTTCAAAGTTGTTTTCAAAGTTGGCG
TATAACATAGTATCGACGGAGCCGATTTTGAAACCGCGGTGATCACAGGCAG
CAACGCTCTGTCATCGTTACAATCAACATGCTACCCTCCGCGAGATCATCCGT
GTTTCAAACCCGGCAGCTTAGTTGCCGTTCTTCCGAATAGCATCGGTAACATG
AGCAAAGTCTGCCGCCTTACAACGGCTCTCCCGCTGACGCCGTCCCGGACT
GATGGGCTGCCTGTATCGAGTGGTGATTTTGTGCCGAGCTGCCGGTCGGGG
AGCTGTTGGCTGGCTGGTGGCAGGATATATTGTGGTGTAAACAAATTGACGC
TTAGACAACTTAATAACACATTGCGGACGTTTTTAATGTACTGAATTAACGCCG
AATTGAATTCGAGCTCGGTACCGGGGGATCTGGATTTTAGTACTGGATTTTGG
TTTTAGGAATTAGAAATTTTATTGATAGAAGTATTTTACAAATACAAATACATAC
TAAGGGTTTCTTATATGCTCAACACATGAGCGAAACCCTATAGGAACCCTAAT
TCCCTTATCTGGGAACTACTCACACATTATTATGGAGAAACTCGAGCTTGTCG
ATCGACTCAAATCTCGGTGACGGGCAGGACCGGACGGGGCGGTACCGGCA
GGCTGAAGTCCAGCTGCCAGAAACCCACGTCATGCCAGTTCCCGTGCTTGAA
GCCGGCCGCCCGCAGCATGCCGCGGGGGGCATATCCGAGCGCCTCGTGCA
TGCGCACGCTCGGGTCGTTGGGCAGCCCGATGACAGCGACCACGCTCTTGA
AGCCCTGTGCCTCCAGGGACTTCAGCAGGTGGGTGTAGAGCGTGGAGCCCA
GTCCCGTCCGCTGGTGGCGGGGGGAGACGTACACGGTCGACTCGGCCGTC
CAGTCGTAGGCGTTGCGTGCCTTCCAGGGGCCCGCGTAGGCGATGCCGGC
GACCTCGCCGTCCACCTCGGCGACGAGCCAGGGATAGCGCTCCCGCAGAC
GGACGAGGTCGTCCGTCCACTCCTGCGGTTCCTGCGGCTCGGTACGGAAGT
TGACCGTGCTTGTCTCGATGTAGTGGTTGACGATGGTGCAGACCGCCGGCAT
GTCCGCCTCGGTGGCACGGCGGATGTCGGCCGGGCGTCGTTCTGGGCTCAT
GGTAGACGATCTGCGAAAGCTCGAGAGAGATAGATTTGTAGAGAGAGACTGG
TGATTTCAGCGTGTCCTCTCCAAATGAAATGAACTTCCTTATATAGAGGAAGG
GTCTTGCGAAGGATAGTGGGATTGTGCGTCATCCCTTACGTCAGTGGAGATA
TCACATCAATCCACTTGCTTTGAAGACGTGGTTGGAACGTCTTCTTTTTCCAC
GATGCTCCTCGTGGGTGGGGGTCCATCTTTGGGACCACTGTCGGCAGAGGC
ATCTTGAACGATAGCCTTTCCTTTATCGCAATGATGGCATTTGTAGGTGCCAC
CTTCCTTTTCTACTGTCCTTTTGATGAAGTGACAGATAGCTGGGCAATGGAAT
CCGAGGAGGTTTCCCGATATTACCCTTTGTTGAAAAGTCTCAATAGCCCTTTG
GTCTTCTGAGACTGTATCTTTGATATTCTTGGAGTAGACGAGAGTGTCGTGCT
CCACCATGTTCACATCAATCCACTTGCTTTGAAGACGTGGTTGGAACGTCTTC
TTTTTCCACGATGCTCCTCGTGGGTGGGGGTCCATCTTTGGGACCACTGTCG
GCAGAGGCATCTTGAACGATAGCCTTTCCTTTATCGCAATGATGGCATTTGTA
GGTGCCACCTTCCTTTTCTACTGTCCTTTTGATGAAGTGACAGATAGCTGGGC
AATGGAATCCGAGGAGGTTTCCCGATATTACCCTTTGTTGAAAAGTCTCAATA
GCCCTTTGGTCTTCTGAGACTGTATCTTTGATATTCTTGGAGTAGACGAGAGT
 
GTCGTGCTCCACCATGTTGGCGTACCCGGGGATCCTCTAGAGTCGACCTGCA
GGCATGCAAGCT
Bovine Collagen alpha-1 (1) Expression vector 7
   SEQ ID NO: 31: 
AGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCC
CTTCGGGAAGCGTGGCGCTTTCTCATAGCTCACGCTGTAGGTATCTCAGTTC
GGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGG
CTACACTAGAAGGACAGTATTTGGTATCTGCGCTCTGCTGAAGCCAGTTACCT
TCGGAAAAAGAGTTGGTAGCTCTTGATCCGGCAAACAAACCACCGCTGGTAG
CGGTGGTTTTTTTGTTTGCAAGCAGCAGATTACGCGCAGAAAAAAAGGATCTC
AAGAAGATCCTTTGATCTTTTCTACGGGGTCTGACGCTCAGTGGAACGAAAAC
TCACGTTAAGGGATTTTGGTCATGCATTCTAGGTACTAAAACAATTCATCCAG
TAAAATATAATATTTTATTTTCTCCCAATCAGGCTTGATCCCCAGTAAGTCAAA
AAATAGCTCGACATACTGTTCTTCCCCGATATCCTCCCTGATCGACCGGACG
CAGAAGGCAATGTCATACCACTTGTCCGCCCTGCCGCTTCTCCCAAGATCAA
TAAAGCCACTTACTTTGCCATCTTTCACAAAGATGTTGCTGTCTCCCAGGTCG
CCGTGGGAAAAGACAAGTTCCTCTTCGGGCTTTTCCGTCTTTAAAAAATCATA
CAGCTCGCGCGGATCTTTAAATGGAGTGTCTTCTTCCCAGTTTTCGCAATCCA
CATCGGCCAGATCGTTATTCAGTAAGTAATCCAATTCGGCTAAGCGGCTGTCT
AAGCTATTCGTATAGGGACAATCCGATATGTCGATGGAGTGAAAGAGCCTGA
TGCACTCCGCATACAGCTCGATAATCTTTTCAGGGCTTTGTTCATCTTCATACT
CTTCCGAGCAAAGGACGCCATCGGCCTCACTCATGAGCAGATTGCTCCAGCC
ATCATGCCGTTCAAAGTGCAGGACCTTTGGAACAGGCAGCTTTCCTTCCAGC
CATAGCATCATGTCCTTTTCCCGTTCCACATCATAGGTGGTCCCTTTATACCG
GCTGTCCGTCATTTTTAAATATAGGTTTTCATTTTCTCCCACCAGCTTATATAC
CTTAGCAGGAGACATTCCTTCCGTATCTTTTACGCAGCGGTATTTTTCGATCA
GTTTTTTCAATTCCGGTGATATTCTCATTTTAGCCATTTATTATTTCCTTCCTCT
TTTCTACAGTATTTAAAGATACCCCAAGAAGCTAATTATAACAAGACGAACTCC
AATTCACTGTTCCTTGCATTCTAAAACCTTAAATACCAGAAAACAGCTTTTTCA
AAGTTGTTTTCAAAGTTGGCGTATAACATAGTATCGACGGAGCCGATTTTGAA
ACCGCGGTGATCACAGGCAGCAACGCTCTGTCATCGTTACAATCAACATGCT
 
ACCCTCCGCGAGATCATCCGTGTTTCAAACCCGGCAGCTTAGTTGCCGTTCT
TCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTT
GTGCCGAGCTGCCGGTCGGGGAGCTGTTGGCTGGCTGGTGGCAGGATATAT
TGTGGTGTAAACAAATTGACGCTTAGACAACTTAATAACACATTGCGGACGTT
TTTAATGTACTGAATTAACGCCGAATTGAATTCGAGCTCGGTGGAGGATCTGG
ATTTTAGTACTGGATTTTGGTTTTAGGAATTAGAAATTTTATTGATAGAAGTATT
TTACAAATACAAATACATACTAAGGGTTTCTTATATGCTCAACACATGAGCGAA
ACCCTATAGGAACCCTAATTCCCTTATCTGGGAACTACTCACACATTATTATG
GAGAAACTATTCACTCTCCTTCTCAAACGTTTCTGTTCCCTCTGGACAGCCCC
GACTGACGTTGGACTCAACCTCAATGGGCGCTAATTGTAATAGCTCCTGTTTG
CTTCTTATGGCCATTTCGCAAAGGTGTTGCAGTGTTCTGCTACCACCACTCAC
TGTAATTGACACACCTCTGAAACGGATGGAGTAAGTGCAACCAATTTGATCAA
ATCCAAAGAAACGACTAGCAGCGTAATTTACTGAGTCACCAGTCCAGGAACC
TAGAACTCGATGGAGAGAGGCCTCTGTCCGATCATAGCGTAGGTAGCGTTTG
AAAACTACCTTGCCAAATCCCCAGGACTCTTTAAATCCCAGTGGGTTGTCCTG
GTTGGAGTTTGTTTCGTCATTGTGTAAACGCCATTCTGTCCAAGATGGGCTCT
CATCGGGTAACTTAAATGGGCTGGTAGTGCCGCCAGAACCACCGTCCCATCT
CTCTGAATTGGCCTGCTCTCTTGCATCATTACCCTGGATTGCTCTCTCCATTG
TCCTCTCCAAATGAAATGAACTTCCTTATATAGAGGAAGGGTCTTGCGAAGGA
TAGTGGGATTGTGCGTCATCCCTTACGTCAGTGGAGATGTCACATCAATCCA
CTTGCTTTGTAGACGTGGTTGGAACCTCTTCTTTTTCCACGATGCTCCTCGTG
GGTGGGGGTCCATCTTTGGGACCACTGTCGGCAGAGAGATCTTGAATGATAG
CCTTTCCTTTATCGCAATGATGGCATTTGTAGGAGCCACCTTCCTTTTCTACT
GTCCTTTCGATGAAGTGACAGATAGCTGGGCAATGGAATCCGAGGAGGTTTC
CCGAAATTATCCTTTGTTGAAAAGTCTCAAACAATAGGCAACCGTGGACTTCT
TCACTAGCTCATCGAGATAGCAATGCCACTAGCTAATTTCTTACGTTGTATCTT
ATTTGTTTTACTTTGGGGCATGACATGGTTAAACCCCATCAAAGAGAAAGTGT
TTCATCCACTAGTCAATATACGAATTCATTCGAATTTATCCCTGTAAATCCTAG
TTCTTAGGATGAACTGGTGTAATAAACAGCAAAAAAAAATAAAAAAAAATAAAT
CTGGAATCATTCGACCACCTCAATAAACTAAAGCTACCAATTACCACAATATA
GTCATCCATATCCACTTAGATATAAAAGATAAAAGTAAACAAATATTAAATTTC
ATATGCACGCATAGGAAACTCATGATCTTATCTTTTAAATAGACATCTAGTTTT
CTTAGGTTATAAATAGACATTTTGTCCTAGAACTTCTTCACTACTAAAACCTAG
CCTCAAAAAGCAGGCTGCCACCATGCATCACCATCATCATCATGGATCTGAG
AATTTATATTTTCAAGGTCAATTGAGTTATGGATACGACGAAAAATCAACTGGA
ATATCAGTGCCAGGACCAATGGGTCCATCAGGACCTAGGGGTCTTCCAGGG
CCACCTGGCGCTCCTGGTCCTCAGGGATTTCAAGGTCCTCCAGGTGAACCTG
 
GAGAGCCTGGTGCTTCTGGTCCAATGGGACCAAGAGGCCCTCCTGGTCCAC
CTGGTAAAAATGGTGATGATGGTGAGGCTGGAAAGCCTGGTCGTCCTGGAG
AAAGAGGACCACCAGGACCACAGGGAGCTAGAGGTTTGCCAGGTACAGCCG
GTCTTCCAGGAATGAAAGGACACAGGGGTTTTTCTGGTCTTGATGGTGCTAA
GGGAGACGCAGGACCTGCTGGACCAAAGGGTGAACCTGGATCTCCTGGTGA
AAATGGTGCTCCTGGACAAATGGGACCTAGAGGATTGCCTGGCGAAAGAGG
AAGACCTGGTGCTCCTGGACCAGCTGGAGCTAGAGGAAATGATGGAGCTAC
TGGTGCTGCCGGACCACCAGGACCAACTGGACCAGCTGGACCACCTGGTTT
TCCAGGAGCAGTTGGGGCTAAGGGAGAAGGTGGACCACAAGGACCTAGAGG
ATCAGAAGGACCTCAAGGAGTTAGGGGAGAACCAGGTCCTCCTGGTCCTGC
TGGAGCTGCTGGACCCGCTGGTAACCCAGGAGCAGATGGACAACCTGGAGC
AAAAGGGGCTAATGGTGCTCCTGGTATTGCAGGTGCTCCTGGATTCCCTGGT
GCTAGAGGACCTTCAGGACCTCAGGGACCATCCGGTCCTCCAGGACCAAAG
GGAAACTCTGGTGAACCAGGGGCTCCTGGATCTAAGGGAGATACTGGAGCT
AAGGGTGAGCCAGGACCTACAGGAATTCAAGGACCACCAGGACCTGCAGGA
GAGGAAGGTAAGAGGGGTGCTAGAGGTGAACCTGGACCAGCTGGATTGCCA
GGCCCACCTGGAGAGAGGGGTGGTCCAGGTTCTAGAGGTTTTCCAGGAGCT
GATGGAGTTGCTGGTCCAAAGGGTCCTGCTGGTGAGAGAGGTGCACCTGGA
CCAGCAGGACCTAAAGGATCACCTGGAGAAGCTGGTCGTCCTGGTGAGGCT
GGTCTTCCAGGTGCTAAGGGCCTTACAGGATCTCCAGGATCACCAGGTCCAG
ATGGAAAGACTGGACCACCAGGTCCTGCAGGACAAGATGGAAGGCCTGGTC
CACCTGGACCACCTGGTGCTAGGGGACAAGCTGGTGTTATGGGATTCCCAG
GTCCAAAGGGTGCAGCTGGTGAGCCTGGTAAAGCTGGTGAAAGAGGAGTTC
CAGGACCTCCTGGTGCTGTTGGACCTGCTGGTAAAGATGGTGAAGCAGGTG
CTCAGGGTCCACCAGGTCCAGCCGGTCCAGCTGGTGAGAGGGGAGAGCAA
GGTCCTGCTGGATCTCCTGGATTTCAAGGTTTGCCTGGACCTGCTGGACCTC
CAGGAGAGGCTGGTAAACCTGGTGAGCAGGGTGTGCCTGGTGATCTTGGTG
CTCCTGGACCTAGTGGCGCAAGAGGCGAAAGGGGATTTCCAGGTGAAAGAG
GAGTTCAGGGACCACCAGGTCCAGCTGGTCCAAGAGGCGCTAATGGAGCTC
CAGGAAATGATGGAGCAAAGGGTGATGCTGGCGCTCCAGGAGCTCCTGGTT
CTCAAGGTGCACCTGGATTGCAAGGAATGCCAGGAGAGAGAGGTGCAGCTG
GTTTGCCGGGACCAAAGGGTGATAGAGGTGATGCTGGTCCTAAGGGAGCTG
ATGGAGCTCCAGGAAAGGATGGTGTGAGAGGATTGACTGGTCCAATTGGAC
CACCAGGTCCTGCTGGAGCACCAGGAGATAAAGGAGAAGCTGGTCCTTCAG
GACCAGCCGGTCCAACTGGAGCTAGAGGAGCTCCAGGAGATAGAGGTGAAC
CAGGACCACCAGGACCTGCTGGGTTTGCAGGTCCACCTGGTGCTGATGGAC
AACCTGGTGCTAAAGGAGAACCAGGCGATGCTGGTGCTAAGGGAGATGCTG
GCCCACCTGGTCCAGCTGGTCCTGCTGGGCCACCAGGTCCTATCGGAAATG
 
TTGGTGCTCCAGGGCCTAAAGGAGCTAGAGGATCAGCAGGTCCCCCAGGTG
CTACAGGTTTTCCAGGGGCTGCTGGAAGAGTGGGTCCCCCAGGTCCTTCTG
GGAATGCAGGACCACCAGGTCCTCCAGGACCAGCTGGAAAAGAAGGAAGTA
AGGGACCAAGGGGAGAAACTGGACCTGCAGGACGTCCAGGTGAAGTTGGTC
CACCTGGACCTCCAGGTCCAGCTGGAGAGAAAGGAGCTCCAGGAGCTGATG
GTCCAGCAGGTGCTCCTGGTACTCCAGGTCCACAAGGTATTGCTGGTCAAAG
GGGAGTGGTTGGTCTCCCAGGTCAGAGAGGTGAAAGAGGTTTCCCAGGTTT
GCCTGGACCATCTGGAGAACCAGGTAAGCAGGGCCCATCTGGAGCTTCTGG
TGAGAGAGGGCCACCTGGACCTATGGGACCTCCTGGGCTTGCTGGACCTCC
AGGGGAATCTGGTAGGGAGGGTGCTCCAGGTGCTGAAGGATCACCTGGTAG
AGATGGATCACCTGGAGCTAAAGGTGATAGAGGGGAAACAGGTCCAGCAGG
TCCACCTGGCGCTCCTGGTGCTCCAGGAGCACCAGGGCCAGTTGGACCTGC
TGGAAAGTCTGGAGATAGGGGAGAGACTGGACCAGCTGGACCTGCTGGACC
AATCGGTCCTGTTGGCGCTAGAGGTCCTGCTGGACCACAAGGTCCTAGGGG
AGATAAAGGTGAAACAGGTGAACAAGGAGATAGAGGAATTAAAGGTCATAGA
GGGTTTTCTGGTTTGCAAGGACCTCCAGGACCTCCTGGATCTCCAGGAGAGC
AAGGACCATCTGGTGCAAGCGGACCTGCTGGTCCTAGAGGACCTCCAGGTT
CAGCTGGCTCACCTGGTAAGGATGGATTAAATGGTTTGCCTGGACCAATAGG
TCCACCAGGACCTAGAGGAAGGACAGGTGATGCAGGACCAGCTGGTCCACC
TGGTCCACCAGGACCACCAGGACCACCAGGTCCACCTTCTGGTGGATATGAT
CTTAGTTTTCTTCCTCAACCTCCCCAGGAGAAAGCTCATGATGGTGGTAGATA
TTATAGGGCTGCATTTGTTTATTAAACCCAGCTTTCTTGTACAAAGTGGTAGCT
CGAATTTCCCCGATCGTTCAAACATTTGGCAATAAAGTTTCTTAAGATTGAATC
CTGTTGCCGGTCTTGCGATGATTATCATATAATTTCTGTTGAATTACGTTAAGC
ATGTAATAATTAACATGTAATGCATGACGTTATTTATGAGATGGGTTTTTATGA
TTAGAGTCCCGCAATTATACATTTAATACGCGATAGAAAACAAAATATAGCGC
GCAAACTAGGATAAATTATCGCGCGCGGTGTCATCTATGTTACTAGATCGGG
AAAGCTTTTAAACTATCAGTGTTTGACAGGATATATTGGCGGGTAAACCTAAG
AGAAAAGAGCGTTTATTAGAATAATCGGATATTTAAAAGGGCGTGAAAAGGTT
TATCCGTTCGTCCATTTGTATGTGCATGCCAACCACAGGGTTCCCCTCGGGA
TCAAAGTACTTTGATCCAACCCCTCCGCTGCTATAGTGCAGTCGGCTTCTGAC
GTTCAGTGCAGCCGTCTTCTGAAAACGACATGTCGCACAAGTCCTAAGTTAC
GCGACAGGCTGCCGCCCTGCCCTTTTCCTGGCGTTTTCTTGTCGCGTGTTTT
AGTCGCATAAAGTAGAATACTTGCGACTAGAACCGGAGACATTACGCCATGA
ACAAGAGCGCCGCCGCTGGCCTGCTGGGCTATGCCCGCGTCAGCACCGAC
GACCAGGACTTGACCAACCAACGGGCCGAACTGCACGCGGCCGGCTGCACC
AAGCTGTTTTCCGAGAAGATCACCGGCACCAGGCGCGACCGCCCGGAGCTG
GCCAGGATGCTTGACCACCTACGCCCTGGCGACGTTGTGACAGTGACCAGG
 
CTAGACCGCCTGGCCCGCAGCACCCGCGACCTACTGGACATTGCCGAGCGC
ATCCAGGAGGCCGGCGCGGGCCTGCGTAGCCTGGCAGAGCCGTGGGCCGA
CACCACCACGCCGGCCGGCCGCATGGTGTTGACCGTGTTCGCCGGCATTGC
CGAGTTCGAGCGTTCCCTAATCATCGACCGCACCCGGAGCGGGCGCGAGGC
CGCCAAGGCCCGAGGCGTGAAGTTTGGCCCCCGCCCTACCCTCACCCCGGC
ACAGATCGCGCACGCCCGCGAGCTGATCGACCAGGAAGGCCGCACCGTGAA
AGAGGCGGCTGCACTGCTTGGCGTGCATCGCTCGACCCTGTACCGCGCACT
TGAGCGCAGCGAGGAAGTGACGCCCACCGAGGCCAGGCGGCGCGGTGCCT
TCCGTGAGGACGCATTGACCGAGGCCGACGCCCTGGCGGCCGCCGAGAAT
GAACGCCAAGAGGAACAAGCATGAAACCGCACCAGGACGGCCAGGACGAAC
CGTTTTTCATTACCGAAGAGATCGAGGCGGAGATGATCGCGGCCGGGTACGT
GTTCGAGCCGCCCGCGCACGTCTCAACCGTGCGGCTGCATGAAATCCTGGC
CGGTTTGTCTGATGCCAAGCTGGCGGCCTGGCCGGCCAGCTTGGCCGCTGA
AGAAACCGAGCGCCGCCGTCTAAAAAGGTGATGTGTATTTGAGTAAAACAGC
TTGCGTCATGCGGTCGCTGCGTATATGATGCGATGAGTAAATAAACAAATAC
GCAAGGGGAACGCATGAAGGTTATCGCTGTACTTAACCAGAAAGGCGGGTC
AGGCAAGACGACCATCGCAACCCATCTAGCCCGCGCCCTGCAACTCGCCGG
GGCCGATGTTCTGTTAGTCGATTCCGATCCCCAGGGCAGTGCCCGCGATTG
GGCGGCCGTGCGGGAAGATCAACCGCTAACCGTTGTCGGCATCGACCGCCC
GACGATTGACCGCGACGTGAAGGCCATCGGCCGGCGCGACTTCGTAGTGAT
CGACGGAGCGCCCCAGGCGGCGGACTTGGCTGTGTCCGCGATCAAGGCAG
CCGACTTCGTGCTGATTCCGGTGCAGCCAAGCCCTTACGACATATGGGCCAC
CGCCGACCTGGTGGAGCTGGTTAAGCAGCGCATTGAGGTCACGGATGGAAG
GCTACAAGCGGCCTTTGTCGTGTCGCGGGCGATCAAAGGCACGCGCATCGG
CGGTGAGGTTGCCGAGGCGCTGGCCGGGTACGAGCTGCCCATTCTTGAGTC
CCGTATCACGCAGCGCGTGAGCTACCCAGGCACTGCCGCCGCCGGCACAAC
CGTTCTTGAATCAGAACCCGAGGGCGACGCTGCCCGCGAGGTCCAGGCGCT
GGCCGCTGAAATTAAATCAAAACTCATTTGAGTTAATGAGGTAAAGAGAAAAT
GAGCAAAAGCACAAACACGCTAAGTGCCGGCCGTCCGAGCGCACGCAGCAG
CAAGGCTGCAACGTTGGCCAGCCTGGCAGACACGCCAGCCATGAAGCGGGT
CAACTTTCAGTTGCCGGCGGAGGATCACACCAAGCTGAAGATGTACGCGGTA
CGCCAAGGCAAGACCATTACCGAGCTGCTATCTGAATACATCGCGCAGCTAC
CAGAGTAAATGAGCAAATGAATAAATGAGTAGATGAATTTTAGCGGCTAAAGG
AGGCGGCATGGAAAATCAAGAACAACCAGGCACCGACGCCGTGGAATGCCC
CATGTGTGGAGGAACGGGCGGTTGGCCAGGCGTAAGCGGCTGGGTTGCCT
GCCGGCCCTGCAATGGCACTGGAACCCCCAAGCCCGAGGAATCGGCGTGA
GCGGTCGCAAACCATCCGGCCCGGTACAAATCGGCGCGGCGCTGGGTGATG
ACCTGGTGGAGAAGTTGAAGGCCGCGCAGGCCGCCCAGCGGCAACGCATC
 
GAGGCAGAAGCACGCCCCGGTGAATCGTGGCAAGCGGCCGCTGATCGAATC
CGCAAAGAATCCCGGCAACCGCCGGCAGCCGGTGCGCCGTCGATTAGGAAG
CCGCCCAAGGGCGACGAGCAACCAGATTTTTTCGTTCCGATGCTCTATGACG
TGGGCACCCGCGATAGTCGCAGCATCATGGACGTGGCCGTTTTCCGTCTGTC
GAAGCGTGACCGACGAGCTGGCGAGGTGATCCGCTACGAGCTTCCAGACGG
GCACGTAGAGGTTTCCGCAGGGCCGGCCGGCATGGCCAGTGTGTGGGATTA
CGACCTGGTACTGATGGCGGTTTCCCATCTAACCGAATCCATGAACCGATAC
CGGGAAGGGAAGGGAGACAAGCCCGGCCGCGTGTTCCGTCCACACGTTGC
GGACGTACTCAAGTTCTGCCGGCGAGCCGATGGCGGAAAGCAGAAAGACGA
CCTGGTAGAAACCTGCATTCGGTTAAACACCACGCACGTTGCCATGCAGCGT
ACGAAGAAGGCCAAGAACGGCCGCCTGGTGACGGTATCCGAGGGTGAAGCC
TTGATTAGCCGCTACAAGATCGTAAAGAGCGAAACCGGGCGGCCGGAGTAC
ATCGAGATCGAGCTAGCTGATTGGATGTACCGCGAGATCACAGAAGGCAAGA
ACCCGGACGTGCTGACGGTTCACCCCGATTACTTTTTGATCGATCCCGGCAT
CGGCCGTTTTCTCTACCGCCTGGCACGCCGCGCCGCAGGCAAGGCAGAAGC
CAGATGGTTGTTCAAGACGATCTACGAACGCAGTGGCAGCGCCGGAGAGTT
CAAGAAGTTCTGTTTCACCGTGCGCAAGCTGATCGGGTCAAATGACCTGCCG
GAGTACGATTTGAAGGAGGAGGCGGGGCAGGCTGGCCCGATCCTAGTCATG
CGCTACCGCAACCTGATCGAGGGCGAAGCATCCGCCGGTTCCTAATGTACG
GAGCAGATGCTAGGGCAAATTGCCCTAGCAGGGGAAAAAGGTCGAAAAGGT
CTCTTTCCTGTGGATAGCACGTACATTGGGAACCCAAAGCCGTACATTGGGA
ACCGGAACCCGTACATTGGGAACCCAAAGCCGTACATTGGGAACCGGTCAC
ACATGTAAGTGACTGATATAAAAGAGAAAAAAGGCGATTTTTCCGCCTAAAAC
TCTTTAAAACTTATTAAAACTCTTAAAACCCGCCTGGCCTGTGCATAACTGTCT
GGCCAGCGCACAGCCGAAGAGCTGCAAAAAGCGCCTACCCTTCGGTCGCTG
CGCTCCCTACGCCCCGCCGCTTCGCGTCGGCCTATCGCGGCCGCTGGCCG
CTCAAAAATGGCTGGCCTACGGCCAGGCAATCTACCAGGGCGCGGACAAGC
CGCGCCGTCGCCACTCGACCGCCGGCGCCCACATCAAGGCACCCTGCCTCG
CGCGTTTCGGTGATGACGGTGAAAACCTCTGACACATGCAGCTCCCGGAGAC
GGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCAC
GTAGCGATAGCGGAGTGTATACTGGCTTAACTATGCGGCATCAGAGCAGATT
GTACTGAGAGTGCACCATATGCGGTGTGAAATACCGCACAGATGCGTAAGGA
GAAAATACCGCATCAGGCGCTCTTCCGCTTCCTCGCTCACTGACTCGCTGCG
CTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAA
GGCCAGCAAA
UBQ10 promotor (Arabidopsis Thaliana)
   SEQ ID NO: 32: 
TCGAGCTGCAGGTCAACGGATCAGGATATTCTTGTTTAAGATGTTGAACTCTA
TGGAGGTTTGTATGAACTGATGATCTAGGACCGGATAAGTTCCCTTCTTCATA
GCGAACTTATTCAAAGAATGTTTTGTGTATCATTCTTGTTACATTGTTATTAATG
AAAAAATATTATTGGTCATTGGACTGAACACGAGTGTTAAATATGGACCAGGC
CCCAAATAAGATCCATTGATATATGAATTAAATAACAAGAATAAATCGAGTCAC
CAAACCACTTGCCTTTTTTAACGAGACTTGTTCACCAACTTGATACAAAAGTCA
TTATCCTATGCAAATCAATAATCATACAAAAATATCCAATAACACTAAAAAATTA
AAAGAAATGGATAATTTCACAATATGTTATACGATAAAGAAGTTACTTTTCCAA
GAAATTCACTGATTTTATAAGCCCACTTGCATTAGATAAATGGCAAAAAAAAAC
AAAAAGGAAAAGAAATAAAGCACGAAGAATTCTAGAAAATACGAAATACGCTT
CAATGCAGTGGGACCCACGGTTCAATTATTGCCAATTTTCAGCTCCACCGTAT
ATTTAAAAAATAAAACGATAATGCTAAAAAAATATAAATCGTAACGATCGTTAA
ATCTCAACGGCTGGATCTTATGACGACCGTTAGAAATTGTGGTTGTCGACGA
GTCAGTAATAAACGGCGTCAAAGTGGTTGCAGCCGGCACACACGAGTCGTGT
TTATCAACTCAAAGCACAAATACTTTTCCTCAACCTAAAAATAAGGCAATTAGC
CAAAAACAACTTTGCGTGTAAACAACGCTCAATACACGTGTCATTTTATTATTA
GCTATTGCTTCACCGCCTTAGCTTTCTCGTGACCTAGTCGTCCTCGTCTTTTC
TTCTTCTTCTTCTATAAAACAATACCCAAAGAGCTCTTCTTCTTCACAATTCAG
ATTTCAATTTCTCAAAATCTTAAAAACTTTCTCTCAATTCTCTCTACCGTGATCA
AGGTAAATTTCTGTGTTCCTTATTCTCTCAAAATCTTCGATTTTGTTTTCGTTCG
ATCCCAATTTCGTATATGTTCTTTGGTTTAGATTCTGTTAATCTTAGATCGAAC
ACGATTTTCTGGGTTTGATCGTTAGATATCATCTTAATTCTCGATTAGGGTTTC
ATAGATATCATCCGATTTGTTCAAATAATTTGAGTTTTGTCGAATAATTACTCTT
CGATTTGTGATTTCTATCTAGATCTGGTGTTAGTTTCTAGTTTGTGCGATCGAA
TTTGTCGATTAATCTGAGTTTTTCTGATTAACAGG
Col1A1 in pET151
   SEQ ID NO: 33: 
TAAAAGGGCGAGCTCAGATCCGGCTGCTAACAAAGCCCGAAAGGAAGCTGA
GTTGGCTGCTGCCACCGCTGAGCAATAACTAGCATAACCCCTTGGGGCCTCT
AAACGGGTCTTGAGGGGTTTTTTGCTGAAAGGAGGAACTATATCCGGATATC
CCGCAAGAGGCCCGGCAGTACCGGCATAACCAAGCCTATGCCTACAGCATC
CAGGGTGACGGTGCCGAGGATGACGATGAGCGCATTGTTAGATTTCATACAC
GGTGCCTGACTGCGTTAGCAATTTAACTGTGATAAACTACCGCATTAAAGCTA
GCTTATCGATGATAAGCTGTCAAACATGAGAATTAATTCTTGAAGACGAAAGG
GCCTCGTGATACGCCTATTTTTATAGGTTAATGTCATGATAATAATGGTTTCTT
 
AGACGTCAGGTGGCACTTTTCGGGGAAATGTGCGCGGAACCCCTATTTGTTT
ATTTTTCTAAATACATTCAAATATGTATCCGCTCATGAGACAATAACCCTGATA
AATGCTTCAATAATATTGAAAAAGGAAGAGTATGAGTATTCAACATTTCCGTGT
CGCCCTTATTCCCTTTTTTGCGGCATTTTGCCTTCCTGTTTTTGCTCACCCAGA
AACGCTGGTGAAAGTAAAAGATGCTGAAGATCAGTTGGGTGCACGAGTGGGT
TACATCGAACTGGATCTCAACAGCGGTAAGATCCTTGAGAGTTTTCGCCCCG
AAGAACGTTTTCCAATGATGAGCACTTTTAAAGTTCTGCTATGTGGCGCGGTA
TTATCCCGTGTTGACGCCGGGCAAGAGCAACTCGGTCGCCGCATACACTATT
CTCAGAATGACTTGGTTGAGTACTCACCAGTCACAGAAAAGCATCTTACGGAT
GGCATGACAGTAAGAGAATTATGCAGTGCTGCCATAACCATGAGTGATAACA
CTGCGGCCAACTTACTTCTGACAACGATCGGAGGACCGAAGGAGCTAACCG
CTTTTTTGCACAACATGGGGGATCATGTAACTCGCCTTGATCGTTGGGAACC
GGAGCTGAATGAAGCCATACCAAACGACGAGCGTGACACCACGATGCCTGC
AGCAATGGCAACAACGTTGCGCAAACTATTAACTGGCGAACTACTTACTCTAG
CTTCCCGGCAACAATTAATAGACTGGATGGAGGCGGATAAAGTTGCAGGACC
ACTTCTGCGCTCGGCCCTTCCGGCTGGCTGGTTTATTGCTGATAAATCTGGA
GCCGGTGAGCGTGGGTCTCGCGGTATCATTGCAGCACTGGGGCCAGATGGT
AAGCCCTCCCGTATCGTAGTTATCTACACGACGGGGAGTCAGGCAACTATGG
ATGAACGAAATAGACAGATCGCTGAGATAGGTGCCTCACTGATTAAGCATTG
GTAACTGTCAGACCAAGTTTACTCATATATACTTTAGATTGATTTAAAACTTCA
TTTTTAATTTAAAAGGATCTAGGTGAAGATCCTTTTTGATAATCTCATGACCAA
AATCCCTTAACGTGAGTTTTCGTTCCACTGAGCGTCAGACCCCGTAGAAAAGA
TCAAAGGATCTTCTTGAGATCCTTTTTTTCTGCGCGTAATCTGCTGCTTGCAAA
CAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTG
TCCTTCTAGTGTAGCCGTAGTTAGGCCACCACTTCAAGAACTCTGTAGCACC
GCCTACATACCTCGCTCTGCTAATCCTGTTACCAGTGGCTGCTGCCAGTGGC
GATAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAG
CGAACGACCTACACCGAACTGAGATACCTACAGCGTGAGCTATGAGAAAGCG
CCACGCTTCCCGAAGGGAGAAAGGCGGACAGGTATCCGGTAAGCGGCAGG
GTCGGAACAGGAGAGCGCACGAGGGAGCTTCCAGGGGGAAACGCCTGGTA
TCTTTATAGTCCTGTCGGGTTTCGCCACCTCTGACTTGAGCGTCGATTTTTGT
GATGCTCGTCAGGGGGGCGGAGCCTATGGAAAAACGCCAGCAACGCGGCCT
TTTTACGGTTCCTGGCCTTTTGCTGGCCTTTTGCTCACATGTTCTTTCCTGCG
TTATCCCCTGATTCTGTGGATAACCGTATTACCGCCTTTGAGTGAGCTGATAC
CGCTCGCCGCAGCCGAACGACCGAGCGCAGCGAGTCAGTGAGCGAGGAAG
CGGAAGAGCGCCTGATGCGGTATTTTCTCCTTACGCATCTGTGCGGTATTTC
 
ACACCGCAATGGTGCACTCTCAGTACAATCTGCTCTGATGCCGCATAGTTAA
GCCAGTATACACTCCGCTATCGCTACGTGACTGGGTCATGGCTGCGCCCCGA
CACCCGCCAACACCCGCTGACGCGCCCTGACGGGCTTGTCTGCTCCCGGCA
TCCGCTTACAGACAAGCTGTGACCGTCTCCGGGAGCTGCATGTGTCAGAGGT
TTTCACCGTCATCACCGAAACGCGCGAGGCAGCTGCGGTAAAGCTCATCAGC
GTGGTCGTGAAGCGATTCACAGATGTCTGCCTGTTCATCCGCGTCCAGCTCG
TTGAGTTTCTCCAGAAGCGTTAATGTCTGGCTTCTGATAAAGCGGGCCATGTT
AAGGGCGGTTTTTTCCTGTTTGGTCACTGATGCCTCCGTGTAAGGGGGATTT
CTGTTCATGGGGGTAATGATACCGATGAAACGAGAGAGGATGCTCACGATAC
GGGTTACTGATGATGAACATGCCCGGTTACTGGAACGTTGTGAGGGTAAACA
ACTGGCGGTATGGATGCGGCGGGACCAGAGAAAAATCACTCAGGGTCAATG
CCAGCGCTTCGTTAATACAGATGTAGGTGTTCCACAGGGTAGCCAGCAGCAT
CCTGCGATGCAGATCCGGAACATAATGGTGCAGGGCGCTGACTTCCGCGTTT
CCAGACTTTACGAAACACGGAAACCGAAGACCATTCATGTTGTTGCTCAGGT
CGCAGACGTTTTGCAGCAGCAGTCGCTTCACGTTCGCTCGCGTATCGGTGAT
TCATTCTGCTAACCAGTAAGGCAACCCCGCCAGCCTAGCCGGGTCCTCAACG
ACAGGAGCACGATCATGCGCACCCGTGGCCAGGACCCAACGCTGCCCGAGA
TGCGCCGCGTGCGGCTGCTGGAGATGGCGGACGCGATGGATATGTTCTGCC
AAGGGTTGGTTTGCGCATTCACAGTTCTCCGCAAGAATTGATTGGCTCCAATT
CTTGGAGTGGTGAATCCGTTAGCGAGGTGCCGCCGGCTTCCATTCAGGTCG
AGGTGGCCCGGCTCCATGCACCGCGACGCAACGCGGGGAGGCAGACAAGG
TATAGGGCGGCGCCTACAATCCATGCCAACCCGTTCCATGTGCTCGCCGAG
GCGGCATAAATCGCCGTGACGATCAGCGGTCCAATGATCGAAGTTAGGCTG
GTAAGAGCCGCGAGCGATCCTTGAAGCTGTCCCTGATGGTCGTCATCTACCT
GCCTGGACAGCATGGCCTGCAACGCGGGCATCCCGATGCCGCCGGAAGCG
AGAAGAATCATAATGGGGAAGGCCATCCAGCCTCGCGTCGCGAACGCCAGC
AAGACGTAGCCCAGCGCGTCGGCCGCCATGCCGGCGATAATGGCCTGCTTC
TCGCCGAAACGTTTGGTGGCGGGACCAGTGACGAAGGCTTGAGCGAGGGC
GTGCAAGATTCCGAATACCGCAAGCGACAGGCCGATCATCGTCGCGCTCCA
GCGAAAGCGGTCCTCGCCGAAAATGACCCAGAGCGCTGCCGGCACCTGTCC
TACGAGTTGCATGATAAAGAAGACAGTCATAAGTGCGGCGACGATAGTCATG
CCCCGCGCCCACCGGAAGGAGCTGACTGGGTTGAAGGCTCTCAAGGGCATC
GGTCGAGATCCCGGTGCCTAATGAGTGAGCTAACTTACATTAATTGCGTTGC
GCTCACTGCCCGCTTTCCAGTCGGGAAACCTGTCGTGCCAGCTGCATTAATG
AATCGGCCAACGCGCGGGGAGAGGCGGTTTGCGTATTGGGCGCCAGGGTG
GTTTTTCTTTTCACCAGTGAGACGGGCAACAGCTGATTGCCCTTCACCGCCT
GGCCCTGAGAGAGTTGCAGCAAGCGGTCCACGCTGGTTTGCCCCAGCAGGC
GAAAATCCTGTTTGATGGTGGTTAACGGCGGGATATAACATGAGCTGTCTTC
 
GGTATCGTCGTATCCCACTACCGAGATATCCGCACCAACGCGCAGCCCGGA
CTCGGTAATGGCGCGCATTGCGCCCAGCGCCATCTGATCGTTGGCAACCAG
CATCGCAGTGGGAACGATGCCCTCATTCAGCATTTGCATGGTTTGTTGAAAAC
CGGACATGGCACTCCAGTCGCCTTCCCGTTCCGCTATCGGCTGAATTTGATT
GCGAGTGAGATATTTATGCCAGCCAGCCAGACGCAGACGCGCCGAGACAGA
ACTTAATGGGCCCGCTAACAGCGCGATTTGCTGGTGACCCAATGCGACCAGA
TGCTCCACGCCCAGTCGCGTACCGTCTTCATGGGAGAAAATAATACTGTTGA
TGGGTGTCTGGTCAGAGACATCAAGAAATAACGCCGGAACATTAGTGCAGGC
AGCTTCCACAGCAATGGCATCCTGGTCATCCAGCGGATAGTTAATGATCAGC
CCACTGACGCGTTGCGCGAGAAGATTGTGCACCGCCGCTTTACAGGCTTCGA
CGCCGCTTCGTTCTACCATCGACACCACCACGCTGGCACCCAGTTGATCGGC
GCGAGATTTAATCGCCGCGACAATTTGCGACGGCGCGTGCAGGGCCAGACT
GGAGGTGGCAACGCCAATCAGCAACGACTGTTTGCCCGCCAGTTGTTGTGC
CACGCGGTTGGGAATGTAATTCAGCTCCGCCATCGCCGCTTCCACTTTTTCC
CGCGTTTTCGCAGAAACGTGGCTGGCCTGGTTCACCACGCGGGAAACGGTC
TGATAAGAGACACCGGCATACTCTGCGACATCGTATAACGTTACTGGTTTCAC
ATTCACCACCCTGAATTGACTCTCTTCCGGGCGCTATCATGCCATACCGCGA
AAGGTTTTGCGCCATTCGATGGTGTCCGGGATCTCGACGCTCTCCCTTATGC
GACTCCTGCATTAGGAAGCAGCCCAGTAGTAGGTTGAGGCCGTTGAGCACC
GCCGCCGCAAGGAATGGTGCATGCAAGGAGATGGCGCCCAACAGTCCCCCG
GCCACGGGGCCTGCCACCATACCCACGCCGAAACAAGCGCTCATGAGCCCG
AAGTGGCGAGCCCGATCTTCCCCATCGGTGATGTCGGCGATATAGGCGCCA
GCAACCGCACCTGTGGCGCCGGTGATGCCGGCCACGATGCGTCCGGCGTA
GAGGATCGAGATCTCGATCCCGCGAAATTAATACGACTCACTATAGGGGAAT
TGTGAGCGGATAACAATTCCCCTCTAGAAATAATTTTGTTTAACTTTAAGAAGG
AGATATACATATGCATCATCACCATCACCATGGTAAGCCTATCCCTAACCCTC
TCCTCGGTCTCGATTCTACGGAAAACCTGTATTTTCAGGGACAATTGAGTTAT
GGATACGACGAAAAATCAACTGGAATATCAGTGCCAGGACCAATGGGTCCAT
CAGGACCTAGGGGTCTTCCAGGCCCACCTGGCGCTCCTGGTCCTCAGGGAT
TTCAAGGTCCTCCAGGTGAACCTGGAGAGCCTGGTGCTTCTGGTCCAATGGG
ACCAAGAGGCCCTCCTGGTCCACCTGGTAAAAATGGTGATGATGGTGAGGCT
GGAAAGCCTGGTCGTCCTGGAGAAAGAGGACCACCAGGACCACAGGGAGCT
AGAGGTTTGCCAGGTACAGCCGGTCTTCCAGGAATGAAAGGACACAGGGGT
TTTTCTGGTCTTGATGGTGCTAAGGGAGACGCAGGACCTGCTGGACCAAAGG
GTGAACCTGGATCTCCTGGTGAAAATGGTGCTCCTGGACAAATGGGACCTAG
AGGATTGCCTGGCGAAAGAGGAAGACCTGGTGCTCCTGGACCAGCTGGAGC
TAGAGGAAATGATGGAGCTACTGGTGCTGCCGGACCACCAGGACCAACTGG
ACCAGCTGGACCACCTGGTTTTCCAGGAGCAGTTGGGGCTAAGGGAGAAGG
 
TGGACCACAAGGACCTAGAGGATCAGAAGGACCTCAAGGAGTTAGGGGAGA
ACCAGGTCCTCCTGGTCCTGCTGGAGCTGCTGGACCCGCTGGTAACCCAGG
AGCAGATGGACAACCTGGAGCAAAAGGGGCTAATGGTGCTCCTGGTATTGCA
GGTGCTCCTGGATTCCCTGGTGCTAGAGGACCTTCAGGACCTCAGGGACCAT
CCGGTCCTCCAGGACCAAAGGGAAACTCTGGTGAACCAGGGGCTCCTGGAT
CTAAGGGAGATACTGGAGCTAAGGGTGAGCCAGGACCTACAGGAATTCAAG
GACCACCAGGACCTGCAGGAGAGGAAGGTAAGAGGGGTGCTAGAGGTGAAC
CTGGACCAGCTGGATTGCCAGGCCCACCTGGAGAGAGGGGTGGTCCAGGTT
CTAGAGGTTTTCCAGGAGCTGATGGAGTTGCTGGTCCAAAGGGTCCTGCTGG
TGAGAGAGGTGCACCTGGACCAGCAGGACCTAAAGGATCACCTGGAGAAGC
TGGTCGTCCTGGTGAGGCTGGTCTTCCAGGTGCTAAGGGCCTTACAGGATCT
CCAGGATCACCAGGTCCAGATGGAAAGACTGGACCACCAGGTCCTGCAGGA
CAAGATGGAAGGCCTGGTCCACCTGGACCACCTGGTGCTAGGGGACAAGCT
GGTGTTATGGGATTCCCAGGTCCAAAGGGTGCAGCTGGTGAGCCTGGTAAA
GCTGGTGAAAGAGGAGTTCCAGGACCTCCTGGTGCTGTTGGACCTGCTGGT
AAAGATGGTGAAGCAGGTGCTCAGGGTCCACCAGGTCCAGCCGGTCCAGCT
GGTGAGAGGGGAGAGCAAGGTCCTGCTGGATCTCCTGGATTTCAAGGTTTG
CCTGGACCTGCTGGACCTCCAGGAGAGGCTGGTAAACCTGGTGAGCAGGGT
GTGCCTGGTGATCTTGGTGCTCCTGGACCTAGTGGCGCAAGAGGCGAAAGG
GGATTTCCAGGTGAAAGAGGAGTTCAGGGACCACCAGGTCCAGCTGGTCCA
AGAGGCGCTAATGGAGCTCCAGGAAATGATGGAGCAAAGGGTGATGCTGGC
GCTCCAGGAGCTCCTGGTTCTCAAGGTGCACCTGGATTGCAAGGAATGCCAG
GAGAGAGAGGTGCAGCTGGTTTGCCGGGACCAAAGGGTGATAGAGGTGATG
CTGGTCCTAAGGGAGCTGATGGAGCTCCAGGAAAGGATGGTGTGAGAGGAT
TGACTGGTCCAATTGGACCACCAGGTCCTGCTGGAGCACCAGGAGATAAAG
GAGAAGCTGGTCCTTCAGGACCAGCCGGTCCAACTGGAGCTAGAGGAGCTC
CAGGAGATAGAGGTGAACCAGGACCACCAGGACCTGCTGGGTTTGCAGGTC
CACCTGGTGCTGATGGACAACCTGGTGCTAAAGGAGAACCAGGCGATGCTG
GTGCTAAGGGAGATGCTGGCCCACCTGGTCCAGCTGGTCCTGCTGGGCCAC
CAGGTCCTATCGGAAATGTTGGTGCTCCAGGGCCTAAAGGAGCTAGAGGATC
AGCAGGTCCCCCAGGTGCTACAGGTTTTCCAGGGGCTGCTGGAAGAGTGGG
TCCCCCAGGTCCTTCTGGGAATGCAGGACCACCAGGTCCTCCAGGACCAGC
TGGAAAAGAAGGAAGTAAGGGACCAAGGGGAGAAACTGGACCTGCAGGACG
TCCAGGTGAAGTTGGTCCACCTGGACCTCCAGGTCCAGCTGGAGAGAAAGG
AGCTCCAGGAGCTGATGGTCCAGCAGGTGCTCCTGGTACTCCAGGTCCACA
AGGTATTGCTGGTCAAAGGGGAGTGGTTGGTCTCCCAGGTCAGAGAGGTGA
AAGAGGTTTCCCAGGTTTGCCTGGACCATCTGGAGAACCAGGTAAGCAGGG
CCCATCTGGAGCTTCTGGTGAGAGAGGGCCACCTGGACCTATGGGACCTCC
 
TGGGCTTGCTGGACCTCCAGGGGAATCTGGTAGGGAGGGTGCTCCAGGTGC
TGAAGGATCACCTGGTAGAGATGGATCACCTGGAGCTAAAGGTGATAGAGG
GGAAACAGGTCCAGCAGGTCCACCTGGCGCTCCTGGTGCTCCAGGAGCACC
AGGGCCAGTTGGACCTGCTGGAAAGTCTGGAGATAGGGGAGAGACTGGACC
AGCTGGACCTGCTGGACCAATCGGTCCTGTTGGCGCTAGAGGTCCTGCTGG
ACCACAAGGTCCTAGGGGAGATAAAGGTGAAACAGGTGAACAAGGAGATAG
AGGAATTAAAGGTCATAGAGGGTTTTCTGGTTTGCAAGGACCTCCAGGACCT
CCTGGATCTCCAGGAGAGCAAGGACCATCTGGTGCAAGCGGACCTGCTGGT
CCTAGAGGACCTCCAGGTTCAGCTGGCTCACCTGGTAAGGATGGATTAAATG
GTTTGCCTGGACCAATAGGTCCACCAGGACCTAGAGGAAGGACAGGTGATG
CAGGACCAGCTGGTCCACCTGGTCCACCAGGACCACCAGGACCACCAGGTC
CACCTTCTGGTGGATATGATCTTAGTTTTCTTCCTCAACCTCCCCAGGAGAAA
GCTCATGATGGTGGTAGATATTATAGGGCT
Bovine Col1A1 expression vector VB
   SEQ ID NO: 34:

CAACTTTGTATAGAAAAGTTGAGATCTAACATCCAAAGACGAAAGGTTGAATG
AAACCTTTTTGCCATCCGACATCCACAGGTCCATTCTCACACATAAGTGCCAA
ACGCAACAGGAGGGGATACACTAGCAGCAGACCGTTGCAAACGCAGGACCT
CCACTCCTCTTCTCCTCAACACCCACTTTTGCCATCGAAAAACCAGCCCAGTT
ATTGGGCTTGATTGGAGCTCGCTCATTCCAATTCCTTCTATTAGGCTACTAAC
ACCATGACTTTATTAGCCTGTCTATCCTGGCCCCCCTGGCGAGGTTCATGTTT
GTTTATTTCCGAATGCAACAAGCTCCGCATTACACCCGAACATCACTCCAGAT
GAGGGCTTTCTGAGTGTGGGGTCAAATAGTTTCATGTTCCCCAAATGGCCCA
AAACTGACAGTTTAAACGCTGTCTTGGAACCTAATATGACAAAAGCGTGATCT
CATCCAAGATGAACTAAGTTTGGTTCGTTGAAATGCTAACGGCCAGTTGGTCA
AAAAGAAACTTCCAAAAGTCGGCATACCGTTTGTCTTGTTTGGTATTGATTGA
CGAATGCTCAAAAATAATCTCATTAATGCTTAGCGCAGTCTCTCTATCGCTTCT
GAACCCCGGTGCACCTGTGCCGAAACGCAAATGGGGAAACACCCGCTTTTT
GGATGATTATGCATTGTCTCCACATTGTATGCTTCCAAGATTCTGGTGGGAAT
ACTGCTGATAGCCTAACGTTCATGATCAAAATTTAACTGTTCTAACCCCTACTT
GACAGCAATATATAAACAGAAGGAAGCTGCCCTGTCTTAAACCTTTTTTTTTAT
CATCATTATTAGCTTACTTTCATAATTGCGACTGGTTCCAATTGACAAGCTTTT
GATTTTAACGACTTTTAACGACAACTTGAGAAGATCAAAAAACAACTAATTATT
CGAAACGACAAGTTTGTACAAAAAAGCAGGCTGCCACCATGAGATTTCCTTCA
ATTTTTACTGCTGTTTTATTCGCAGCATCCTCCGCATTAGCTGCTCCAGTCAA
CACTACAACAGAAGATGAAACGGCACAAATTCCGGCTGAAGCTGTCATCGGT
TACTCAGATTTAGAAGGGGATTTCGATGTTGCTGTTTTGCCATTTTCCAACAG

CACAAATAACGGGTTATTGTTTATAAATACTACTATTGCCAGCATTGCTGCTAA
AGAAGAAGGGGTATCTCTCGAGAAAAGAGAGGCTGAAGCTCATCACCATCAC
CATCACGAAAACCTGTATTTTCAGGGCCAATTGTCTTATGGTTACGATGAAAA
GTCCACTGGTATTTCTGTCCCTGGACCAATGGGTCCATCAGGTCCTAGAGGT
TTGCCAGGTCCTCCAGGTGCTCCAGGTCCACAAGGTTTTCAAGGTCCACCAG
GTGAACCAGGAGAACCCGGTGCTTCTGGTCCAATGGGTCCAAGAGGTCCAC
CTGGACCTCCTGGTAAGAACGGTGATGATGGTGAGGCTGGTAAGCCAGGAA
GACCTGGAGAAAGAGGACCACCTGGTCCACAAGGAGCTAGAGGTTTACCCG
GTACAGCTGGTTTGCCTGGTATGAAGGGTCATAGAGGTTTCTCCGGTTTGGA
TGGAGCTAAGGGTGATGCTGGACCTGCTGGTCCTAAGGGAGAGCCAGGATC
TCCAGGTGAGAACGGAGCTCCTGGACAAATGGGACCAAGAGGTCTGCCTGG
TGAAAGAGGTAGACCAGGTGCTCCTGGTCCTGCCGGAGCCAGAGGTAATGA
TGGTGCTACTGGTGCTGCTGGTCCTCCAGGTCCAACTGGTCCTGCTGGTCCT
CCAGGTTTTCCAGGTGCTGTTGGAGCCAAAGGTGAAGGTGGCCCACAAGGT
CCTAGAGGATCTGAAGGTCCACAGGGTGTTAGAGGTGAACCAGGTCCACCA
GGTCCAGCTGGAGCTGCTGGTCCTGCTGGTAACCCAGGTGCAGATGGTCAA
CCAGGTGCTAAGGGTGCCAACGGTGCTCCAGGCATTGCTGGAGCTCCAGGT
TTTCCAGGAGCTAGAGGTCCTTCTGGACCTCAAGGACCATCTGGTCCACCTG
GACCAAAAGGTAACTCTGGAGAACCAGGTGCTCCTGGTTCCAAAGGTGATAC
CGGTGCTAAAGGAGAGCCAGGTCCTACTGGTATTCAAGGTCCTCCTGGTCCT
GCTGGTGAAGAGGGTAAAAGAGGAGCCAGAGGTGAACCTGGTCCAGCCGGT
TTGCCAGGTCCACCAGGTGAAAGAGGAGGTCCAGGATCTAGAGGTTTTCCAG
GTGCTGACGGTGTTGCTGGTCCAAAGGGTCCTGCTGGTGAGAGAGGAGCCC
CAGGTCCAGCTGGACCTAAAGGATCTCCAGGAGAAGCTGGTCGTCCTGGTG
AAGCTGGTCTTCCAGGTGCTAAGGGTTTGACAGGCTCCCCAGGATCCCCTGG
TCCAGATGGTAAGACTGGACCACCTGGACCAGCTGGTCAAGATGGAAGACC
AGGTCCTCCAGGACCACCTGGAGCCAGAGGCCAAGCTGGTGTTATGGGTTT
CCCTGGTCCAAAAGGTGCTGCTGGAGAACCGGGTAAAGCCGGTGAAAGAGG
TGTTCCAGGTCCTCCAGGTGCTGTTGGTCCTGCTGGTAAAGATGGAGAAGCC
GGTGCTCAAGGACCACCAGGTCCAGCTGGTCCAGCTGGAGAGAGAGGTGAA
CAAGGTCCTGCTGGATCCCCAGGTTTCCAAGGTTTGCCTGGTCCTGCTGGAC
CACCAGGTGAAGCTGGTAAGCCAGGTGAACAAGGAGTTCCAGGTGATTTGG
GTGCTCCTGGTCCATCCGGTGCTAGAGGTGAAAGAGGATTTCCAGGAGAGA
GAGGTGTTCAAGGACCACCTGGTCCTGCTGGACCAAGAGGAGCAAACGGTG
CTCCAGGAAACGATGGTGCTAAAGGTGATGCTGGTGCTCCAGGAGCTCCAG
GTTCACAAGGTGCTCCAGGTTTGCAAGGAATGCCTGGAGAAAGAGGTGCTG
CTGGATTACCAGGTCCAAAAGGAGACAGAGGTGATGCCGGTCCAAAGGGTG
CTGATGGTGCTCCAGGTAAGGACGGTGTGCGAGGTTTGACTGGACCAATTG

GACCTCCAGGTCCAGCTGGAGCCCCAGGAGATAAGGGTGAAGCTGGTCCAT
CGGGTCCTGCTGGTCCTACTGGTGCTAGAGGAGCTCCAGGAGATAGAGGTG
AACCTGGTCCACCTGGACCCGCTGGATTTGCTGGTCCACCAGGTGCTGATG
GTCAACCAGGGGCTAAAGGTGAACCAGGTGACGCTGGAGCTAAAGGAGATG
CCGGTCCACCAGGTCCAGCCGGTCCAGCTGGTCCTCCTGGTCCAATTGGTA
ATGTTGGCGCTCCTGGTCCTAAGGGTGCCAGAGGTTCAGCTGGACCACCAG
GTGCTACTGGTTTCCCAGGTGCTGCCGGTAGAGTTGGTCCACCAGGTCCATC
TGGTAACGCTGGTCCTCCAGGTCCACCAGGTCCTGCTGGAAAGGAGGGATC
TAAGGGACCAAGAGGTGAAACTGGTCCAGCTGGAAGACCTGGTGAAGTTGG
TCCTCCAGGACCACCAGGTCCTGCTGGAGAAAAAGGTGCTCCAGGTGCTGAT
GGACCAGCTGGTGCTCCTGGTACCCCAGGTCCACAAGGTATTGCTGGTCAAA
GAGGTGTGGTTGGTCTTCCTGGACAAAGAGGTGAGAGAGGTTTTCCTGGTTT
GCCAGGTCCTTCTGGAGAACCTGGAAAACAGGGTCCCTCTGGTGCTTCTGGT
GAGAGAGGTCCTCCAGGACCAATGGGACCTCCAGGTTTGGCTGGTCCACCA
GGTGAGTCTGGTAGAGAGGGTGCTCCAGGTGCTGAGGGTTCTCCAGGTAGA
GATGGTAGTCCAGGTGCTAAAGGAGACAGAGGAGAGACTGGTCCAGCTGGT
CCTCCAGGTGCTCCTGGTGCTCCCGGTGCTCCTGGTCCTGTCGGTCCTGCT
GGAAAGTCTGGTGATAGAGGTGAGACTGGTCCAGCTGGACCAGCTGGTCCA
ATCGGACCAGTTGGAGCTAGAGGTCCTGCTGGTCCTCAAGGTCCAAGAGGT
GACAAGGGTGAAACAGGTGAGCAAGGTGACAGAGGTATTAAAGGACATAGA
GGTTTCTCTGGTCTACAGGGACCTCCTGGTCCTCCAGGTTCCCCAGGTGAGC
AAGGTCCTTCTGGTGCTTCAGGTCCTGCTGGTCCAAGAGGTCCACCAGGTTC
TGCTGGTTCTCCAGGAAAGGATGGTTTGAATGGTTTGCCTGGACCTATTGGT
CCTCCTGGACCTAGAGGTAGAACAGGTGATGCTGGTCCTGCTGGTCCACCTG
GTCCTCCTGGTCCTCCTGGTCCCCCTGGACCTCCATCTGGAGGTTACGATTT
GTCTTTTTTGCCACAACCTCCACAGGAAAAAGCCCATGACGGTGGAAGATATT
ATAGAGCTTAAACCCAGCTTTCTTGTACAAAGTGGTGTTTGTAGCCTTAGACA
TGACTGTTCCTCAGTTCAAGTTGGGCACTTACGAGAAGACCGGTCTTGCTAG
ATTCTAATCAAGAGGATGTCAGAATGCCATTTGCCTGAGAGATGCAGGCTTCA
TTTTTGATACTTTTTTATTTGTAACCTATATAGTATAGGATTTTTTTTGTCATTTT
GTTTCTTCTCGTACGAGCTTGCTCCTGATCAGCCTATCTCGCAGCTGATGAAT
ATCTTGTGGTAGGGGTTTGGGAAAATCATTCGAGTTTGATGTTTTTCTTGGTA
TTTCCCACTCCTCTTCAGAGTACAGAAGATTAAGTGAGACCTTCGTTTGTGCG
GATCCCCCACACACCATAGCTTCAAAATGTTTCTACTCCTTTTTTACTCTTCCA
GATTTTCTCGGACTCCGCGCATCGCCGTACCACTTCAAAACACCCAAGCACA
GCATACTAAATTCCCCCTCTTTCTTCCTCTAGGGTGTCGTTAATTACCCGTACT
AAAGGTTTGGAAAAGAAAAAAGAGACCGCCTCGTTTCTTTTTCTTCGTCGAAA
AAGGCAATAAAAATTTTTATCACGTTTCTTTTTCTTGAAAATTTTTTTTTTTGATT

TTTTTCTCTTTCGATGACCTCCCATTGATATTTAAGTTAATAAATGGTCTTCAAT
TTCTCAAGTTTCAGTTTCATTTTTCTTGTTCTATTACAACTTTTTTTACTTCTTGC
TCATTAGAAAGAAAGCATAGCAATCTAATCTAAGGGCGGTGTTGACAATTAAT
CATCGGCATAGTATATCGGCATAGTATAATACGACAAGGTGAGGAACTAAAC
CATGGCCAAGTTGACCAGTGCCGTTCCGGTGCTCACCGCGCGCGACGTCGC
CGGAGCGGTCGAGTTCTGGACCGACCGGCTCGGGTTCTCCCGGGACTTCGT
GGAGGACGACTTCGCCGGTGTGGTCCGGGACGACGTGACCCTGTTCATCAG
CGCGGTCCAGGACCAGGTGGTGCCGGACAACACCCTGGCCTGGGTGTGGG
TGCGCGGCCTGGACGAGCTGTACGCCGAGTGGTCGGAGGTCGTGTCCACGA
ACTTCCGGGACGCCTCCGGGCCGGCCATGACCGAGATCGGCGAGCAGCCG
TGGGGGCGGGAGTTCGCCCTGCGCGACCCGGCCGGCAACTGCGTGCACTT
CGTGGCCGAGGAGCAGGACTGACACGTCCGACGCGGCCCGACGGGTCCGA
GGCCTCGGAGATCCGTCCCCCTTTTCCTTTGTCGATATCATGTAATTAGTTAT
GTCACGCTTACATTCACGCCCTCCCCCCACATCCGCTCTAACCGAAAAGGAA
GGAGTTAGACAACCTGAAGTCTAGGTCCCTATTTATTTTTTTATAGTTATGTTA
GTATTAAGAACGTTATTTATATTTCAAATTTTTCTTTTTTTTCTGTACAGACGCG
TGTACGCATGTAACATTATACTGAAAACCTTGCTTGAGAAGGTTTTGGGACGC
TCGAAGGCTTTAATTTGCAAGCTGGAGACCAACATGTGAGCAAAAGGCCAGC
AAAAGGCCAGGAACCGTAAAAAGGCCGCGTTGCTGGCGTTTTTCCATAGGCT
CCGCCCCCCTGACGAGCATCACAAAAATCGACGCTCAAGTCAGAGGTGGCG
AAACCCGACAGGACTATAAAGATACCAGGCGTTTCCCCCTGGAAGCTCCCTC
GTGCGCTCTCCTGTTCCGACCCTGCCGCTTACCGGATACCTGTCCGCCTTTC
TCCCTTCGGGAAGCGTGGCGCTTTCTCATAGCTCACGCTGTAGGTATCTCAG
TTCGGTGTAGGTCGTTCGCTCCAAGCTGGGCTGTGTGCACGAACCCCCCGTT
CAGCCCGACCGCTGCGCCTTATCCGGTAACTATCGTCTTGAGTCCAACCCGG
TAAGACACGACTTATCGCCACTGGCAGCAGCCACTGGTAACAGGATTAGCAG
AGCGAGGTATGTAGGCGGTGCTACAGAGTTCTTGAAGTGGTGGCCTAACTAC
GGCTACACTAGAAGAACAGTATTTGGTATCTGCGCTCTGCTGAAGCCAGTTA
CCTTCGGAAAAAGAGTTGGTAGCTCTTGATCCGGCAAACAAACCACCGCTGG
TAGCGGTGGTTTTTTTGTTTGCAAGCAGCAGATTACGCGCAGAAAAAAAGGAT
CTCAAGAAGATCCTTTGATCTTTTCTACGGGGTCTGACGCTCAGTGGAACGAA
AACTCACGTTAAGGGATTTTGGTCATGAGATC

**Table 1. Further sequences that may be used in the context of the present invention as collagen as expressed by a non-animal organism**

| **Name** | **Description** | **Sequence length** | **Accession** | **Genetic Code** | **Molecule Type** |
|---|---|---|---|---|---|
| 2X ARE | 2X antioxidant response element (ARE) from Mus musculus glutathione S-transferase | 82 | JQ858521.1 | Bacterial | DNA |
| 2X p53 RE | 2X p53 response element | 58 | JQ858522.1 | Bacterial | DNA |
| 3X Sp1 | 3 binding sites for transcriptional factor Sp1 | 48 | AF104248.3 | Bacterial | DNA |
| 3X FLAG | 3X FLAG tag used for protein detection and purification | 22 | AGU99855.1 | Bacterial | AA |
| 3X NFAT RE | 3X nuclear factor of activated T-cells (NFAT) response element | 90 | DQ904462.1 | Bacterial | DNA |
| 3X SBE | 3X Smad-binding element (SBE) | 48 | JQ858517.1 | Bacterial | DNA |
| 3X XRE | 3X xenobiotic response element (XRE) | 63 | JQ858513.1 | Bacterial | DNA |
| 3' AcPH | 3' end fragment of Polyhedrin gene in Autographa californica nucleopolyhedrovirus (AcMNPV) | 102 | LT727492.1 | Standard | DNA |
| 3' AOX1 | 3' end region of AOX1 from Pichia pastoris | 750 | AY178634.1 | Standard | DNA |
| AcPH 3' flank | 3' flanking sequence of Autographa californica Multiple Nucleopolyhedrovirus (AcMNPV) polyhedrin gene | 1434 | JN029539.1 | Standard | DNA |
| 3' β-globin insulator | 3' insulator/UTR of Homo sapiens β-globin gene | 72 | NG_042165.1 | Standard | DNA |
| 3' LTR | 3' long terminal repeat (LTR) from HIV-1 | 634 | MH325104.1 | Standard | DNA |
| 3' LTR (ΔU3) | 3' long terminal repeat (LTR) from HIV-1 (self-inactivating) | 234 | EU048697.1 | Standard | DNA |
| MESV 3' LTR | 3' long terminal repeat (LTR) from murine embryonic stem cell virus (MESV) | 515 | LT726944.1 | Standard | DNA |
| BYDV 3' TE | 3' translation enhancer sequence from barley yellow dwarf virus (BYDV) | 107 | AY349044.1 | Standard | DNA |
| HIS3 3'UTR | 3' UTR and termination region of HIS3 | 640 | AY061966.1 | Bacterial | DNA |
| cspA 3' UTR | 3' UTR from Escherichia coli cold shock protein CspA | 145 | AB213654.1 | Bacterial | DNA |
| Elk13' UTR | 3' UTR of Homo sapiens Elk1 transcription factor | 1311 | AB016193.1 | Standard | DNA |
| ADE2 3' UTR | 3' UTR of S. cerevisiae ADE2 | 483 | M58324.1 | Standard | DNA |
| 4X CRE | 4X cyclic AMP response element (CRE) | 99 | KY025563.1 | Bacterial | DNA |
| 4X HSE | 4X heat shock response element (HSE) | 41 | JQ858520.1 | Bacterial | DNA |
| 4X HRE | 4X hypoxia response element (HRE) | 76 | JQ858518.1 | Bacterial | DNA |
| 5X gal4 DBD | 5 17nt GAL4 binding elements - upstream activating sequence (UAS) | 95 | KF545600.1 | Standard | DNA |
| 5X ATF6 RE | 5X ATF6 response element | 135 | JQ858519.1 | Bacterial | DNA |
| 5X SRE | 5X c-fos serum response element (SRE) | 115 | FJ773212.1 | Bacterial | DNA |
| 5X MRE | 5X metal response element (MRE) | 75 | JQ858515.1 | Bacterial | DNA |
| 5X NF-κB RE | 5X nuclear factor kappa-light-chain-enhancer of activated B cells (NF-κB) response element | 52 | JQ513377.1 | Bacterial | DNA |
| 5X SIE | 5X sis-inducible element (SIE) | 125 | JQ858512.1 | Bacterial | DNA |
| AcPH 5' flank | 5' flanking sequence of Autographa californica Multiple Nucleopolyhedrovirus (AcMNPV) polyhedrin gene | 1250 | JN029539.1 | Standard | DNA |
| 5' β-globin insulator | 5' insulator/UTR of Homo sapiens β-globin gene | 72 | NG_052895.1 | Standard | DNA |
| MESV 5' LTR | 5' long terminal repeat (LTR) from murine embryonic stem cell virus (MESV) | 516 | DQ465352.1 | Standard | DNA |
| 5' LTR | 5' LTR containing CMV (cytomegalovirus) promoter | 727 | AF311737.1 | Standard | DNA |
| 5' LTR2 | 5' LTR containing viral promoter | 592 | LT726818.1 | Standard | DNA |
| BYDV 5' UTR | 5' UTR from barley yellow dwarf virus (BYDV) | 137 | AY349044.1 | Standard | DNA |
| cspA 5' UTR | 5' UTR from Escherichia coli cold shock protein CspA | 134 | AB213654.1 | Bacterial | DNA |
| TE5'UTR | 5' UTR from tobacco etch virus used as a translational enhancer | 142 | DQ370427.1 | Standard | DNA |
| Elk15' UTR | 5' UTR of Homo sapiens Elk1 transcription factor | 100 | AB016193.1 | Standard | DNA |
| ADE2 | 5' UTR of S. cerevisiae ADE2 (promoter region) | 504 | M58324.1 | Standard | DNA |
| HSV TK 5' UTR | 5' UTR region from the herpes simplex virus thymidine kinase gene | 40 | MG711910.1 | Standard | DNA |
| 6X AP-1 RE | 6X AP-1 response element | 48 | | Bacterial | DNA |
| polyhistidine | 6X polyhistidine tag used for protein purification | 6 | 1KTR_P | Standard | AA |
| 8X TCF/LEF RE | 8X TCF/LEF response element | 120 | JX099537.1 | Bacterial | DNA |
| 12X ZFHD1 BS | 12 binding sites for the composite human DNA-binding domain ZFHD1 | 210 | | Standard | DNA |
| repE | 29kD protein essential for replication of mini F plasmid | 756 | AZP53506.1 | Bacterial | DNA |
| CaMV35S | 35S promoter sequence from cauliflower mosaic virus (CaMV) | 420 | MH450173.1 | Standard | DNA |
| CaMV 35S | 35S terminator sequence from Cauliflower mosaic virus (CaMV) | 212 | MG687282.1 | Standard | DNA |
| h β 1,4-GT | 81aa amino terminal of Homo sapiens beta-1,4-galactosyltransferase for Golgi localization | 81 | XP_005251497.1 | Standard | AA |
| 186 int | 186 phage integrase - enables integration of genetic material | 1011 | AGN12530.1 | Bacterial | DNA |
| amdS | acetamidase from Aspergillus nidulans | 1647 | XP_682046.1 | Standard | DNA |
| AsCpf1 | Acidaminococcus sp. type V CRISPR-associated protein | 3957 | ATB19153.1 | Bacterial | DNA |
| ATF AD | activation domain of activating transcription factor | 290 | KJ890967.1 | Bacterial | DNA |
| CREB AD | Activation domain of cyclic AMP-responsive element-binding protein (CREB) | 365 | XP_013376146.1 | Standard | AA |
| ACP | acyl carrier protein tag used for protein labelling | 78 | AAC24154.1 | Bacterial | AA |
| Ad5 left | Ad5 homology arm (left) - facilitates homologous recombination | 902 | AF334399.1 | Bacterial | DNA |
| Ad5 right | Ad5 homology arm (right) - facilitates homologous recombination | 1999 | AF334399.1 | Bacterial | DNA |
| AAV-2 cap | Adeno-associated virus 2 capsid protein used for transfection | 2208 | AAK76419.1 | Standard | DNA |
| AAV-2 rep | Adeno-associated virus 2 replication proteins | 1866 | AAK76418.1 | Standard | DNA |
| AAV2 ITR | adeno-associated virus (AAV-2) inverted terminal repeat (ITR) region required for genome multiplication | 141 | MN224160.1 | Standard | DNA |
| 22K | adenoviral 22K protein | 585 | AZP56160.1 | Bacterial | DNA |
| 33K | adenoviral 33K protein | 684 | AZP56159.1 | Bacterial | DNA |
| 52K | adenoviral 52K protein | 1248 | AZP56149.1 | Bacterial | DNA |
| 100K | adenoviral 100K protein | 2424 | AZP56158.1 | Bacterial | DNA |
| DBP | adenoviral DNA-binding protein (DBP) | 1590 | AZP56157.1 | Bacterial | DNA |
| E2B pTP 75K | adenoviral E2B pTP 75K protein | 1962 | AZP56148.1 | Bacterial | DNA |
| E3 12.5K | adenoviral E3 12.5k protein | 324 | AZP56162.1 | Bacterial | DNA |
| E4 ORF 1 | adenoviral E4 ORF 1 | 387 | AZP56171.1 | Bacterial | DNA |
| E4 ORF 3 | adenoviral E4 ORF 3 | 351 | AZP56169.1 | Bacterial | DNA |
| E4 ORF 3/4 | adenoviral E4 ORF 3/4 | 186 | AZP56167.1 | Bacterial | DNA |
| E4 ORF 4 | adenoviral E4 ORF 4 | 345 | AZP56168.1 | Bacterial | DNA |
| E4 ORF 6 | adenoviral E4 ORF 6 protein | 885 | AZP56166.1 | Bacterial | DNA |
| E4 ORF 6/7 | adenoviral E4 ORF 6/7 protein | 453 | AZP56165.1 | Bacterial | DNA |
| E4 ORFB | adenoviral E4 ORFB | 393 | AZP56170.1 | Bacterial | DNA |
| IVa2 | adenoviral IVa2 | 1350 | AZP56146.1 | Bacterial | DNA |
| L2 piII penton | adenoviral L2 piII penton capsid protein | 1716 | AZP56151.1 | Bacterial | DNA |
| L3 pII hexon | adenoviral L3 pII hexon capsid protein | 2859 | AZP56156.1 | Bacterial | DNA |
| L5 pIV fiber | adenoviral L5 pIV fiber protein | 1746 | AZP56164.1 | Bacterial | DNA |
| pIIIa | adenoviral pIIIa protein | 1758 | AZP56150.1 | Bacterial | DNA |
| pIX | adenoviral PIX cement protein | 423 | AZP56145.1 | Bacterial | DNA |
| PIX | adenoviral PIX promoter | 33 | MH325116.1 | Bacterial | DNA |
| pVI | adenoviral pVI protein | 753 | AZP56155.1 | Bacterial | DNA |
| pVII | adenoviral pVII protein | 597 | AZP56152.1 | Bacterial | DNA |
| pVIII | adenoviral pVIII protein | 684 | AZP56161.1 | Bacterial | DNA |
| pX | adenoviral pX protein | 243 | AZP56154.1 | Bacterial | DNA |
| V | adenoviral V protein | 1107 | AZP56153.1 | Bacterial | DNA |
| AdV E2A | Adenovirus E2A DNA-binding protein required for replication | 1590 | AFS50244.1 | Standard | DNA |
| virB11 | Agrobacterium tumefaciens Ti plasmid ATPase VirB11 involved in T-DNA transfer to plants | 1032 | AAZ50528.1 | Bacterial | DNA |
| virB3 | Agrobacterium tumefaciens Ti plasmid VirB3 protein involved in T-DNA transfer to plant cells | 327 | AAZ50520.1 | Bacterial | DNA |
| virB5 | Agrobacterium tumefaciens Ti plasmid VirB5 protein involved in T-DNA transfer to plant cells | 663 | AAZ50522.1 | Bacterial | DNA |
| virB6 | Agrobacterium tumefaciens Ti plasmid VirB6 protein involved in T-DNA transfer to plant cells | 888 | AAZ50523.1 | Bacterial | DNA |
| virB7 | Agrobacterium tumefaciens Ti plasmid VirB7 protein required for T-pilus biogenesis | 168 | AAZ50524.1 | Bacterial | DNA |
| virB8 | Agrobacterium tumefaciens Ti plasmid VirB8 protein involved in T-DNA transfer to plant cells | 714 | AAZ50525.1 | Bacterial | DNA |
| virB9 | Agrobacterium tumefaciens Ti plasmid VirB9 protein required for T-pilus biogenesis | 882 | AAZ50526.1 | Bacterial | DNA |
| virB10 | Agrobacterium tumefaciens Ti plasmid VirB10 protein involved in T-DNA transfer to plant cells | 1134 | AAZ50527.1 | Bacterial | DNA |
| virJ | Agrobacterium tumefaciens Ti plasmid VirJ protein | 744 | AAZ50516.1 | Bacterial | DNA |
| virK | Agrobacterium tumefaciens Ti plasmid VirK protein | 438 | AAZ50511.1 | Bacterial | DNA |
| MAS | Agrobactrium tumefaciens mannopine synthase promoter | 382 | KY825159.1 | Standard | DNA |
| ABP | albumin-binding protein tag | 52 | 2N35_A | Standard | AA |
| ScADH1 | Alcohol dehydrogenase 1 (ADH1) promoter from S. cerevisiae | 403 | KY131987.1 | Standard | DNA |
| SpADH1 | Alcohol dehydrogenase 1 (ADH1) promoter from S. pombe | 742 | NM_001023234.2 | Standard | DNA |
| yADH2 | Alcohol dehydrogenase 1 (ADH1) terminator 2 region from Saccharomyces cerevisiae | 327 | FJ696408.1 | Standard | DNA |
| yADH1 | Alcohol dehydrogenase 1 (ADH1) terminator region from Saccharomyces cerevisiae | 193 | U57443.1 | Standard | DNA |
| AP | alkaline phosphatase tag used for protein expression | 471 | AEI59074.1 | Bacterial | AA |
| lacZα | alpha fragment of lacZ from LITMUS28i. Used for alpha complementation alongside omega fragment (usually in host) | 372 | AGS09126.1 | Bacterial | DNA |
| lacZα | alpha fragment of lacZ from pAZILcos. Used for alpha complementation alongside omega fragment (usually in host) | 507 | CCB84606.1 | Bacterial | DNA |
| lacZα | alpha fragment of lacZ from pBLUESCRIBE vector. Used for alpha complementation alongside omega fragment (usually in host) | 384 | AWX67510.1 | Bacterial | DNA |
| lacZα | alpha fragment of lacZ from pDN19. Used for alpha complementation alongside omega fragment (usually in host) | 267 | AAK73367.1 | Bacterial | DNA |
| lacZα | alpha fragment of lacZ from pELS100. Used for alpha complementation alongside omega fragment (usually in host) | 381 | AAS77680.1 | Bacterial | DNA |
| lacZα | alpha fragment of lacZ from pJAZZ-OC. Used for alpha complementation alongside omega fragment (usually in host) | 273 | ABQ45977.1 | Bacterial | DNA |
| lacZα | alpha fragment of lacZ from pSpcP-lac. Used for alpha complementation alongside omega fragment (usually in host) | 363 | AAQ08405.1 | Bacterial | DNA |
| lacZα | alpha fragment of lacZ from pTZ19U. Used for alpha complementation alongside omega fragment (usually in host) | 477 | CAA75108.1 | Bacterial | DNA |
| lacZα | alpha fragment of lacZ from pUC19 cloning vector. Used for alpha complementation alongside omega fragment (usually in host) | 324 | AWX67510 | Bacterial | DNA |
| lacZα | alpha fragment of lacZ from pUvBBAC. Used for alpha complementation alongside omega fragment (usually in host) | 381 | CAD50590.1 | Bacterial | DNA |
| lacZα | alpha fragment of lacZ from pVv3. Used for alpha complementation alongside omega fragment (usually in host) | 468 | CDG15339.1 | Bacterial | DNA |
| lacZa | alpha fragment of lacZ GenBank: J01636 derived from Escherichia coli for alpha complementation in Escherichia coli blue/white screen; uninterrupted by a MCS | 231 | AII99647.1 | Bacterial | DNA |
| lacZα | alpha fragment of lacZ. Used for alpha complementation alongside omega fragment (usually in host) | 480 | AFQ39733.1 | Bacterial | DNA |
| alpha | alpha-factor N-terminal secretion signal from Kluyveromyces lactis | 255 | XM_454814.1 | Standard | DNA |
| AMA1 | AMA1 origin of replication - required for Aspergillus replication | 5721 | KT031986.1 | Bacterial | DNA |
| AOX1 | AOX1 promoter and 5' UTR | 941 | EU285586.1 | Bacterial | DNA |
| HSP | Arabidopsis thaliana HSP18.2 termination sequence | 250 | LC217877.1 | Standard | DNA |
| U6-26 | Arabidopsis thaliana U6-26 snRNA promoter region | 450 | MF375491.1 | Standard | DNA |
| araO1 | Arabinose operator 1 region - involved in arabinose operon regulation | 12 | MH101733.0 | Bacterial | DNA |
| araO2 | Arabinose operator 2 region - involved in arabinose operon regulation | 17 | MH101733.1 | Bacterial | DNA |
| aurR | Aspergillus nidulans aureobasidin-resistance protein | 1320 | AAD22749.1 | Standard | DNA |
| ptrR | Aspergillus oryzae pyrithiamine-resistance protein | 984 | XP_001819323.1 | Standard | DNA |
| polyaspartate | asp-tag (polyaspartate) used for protein immobilization and purification | 5 | | Standard | AA |
| virC1 | ATPase from Agrobacterium tumefaciens Ti plasmid | 696 | AAZ50531.1 | Bacterial | DNA |
| virB4 | ATPase involved in transferring T-DNA-protein complexes across the Agrobacterium tumefaciens membrane | 2370 | AAZ50521.1 | Bacterial | DNA |
| 186 attP | attachment site of 186 phage | 307 | KF030466.1 | Bacterial | DNA |
| HK022 attP | attachment site of HK022 phage | 258 | KF030457.1 | Bacterial | DNA |
| λ attP | attachment site of λ phage | 387 | KF030461.1 | Bacterial | DNA |
| ϕ21 attP | attachment site of ϕ21 phage | 567 | KF030467.1 | Bacterial | DNA |
| ϕ31 attP | attachment site of ϕ31 phage | 258 | KX278432.1 | Bacterial | DNA |
| AU1 | AU1 epitope used for protein detection and purification | 6 | | Standard | AA |
| AU5 | AU5 epitope used for protein detection and purification | 6 | | Standard | AA |
| hr5 | Autographa californica hr5 enhancer | 483 | KC991096.1 | Standard | DNA |
| gp64 | Autographa californica nucleopolyhedrovirus gp64 promoter region | 100 | KY792989.1 | Standard | DNA |
| IE0 | Autographa californica nucleopolyhedrovirus IE0 promoter region | 355 | KY792989.1 | Standard | DNA |
| IE-1 | Autographa californica nucleopolyhedrovirus IE-1 promoter region | 592 | KY792989.1 | Standard | DNA |
| IE-1 | Autographa californica nucleopolyhedrovirus IE-1 termination region | 307 | MG051710.1 | Standard | DNA |
| p6.9 | Autographa californica nucleopolyhedrovirus p6.9 promoter region | 316 | KY792989.1 | Standard | DNA |
| p10 | Autographa californica nucleopolyhedrovirus p10 promoter region | 99 | KY792989.1 | Standard | DNA |
| PH | Autographa californica nucleopolyhedrovirus polyhedrin promoter region | 92 | JN029539.1 | Standard | DNA |
| ars1 | Autonomously replicating sequence 1 (ars1) - site where DNA replication is initiated | 1206 | KT033769.1 | Bacterial | DNA |
| B42 AD | B42 activation domain used for transcription activation | 94 | AAB68652.1 | Bacterial | AA |
| sacB | Bacillus subtilis sacB (glycoside hydrolase family 68) promoter region | 446 | MF287370.1 | Standard | DNA |
| HBH | bacterial biotinylation signal flanked by 6X His tags | 88 | | Standard | AA |
| repB | bacterial repB replication initiator protein. Essential for replication | 1005 | AYA22195.1 | Bacterial | DNA |
| CRE | bacteriophage P1 recombinase that targets loxP sequences | 1047 | BAV82214.1 | Bacterial | DNA |
| T4-32 | bacteriophage T4 gene 32 transcription terminator | 32 | MF510148.1 | Bacterial | DNA |
| T7 | bacteriophage T7 tag used for protein purification and expression | 11 | ABS70473.1 | Bacterial | AA |
| T7 | bacteriophage T7 terminator | 48 | KC991095.1 | Bacterial | DNA |
| V5 | bacteriophage V5 tag | 14 | | Bacterial | AA |
| basic protein | baculovirus basic protein promoter, allowing expression during late viral infection | 332 | EF050536.1 | Standard | DNA |
| lef2 | baculovirus late expression factor 2 | 633 | ARJ58692.1 | Standard | DNA |
| barnase | barnase bacterial ribonuclease from Bacillus amyloliquefaciens - lethal without expression of barstar inhibitor | 336 | AAV87646.1 | Bacterial | DNA |
| bom | basis of mobility (bom) from pBR322 | 140 | LC318058.1 | Bacterial | DNA |
| BioBrick Prefix | BioBrick prefix - attached to 5' end of a BioBrick sequence | 22 | MH492453.1 | Bacterial | DNA |
| BioBrick Suffix | BioBrick suffix - attached to 3' end of a BioBrick sequence | 21 | MH492453.1 | Bacterial | DNA |
| BirA | biotin ligase and biotin-operator repressor from Escherichia coli | 966 | QGQ68377.1 | Bacterial | DNA |
| BCCP | biotin-carboxy carrier protein tag used for protein detection and purification | 67 | AAA89090.1 | Bacterial | AA |
| AviTag^{™} | biotinylation tag used for protein purification | 15 | QBQ88493.1 | Bacterial | AA |
| EBFP2 | Blue fluorescent reporter protein - Ex:383, Em:448 | 720 | ABP88743.1 | Bacterial | DNA |
| Azurite | Blue fluorescent reporter protein - Ex:383, Em:450 | 735 | ABK79091.1 | Bacterial | DNA |
| mKalama1 | Blue fluorescent reporter protein - Ex:385, Em:456 | 720 | ABP88742 | Bacterial | DNA |
| mTagBFP2 | Blue fluorescent reporter protein - Ex:399, Em:454 | 702 | AIQ82697.1 | Bacterial | DNA |
| TagBFP | Blue fluorescent reporter protein - Ex:402, Em:457 | 693 | AIZ65962.1 | Bacterial | DNA |
| BoBS | Bunch of baby spinach (BoBS) fluorescent RNA | 129 | | | RNA |
| hsp16-48 | Caenorhabditis elegans heat shock protein 16-48a promoter region | 305 | K03273.1 | Standard | DNA |
| CBP | calmodulin binding peptide (CBP) used for protein purification and expression | 26 | AAC71016.1 | Bacterial | AA |
| CAP BS | catabolite activator protein binding site | 22 | MK816965.1 | Bacterial | DNA |
| 35SPPDK | cauliflower mosaic virus 35S enhancer and maize C4PPDK hybrid promoter | 557 | EF090408.1 | Standard | DNA |
| eCaMV 35S | cauliflower mosaic virus 35S promoter with a duplicated enhancer region | 809 | MG719602.1 | Standard | DNA |
| c-Ha-Ras farnesylation | cell membrane localisation signal | 63 | JN717245.1 | Bacterial | DNA |
| CBD | cellulose binding domain from Cellulomonas fimi | 100 | AAA23084.1 | Bacterial | AA |
| CBD | cellulose binding domain from Cellulomonas fimi | 97 | AAA23084.1 | Bacterial | AA |
| CBD | cellulose binding domain from Cellulomonas fimi | 99 | AAA23086.1 | Bacterial | AA |
| CBD | cellulose binding domain from Cellulomonas fimi | 73 | AAA23086.1 | Bacterial | AA |
| CBD | cellulose binding domain from Cellulomonas fimi | 125 | CAA40993.1 | Bacterial | AA |
| CBD | cellulose binding domain from Cellulomonas fimi | 118 | CAA40993.1 | Bacterial | AA |
| CEN6 ARS4 | CEN6 centromere fused to an autonomously replicating sequence (ARS) used to maintain low plasmid copy number | 504 | MG833229.1 | Standard | DNA |
| CEN4 | centromere of Saccharomyces cerevisiae chromosome IV | 111 | MK416190.1 | Standard | DNA |
| sopC | centromere partitioning particle used in F plasmids | 703 | AY643800.1 | Bacterial | DNA |
| cer | cer recombination site - converts multimers to monomers in multicopy plasmids | 286 | AY349044.1 | Standard | DNA |
| virE1 | chaperone protein for VirE2 from Agrobacterium tumefaciens Ti plasmid involved in DNA transfer | 198 | AAZ50537.1 | Bacterial | DNA |
| HAT | chicken lactate dehydrogenase histidine affinity tag (HAT) used for protein purification | 19 | P00340 | Standard | AA |
| chimeric | chimera of human β-globin and immunoglobulin heavy chain genes | 133 | KX830961.1 | Standard | DNA |
| CBD | chitin-binding protein used for protein purification | 51 | AAD49604.1 | Bacterial | AA |
| gypsy | chromatin insulator from Drosophila | 431 | MK424968.1 | Standard | DNA |
| CilioKozak | Ciliate kozak sequence | 12 | | Ciliate | DNA |
| cis oriR-RepA | CIS element needed for cis-activation of oriR by the RepA protein | 171 | L05669.1 | Bacterial | DNA |
| HIV-1 ψ | cis-acting HIV-1 psi packaging sequence | 126 | MK318529.1 | Standard | DNA |
| CDF | CloDF13 (CDF) origin of replication | 739 | MH473345.1 | Bacterial | DNA |
| colA | colA origin of replication | 1033 | M12574.1 | Bacterial | DNA |
| colA | colA origin of replication | 636 | M37402.1 | Bacterial | DNA |
| ampR | confers ampicillin resistance | 861 | WP_000027060.1 | Standard | DNA |
| blastR | confers blasticidin resistance | 402 | ABG81367.1 | Bacterial | DNA |
| blastR | confers blasticidin resistance | 423 | ACG80842.1 | Bacterial | DNA |
| catR | Confers chloramphenicol resistance - also used as a reporter | 660 | CAJ00344.1 | Bacterial | DNA |
| erythR | confers erythromycin resistance | 735 | AEQ67353.1 | Bacterial | DNA |
| gentR | confers gentamicin resistance | 534 | AAB60000.1 | Bacterial | DNA |
| hygR | confers hygromycin resistance | 1035 | AAB49979.1 | Bacterial | DNA |
| kanR | confers kanamycin resistance | 795 | ATE88998.1 | Bacterial | DNA |
| kanR | confers kanamycin resistance | 816 | QFQ66226.1 | Bacterial | DNA |
| puroR | confers puromycin resistance | 600 | AAF01142.1 | Bacterial | DNA |
| puroR | confers puromycin resistance | 633 | BAM95187.1 | Bacterial | DNA |
| apmR | confers resistance to apramycin | 777 | AFD02329.1 | Bacterial | DNA |
| blpR | confers resistance to bialophos and phosphinothricin | 552 | AHG97684.1 | Bacterial | DNA |
| zeoR/bleoR | confers resistance to bleomycin, phleomycin and zeocin | 372 | AFV14773.1 | Bacterial | DNA |
| CAH | confers resistance to cyanamide herbicides | 735 | AAA33429.1 | Standard | DNA |
| neoR | confers resistance to neomycin and kanamycin | 804 | AAL78958.1 | Bacterial | DNA |
| nrsR | confers resistance to nourseothricin | 576 | ABL09005.1 | Bacterial | DNA |
| specR/strepR | confers resistance to spectinomycin and streptomycin | 1011 | QBF76421.1 | Bacterial | DNA |
| tetR | confers resistance to tetracycline | 1191 | AAA73378.1 | Bacterial | DNA |
| tetR | confers resistance to tetracycline | 1200 | WP_011645018.1 | Bacterial | DNA |
| tetAR | confers resistance to tetracycline, but sensitivity to fusaric and quinalic acids - from Tn10 region of Salmonella enterica | 669 | YP_009061935.1 | Bacterial | DNA |
| PAT | confers resistance to the herbicicde glufosinate | 552 | AAA72709.1 | Bacterial | DNA |
| bxn | confers resistance to the herbicide bromoxynil | 1050 | AAA25057.1 | Bacterial | DNA |
| tetL | confers tetracycline resistance | 1377 | ALT14586.1 | Bacterial | DNA |
| virC2 | conjugal transfer protein from Agrobacterium tumefaciens Ti plasmid | 609 | AAZ50530.1 | Bacterial | DNA |
| traJ | conjugal transfer transcriptional regulator | 372 | AAA91588.1 | Bacterial | DNA |
| AdV E4 | Contains multiple E4 ORFs - required for viral transcription | 3201 | AF369965.1 | Bacterial | DNA |
| CopA | CopA antisense RNA inhibits translation of RepA indirectly through tap and prevents initiation of plasmid replication | 91 | L05669.1 | Bacterial | DNA |
| CopB | CopB trascriptional repressor that regulates RNA II synthesis - frameshifted, so increases copy number | 265 | L05669.1 | Bacterial | DNA |
| pheS CS | counterselectable marker - introduces p-chlorophenylalanine sensitivity | 984 | AAB61946.1 | Bacterial | DNA |
| lacY CS | counterselectable marker - introduces t-o-nitrophenyl-b-D-galactopyranoside sensitivity | 417 | AMC97587.1 | Bacterial | AA |
| thyA CS | counterselectable marker from Bacillus subtilis - introduces ganciclovir and trimethoprim susceptibility | 795 | AAA22852.1 | Bacterial | DNA |
| thyA CS | counterselectable marker from Bacillus subtilis - introduces ganciclovir and trimethoprim susceptibility | 840 | CAA55307.1 | Bacterial | DNA |
| sacB CS | counterselectable marker from Bacillus subtilis that converts sucrose to levans | 1419 | AAA73429.1 | Bacterial | DNA |
| sacB CS | counterselectable marker from Bacillus subtilis that converts sucrose to levans | 1422 | QDK64795.1 | Bacterial | DNA |
| ccdB CS | counterselectable marker from Escherichia coli - inhibits bacterial gyrase (inhibited by ccdA) | 306 | ACA62826.1 | Standard | DNA |
| thyA CS | counterselectable marker from Escherichia coli - introduces ganciclovir and trimethoprim susceptibility | 795 | AAA24675.1 | Bacterial | DNA |
| gata-1 CS | counterselectable marker from Mus musculus - inhibits initiation of bacterial replication | 1242 | NP_032115.1 | Bacterial | DNA |
| strep CS | counterselectable marker that reintroduces sensitivity to streptomycin resistance | 375 | KFA84265.1 | Bacterial | DNA |
| CP4 | CP4 gene from Agrobacterium sp.that confers resistance to glyphosate herbicides | 1368 | AII71485.1 | Bacterial | DNA |
| CRE NLS | CRE recombinase that contains a nuclear localization signal | 1056 | AAK11472.1 | Bacterial | DNA |
| gRNA | CRISPR/Cas9 system guide RNA scaffold | 76 | MH037009.1 | Bacterial | DNA |
| hCas9 | CRISPR/Cas system Type II associated protein, contains McrA/HNH and RuvC-like nuclease domains [Defense mechanisms]; COG3513 | 4140 | ASG92122.1 | Bacterial | DNA |
| C | C-tag used for protein purification | 4 | LP889097.1 | Standard | AA |
| shBFP | Cyan fluorescent reporter protein - Ex:401 Em:458 | 228 | 6JC5 | Standard | AA |
| SCFP3A | Cyan fluorescent reporter protein - Ex:433, Em:474 | 720 | AAZ65848.1 | Bacterial | DNA |
| Cerulean | Cyan fluorescent reporter protein - Ex:433, Em:475 | 720 | AXC07678.1 | Bacterial | DNA |
| ECFP | Cyan fluorescent reporter protein - Ex:433, Em:475 | 720 | AMO27223.1 | Bacterial | DNA |
| mCerulean3 | Cyan fluorescent reporter protein - Ex:433, Em:475 | 720 | ATP07149.1 | Bacterial | DNA |
| mTurquoise | Cyan fluorescent reporter protein - Ex:434, Em:474 | 720 | AEL12175.1 | Bacterial | DNA |
| mTurquoise2 | Cyan fluorescent reporter protein - Ex:434, Em:474 | 720 | ATP60648.1 | Bacterial | DNA |
| CyPet | Cyan fluorescent reporter protein - Ex:435, Em:477 | 801 | AEH43768.1 | Bacterial | DNA |
| AmCyan | Cyan fluorescent reporter protein - Ex:453, Em:486 | 690 | ALQ43923.1 | Bacterial | DNA |
| TagCFP | Cyan fluorescent reporter protein - Ex:458, Em:480 | 717 | ADK12945.1 | Bacterial | DNA |
| mTFP1 | Cyan fluorescent reporter protein - Ex:462, Em:492 | 717 | AKA95303.1 | Bacterial | DNA |
| MiCy | Cyan fluorescent reporter protein - Ex:470, Em:496 | 720 | BAG31928.1 | Bacterial | DNA |
| CYC1 | CYC1 transcription termination region | 319 | AB379557.1 | Bacterial | DNA |
| GFP cyc3 | Cycle3 mutant of green fluorescent protein (GFP) | 744 | AAX31732.1 | Bacterial | DNA |
| cLuc | Cypridina noctiluca secreted luciferase | 1662 | BAD08210.1 | Standard | DNA |
| CMV | cytomegalovirus (CMV) enhancer sequence | 380 | MH107059.1 | Standard | DNA |
| CMV | cytomegalovirus (CMV) enhancer sequence | 204 | K03104.1 | Standard | DNA |
| hCyto β-actin | Cytoskeletal actin from H. sapiens used as a control in PCR and Western blotting | 1128 | CAA25099.1 | Standard | DNA |
| hPEST | degradation sequence from Mus musculus ornithine decarboxylase PEST | 120 | AFI79291.1 | Bacterial | DNA |
| LbCpf1 | derived from Lachnospiraceae bacterium ND2006 (LbCpf1); optimized for expression in S. cerevisiae | 3720 | ATB19154.1 | Bacterial | DNA |
| d1EGFP | Destabilized green fluorescent reporter protein - Ex:488, Em:509 | 840 | ACU30028.1 | Bacterial | DNA |
| d2EYFP | Destabilized yellow fluorescent reporter protein | 846 | AGJ84355.1 | Bacterial | DNA |
| DHFR | Dihydrofolate reductase (DHFR) tag from Mus musculus used for increased protein expression | 187 | NP_034179.1 | Standard | AA |
| lexA DBD | DNA-binding domain of transcriptional repressor LexA | 693 | AAB68649.1 | Bacterial | DNA |
| DS | double stranded replication origin | 21 | AB042431.1 | Bacterial | DNA |
| BiP signal | Drosophila binding protein (BiP) signal sequence for ER localization | 54 | AM408495.1 | Bacterial | DNA |
| vermillion | Drosophila eye-colour gene vermillion (tryptophan oxygenase) | 1880 | MK424968.1 | Standard | DNA |
| Ac5 | Drosophila melanogaster actin 5c promoter region | 2452 | X15730.1 | Standard | DNA |
| MT | Drosophila melanogaster metallothionein promoter | 427 | KF444903.1 | Standard | DNA |
| dU6-1 | dU6-1 promoter region | 796 | MK908408.1 | Bacterial | DNA |
| dU6 | dU6 promoter region | 400 | KU212289.1 | Bacterial | DNA |
| CaMVd35S | Dual 35S promoter sequence from cauliflower mosaic virus (CaMV) | 755 | AY995145.1 | Standard | DNA |
| E2 | E2 epitope tag used for protein detection and purification | 12 | Icosagen | Standard | AA |
| E2 | E2 epitope tag used for protein detection and purification | 10 | Icosagen | Standard | AA |
| E | E epitope tag used for protein analysis and visualisation | 13 | BAT46708.1 | Bacterial | AA |
| EtoL | Early to late promoter from Autographa californica multiple nucleopolyhedrovirus (AcMNPV) | 275 | LT727224.1 | Standard | DNA |
| E/GRE | ecdysone/glucocorticoid response element | 13 | | Standard | DNA |
| eco47IR | Eco47I restriction endonuclease that recognizes the double-stranded sequence GGWCC | 738 | ABR53874.1 | Bacterial | DNA |
| Glu-Glu | EE tag used for protein detection and purification | 6 | | Standard | AA |
| gapdh | Eggerthella lenta glyceraldehyde-3-phosphate dehydrogenase promoter region | 285 | CP001726.1 | Bacterial | DNA |
| Elk1 | ELK1 transcription factor that binds to purine-rich DNA sequences from Homo sapiens | 1287 | BAA36616.1 | Standard | DNA |
| EM7 | EM7 promoter derived from T7 | 48 | MH325105.1 | Bacterial | DNA |
| VDE | Endonuclease PI-Scel/VDE (VMA1-derived endonuclease) - See VMA intein | 37 | U75992.1 | Bacterial | DNA |
| rhoB | Endosomal targeting - ras homolog gene family, member B (RhoB) | 591 | CAA29968.1 | Standard | DNA |
| EGFP-F | Enhanced green fluorescent protein (EGFP) with membrane-targeted farnesylation modification | 795 | AEO31555.1 | Bacterial | DNA |
| EGFP | Enhanced green fluorescent reporter protein - Ex:488, Em:507 | 717 | AAF62891.1 | Standard | DNA |
| EYFP | Enhanced yellow/green fluorescent reporter protein - Ex:513, Em:527 | 720 | AAX97736.1 | Bacterial | DNA |
| EBNA1 | Epstein-Barr nuclear antigen 1, also known as EBNA-1 | 1926 | YP_401677.1 | Standard | DNA |
| EBV | Epstein-Barr virus (EBV) origin of replication | 2183 | U75992.1 | Bacterial | DNA |
| EBV oriP | Epstein-Barr virus (EBV) oriP | 1772 | LT727609.1 | Bacterial | DNA |
| calreticulin targeting | ER targeting signal | 51 | AB451408.1 | Standard | DNA |
| LexA | Escherichia coli DNA-binding protein that represses genes involved in the SOS response to DNA damage | 609 | ACI73966.1 | Bacterial | DNA |
| tnpA | Escherichia coli insertion sequence IS10 TnpA | 1209 | AAS67729.1 | Bacterial | DNA |
| EcCI | Escherichia coli promoter and CI binding site | 41 | U75992.1 | Bacterial | DNA |
| DsbA | Escherichia coli protein required for disulfide bond formation for some periplasmic proteins | 627 | ACI74741.1 | Bacterial | DNA |
| recA (ΔLexA) | Escherichia coli recA promoter lacking the LexA binding site | 69 | CP041300.1 | Bacterial | DNA |
| NusA | Escherichia coli transcription termiantion/antitermination protein | 1488 | QGL42391.1 | Bacterial | DNA |
| ileX | Escherichia coli tRNA-Ile recognizing AUA codons | 76 | CP041359.1 | Bacterial | DNA |
| F1 | f1 replication origin derived from bacteriophage f1 | 456 | MN019114.1 | Bacterial | DNA |
| DmrD | F36M mutant of FK506-binding protein FKBP12 from Homo sapiens | 107 | 1EYM_A | Standard | AA |
| DmrB | F36V mutant of Homo sapiens FK506-binding protein FKBP12 | 327 | XP_012970645.2 | Standard | DNA |
| VP22 | facilitates virus spread | 906 | QAU09774.1 | Standard | DNA |
| factor XA site | factor Xa recognition and cleavage site | 4 | | Standard | AA |
| mKate2 | Far red fluorescent reporter protein - Ex:588, Em:633 | 699 | AIZ66133.1 | Bacterial | DNA |
| mPlum | Far red fluorescent reporter protein - Ex:590, Em:649 | 681 | AMO27242.1 | Bacterial | DNA |
| mRaspberry | Far red fluorescent reporter protein - Ex:598, Em:625 | 681 | AAV65486.1 | Standard | DNA |
| mNeptune | Far red fluorescent reporter protein - Ex:600, Em:650 | 735 | CBH32884.1 | Bacterial | DNA |
| mCardinal | Far red fluorescent reporter protein - Ex:604, Em:659 | 735 | AHL19967.1 | Bacterial | DNA |
| mMaroon1 | Far red fluorescent reporter protein - Ex:609, Em:657 | 735 | AOY07814.1 | Bacterial | DNA |
| crimson | Far red fluorescent reporter protein - Ex:611, Em:646 | 717 | AMO27221.1 | Bacterial | DNA |
| fd | fd terminator sequence derived from bactriophage fd | 51 | E03514.1 | Standard | DNA |
| fLuc | Firefly luciferase bioluminescent reporter | 1653 | AFE85520.1 | Bacterial | DNA |
| FKBP DD | FKBP-derived destabilizing domain that leads to protein degradation | 324 | APM86966.1 | Bacterial | DNA |
| FLAG | FLAG epitope tag for protein purification | 8 | ACH81550.1 | Bacterial | AA |
| FRT | Flp recombinase target site used for site-directed recombination | 48 | MK356269.1 | Bacterial | DNA |
| Baby spinach | Fluorescent RNA | 51 | | | RNA |
| Broccoli | Fluorescent RNA | 49 | | | RNA |
| Corn | Fluorescent RNA | 36 | 5BJP_E | | RNA |
| Mango | Fluorescent RNA | 31 | 5V3F_A | | RNA |
| Orange broccoli | Fluorescent RNA | 49 | | | RNA |
| Red broccoli | Fluorescent RNA | 49 | | | RNA |
| Spinach | Fluorescent RNA | 98 | | | RNA |
| Spinach2 | Fluorescent RNA | 95 | | | DNA |
| TEF1 | fragment containing translation elongation factor EF-1 alpha (TEF1) promoter sequence | 412 | MH142259.1 | Bacterial | DNA |
| Fc | fragment crystallizable (Fc) tag used for protein purification (from human IgG1) | 232 | AEV43323.1 | Standard | AA |
| Tn3R | Fragment of Tn3 tnpR resolvase gene | 491 | MH325469.1 | Bacterial | DNA |
| OLLAS | Fusion between the Escherichia coli OmpF linker and Mus musculus langerin | 14 | AFS33207.1 | Ciliate | AA |
| HIV-1 gag | Gag polyprotein from human immunodeficiency virus type 1 (HIV-1) - essential for virion assembly | 1503 | JQ686832.1 | Standard | DNA |
| GAL1 | GAL1 promoter used to drive galactose-inducible expression | 453 | KM407511.1 | Bacterial | DNA |
| Gal4 AD | Gal4 transcription factor activation domain | 339 | KX696451.1 | Bacterial | DNA |
| Gal4 DBD | Gal4 transcription factor activation domain | 441 | AAA83258.1 | Bacterial | DNA |
| GLuc | Gaussia princeps secreted luciferase | 558 | AAG54095.1 | Standard | DNA |
| GRE | Gluococorticoids activate transcription through gluococorticoid response elements (GREs) located in the promoter region. | 15 | | Standard | DNA |
| GST | glutathione S-transferase tag used in pull-down assays | 232 | AAA57092.1 | Bacterial | AA |
| GAP | Glyceraldehyde-3-phosphate dehydrogenase (GAPDH) promoter sequence - constitutive promoter | 487 | JQ519686.1 | Bacterial | DNA |
| EF-1α core | Gorilla gorilla gorilla eukaryotic translation elongation factor 1 alpha 1 promoter | 212 | XM_019028903.1 | Standard | DNA |
| gp64 | gp64 signal sequence from baculovirus envelope glycoprotein GP64 | 114 | X58376.1 | Bacterial | DNA |
| CBG68luc | Green click beetle luciferase | 1629 | AAP83307.1 | Bacterial | DNA |
| CBG99luc | Green click beetle luciferase | 1629 | AAP83311.1 | Bacterial | DNA |
| TurboGFP | Green fluorescent reporter protein - Ex:482, Em:502 | 714 | ASW25895.1 | Bacterial | DNA |
| TagGFP2 | Green fluorescent reporter protein - Ex:483, Em:506 | 717 | AMM04547.1 | Bacterial | DNA |
| Superfolder GFP | Green fluorescent reporter protein - Ex:485, Em:510 | 750 | ASL68970.1 | Bacterial | DNA |
| Emerald | Green fluorescent reporter protein - Ex:487, Em:509 | 717 | AIA24532.1 | Bacterial | DNA |
| EGFP | Green fluorescent reporter protein - Ex:488, Em:507 | 726 | AFA52654.1 | Bacterial | DNA |
| GFP | Green fluorescent reporter protein - Ex:488, Em:510 | 717 | AAB51347.1 | Bacterial | DNA |
| Azami-Green | Green fluorescent reporter protein - Ex:492, Em:505 | 678 | BAD52001.1 | Bacterial | DNA |
| mWasabi | Green fluorescent reporter protein - Ex:493, Em:509 | 711 | ABW74902.1 | Bacterial | DNA |
| NowGFP | Green fluorescent reporter protein - Ex:494, Em:502 | 753 | AQY79140.1 | Bacterial | DNA |
| ZsGreen | Green fluorescent reporter protein - Ex:496, Em:506 | 696 | AAF03372.1 | Standard | DNA |
| Clover | Green fluorescent reporter protein - Ex:505, Em:515 | 684 | AFR60231.1 | Bacterial | DNA |
| mNeonGreen | Green fluorescent reporter protein - Ex:506, Em:517 | 711 | BBB44438.1 | Bacterial | DNA |
| mClover3 | Green fluorescent reporter protein - Ex:506, Em:518 | 717 | ATE88096.1 | Bacterial | DNA |
| AcGFP1 | Green fluorescent reporter protein from Aequorea coerulescens Ex:475, Em:505 | 720 | ASK86109.1 | Bacterial | DNA |
| ppluGFP2 | Green fluorescent reporter protein from Pontellina plumata - Ex:482, Em:502 | 669 | AAQ01184.1 | Standard | DNA |
| HA/NLS | HA epitope tag and nuclear localization sequence | 40 | | | AA |
| HaloTag^{®} | HaloTag used for protein expression and purification | 296 | AAV70825.1 | Bacterial | AA |
| hsp70 | heat shock protein 70 promoter region | 475 | AM887687.1 | Standard | DNA |
| cI857 LR | Heat-sensitive lambda repressor used in lambda-based expression vectors | 714 | AAA99919.1 | Standard | DNA |
| S1 | Hepatitis B virus S1 tag used for protein detection and purification | 9 | ACO05432.1 | Standard | AA |
| HSV | herpes simplex virus (HSV) tag used for protein purification | 12 | | Standard | AA |
| VP16 AD | herpes simplex virus VP16 activation domain | 261 | U89963.1 | Bacterial | DNA |
| HN | histidine-asparagine (HN) tag for protein purification | 12 | | Standard | AA |
| cPPT/CTS | HIV-1 central polypurine tract and central termination sequence | 118 | MH325100.1 | Standard | DNA |
| RRE | HIV-1 rev response element (RRE) - cis-acting RNA needed for viral replication | 234 | MK801286.1 | Bacterial | DNA |
| HK022 int | HK022 phage integrase - enables integration of genetic material | 1074 | AGN12526.1 | Bacterial | DNA |
| CD4 | Homo sapiens CD4 cell surface glycoprotein | 1377 | NP_000607.1 | Standard | DNA |
| FKPB (DmrA) | Homo sapiens FK506-binding protein FKBP12 | 327 | NP_000792.1 | Standard | DNA |
| HRP | horseradish peroxidase (HRP) tag used for protein detection | 309 | AAA72223.1 | Bacterial | AA |
| HQ | HQ tag used for protein expression and purification | 6 | | Standard | AA |
| hb glob PA | human beta (hb) globin polyadenylation (PA) signal | 401 | KC176268.1 | Standard | DNA |
| CMV IE94 | Human cytomegalovirus (CMV) immediate early promoter | 987 | KY435761.1 | Standard | DNA |
| CMV | Human cytomegalovirus immediate early enhancer | 286 | MG550105.1 | Standard | DNA |
| EF1α | Human elongation factor 1 alpha (EF1α) promoter | 1184 | HQ644134.1 | Standard | DNA |
| hGH | Human growth hormone (hGH) intron | 271 | AF369964.1 | Standard | DNA |
| HA | human influenza hemagglutinin (HA) (amino acids 98-106) tag | 9 | AAB02235.1 | Standard | AA |
| hSos | human Son of sevenless (hSos) CDS. Membrane-bound guanine nucleotide-binding protein | 3957 | XM_005264515.4 | Standard | DNA |
| hU6 | Human U6 snRNA promoter | 241 | MK318530.1 | Standard | DNA |
| hUbC | human ubiquitin C (UbC) promoter for use in mammalian expression vectors | 1204 | KJ796484.1 | Standard | DNA |
| h α-tubulin | human α-tubulin sequence | 1353 | NP_006073.2 | Standard | DNA |
| hcas9 | human-codon optimised Cas9 endonuclease | 4131 | AKS40380.1 | Bacterial | DNA |
| hybrid | hybrid introns of chicken β-actin (CBA) and minute virus of mice (MMV) | 228 | MG550105.1 | Standard | DNA |
| trc | hybrid of the trp and lac promoters that is stronger than the lac promoter | 74 | U19585.1 | Bacterial | DNA |
| virD3 | hypothetical protein from Agrobacterium tumefaciens Ti plasmid | 639 | AAZ50534.1 | Bacterial | DNA |
| Aral1 | I1 operator of the arabinose operon | 16 | MH101733.1 | Bacterial | DNA |
| AraI2 | I2 operator of the arabinose operon | 12 | MH101733.1 | Bacterial | DNA |
| protA | IgG-binding unit of Staphylococcus aureus protein A | 174 | ALO52730.1 | Bacterial | DNA |
| IgK secretion | IgK leader signal for protein secretion | 63 | MG437047.1 | Standard | DNA |
| rtTA-advanced | improved tetracycline-controlled transactivator | 747 | ABC65842.1 | Bacterial | DNA |
| tTA-advanced | improved tetracycline-controlled transactivator | 747 | AZP55973.1 | Bacterial | DNA |
| incA | incA cis-acting incompatibility locus required for stable plasmid maintenance | 63 | L05669.1 | Bacterial | DNA |
| incC | incompatibility site used in F plasmids | 251 | KT362048.1 | Bacterial | DNA |
| incP oriT | incP origin of transfer | 122 | MN057686.1 | Bacterial | DNA |
| hsp70 | Inducible eukaryotic minimal heat shock promoter derived from Drosophila melanogaster hsp70 | 283 | MG550104.1 | Standard | DNA |
| Gam | inhibitor of λ red recombinase | 417 | AFC75892.1 | Bacterial | DNA |
| trfA | initiates plasmid replication by binding to oriV | 1149 | AGN30568.1 | Bacterial | DNA |
| GAS | interferon-gamma activation sequence (GAS) | 18 | | Standard | DNA |
| ISRE | interferon-stimulated response element (ISRE) | 12 | | Standard | DNA |
| IRES EMCV | Internal ribosomal entry site (IRES) from encephalomyocarditis virus (EMCV) involved in cap-dependent translation | 598 | AF264696.2 | Standard | DNA |
| IRES | Internal ribosomal entry site (IRES) involved in cap-dependent translation | 587 | MG833229.1 | Standard | DNA |
| bGlob | Intron 2 of rabbit gene for beta globin - enhancer of transgene expression | 573 | V00882.1 | Standard | DNA |
| chsA | intron from petunia chalcone synthase gene | 1353 | EU049865.1 | Standard | DNA |
| ITR repeat region | inverted terminal repeat of human adenovirus serotype 5 | 103 | MH121116.1 | Standard | DNA |
| Ras | Involved in signal transduction - controls cell growth and death | 570 | NP_001091711.1 | Standard | DNA |
| trpE | Involved in the biosynthesis of anthranilate, an intermediate of L-tryptophan | 1563 | AAA57297.1 | Bacterial | DNA |
| Jun AD | Jun activation domain, involved in activation of AP-1 proteins | 237 | 1404381A | Standard | AA |
| kemptide | Kemptide target that serves as a substrate for cAMP-dependent protein kinase (PKA) | 7 | | Standard | AA |
| KSI | Ketosteroid isomerase (KSI) tag used for protein expression | 121 | AAA25872.1 | Bacterial | AA |
| 3' LAC4 | Kluyveromyces lactis LAC4 promoter (3' end) | 1633 | JF327849.1 | Standard | DNA |
| 5' LAC4 | Kluyveromyces lactis LAC4 promoter (5' end) | 581 | JF327849.1 | Standard | DNA |
| LAC4 | Kluyveromyces lactis LAC4 termination region | 583 | JF327849.1 | Standard | DNA |
| KITRP1 | Kluyveromyces lactis TRP1 involved in tryptophan biosynthesis | 633 | XP_454745.1 | Standard | DNA |
| PpADE2 | Komagataella phaffii ADE2 gene required for purine nucleotide biosynthesis | 1692 | XP_002492296.1 | Standard | DNA |
| PpTRP2 | Komagataella phaffii TRP2 gene required for tryptophan biosynthesis | 1617 | XP_002491044.1 | Standard | DNA |
| AmKozak | Kozak sequence of Dictyostelium discoideum | 12 | | Standard | DNA |
| DroKozak | Kozak sequence of Drosophila | 12 | | Standard | DNA |
| DsKozak | Kozak sequence of Dunaliella salina | 12 | | Standard | DNA |
| Plant Kozak | Kozak sequence of plants | 11 | | Standard | DNA |
| PlasKozak | Kozak sequence of Plasmodium | 12 | | Standard | DNA |
| ScKozak | Kozak sequence of Saccharomyces cerevisiae | 12 | | Standard | DNA |
| ToxoKozak | Kozak sequence of Toxoplasma gondii | 10 | | Standard | DNA |
| TrypKozak | Kozak sequence of Trypanosomatidae | 11 | | Standard | DNA |
| rrk1 | K-RNA component of RNase P from Schizosaccharomyces pombe (contains promoter region and leader RNA) | 357 | CU329672.1 | Standard | DNA |
| KRAB | krueppel-associated box from Pan troglodytes | 180 | NP_001233376.1 | Standard | DNA |
| KT3 | KT3 epitope tag used for protein detection and purification | 11 | JC052327.1 | Standard | AA |
| lacO | Lac operon operator | 17 | LC459975.1 | Bacterial | DNA |
| lacUV5 | Lac promoter of Escherichia coli containing 2 base pair mutations | 31 | MK787297.1 | Bacterial | DNA |
| laclq | laclq promoter region | 78 | MH290813.1 | Bacterial | DNA |
| lacl | lactose operon repressor | 1083 | AWN09465.1 | Bacterial | DNA |
| lacZa | lacZ 5'-region | 177 | CAA34380.1 | Bacterial | DNA |
| lacZa | lacZ alpha fragment | 183 | BAV38147.1 | Bacterial | DNA |
| lacZα | lacZ alpha fragment from pNG168 vector. Used for alpha complementation alongside omega fragment (usually in host) | 576 | AAP44986.1 | Bacterial | DNA |
| lacZa | lacZ alpha peptide | 177 | ASG92102.1 | Bacterial | DNA |
| lacZ | lacZ tag used for protein detection, expression and purification | 1024 | AAO48720.1 | Bacterial | AA |
| lacZa | lacZ-alpha | 548 | ACA63830.1 | Bacterial | DNA |
| λT0 | lambda T0 terminator region | 106 | MK756317.1 | Bacterial | DNA |
| preprotrypsin | leader sequence from mouse preprotrypsin | 45 | BAD83864.1 | Standard | DNA |
| T7 gene 10 leader | Leader sequence of gene 10 in T7 phage - ribosome-binding site and enhancer | 33 | LC363502.1 | Standard | DNA |
| LB T-DNA repeat | left border repeat from nopaline C58 T-DNA | 25 | AP019003.1 | Bacterial | DNA |
| Tn7L | left segment of Tn7 transposon | 166 | MK356269.1 | Bacterial | DNA |
| telL | left terminal hairpin loop from bacteriophage N15; telL | 28 | U63086.1 | Bacterial | DNA |
| LEU2 | Leucine auxotrophic marker | 1107 | AJD87329.1 | Bacterial | DNA |
| HIS3 | L-histidine auxotrophic marker | 660 | AAA67141.1 | Bacterial | DNA |
| HIS4 | L-histidine auxotrophic marker | 2529 | AAA67001.1 | Standard | DNA |
| mKeima | Long stokes shift fluorescent reporter protein - Ex:440, Em:620 | 669 | AMO27260.1 | Bacterial | DNA |
| CyOFP1 | Long stokes shift fluorescent reporter protein - Ex:497, Em:589 | 702 | ANG09186.1 | Bacterial | DNA |
| loxH | loxH recombination site | 34 | FJ750581.1 | Standard | DNA |
| loxP | LoxP site for Cre-Lox recombination | 34 | MK044343.1 | Bacterial | DNA |
| Ipp | Ipp promoter sequence | 30 | AF361441.1 | Bacterial | DNA |
| TRP1 | L-tryptophan auxotrophic marker | 705 | ABQ43169.1 | Bacterial | DNA |
| LcLuc | Luciola cruciata luciferase | 1647 | AAA29135.1 | Standard | DNA |
| T7 lysozyme | lysozyme from bacteriophage T7 | 456 | AAB32819.1 | Bacterial | DNA |
| M13 | M13 origin of replication | 510 | KC860515.1 | Bacterial | DNA |
| MBP | maltose-binding protein used for protein detection, purification and expression | 387 | AMW03664.1 | Bacterial | AA |
| MAS | mannopine synthase termination region | 252 | KM507060.1 | Standard | DNA |
| MetLuc | Metridia longa secreted luciferase; produces light with a wavelength of 485 nm | 660 | AAR17541.1 | Bacterial | DNA |
| minicis | minicistron - enhances translation efficiency | 27 | | Standard | DNA |
| min FRT | minimal FRT site that allows excision but not integration | 34 | MK425748.1 | Bacterial | DNA |
| MiniTK | minimal herpes simplex virus (HSV) thymidine kinase promoter | 68 | AJ277959.1 | Standard | DNA |
| ARA | Minimal promoter region of arabinose (araBAD) operon | 28 | MK637406.1 | Bacterial | DNA |
| MinP | minimal TATA-box promoter | 54 | MK484106.1 | Standard | DNA |
| CMV | Minimum cytomegalovirus (CMV) promoter sequence required for expression | 125 | MG437024.1 | Standard | DNA |
| miR30-shRNA | miR30-shRNA (short hairpin RNA) used to silence Renilla luciferase | 348 | HQ456319.1 | Bacterial | DNA |
| mito targeting | mitochondrial targeting sequence | 87 | MG520665.1 | Standard | DNA |
| MEK1 | Mitogen-activated protein kinase (MAPK)/extracellular signal-related kinase (ERK) - part of RAS/MAPK pathway | 1116 | XP_011520085.1 | Standard | DNA |
| MEKK1 | Mitogen-activated protein kinase kinase kinase of Mus musculus - part of signal transduction cascade | 4482 | AAD25049.1 | Standard | DNA |
| MMLV pol | MMLV polymerase region (contains splice acceptor site) | 375 | AB041928.1 | Standard | DNA |
| cat1 | modified castor bean catalase intron | 190 | KY420087.1 | Standard | DNA |
| modSV40 late 16s | modified simian vacuolating virus 40 (SV40) late 16s mRNA intron | 99 | AY122060.1 | Standard | DNA |
| 3' MoMuLV LTR | Moloney murine leukemia virus (MoMuLV) 3' long terminal repeat region | 594 | M64754.1 | Standard | DNA |
| 5' MoMuLV LTR | Moloney murine leukemia virus (MoMuLV) 5' long terminal repeat region | 589 | M64754.1 | Standard | DNA |
| mPKA | mouse protein kinase A (PKA) | 1056 | ABK42341.1 | Standard | DNA |
| MESV ψ | murine embryonic stem cell virus (MESV) packaging signal | 889 | LT726944.1 | Standard | DNA |
| MODC | Mus musculus orthinine decarboxylase that converts orthinine to putrescine | 1386 | AAB27809.1 | Standard | DNA |
| PGK | Mus musculus phosphoglycerate kinase 1 promoter | 500 | MH325104.1 | Standard | DNA |
| U6 | Mus musculus U6 snRNA promoter | 315 | X06980.1 | Standard | DNA |
| myc | myc protein tag derived from c-myc oncogene | 10 | AZP55974.1 | Bacterial | AA |
| N25O2 | N25O2 promoter sequence | 83 | LT727462.1 | Bacterial | DNA |
| Nluc | NanoLuc^{®} luciferase | 516 | AFI79290.1 | Bacterial | DNA |
| miRFP670 | Near IR fluorescent reporter protein - Ex:642, Em:670 | 948 | AOD74926.1 | Bacterial | DNA |
| TDsmURFP | Near IR fluorescent reporter protein - Ex:642, Em:670 | 870 | ANW47199.1 | Bacterial | DNA |
| iRFP670 | Near IR fluorescent reporter protein - Ex:643, Em:670 | 936 | AGN32863.1 | Bacterial | DNA |
| iRFP682 | Near IR fluorescent reporter protein - Ex:663, Em:682 | 951 | AGN32864.1 | Bacterial | DNA |
| iRFP702 | Near IR fluorescent reporter protein - Ex:673, Em:702 | 936 | AGN32865.1 | Bacterial | DNA |
| mlFP | Near IR fluorescent reporter protein - Ex:683, Em:704 | 960 | AKH03689.1 | Bacterial | DNA |
| iFP1.4 | Near IR fluorescent reporter protein - Ex:684, Em:708 | 321 | 4O8G | Standard | AA |
| iFP2.0 | Near IR fluorescent reporter protein - Ex:690, Em:711 | 329 | 4CQH | Standard | AA |
| iRFP | Near IR fluorescent reporter protein - Ex:690, Em:713 | 951 | AIZ66003.1 | Bacterial | DNA |
| iRFP720 | Near IR fluorescent reporter protein - Ex:702, Em:720 | 951 | AGN32866.1 | Bacterial | DNA |
| NmtracrRNA | Neisseria meningitidis CRISPR/Cas9 trans-activating RNA | 93 | LR134528.1 | Bacterial | DNA |
| NmCas9 | Neisseria meningitidis Type II Cas9 endonuclease | 3249 | VEJ38538.1 | Bacterial | DNA |
| nptII | neomycin phosphotransferase (NEOKAN) promoter sequence | 365 | MK562405.1 | Standard | DNA |
| NE | NE-tag used for protein detection, quantification and purification | 18 | | Standard | AA |
| neuromodulin | neuromodulin amino terminal sequence for localization to growth cones in neurons | 60 | AB161231.1 | Bacterial | DNA |
| p65 AD | NFκB transcription factor p65 activation domain | 191 | SJL87367.1 | Bacterial | AA |
| N-myristoylation | N-myristoylation signal from Src kinase | 42 | SJL87546.1 | Bacterial | DNA |
| NMT | no message in thiamine (nmt) promoter - subject to repression by thiamine | 1169 | AB871644.1 | Standard | DNA |
| AdV VA | non-coding RNA involved in regulating translation in adenovirus (VAI and VAII) | 744 | AF369965.1 | Bacterial | DNA |
| NOS | nopaline synthase promoter | 319 | MK317973.1 | Standard | DNA |
| CIB-N | N-terminal of CIB1 used in light activated CRISPR-case effector (LACE) systems - induces expression in the presence of blue light | 510 | AAO63377.1 | Standard | DNA |
| alpha | N-terminal secretion signal from S. cerevisiae alpha-factor | 279 | HQ398363.1 | Bacterial | DNA |
| MAPKK NES | nuclear export signal (NES) of Map Kinase Kinase (MAPKK) | 57 | KP030821.1 | Bacterial | DNA |
| NF-KB AD | Nuclear factor kappa-light-chain-enhancer of activated B cells (NF-KB) activation domain | 565 | AF151087.1 | Bacterial | DNA |
| IκB | nuclear factor of kappa light polypeptide gene enhancer in B-cells inhibitor (NFKBI) | 954 | AAA16489.1 | Standard | DNA |
| HIV Rev NES | Nuclear localisation signal of the HIV Rev protein | 12 | MK490661.1 | Standard | AA |
| EGL-13 NLS | nuclear localization signal from the C. elegans EGL-13 transcription factor | 25 | | | AA |
| REX NLS | nuclear localization signal from the human T-cell leukemia virus type 1 (HTLV-1) Rex protein | 17 | P0C208.1 | Standard | AA |
| dCAS9 | nuclease deficient cas9 from Streptococcus pyogenes | 4107 | AKA60242.1 | Bacterial | DNA |
| nucleoplasmin NLS | nucleoplasmin nuclear localisation signal | 48 | KF264451.1 | Bacterial | DNA |
| CLIP | O6-alkylguanine-DNA alkyltransferase protein tag - reacts with benzylcytosine | 182 | AQS79240.1 | Bacterial | AA |
| SNAP | O6-alkylguanine-DNA alkyltransferase protein tag - reacts with benzylguanine | 182 | AQS79239.1 | Standard | AA |
| ScMET25 | O-acetyl homoserine - O-acetyl serine sulfhydrylase (MET25) promoter region from S. cerevisiae | 577 | LT727226.1 | Standard | DNA |
| OCS | octopine synthase terminator | 708 | LT725641.1 | Bacterial | DNA |
| lacZ | One of three structural genes in the lac operon - responsible for cleaving lactose | 3075 | AAD11974.1 | Bacterial | DNA |
| lacY | One of three structural genes of the lac operon - involved in transport of lactose into the cell | 1254 | AIZ92505.1 | Bacterial | DNA |
| mKO1 | Orange fluorescent reporter protein - Ex:548, Em:559 | 657 | AGM53875.1 | Bacterial | DNA |
| mOrange | Orange fluorescent reporter protein - Ex:548, Em:562 | 711 | AUO38282.1 | Bacterial | DNA |
| mOrange2 | Orange fluorescent reporter protein - Ex:549, Em:565 | 708 | AIL28758.1 | Bacterial | DNA |
| dTomato | Orange fluorescent reporter protein - Ex:554, Em:581 | 705 | AAV52168.1 | Bacterial | DNA |
| tdTomato | Orange fluorescent reporter protein - Ex:554, Em:581 | 1431 | AAV52169.1 | Bacterial | DNA |
| ORF603 | ORF603 from Autographa californica nucleopolyhedrovirus | 606 | BAA00606.1 | Standard | DNA |
| ORF1629 | ORF1629 capsid protein from Plutella xylostella multiple nucleopolyhedrovirus | 1632 | ABE68394.1 | Standard | DNA |
| OpIE1 | Orgyia pseudotsugata multicapsid polyhedrosis virus (Op) IE 1 promoter | 292 | AB711114.1 | Standard | DNA |
| OplE2 PA | Orgyia pseudotsugata multicapsid polyhedrosis virus (Op) IE 2 polyadenylation signal | 130 | MG356853.1 | Standard | DNA |
| OplE2 | Orgyia pseudotsugata multicapsid polyhedrosis virus (Op) IE 2 promoter | 548 | MG356853.1 | Standard | DNA |
| N15 | origin of replication derived from coliphage N15 | 90 | EF583812.1 | Bacterial | DNA |
| pRiA4 | origin of replication from Agrobacterium plasmid pRiA4 | 4636 | GU574780.1 | Bacterial | DNA |
| ColE1 | origin of replication from pBR322 (also known as pMB1, pUC origin) | 589 | MN019113.1 | Bacterial | DNA |
| pSA | origin of replication from pSA | 436 | MH445409.1 | Bacterial | DNA |
| pVS1 oriV | origin of replication from Pseudomonas pVS1 plasmid | 195 | MG836292.1 | Bacterial | DNA |
| RSF1010 oriT | origin of transfer - requires repA, repB and repC | 88 | LT727569.1 | Bacterial | DNA |
| RP4 oriT | origin of transfer for RP4 plasmid | 99 | X14165.1 | Bacterial | DNA |
| F oriV | origin of vegetative replication | 625 | KX264182.1 | Bacterial | DNA |
| RSF1010 oriV | origin of vegetative replication - requires repA, repB and repC | 395 | MH423581.1 | Bacterial | DNA |
| oriS | oriS origin of replication | 266 | MH325116.1 | Bacterial | DNA |
| OsmY | osmotically inducible protein OsmY from Escherichia coli | 606 | ACI72779.1 | Bacterial | DNA |
| p15A | P15A origin of replication | 549 | X06402.1 | Bacterial | DNA |
| p300 | p300 histone acetyltransferase involved in regulation of transcription | 7263 | XP_003932886.1 | Standard | DNA |
| Ad5 ψ | packaging signal for adenovirus serotype 5 | 151 | MH325116.1 | Standard | DNA |
| MMLV ψ | packaging signal of murine leukemia virus (MMLV) | 905 | AB296084.1 | Standard | DNA |
| ERE | Palindromic estrogen hormone response element (ERE) involved in regulating transcription | 15 | | Standard | DNA |
| virA | part of a two-component signal transduction system from Agrobacterium tumefaciens Ti plasmid | 2490 | AAZ50512.1 | Bacterial | DNA |
| Raf1 | Part of RAS/MAPK signaling pathway | 1945 | AIC55012.1 | Standard | DNA |
| Pc | Pc class 1 integron promoter | 29 | MK809155.1 | Bacterial | DNA |
| PDZ | PDZ domain used for protein detection, immobilization and purification | 83 | Q9Z0J4 | Standard | AA |
| pelB | periplasm localization signal | 66 | CAA74922.1 | Bacterial | DNA |
| yPGK | PGK terminator from Saccharomyces cerevisiae | 276 | KJ502284.1 | Standard | DNA |
| PL | phage lambda major leftward promoter PL - initiates transcription of gam, red, xis and int genes | 243 | L11614.1 | Bacterial | DNA |
| Polyphenylalanine | phe-tag (polyphenylalanine) used for protein purification | 11 | | Standard | AA |
| PHO1 | PH01 secretion signal | 66 | XP_002490985.1 | Standard | DNA |
| Dendra2 | Photoswitchable (green/red) fluorescent reporter protein - Ex:490/553, Em:507/573 | 690 | ADE48820.1 | Bacterial | DNA |
| Dronpa | Photoswitchable green fluorescent reporter protein - Ex:503, Em:518 | 673 | ADE48854.1 | Bacterial | DNA |
| Gamillus | Photoswitchable green fluorescent reporter protein - Ex:504, Em:519 | 720 | BBC28144.1 | Bacterial | DNA |
| FLD1 | Pichia pastoris FLD1 promoter region | 597 | AF066054.1 | Standard | DNA |
| virB2 | pilin major subunit from Agrobacterium tumefaciens Ti plasmid | 366 | AAZ50519.1 | Bacterial | DNA |
| Pinpoint Xa | Pinpoint^{™} Xa biotin purification tag | 129 | AAA89090.1 | Bacterial | AA |
| ParB/RepB | plasmid partitioning protein | 972 | QGF18915.1 | Bacterial | DNA |
| sopA | plasmid partitioning protein | 1167 | QGQ43739.1 | Bacterial | DNA |
| galK | plays a role in galactose metabolism through phosphorylation of α-D-glactose | 1149 | U66885.1 | Bacterial | DNA |
| PNO3097 | pNO3097 origin of replication | 188 | L05669.1 | Bacterial | DNA |
| HIV-1 pol | pol protein from human immunodeficiency virus type 1 (HIV-1) involved in cleavage of RNA in RNA-DNA hybrids | 3012 | AAA44988.2 | Standard | DNA |
| hGH PA | poly A signal/terminator region from human growth hormone | 487 | AF369966.1 | Standard | DNA |
| hsp70 PA | polyadenylation signal of 70K heat shock protein | 1160 | AM887687.1 | Standard | DNA |
| TK PA | polyadenylation signal of Herpes simplex virus (HSV) thymidine kinase (TK) | 49 | MH325116.1 | Standard | DNA |
| polyArg | polyarginine tag used for protein purification (may sometime have 6 R residues instead of 5) | 5 | 4D49_C | Standard | AA |
| PH 3'UTR | polyhedrin 3' UTR sequence | 373 | AB713997.1 | Standard | DNA |
| VP3 | polyoma viral coat protein VP3 involved in facilitating entry into host cells | 615 | CAA24467.1 | Standard | DNA |
| PreScission | PreScission protease (human rhinovirus 3C protease) recognition/cleavage region - cleaves between Gln and Gly residues | 7 | | Standard | AA |
| RSF1010 RepB | primase required by RSF1010 ori for replication | 972 | AAA72888.1 | Bacterial | DNA |
| Profinity eXact | Profinity eXact tag used for protein purification and expression | 75 | Bio-Rad | Bacterial | AA |
| IS1 | prokaryotic transposable element IS1 | 770 | MK204379.1 | Bacterial | DNA |
| amp | Promoter for ampicillin resistance gene (b-lactamase) | 105 | MK816965.1 | Standard | DNA |
| cat | Promoter for chloramphenicol acetyl transferase | 103 | KX273378.1 | Bacterial | DNA |
| rpsL | promoter for the Xylanimonas cellulosilytica ribosomal protein S12 gene | 302 | CP001821.1 | Standard | DNA |
| URA3 | promoter for URA3 (pyrimidine auxotrophic marker) | 226 | MK036504.1 | Bacterial | DNA |
| TEF | promoter from Ashbya gossypii TEF | 369 | MG680582.1 | Bacterial | DNA |
| gpdA | Promoter from Aspergillus nidulans | 2301 | MF169983.1 | Standard | DNA |
| cspA | Promoter region for Escherichia coli cspA gene | 67 | AB248603.1 | Bacterial | DNA |
| SNR52 | promoter region for the Saccharomyces cerevisiae small nucleolar RNAgene (SNR52) | 269 | KX981587.1 | Standard | DNA |
| HSV TK | Promoter region from human alphaherpesvirus thymidine kinase (UL23) | 753 | KM359774.1 | Standard | DNA |
| PpADE2 | promoter region from Pichia pastoris ADE2 | 13 | FR839630.1 | Standard | DNA |
| PpTRP2 | promoter region from Pichia pastoris TRP2 | 150 | FR839629.1 | Standard | DNA |
| ScTDH3 | promoter region from Saccharomyces cerevisiae Glyceraldehyde-3-phosphate dehydrogenase (GAPDH) | 673 | KY131997.1 | Standard | DNA |
| RPR1 | promoter region from Saccharomyces cerevisiae RPR1 gene | 407 | GCF_000146045.2 | Standard | DNA |
| copia | promoter region from the Drosophila melanogaster transposable element copia | 280 | X02599.1 | Standard | DNA |
| SFFV | promoter region of a spleen focus-forming virus LTR | 483 | KJ697753.1 | Standard | DNA |
| araBAD | Promoter region of araBAD operon | 166 | CP041110.1 | Bacterial | DNA |
| rhaB | Promoter region of Escherichia coli rhaB gene | 282 | AY236525.1 | Bacterial | DNA |
| hPGK | promoter region of human phosphoglycerate kinase 1 | 520 | KT351864.1 | Standard | DNA |
| hSyn | promoter region of human synapsin I | 471 | MH883617.1 | Standard | DNA |
| LEU2 | Promoter region of LEU2 selectable marker | 405 | KY132044.1 | Bacterial | DNA |
| OsUbi | promoter region of Oryza sativa polyubiquitin gene UBQ2 | 2618 | KU252586.1 | Standard | DNA |
| OsU3 | promoter region of Oryza sativa snRNA U3 | 506 | LC460477.1 | Standard | DNA |
| HIS3 | promoter region of S. cerevisiae HIS3 | 237 | KR232300.1 | Standard | DNA |
| trpC | promoter region of the Aspergillus nidulans trpC gene | 361 | X02390.1 | Standard | DNA |
| TBG | promoter region of the human thyroxine-binding globulin gene | 410 | L13470.1 | Standard | DNA |
| tnpA | Promoter region of the transposase gene A from Pasteurella multocida | 107 | CP041111.1 | Bacterial | DNA |
| cBA | Promoter sequence from chicken beta actin | 278 | EU733646.1 | Standard | DNA |
| lac | promoter sequence of the lac operon | 31 | MK816965.1 | Bacterial | DNA |
| protA | protein A promoter from Staphylococcus aureus | 185 | M74186.1 | Bacterial | DNA |
| Protein C | protein C epitope tag used for protein detection and purification | 12 | BAC21170.1 | Standard | AA |
| PKCα | Protein kinase C alpha subunit involved in regulation of cell proliferation and growth | 2019 | CAA36718.1 | Standard | DNA |
| PKCy | Protein kinase C gamma (γ) involved in neuronal cells and eye tissues | 2094 | CAA47608.1 | Standard | DNA |
| PKCθ | Protein kinase C theta (θ) involved in signal transduction | 2121 | XM_024448076.1 | Standard | DNA |
| PKCζ | Protein kinase C zeta (ζ) involved in cell proliferation, differentiation and secretion | 1755 | CAA78813.1 | Standard | DNA |
| PKCβ | Protein kinase C β I involved in cell signaling | 2022 | AAA60095.1 | Standard | DNA |
| softag 1 | protein tag used for mammalian expression | 13 | | Standard | AA |
| softag 3 | protein tag used for prokaryotic expression | 8 | | Bacterial | AA |
| gp29 | protelomerase of coliphage N15 | 1896 | ACI02335.1 | Bacterial | DNA |
| pSC101 | pSC101 origin of replication | 1843 | MH551153.1 | Bacterial | DNA |
| Csy4 | Pseudomonas aeruginosa CRISPR-associated endoribonuclease | 564 | ALY39308.1 | Bacterial | DNA |
| pVS1 repA | Pseudomonas pVS1 plasmid replication protein | 1074 | AAD19680.1 | Bacterial | DNA |
| pVS1 staA | Pseudomonas pVS1 plasmid stability protein | 630 | AAD19678.1 | Bacterial | DNA |
| ψ plus pack | psi plus extended packaging signal of retroviruses | 810 | M77239.1 | Standard | DNA |
| ψ plus pack2 | psi plus packaging signal | 1287 | AF311737.1 | Standard | DNA |
| URA3 | pyrimidine auxotrophic marker | 804 | QDQ17562.1 | Bacterial | DNA |
| PDK | pyruvate orthophosphate dikinase intron | 742 | AJ311873.1 | Standard | DNA |
| R6Kg | R6K gamma (γ) origin of replication | 389 | MK787297.1 | Bacterial | DNA |
| rb glob PA | rabbit beta (rb) globin polyadenylation (PA) signal | 99 | MF174870.1 | Standard | DNA |
| rb glob PA | rabbit beta (rb) globin polyadenylation (PA) signal | 527 | MH210859.1 | Standard | DNA |
| argU | rare Escherichia coli tRNA-Arg that decodes AGA and AGG codons | 77 | CP041359.1 | Bacterial | DNA |
| HRV 3C site | recognition and cleavage site for human rhinovirus 3C protease | 8 | | Standard | AA |
| recA | recombinase A from Escherichia coli that makes any strain recA+ | 1062 | QGL36765.1 | Bacterial | DNA |
| FlpRec | recombinase flippase used in site-directed recombination | 1272 | AAC53669.1 | Bacterial | DNA |
| CBR!uc | Red click beetle luciferase | 1629 | AAP83303.1 | Standard | DNA |
| TurboRFP | Red fluorescent reporter protein - Ex:553, Em:574 | 696 | ADK12947.1 | Bacterial | DNA |
| TagRFP | Red fluorescent reporter protein - Ex:555, Em:584 | 702 | AKA95307.1 | Bacterial | DNA |
| TagRFP-T | Red fluorescent reporter protein - Ex:555, Em:584 | 735 | ACD03281.1 | Bacterial | DNA |
| mNectarine | Red fluorescent reporter protein - Ex:558, Em:578 | 711 | ACR78132.1 | Bacterial | DNA |
| mRuby3 | Red fluorescent reporter protein - Ex:558, Em:592 | 711 | ATE88097.1 | Bacterial | DNA |
| mRuby2 | Red fluorescent reporter protein - Ex:559, Em:600 | 714 | ANW97526.1 | Standard | DNA |
| DsRed2 | Red fluorescent reporter protein - Ex:561, Em:587 | 717 | AEX37894.1 | Bacterial | DNA |
| mTangerine | Red fluorescent reporter protein - Ex:568, Em:585 | 678 | AAV52170.1 | Bacterial | DNA |
| mApple | Red fluorescent reporter protein - Ex:568, Em:592 | 711 | AEM37572.1 | Bacterial | DNA |
| mScarlet-I | Red fluorescent reporter protein - Ex:569, Em:593 | 696 | APD76536.1 | Bacterial | DNA |
| mScarlet | Red fluorescent reporter protein - Ex:569, Em:594 | 696 | APD76535.1 | Bacterial | DNA |
| mStrawberry | Red fluorescent reporter protein - Ex:574, Em:596 | 711 | AAV52166.1 | Bacterial | DNA |
| FusionRed | Red fluorescent reporter protein - Ex:580, Em:608 | 1251 | AUO28692.1 | Bacterial | DNA |
| mCherry | Red fluorescent reporter protein - Ex:587, Em:610 | 711 | AAV52164.1 | Bacterial | DNA |
| HcRed1 | Red fluorescent reporter protein - Ex:592, Em:645 | 687 | AAX29983.1 | Bacterial | DNA |
| virG | regulator of at least two vir loci in Ti plasmid of Agrobacterium tumefaciens Ti plasmid | 804 | AAZ50529.1 | Bacterial | DNA |
| virE3 | regulatory protein from Agrobacterium tumefaciens Ti plasmid | 2019 | AAZ50539.1 | Bacterial | DNA |
| AraC | Regulatory protein that plays a part of the L-arabinose operon. Acts as an activator in the presence of arabinose | 888 | AAK85197.1 | Bacterial | DNA |
| Rluc | Renilla reniformis luciferase | 2049 | BAV38140.1 | Bacterial | DNA |
| hR!uc | Renilla reniformis luciferase (human codon-optimised) | 945 | AAO48589.1 | Bacterial | DNA |
| repA | RepA protein activates initiation of DNA replication at oriR. Translation is coupled to the tap gene | 858 | AAA99917.1 | Bacterial | DNA |
| repB | repB replication initiator from Streptococcus. Essential for replication | 678 | BAA83676.1 | Bacterial | DNA |
| repA4 | replication protein A4 from Escherichia coli | 387 | AAA72150.1 | Bacterial | DNA |
| rep | replication protein from pl-Scel vector | 675 | ACC63381.1 | Bacterial | DNA |
| β-glu | reporter gene from Escherichia coli used to monitor expression in GUS assays | 1812 | AAB30197.1 | Standard | DNA |
| SEAP | Reporter system used to study promoter activity and gene expression | 1560 | AAB64400.1 | Standard | DNA |
| ROP | repressor of primer (Rop) RNA-binding protein from Escherichia coli involved in low copy number maintenance | 74 | WP_032210647.1 | Bacterial | AA |
| ROP | repressor of primer (Rop) RNA-binding protein used for low plasmid copy number maintenance | 63 | KIG91028.1 | Bacterial | AA |
| gp38 | repressor protein of coliphage N15 | 609 | NP_046933.1 | Bacterial | DNA |
| RSF1010 RepA | required by RSF1010 ori for replication | 840 | AWD72714.1 | Bacterial | DNA |
| RSF1010 RepC | required by RSF1010 ori for replication | 951 | AEM76693.1 | Bacterial | DNA |
| AP1 | Response Element AP1 | 42 | | Standard | DNA |
| RXR rec | retinoic acid receptor RXR | 1419 | AAG02188.1 | Bacterial | DNA |
| rhaR | rhamnose binding transcriptional activator from Escherichia coli | 1023 | AAX99114.1 | Bacterial | DNA |
| rhaS | rhamnose binding transcriptional activator from Escherichia coli | 837 | QGL43075.1 | Bacterial | DNA |
| 1D4 | rho1D4 tag used for protein purification | 9 | | Standard | AA |
| RNAseIII PS | Ribonuclease III (RNAselll) processing site | 77 | AY599233.1 | Bacterial | DNA |
| RiboJ | ribozyme insulator used in synthetic genetic programs | 75 | MN005206.1 | Bacterial | DNA |
| RB T-DNA repeat | right border repeat from nopaline C58 T-DNA | 25 | AP019003.1 | Bacterial | DNA |
| Tn7R | right segment of Tn7 transposon | 225 | KT351864.1 | Bacterial | DNA |
| telR | right terminal hairpin loop from bacteriophage N15; telR | 28 | U91583.1 | Bacterial | DNA |
| RK2 oriV | RK2 origin of vegetative replication | 699 | DQ225750.1 | Standard | DNA |
| RPA | RNA polymerase alpha subunit; used for expressing fusion proteins with transcription factors | 750 | AF361441.1 | Bacterial | DNA |
| HA-L | Rosa26 intron 1 left homology arm | 1085 | MG763233.1 | Bacterial | DNA |
| HA-R | Rosa26 intron 1 right homology arm | 3611 | MG763233.1 | Bacterial | DNA |
| RSV | Rous sarcoma virus (RSV) long terminal repeat enhancer promoter | 527 | DQ075935.1 | Standard | DNA |
| RSV | Rous sarcoma virus (RSV) promoter | 227 | MH325102.1 | Standard | DNA |
| rrnB T1 | rrnB T1 transcription termination sequence | 87 | MK234843.1 | Bacterial | DNA |
| rrnB T2 | rrnB T2 transcription termination sequence | 28 | MH015246.1 | Bacterial | DNA |
| rrnB | rrnB transcriptional termination region | 411 | MH651726.1 | Bacterial | DNA |
| RSF ori | RSF1030 origin of replication | 750 | KJ641600.1 | Bacterial | DNA |
| S | S fragment of RNase S used for protein detection and purification | 15 | CAA68965.1 | Bacterial | AA |
| 2micron | S. cerevisiae 2 micron plasmid origin of replication | 878 | KY132088.1 | Standard | DNA |
| ADE2 | S. cerevisiae ADE2 required for purine biosynthesis | 1713 | AAA34407.1 | Standard | DNA |
| Sc-ars1 | S. cerevisiae autonomously replicating sequence ARS1 | 838 | LT727554.1 | Bacterial | DNA |
| Gal1, 10 | S. cerevisiae gal-inducible promoter | 615 | JN201971.1 | Standard | DNA |
| URA3 | S. cerevisiae URA3 promoter (pyrimidine auxotrophic marker) | 217 | LT727521.1 | Standard | DNA |
| ura4 | S. pombe URA4 - required for uracil biosynthesis | 795 | NP_587705.1 | Standard | DNA |
| RPR1 | Saccharomyces cerevisiae RPR1 termination sequence | 63 | BK006939.2 | Standard | DNA |
| mAmetrine | Sapphire fluorescent reporter protein - Ex:406, Em:526 | 720 | AQZ21583.1 | Bacterial | DNA |
| Sp-ars1 | Schizosaccharomyces pombe autonomously replicating sequence ars1 | 928 | EU665638.1 | Bacterial | DNA |
| SpH!S5 | Schizosaccharomyces pombe HISS required for histidine biosynthesis | 654 | AAG34536.1 | Bacterial | DNA |
| IL-6 | secretion signal from interleukin 6 | 87 | AFI79293.1 | Bacterial | DNA |
| SIN | self inactivating deletion (SIN) rendering the virus self-inactivating after integration | 38 | AF264696.2 | Standard | DNA |
| P2A | self-cleaving 2A peptide from porcine teschovirus-1 | 60 | A!E57569.1 | Bacterial | DNA |
| bGH PA | Sequence used for polyadenlyation and termination of transcripts | 224 | MK160997.1 | Standard | DNA |
| HSA | serum albumin precursor from Homo sapiens (cell growth inhibiting protein 42) | 1830 | AAU21642.1 | Standard | DNA |
| SRE | serum response element (SRE) - a regulatory element bound by transcription factor | 60 | | Standard | DNA |
| melittin | signal sequence from honeybee melittin used for protein secretion | 57 | AY598466.1 | Bacterial | DNA |
| CMV enhancer | simian cytomegalovirus major immediate early transcription unit IE94 promoter region | 380 | K03104.1 | Standard | DNA |
| SV40ER | simian vacuolating virus 40 (SV40) enhancer region | 237 | AY170009.1 | Standard | DNA |
| SV40 NLS | simian vacuolating virus 40 (SV40) nuclear localization signal (NLS) from large T antigen | 57 | LT727380.1 | Bacterial | DNA |
| SV40 | simian vacuolating virus 40 (SV40) origin of replication | 136 | MH325111.1 | Bacterial | DNA |
| SV40 polyA | simian vacuolating virus 40 (SV40) polyA signal | 122 | MK816965.1 | Standard | DNA |
| SV40 | simian vacuolating virus 40 (SV40) promoter sequence | 197 | MG547974.1 | Standard | DNA |
| SV40 IrgT | simian vacuolating virus 40 TAg (SV40) large T antigen - allows amplification of plasmids with SV40 ORIs | 1881 | LT727634.1 | Standard | DNA |
| virE2 | single-stand DNA-binding protein involved in nuclear uptake of single-stranded DNA from Agrobacterium tumefaciens Ti plasmid | 1650 | AAZ50538.1 | Bacterial | DNA |
| SS | single-stranded replication origin | 142 | AB042431.1 | Bacterial | DNA |
| EK | Site cleaved by enterokinase (EK) - cleavage occurs after lys residue | 5 | | Standard | AA |
| thromb targ | Site cleaved by thrombin protease - cleaved between Arg and Gly | 6 | | Standard | AA |
| TEV | Site cleaved by Tobacco Etch Virus (TEV) protease - cleaved between Glu and Gly | 7 | | Standard | AA |
| PKA site | site for labeling with protein kinase A | 5 | | Standard | AA |
| TEV | site recognised by tobacco etch virus (TEV) protease | 7 | JN874651.1 | Bacterial | AA |
| SP6 | SP6 RNA polymerase promoter | 20 | AY349044.1 | Standard | DNA |
| Spel | Spel promoter region | 35 | MH319949.1 | Bacterial | DNA |
| spot | spot-tag used for protein detection and purification | 12 | | Standard | AA |
| protA | staphylococcal protein A tag used for increased protein solubility and expression | 68 | BAC76617.1 | Standard | AA |
| protG | staphylococcal protein G tag used for increased protein solubility and expression | 119 | ABO76907.1 | Bacterial | AA |
| ShCas9 | Staphylococcus aureus subsp. aureus type II Cas9 endonuclease | 3162 | CCK74173.1 | Bacterial | DNA |
| STATS RE | STATS response element | 9 | | Standard | DNA |
| stbA | StbA and StbB are both required for stable plasmid maintenance | 963 | AAA99912.1 | Bacterial | DNA |
| stbB | StbA and StbB are both required for stable plasmid maintenance | 459 | AAA99913.1 | Bacterial | DNA |
| T7E | stem loop early T7 transcriptional terminator | 41 | KU523973.1 | Bacterial | DNA |
| strep | strep-Tactin tag used for protein detection and purification | 8 | ARU77561.1 | Bacterial | AA |
| SBP | Streptavadin-binding peptide used for protein purification | 38 | ACF35721.1 | Bacterial | AA |
| cas9VQR | Streptococcus pyogenes Cas9 endonuclease (D1135V/R1335Q/T1337R mutations) | 4107 | AVR59331.1 | Bacterial | DNA |
| cas9 D10A | Streptococcus pyogenes Cas9 endonuclease with the D10A mutation (only cleaves the target strand) | 4140 | AWD73737.1 | Bacterial | DNA |
| tracrRNA | Streptococcus pyogenes CRISPR/Cas9 trans-activating RNA | 171 | LC127310.1 | Bacterial | DNA |
| cRNA leader | Streptococcus pyogenes cRNA leader sequence | 132 | MK214497.1 | Bacterial | DNA |
| pCoCas9 | Streptococcus pyogenes Type II Cas9 endonuclease - plant codon optimised | 4254 | AGZ01981.1 | Bacterial | DNA |
| StCas9 | Streptococcus thermophilus type II Cas9 endonuclease | 3366 | AKB97299.1 | Bacterial | DNA |
| Sv rep | Streptomyces viridosporus replication initiator protein | 1443 | YP_001004139.1 | Bacterial | DNA |
| SUMO3 | SUMO3 tag used for protein expression | 111 | XP_027438670.1 | Standard | AA |
| SUMO | SUMO-tag used for increased protein solubility and expression | 98 | BAO66634.1 | Bacterial | AA |
| SUP4 | sup4 gene terminator sequence from Saccharomyces cerevisiae | 20 | KX981587.1 | Standard | DNA |
| ALS | suRB gene from Nicotiana tabacum that confers resistance to sulfonylurea herbicides | 1995 | CAA30485.1 | Standard | DNA |
| SV40 small T | SV40 early 19s mRNA (small t) intron | 65 | Y11034.1 | Standard | DNA |
| SV40 | SV40 intron | 196 | JX445134 | Standard | DNA |
| Gal4 AD | SV40 NLS fused to the gal4 activation domain | 136 | SJL88293.1 | Bacterial | AA |
| VgEcR | Synthetic fusion of Drosophila ecdysone receptor, DNA binding domain of the glucocorticoid receptor and activation domain of herpes simplex virus VP16 | 756 | AF264696.2 | Standard | AA |
| synth | synthetic intron - facilitates expression of mammalian transcripts | 294 | Y07702.1 | Standard | DNA |
| synth PA | Synthetic poly(A) signal/transcriptional pause site | 154 | KY025566.1 | Bacterial | DNA |
| tac | synthetic promoter produced from a combination of the trp and lac operon promoters | 29 | MK130721.1 | Bacterial | DNA |
| Lucia | Synthetic secreted luciferase | 630 | AGC79556.1 | Bacterial | DNA |
| PLtet | Synthetic tet-sensitive phage lambda tetracycline O-1 promoter | 74 | KX077536.1 | Bacterial | DNA |
| T1/TE | T1/TE bidirectional terminator | 129 | MH488950.1 | Bacterial | DNA |
| T2A | T2A ribosomal skipping sequence | 60 | AHZ97961.1 | Bacterial | DNA |
| T3 | T3 bacteriophage terminator sequence | 78 | KM018297.1 | Bacterial | DNA |
| T3 | T3 RNA polymerase promoter | 17 | LT726831.1 | Bacterial | DNA |
| T5 | T5 promoter sequence | 45 | KX147099.1 | Standard | DNA |
| T7G10RBSL | T7 gene 10 RBS leader | 47 | V01146.1 | Bacterial | DNA |
| T7 transl en RBS | T7 phage, gene 10 translational enhancer sequence - ribosome-binding site | 17 | MG437007.1 | Standard | DNA |
| T7 | T7 RNA polymerase promoter | 18 | AY349044.1 | Bacterial | DNA |
| tag-100 | tag-100 used for protein expression | 12 | | Standard | AA |
| TAP | tandem affinity purification (TAP) tag used for protein purification | 185 | 5UZ5_C | Standard | AA |
| TAP | tandem affinity purification (TAP) tag used for protein purification | 183 | AAV33421.1 | Bacterial | AA |
| TAP | tandem affinity purification (TAP) tag used for protein purification | 182 | AUZ17109.1 | Bacterial | AA |
| virD1 | T-DNA border endonuclease from Agrobacterium tumefaciens Ti plasmid | 444 | AAZ50532.1 | Bacterial | DNA |
| virD2 | T-DNA border endonuclease from Agrobacterium tumefaciens Ti plasmid | 1275 | AAZ50533.1 | Bacterial | DNA |
| TEF | TEF terminator sequence from Saccharomyces cervisiae | 265 | MG680557.1 | Standard | DNA |
| rep101 (TS) | temperature-sensitive replication protein | 951 | AAD51641.1 | Bacterial | DNA |
| AOX1 | Termination sequence for AOX1 | 333 | AY178634.1 | Standard | DNA |
| trpC | Termination sequence for Aspergillus nidulans trpC | 563 | LT726870.1 | Standard | DNA |
| NOS | termination sequence of Agrobacterium tumefaciens nopaline synthase gene | 256 | MK078637.1 | Bacterial | DNA |
| tonB | termination sequence of Escherichia coli tonB gene | 32 | LR595691.1 | Bacterial | DNA |
| soxR | termination sequence of the soxR gene from Escherichia coli | 29 | CP040667.1 | Bacterial | DNA |
| bla | terminator of beta-lactamase (bla) | 33 | MG595924.1 | Bacterial | DNA |
| rrnG | terminator region from Escherichia coli ribosomal RNA genes | 137 | LT906474.1 | Bacterial | DNA |
| ADH2 | terminator region of ADH2 | 316 | KX981587.1 | Bacterial | DNA |
| HIS | terminator region of the HIS operon | 72 | MH492456.1 | Bacterial | DNA |
| tet | tetracycline efflux transporter promoter region from pBR322 | 66 | MK416190.1 | Bacterial | DNA |
| TetO | tetracycline operator sequence (TetO) | 19 | MG437024.1 | Bacterial | DNA |
| TRE | tetracycline responsive element (TRE) | 318 | MG437024.1 | Bacterial | DNA |
| rtTA | tetracycline-controlled transactivator, comprising a fusion of the reverse tetracycline repressor rTetR with the C-terminal activation domain of herpes simplex virus VP16 | 1008 | AIU94961.1 | Bacterial | DNA |
| tTA | tetracycline-controlled transactivator, comprising a fusion of the tetracycline repressor TetR with the C-terminal activation domain of herpes simplex virus VP16 | 1008 | ACG80850.1 | Bacterial | DNA |
| pTRE | tetracycline-responsive promoter | 451 | KY053834.1 | Bacterial | DNA |
| TC | tetracysteine tag used for protein detection | 6 | AZP56002.1 | Bacterial | AA |
| AdPol | The adenovirus DNA polymerase containing an internal deletion | 2598 | AZP56147.1 | Bacterial | DNA |
| trx | thioredoxin tag from Escherichia coli used for improving solubility | 109 | AAA24693.1 | Bacterial | AA |
| TK2 | thymidine kinase (TK2) promoter sequence | 277 | U43612.1 | Bacterial | DNA |
| TK | thymidine kinase (TK) promoter sequence | 248 | LT727541.1 | Bacterial | DNA |
| TRE | thyroid hormone response element (TRE) | 16 | | Standard | DNA |
| tight TRE | tight tet-responsive promoter - contains multiple tet operator sequences, plus the minimal CMV promoter | 291 | MK816965.1 | Bacterial | DNA |
| tnpA | tnpA transposase from pMiniT | 231 | AAK28028.1 | Bacterial | DNA |
| TVMV protease | tobacco vein mottling virus (TVMV) Nla protease | 720 | NP_734334.1 | Standard | DNA |
| TVMV site | tobacco vein mottling virus (TVMV) Nla protease recognition and cleavage site | 8 | 3MMG_C | Standard | AA |
| p53 | TP53 from Homo sapiens | 1182 | CAA26306.1 | Standard | DNA |
| URA4 | transcription termination sequence for URA4 from Schizosaccharomyces pombe | 436 | AB601903.1 | Standard | DNA |
| nmt1-2 | transcription termination sequence of no message in thiamine (nmt1-2) | 142 | KU725771.1 | Standard | DNA |
| g7 | transcription terminator from Agrobacterium tumefaciencs Ti plasmid | 201 | LC482137.1 | Standard | DNA |
| λTL3 | transcription terminator tL3 from phage λ | 272 | KF030467.1 | Bacterial | DNA |
| copG TR | transcriptional repressor that regulates synthesis of itself and RepB | 138 | BAB03243.1 | Bacterial | DNA |
| Tfr membrane anchor | transferrin receptor signal-anchor region | 186 | XP_024309499.1 | Bacterial | DNA |
| TEE | translating enhancing element (TEE) | 15 | AB213654.1 | Standard | DNA |
| tap | translational activator peptide (tap) required for translation of repA protein | 72 | AXC59592.1 | Bacterial | DNA |
| AtADH 5'-UTR | translational enhancer from the 5'-UTR of the Arabidopsis thaliana alcohol dehydrogenase gene | 58 | X77943.1 | Standard | DNA |
| OsADH 5'-UTR | translational enhancer from the 5'-UTR of the Oryza sativa alcohol dehydrogenase gene | 101 | KF684948.1 | Standard | DNA |
| TMV Ω | translational enhancer from the tobacco mosaic virus (TMV) 5' leader sequence | 57 | | Standard | DNA |
| trans en | Translational enhancer of vascular endothelial growth factor from Mus musculus | 163 | XM_028766747.1 | Standard | DNA |
| T7g10 TE | translational enhancher from T7g10 | 9 | | Standard | DNA |
| KASH | transmembrane KASH domain of nesprin-2 from Mus musculus | 246 | NM_001005510.2 | Standard | DNA |
| tnpR | transposon resolvase involved in DNA recombination | 366 | EGB39263.1 | Bacterial | DNA |
| Tn7 att | transposon Tn7 attachment site | 65 | CVU75992.1 | Bacterial | DNA |
| tig | trigger factor involved in protein export | 1296 | BAD98926.1 | Bacterial | DNA |
| TRP1 | TRP1 promoter region | 281 | MG637043.1 | Bacterial | DNA |
| Trp53 DBD | Trp53 DNA-binding domain from Mus musculus murine p53 | 1173 | NP_035770.2 | Standard | DNA |
| truncRSA | truncated S-layer protein (RSA) from Caulobacter vibrioides (aa 690-1026) | 1011 | AF193064.1 | Bacterial | DNA |
| truncVP22 | truncated VP22 tegument protein (aa 159-301) | 142 | AWW13413.1 | Standard | AA |
| truncated gag | trungated gag protein - involved in capsid/matrix proteins | 426 | EF394360.1 | Standard | DNA |
| Ty | Ty1 tag used for protein detection | 10 | Z72946.1 | Standard | AA |
| virB1 | type IV secretion system from Agrobacterium tumefaciens Ti plasmid involved in T-DNA transfer | 720 | AAZ50518.1 | Bacterial | DNA |
| Lck | tyrosine-protein kinase from Homo sapiens | 1530 | CAA31884.1 | Standard | DNA |
| Universal | universal tag used for detection and purification | 6 | | Standard | AA |
| UAS | upstream activating sequence (UAS) from pSCUDMFE6L2 | 17 | LT727450 | Bacterial | DNA |
| UAS | upstream activating sequence (UAS) from pSCUDMFE6L2 | 17 | LT727450 | Bacterial | DNA |
| UAS | upstream activating sequence (UAS) from pSCUDMFE6L2 | 17 | LT727450 | Bacterial | DNA |
| UBQ10 | Ubiquitin10 promotor sequence from Arabidopsis thaliana | 1326 | | Standard | DNA |
| Sirius | UV fluorescent reporter protein - Ex:355, Em:424 | 720 | BAH29934.1 | Bacterial | DNA |
| Sandercyanin | UV fluorescent reporter protein - Ex:375, Em:630 | 170 | 5F6Z | | AA |
| VertKozak | Vertebrate kozak sequence | 10 | | Standard | DNA |
| VSV-G | vesicular stomatitis virus glycoprotein (VSV-G) tag used for protein detection and purification | 11 | HI649965.1 | Standard | AA |
| virD4 | VirD4 protein from Agrobacterium tumefaciens Ti plasmid | 1959 | AAZ50535.1 | Bacterial | DNA |
| virD5 | VirD5 protein from Agrobacterium tumefaciens Ti plasmid | 2511 | AAZ50536.1 | Bacterial | DNA |
| B | VP7 region of bluetongue virus used for protein detection and purification | 6 | | Standard | AA |
| rtTA3 | when doxycycline is present, this protein will bind to promoters containing the tet operator | 747 | ADR71685.1 | Bacterial | DNA |
| WPRE | woodchuck hepatitis virus posttranscriptional regulatory element | 588 | MH458082.1 | Bacterial | DNA |
| Xenopus globin 3' UTR | Xenopus globin 3' UTR sequence | 141 | LT727607.1 | Standard | DNA |
| Xenopus globin 5' UTR | Xenopus globin 5' UTR sequence | 43 | LT727607.1 | Standard | DNA |
| Xpress EK | Xpress epitope tag with enterokinase site used for protein purification | 8 | | Bacterial | AA |
| hCL1 | yeast peptide - ubiquitin-dependent degradation signal | 48 | AAR29593.1 | Bacterial | DNA |
| EYFP | Yellow fluorescent reporter protein - Ex:513, Em:527 | 720 | AMO27254.1 | Bacterial | DNA |
| Topaz | Yellow fluorescent reporter protein - Ex:514, Em:527 | 720 | Addgene | Bacterial | DNA |
| SYFP2 | Yellow fluorescent reporter protein - Ex:515, Em:527 | 720 | AAZ65845.1 | Bacterial | DNA |
| Venus | Yellow fluorescent reporter protein - Ex:515, Em:528 | 1776 | ANF29831.1 | Standard | DNA |
| Citrine | Yellow fluorescent reporter protein - Ex:516, Em:529 | 720 | BBD34379.1 | Bacterial | DNA |
| Ypet | Yellow fluorescent reporter protein - Ex:517, Em:530 | 723 | AVO64728.1 | Bacterial | DNA |
| mCyRFP1 | Yellow fluorescent reporter protein - Ex:528, Em:594 | 705 | AOY07765.1 | Bacterial | DNA |
| ZsYellow | Yellow fluorescent reporter protein - Ex:529, Em:539 | 696 | AAF03373.1 | Standard | DNA |
| mPapaya1 | Yellow fluorescent reporter protein - Ex:530, Em:541 | 714 | AGX93076.1 | Bacterial | DNA |
| Z domain | Z domain tag derived from staphylococcal protein A | 58 | ALO52730.1 | Bacterial | AA |
| cos | λ cos site; allows packaging into phage λ particles | 399 | JX069762.1 | Bacterial | DNA |
| λ cosN | λ cosN site, allows packaging into phage λ particles | 90 | FJ160466.1 | Bacterial | DNA |
| λ int | λ phage integrase - enables integration of genetic material | 1071 | AG N 12510.1 | Bacterial | DNA |
| Exo | λ red exonuclease | 681 | AFC75894.1 | Bacterial | DNA |
| Beta | λ red recombinase | 786 | AFC75893.1 | Bacterial | DNA |
| ϕ21 int | ϕ21 phage integrase - enables integration of genetic material | 1143 | AGN12534.1 | Bacterial | DNA |
| ϕ31 int | ϕ31 phage integrase - enables integration of genetic material | 1818 | NP_047974.1 | Bacterial | DNA |
| ϕ80 attP | ϕ80 phage attachment site | 480 | KF030463.1 | Bacterial | DNA |
| ϕ80 int | ϕ80 phage integrase - enables integration of genetic material | 1209 | AGN12518.1 | Bacterial | DNA |
| RNAP ω | ω subunit of DNA-directed RNA polymerase | 276 | AMC96537.1 | Bacterial | DNA |

**Table 2. Strains that may be use in the context of the present invention as non-animal organism (DSP Host organisms and examples of expressable collagens. This list only mentions strains that were generated for recombinant protein production. E. coli JM109 that is also usable to amplify and purify plasmids for further applications is not enlisted):**

| **Strain Species** | **Backbone** | **Expressed protein** | **Protein details** |
|---|---|---|---|
| BL21(DE3) pLysS | pET151 | | |
| *Escherichia coli* | | | No codon optimizations, sequence like in plant recombinant expression |
| | | Col1A1 | Bovine Collagen Col1A1 without N- and C-terminal propeptides. Same as used in plants |
| PPS-9010 or BG10 (from ATUM) | VB standard backbone for Pichia | Col1A1 | α-Factor-HIS-TEV-Col1A1 - |
| | | | Bovine Collagen Col1A1 without N- and C-terminal propeptides |
| *Pichia pastoris* | | | |
| N. benthamiana | p-Cambia | Col1A1 | His-TEV-Col1A1; Col1A1 |
| | | P4Ha | |
| | | P4Hb | |
| N. tobacum | p-Cambia | Col1A1 | His-TEV-Col1A1; Col1A1 |
| | | P4Ha | |
| | | P4Hb | |

In a preferred embodiment, the collagen expressed by the non-animal organism is selected from Table 1.

In a preferred embodiment, the non-animal organism is selected from those depicted in Table 2 - which may be independent of the exemplified collagen.

The non-animal organism preferably contains genetic material that encodes for the collagen that naturally occurs extracellularly in one or more animal species. Such genetic material is a foreign genetic material. Its sequence at least partly corresponds to a sequence of animal origin, while it is expressed in a non-animal organism. The genetic material may be any genetic material known in the art. The genetic material may, for instance, be deoxyribonucleic acid (DNA), ribonucleic acid (RNA), an analogue thereof such as peptide nucleic acid (PNA), morpholino and locked nucleic acid (LNA), glycol nucleic acid (GNA), threose nucleic acid (TNA) and hexitol nucleic acids (HNA), or a combination of two or more thereof. It will be understood that the DNA, RNA, analogue thereof, or combination thereof may be optionally stabilized by any means such as, e.g., terminal (e.g., 3'-terminal and/or 5' terminal) capping.

DNA may be double stranded DNA or single stranded DNA, preferably double stranded DNA. RNA may be double stranded RNA or single stranded RNA. Also, a nucleic acid analogue may be double stranded or single stranded. DNA, RNA, analogue thereof, or combination thereof may be linear or circular. For instance, circular DNA may be a plasmid.

The present invention is not limited to a certain type of gene vector. The present invention is not limited to a certain type of plasmid.

A large variety of examples of plasmids that may be used in the context of the present invention are well-known by the person skilled in the art. It will be understood that the plasmid, including its enhancers and optional enhancer sequences, start and stop sequences may be adapted to the non-animal organism as used. Optionally, a plasmid or other vector may further lead to resistance against one or more antibiotics. This allows selection of successfully transfected cells from non-transfected cells. While non-transfected cells are killed or at least growth-arrested, the transfected cells are viable in a culture medium containing the respective antibiotic. This is well-known by the person skilled in the art.

Examples that may be used in the context of the present invention may be selected from Tables 1 and 2 herein, without being bound to certain collagen cargos, which can be freely adapted by routine means.

The inclusion of DNA, RNA, analogue thereof, or combination thereof into the non-animal organism may also be considered as "transfection". Such transfection may be achieved by any means known in the art such as, e.g., electroporation, using a gene gun or a vector (e.g., a viral vector, a cell-penetrating peptide, etc.). A transfection may be a permanent or a transient transfection. For the expression in vectors with N-terminal tags, such as, e.g., pET151, a start codon may be omitted, because there may be already one before the tag. Methionin as the first amino acid may be optionally cleaved anyway. However, when it is desirable, the start codon may be kept, because there is only one RBS, before the tag, so there should be no recognition of two different open reading frames (ORFs). However, pET151 may need one or two stop codons. Optionally, two TAAs may be added at the end of the sequence.

When the non-animal organism is a plant, transfection may also involve the generation of a callus. Optionally, the cell wall of a cell to be transfected may be partly or completely removed.

The person skilled in the art is well aware of a variety of methods for heterologous expression of genes in non-animal organisms as such. Examples are mentioned below in the example section.

In a transient transfection, the genetic material is incorporated into the organism for a limited time such as for few hours, view days, few weeks or few months. A permanent transfection leads to a longer duration of the genetic material in the cells. Typically, a non-animal organism that expresses the genetic material encoding for the collagen permanently can also pass the genetic material to subsequent generations upon breeding. Optionally, DNA, RNA, analogue thereof, or combination thereof, in particular DNA, may be incorporated into the non-animal organism' genome. This may be preferably passed to subsequent generations of the non-animal organism. A permanent expression may also be achieved by using one or more plasmids that may optionally replicate in the cells of the non-animal organism expressing the collagen.

In a preferred embodiment, the non-animal organism comprises the genetic material encoding for the collagen permanently. In a preferred embodiment, the genetic material is incorporated in the non-animal organism's genome. In a preferred embodiment, the non-animal organism comprises the genetic material encoding for the collagen permanently and the genetic material is incorporated in the non-animal organism's genome.

In an alternative preferred embodiment, the non-animal organism comprises the genetic material encoding for the collagen transiently.

In a preferred embodiment, the non-animal organism as a whole is comestible, i.e., edible and/or drinkable and/or suitable to prepare a beverage or foodstuff of it.

As used in the context of the present invention, the term "comestible" may be understood as generally understood in the art as consumable without (substantial) health risk when consuming reasonable amounts as indicated herein. Preferably, "comestible" means components suitable for animal and, in particular human, food consumption. This may also be interpreted in the context of accreditation of official regulatory offices. As used in this context, "reasonable amounts" may depend on the properties of the material. Typically, a reasonable consumable amount may range in the oral dose range of the collagen of milligrams or grams per day, such as, e.g., 0.0001 to 1000 g/day of the collagen, 0.005 to 100 g/day of the collagen, 0.001 to 10 g/day of the collagen, 0.05 to 5 g/day of the collagen, or 0.1 to 1 g/day of the collagen. Specific ranges will be understood by a person skilled in the art.

In a preferred embodiment, the collagen occurs in or is used in natural animal-derived food or comestible nutrient. Preferably, such animal-based comestible nutrient is vegetarian.

In a preferred embodiment, the collagen is used as a gelling agent in food, a beverage, a medication, a capsule (e.g. a vitamin, nutrient or medicament capsule), a photographic film, paper, and/or a cosmetic (e.g., as texture conditioner, moisturizer, and/or filler).

In a preferred embodiment, the collagen occurs in or is used in a composition that is comprised in at least one vegetarian animal-based comestible nutrient, in particular a dairy and/or an egg-based product.

In a preferred embodiment, the collagen occurs in or is used in a dessert such as, e.g., candy (e.g., gummy candy, marshmallows), ice cream, a bakery good (e.g., torte, sweetie, cake, baklava, cake glaze, frostings, cake fillings, no-bake cakes), a dip, a jam, a diary product (e.g., yogurt or creams, desserts). In a preferred embodiment, the collagen occurs in or is used in a spicy food such as in aspic, a (optional vergetarian) sausage, cream cheese, spread, margarine and/or a sauce or soup.

In a preferred embodiment, the collagen occurs in or is used in a composition that is comprised in at least one vegetarian animal-based comestible nutrient, in particular a vegetarian animal-based comestible nutrient selected from the group consisting of candy (e.g., gummy candy, marshmallows), ice cream, a bakery good (e.g., torte, sweetie, cake, baklava, cake glaze, frostings, cake fillings, no-bake cakes), a dip, a diary product (e.g., yogurt or creams, desserts ), aspic, a vegetarian sausage, cream cheese, jam, spread, margarine and/or a sauce or soup.

In a preferred embodiment, the collagen is used as a stabilizer, thickener, or texturizer such as, e.g., in food, medical uses, cosmetic uses. Collagen may also be used as binder and/or as glue.

In a preferred embodiment, the collagen is usable for cooking such as, e.g., as an instant ingredient, and may be provided as powder, granule, or sheet.

Accordingly, a "comestible nutrient" may be understood in the broadest sense as any material that is consumable without (substantial) health risk when consuming reasonable amounts. Preferably, a "comestible nutrient" may be understood in the broadest sense as any foodstuff composition that may be consumed by drinking and/or eating.

The collagen that naturally occurs extracellularly in one or more animal species may be located in the non-animal organism expressing it in at any location.

The intracellular protein may be a soluble collagen or may form collagen aggregates, which may optionally precipitate.

In a preferred embodiment, the expressed collagen is located intracellularly in the non-animal organism.

For instance, the expressed collagen may be located in the cytoplasm of cells of non-animal organism and/or may be located attached to a membrane of cells of non-animal organism or included in a membrane of cells of non-animal organism. The intracellular collagen may be a soluble collagen or may be a membrane-bound collagen and/or may form collagen aggregates. Alternatively, the expressed collagen may be located in protein storage vacuoles, endoplasmic reticulum (ER) or chloroplasts.

In another preferred embodiment, the expressed collagen is located extracellularly in the non-animal organism.

The extracellular collagen may be secreted by cells of the non-animal organism. In the case that the non-animal organism is a monocellular organism such as, e.g., a bacterium or a yeast, the extracellular collagen may be secreted into the cell culture medium in which the cells are cultivated. In the case that the non-animal organism is a multicellular organism such as, e.g., a plant or a fungus, the extracellular collagen may be secreted outside the organism, e.g., via a gland, or may be located in the extracellular space inside the non-animal organism. The extracellular collagen may be a soluble collagen or may be a membrane-bound collagen and/or may form collagen aggregates.

The expressed collagen may optionally comprise one or more means for its purification.

In a preferred embodiment, the expressed collagen contains a tag, in particular a His-tag.

Such a tag may allow affinity purification of the expressed collagen. A tag may be any sequence that allows specific binding. For instance, a tag may be a His-tag, a (poly)peptide sequence that may specifically bind to a target structure such as, e.g, streptavidin, or may be a sequence that is detected by an antibody. A His-tag may comprise any number of histidine (His) residues such as, e.g., five, six, seven or eight His residues. In a preferred embodiment, the collagen comprises a hexahistidine (6His) tag that may contain six consecutive histidine residues. Such tag (e.g., a His-tag) may be located N-terminally or C-terminally of the collagen strand.

A cleavage site for polypeptide purification may be any method known in the art for purifying polypeptides. For instance, a cleavage site for polypeptide purification purposes may be TEV cleavage site (as recognized by TEV (Tobacco Etch Virus) cysteine protease) or a functional homologue thereof, in particular TEV.

The collagen and optionally further parts of the non-animal organism is expressed by forming part of a comestible nutrient composition.

The obtained comestible nutrient product (e.g., a powder or a liquid or a gel) may optionally be hermetically sealed. The product may be prepared at and/or subsequently treated below room temperature (i.e., <20°C), at room temperature (e.g., (approximately) 20°C), or at increased temperature (>20°C, such as exemplarily at 60-120°C for faster dissolving). The product may optionally be sterilized (e.g., by pasteurization, heating, irradiation with ultraviolet (UV) light or X-rays, gamma-rays, etc.).

The collagen, or non-animal organism comprising it or comestible nutrient composition comprising the collagen and/or non-animal organism or parts thereof may be consumed directly. Optionally, the collagen or comestible nutrient composition comprising it may be processed further by any means known in the art. In one embodiment, the method further comprises a step of:
(a) fermenting the comestible nutrient composition;
(b) smoking the comestible nutrient composition;
(c) pickling a comestible good with a composition comprising the comestible nutrient composition, preferably in combination with one or more further component such as at least 1% by weight, referred to the composition, of sodium chloride, at least 1% by weight, referred to the composition, of one or more types of sugar, at least 1% by weight, referred to the composition, of acetic acid, at least 0.5% by weight, referred to the composition, of ethanol, or at least 5% by weight, referred to the composition, one or more types of edible oil;
(d) thickening the comestible nutrient composition to obtain a syrup, in particular wherein sugar and/or other sweetener is added to obtain a sweet syrup; and/or
(e) combining one or more collagens or collagen fragments in a comestible nutrient composition

As used herein, fermenting may be understood in the broadest sense as any kind of metabolic process that produces chemical changes in the organic substrates as contained in the composition through the action of enzymes. Such enzymes may optionally originate from the non-animal organism, may be added and/or may be present in the form of microorganisms and/or secreted from such. In one embodiment, fermenting may be used for increasing umami taste. In one embodiment, fermenting may be used for increasing shelf-life. In one embodiment, a fermented comestible nutrient composition may, after the step of fermenting, comprise at least 0.5% by weight, at least 1% by weight, at least 2% by weight, at least 5% by weight, at least 10% by weight, based on the total weight of the comestible nutrient composition.

A step of thickening may be conducted by any means. It may comprise partial removal of water and/or addition of further ingredient having thickening properties such as, e.g. one or more sugars, one or more other (poly)saccharides (e.g. starch, resistant starch, agar, pectin, inulin, fructans, raffinose, polydextrose), one or more thickening sweeteners, one or more thickening agents (e.g., cellulose, hemicellulose, carboxymethyl cellulose, carboxy ethyl cellulose, carboxypropyl cellulose, chitin, beta-glucan, raw guar gum, xanthan gum, lignin, a polyuronide, alginic acid or a salt thereof (e.g., sodium alginate), or a combination of two or more thereof) or a combination of two or more thereof.

A step of coagulating the collagen, optionally comprised in a comestible nutrient composition, may be conducted by any means. For instance, it may be conducted by adding one or more components facilitating coagulation such as, e.g., one or more collagens (e.g., one or more enzymes, preferably one or more (poly)peptidases/proteases (e.g., chymosin, pepsin) and/or one or more lipases, in particular enzyme mixtures such as, e.g., lab (also: rennet)), acidification, heating, cooling, one or more freeze/thaw cycles, or a combination of two or more thereof. Enzymes used for this purpose, including lab, may be obtained from any natural sources or from any biotechnological means. This may optionally provide a cheese-like semi-solid product ("curd") and separate it from a liquid phase ("whey"). For instance, casein may be coagulated by addition of one or more enzymes (e.g., lab). This may allow preparing a curd containing casein and a whey fraction containing the non-coagulated components. This may allow preparing a cheese-like product from casein. Herein, the casein and/or the one or more enzymes used for coagulation may be obtained as the collagen from the method of the present invention.

Optionally, such comestible nutrient composition may form a dried powder. If so, such dried powder may be the final product (comestible nutrient composition). Alternatively or additionally, such powder may be re-dissolved or resuspended in a suitable liquid such as, e.g, water, a consumable aqueous buffer, or a mixture of one thereof comprising ethanol. Accordingly, on a preferred embodiment, the comestible nutrient composition is a drinkable composition, and the method includes the further step of suspending the powder in an aqueous liquid, in particular in mineral or tap water. Alternatively or additionally, such powder may be further processed into a granulate.

In one embodiment, the comestible nutrient composition is:
(a) a drinkable composition such as a drink;
(b) a powder or granulate composition;
(c) a gel; or
(d) a frozen or partly frozen composition,
wherein the comestible nutrient composition may optionally form part of a filling of a capsule or may optionally form part of a drink, a dairy product, a non-dairy cream, a sauce, or a bakery good.

The collagen and optionally further parts of the non-animal organism may optionally form part of a drink or foodstuff or a gel.

As used herein, the terms "drink" and "beverage" may be understood interchangeably in the broadest sense as generally understood in the art as a liquid or syrup-like orally consumable composition. It may or may not contain alcohol. A drinkable composition may be in liquid or pasty form, preferably in liquid form.

Optionally, a lyophilized or dried product (which may be, e.g., a powder or foam-like) may be milled to form a homogeneous powder. Such optional milling process may be applied using a pulverizer.

As used herein, the term "gel" may be understood in the broadest sense and may also comprise a gel as such, any kind of jelly composition, a filling of a capsule, filling of a bakery good, a pudding form, etc.

The comestible nutrient composition may also be frozen or partly frozen composition, including an ice cube, frozen yogurt-like, soft ice like, slushies and the like.

A further aspect of the present invention refers to the use of a comestible nutrient composition of the present invention for providing a well-defined nutrient composition, in particular a well-defined protein composition, to a consumer.

The comestible nutrient composition may optionally further comprise one or more further consumable ingredients selected from the group consisting of one or more vitamins, one or more minerals, one or more aroma compounds, one more food colors, one or more types of fibers, ethanol, acetic acid, carbonic acid, and combinations of two or more thereof. Ingredients may interchangeably also be designated as components, additives, etc.

This may allow compensating for common micronutrient deficiencies of consumers (e.g., iron, vitamin A and iodine, but possibly also zinc, folate, vitamin B12, other B vitamins, vitamin C, vitamin D, calcium, selenium and fluoride).

A vitamin may be any vitamin known in the art. For instance, vitamins that may be added are selected from the group consisting of vitamin A, vitamins B (e.g., vitamin B1, vitamin B2, Vitamin B3, vitamin B5, vitamin B6, folate, vitamin B12), vitamin C, vitamin D, and metabolic precursors and combinations of two or more thereof. A vitamin or metabolic precursor thereof may be of natural or synthetical origin (nature-identical in structure or artificial in structure). It may be commercially available.

Preferably, the comestible nutrient composition comprises not more than 20% by weight, not more than 10% by weight, not more than 5% by weight, not more than 2% by weight, or not more than 1% by weight, referred to the total mass of the comestible nutrient composition, of total amount of vitamins.

A mineral may be any nutrient that may be of nutrient value for consumers. Typically and preferably, minerals comprise ions or complexes of metals such as, e.g., a metal selected from the group consisting of iron, copper, calcium, zinc, and a combination of two or more thereof. Furthermore, minerals may also be selenium, iodine, and/or fluoride. A mineral may be commercially available. Preferably, the comestible nutrient composition comprises not more than 20% by weight, not more than 10% by weight, not more than 5% by weight, not more than 2% by weight, or not more than 1% by weight, referred to the total mass of the comestible nutrient composition, of total amount of minerals.

An aroma compound may be any comestible compound known in the art that alters the flavor or taste. Preferably, an aroma compound has a significant impact on the comestible nutrient composition's taste, when it is applied in low amounts. Preferably, the comestible nutrient composition comprises not more than 20% by weight, not more than 10% by weight, not more than 5% by weight, not more than 2% by weight, or not more than 1% by weight, referred to the total mass of the comestible nutrient composition, of total amount of aroma compounds. An aroma compound may be of natural or synthetical origin (nature-identical in structure or artificial in structure). For example, an aroma compound may be menthol furaneolhexyl cinnamaldehyde, isovaleraldehyde anisic aldehyde cuminaldehyde, glutamate, fructone, ethyl methylphenylglycidate, dihydrojasmone, oct-1-en-3-one, 2-acetyl-1-pyrroline, 6-acetyl-2,3,4,5-tetrahydropyridine, delta-octalactone, massoia lactone, diacetyl acetoin, nerolin, and combinations of two more thereof. Many aroma compounds are known by those skilled in the art and may be commercially available.

A food color may be any comestible dye known in the art that alters the color of food, also designatable as food coloring. Preferably, a food color has a significant impact on the comestible nutrient composition's color, when it is applied in low amounts. The coloring may be freely selectable.

The comestible nutrient composition may have any texture, which may be defined by optionally added to the comestible nutrient composition. For example, as texturizing and/or nutrient, one or more further collagens of extracellular matrix (e.g., collagen, elastin, etc.) may be added to the comestible nutrient composition.

Preferably, the comestible nutrient composition comprises not more than 20% by weight, not more than 10% by weight, not more than 5% by weight, not more than 2% by weight, or not more than 1% by weight, referred to the total mass of the comestible nutrient composition, of total amount of food colors. A food color may be of natural or synthetical origin (nature identical in structure or artificial in structure). For example, a food color may be beetroot juice, beta-carotene, quinoline yellow, Ponceau 4R, Patent blue V, Green S, Brilliant blue FCF, Citrus red 2, Orange B Indigotine, Fast green FCF, Erythrosine, Allura red AC, Tartrazine, Sunset yellow FCF, or a combination of two or more thereof. A food color may even be fluorescent such as fluorescein. Many food colors are known by those skilled in the art and may be commercially available.

Fibers may be any comestible fibers, which may also be designated as dietary fibers. Typically, fibers in the context of the present invention are plant-derived food ingredients that cannot be completely broken down by human digestive enzymes. For example, it may originate from legumes, whole grains and cereals, vegetables, fruits, nuts or seeds. For example, fibers may comprise or consist of cellulose, hemicellulose, chitin, pectin, resistant starch, or a combination of two or more thereof. Fibers may also comprise inulin, beta-glucan, raw guar gum, xanthan gum, fructans, lignin, a polyuronide, an alginic acid or a salt thereof (e.g., sodium alginate), agar, carrageen, raffinose, polydextrose, or a combination of two or more thereof. Many fibers are known by those skilled in the art and may be commercially available.

Based on the production process of the present invention, but also as component of the admixed finished comestible, proteins of the extracellular matrix (ECM) may be comprised in the composition, such as, e.g., collagen type I, hyaluronic acid, polylysin, vinculin, laminin, fibronectin.

Optionally, the comestible nutrient composition may comprise one or more juices, optionally in addition to one or more of the further ingredients mentioned herein. For instance, it may comprise one or more fruit or vegetable juices.

The pH of the comestible nutrient composition may be adjusted to a desired range. Preferably, the pH is essentially neutral or sour. In one embodiment, the pH is in the range of pH 2-9, pH 2-8, pH 3-7, pH 4-7, pH 5-7, or pH 4-6. The pH may be adjusted by comestible acids, bases, buffer agents and/or acidity regulators.

In a preferred embodiment, a high-grade nutritional liquid product for individual demands may be obtained. The comestible nutrient composition may be adjusted to individual lifestyle demands, thus, its content ranges may be adapted to the desire of a specific consumer or group of consumers. This may be considered as "customizable food" and/or "personalized food".

The present invention also refers to a method of preparing the collagen that naturally occurs extracellularly in one or more animal species. Thus, a further aspect of the present invention relates to a method for preparing a collagen that naturally occurs extracellularly in one or more animal species, comprising the steps of:
(i) providing a non-animal organism comprising genetic information encoding for the collagen that naturally occurs extracellularly in one or more animal species;
(ii) expressing the collagen in the non-animal organism and optionally subjecting the collagen to one or more posttranslational modifications.

It will be understood that the definitions and preferred embodiments laid out in the context of the non-animal organism as laid out above *mutatis mutandis* apply to the method for preparing the collagen.

It will be understood that the process may be conducted in various size ranges. It may optionally be conducted in a laboratory scale such as, e.g, in a flask, or in a large industrial scale such as, e.g., in a fermenter. Likewise, the mass of non-animal organism may be adapted.

The conditions may be adapted to the non-animal organism chosen. For example, some organisms may be supported by adding oxygen, others may prefer the essential absence of gaseous oxygen. Likewise, the temperature may be adapted to each organism. A temperature range at which the non-animal organism expresses a collagen may be chosen depending on the conditions. In certain embodiments, it is in the range of 18 to 40°C, 18 to 22°C, 22 to 30°C, or 35 to 38°C.

Providing the non-animal organism may be conducted by any means. The non-animal organism may be optionally directly prepared such as, e.g., by transfecting the non-animal organism with genetic material encoding for the collagen. Alternatively, it may be a permanently genetically modified organism that may optionally also be breeded. Optionally, the non-animal organism may be obtained from a stock and may optionally be (deep)frozen.

Expressing the collagen may be performed permanently or may be induced in case of using an inducible promoter and/or enhancer. The collagen expression may also be transient in case of using plant hosts (e.g. *N. tabacum, N. benthamiana)*

In a preferred embodiment, the method further comprises one or more of the following:
harvesting the non-animal organism that has expressed the collagen;
isolating the collagen from the non-animal organism or cell-culture medium in which the non-animal organism is cultivated;
preserving the collagen or composition comprising such from spoiling; and/or freeze-drying or drying of the collagen or composition comprising such.

As used herein, the term "harvesting" may be understood in the broadest sense as any means for obtaining the non-animal organism. For instance, a monocellular organism such as, e.g., bacterium or yeast, may be harvested by centrifugation and optional washing steps. For instance, a multicellular organism such as, e.g., a plant or a fungus, may be harvested by chopping or picking the parts of interest. The parts of interest of a plant may exemplarily be the aerial parts or the roots, the leaves, the stem, the blossoms, the flowers, and/or the fruits. Harvesting may include one or more steps of releasing material therefrom and optionally lysing cells. Thus, harvesting may include exposing of cells of the non-animal organism, parts of the non-animal organism, or the non-animal organism as a whole to a lysis buffer (e.g., containing one or more surfactants) and/or sonication. This may optionally be combined with one or more further centrifugation steps.

As noted above, the collagen may be located inside the non-animal organism or may be located outside. The collagen may be isolated from the non-animal organism or the surrounding environment such as the cell culture medium in which the non-animal organism is cultivated. As used herein, the term "isolating" may be understood in the broadest sense as obtaining the collagen and separating it from other parts of the non-animal organism or the surrounding environment. It does not have to be entirely pure. Isolating may also comprise a step of precipitation such as, e.g, by addition of salt (salting out, e.g., by means of ammonium chloride, such as 20 to 70%, or 40 to 60% of ammonium chloride), or addition of an anti-solvent (e.g. ethanol or other organic solvents), or reducing the salt concentration (salting in). The person skilled in the art will be aware of means and will be able to adapt these to the respective collagen to be purified.

Optionally, isolating may also include the coagulation. Coagulation may be conducted by any means such as, e.g., adding one or more components facilitating coagulation such as, e.g., one or more collagens (e.g., one or more enzymes, (e.g., one or more (poly)peptidases/proteases such as, e.g., chymosin, pepsin) and/or one or more lipases), acidification, heating, cooling, one or more freeze/thaw cycles, or a combination of two or more thereof. Coagulation may separate a fraction containing one or more coagulated components (curd-like fraction) and a fraction containing one or more components that remain soluble in the liquid fraction (whey-like fraction). Depending on the characteristics of the collagen of interest expressed by the non-animal organism, this may be located in the coagulated fraction or the liquid fraction. Optionally, the fraction of interest may be treated further by any means.

Optionally, isolating may also include the removal of salt such as, e.g., via dialysis and/or chromatographic means (e.g., size-exclusion chromatography (SEC)). Dialysis and/or chromatographic means (e.g., size-exclusion chromatography (SEC)) may optionally also be used for increasing the concentration of the collagen. As used herein, the term "preserving" may be understood in the broadest sense as any means for preventing spoiling. For instance, one or more preserving agents may be added which may be preferably comestible preserving agents such as, e.g., benzoic acid. As indicated above, the collagen or a composition containing such may be subjected to fermenting, smoking, pickling and/or thickening to preserve. The collagen or a composition containing such may be optionally heated such as, e.g., pasteurized. The collagen or a composition containing such may optionally also be freeze-dried, frozen, dried, or cooled to preserve it.

The collagen or a composition containing such may optionally subjected to freeze-drying or drying. This may be achieved by routine means. In a preferred embodiment, this is achieved by chromatographic means such as, e.g., affinity chromatography, size-exclusion chromatography (SEC), ion exchange chromatography (IEX), reverse-phase chromatography, high-performance liquid chromatography (HPLC), ultra high-performance liquid chromatography (UPLC), fast protein liquid chromatograph (FPLC), or a combination of two or more thereof. Such chromatographic step may be conducted at any suitable temperature, preferably in a temperature range of 0 to 30°C, 3 to 15°C or 15 to 25°C. Optionally, the purity of the collagen may be determined.

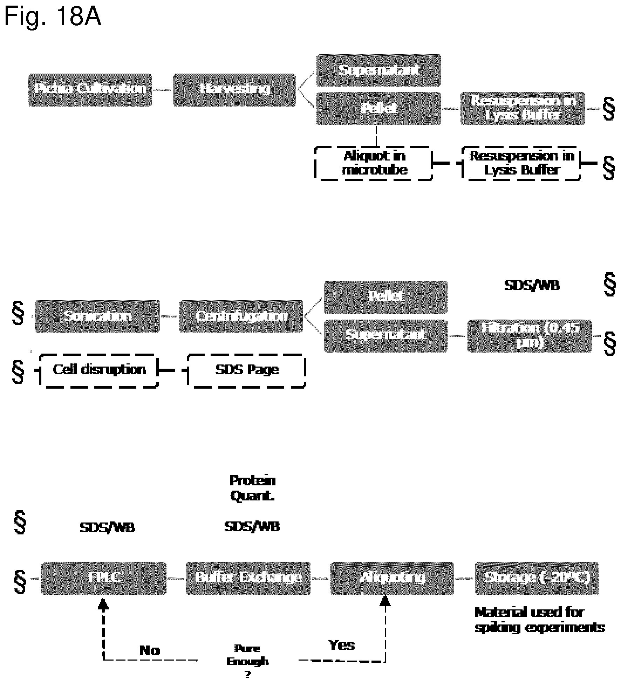

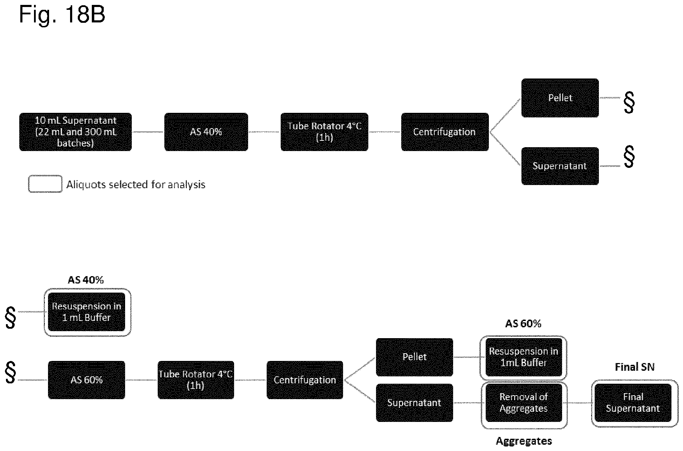

An exemplified, non-limiting example flow scheme is shown in Figure 18A, where it will be understood that such process may be modified and is not limited to *Pichia pastoris* and not limited to collagen purification. In addition, an exemplified, non-limiting example flow scheme depicting the complementary harvesting of POI from supernatant, is shown in Figure 18B.

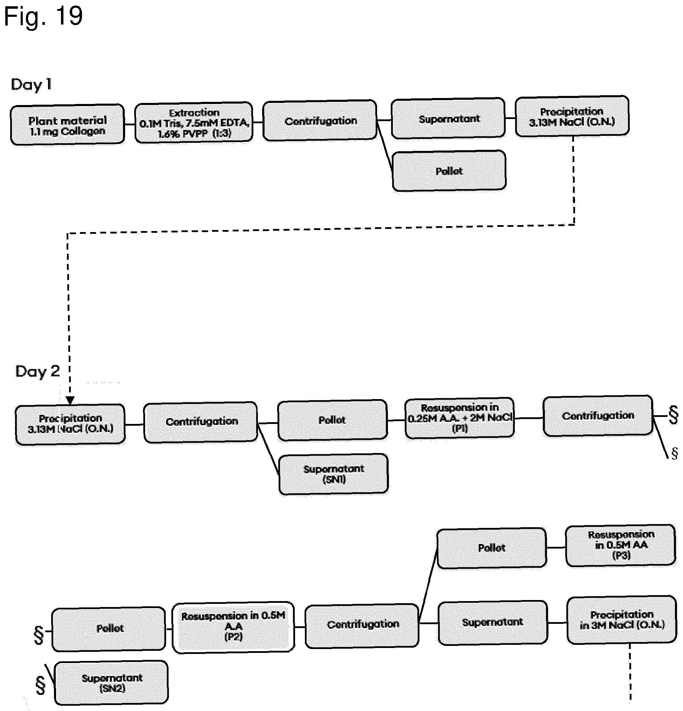

A further exemplified, non-limiting example flow scheme is shown in Figure 19, where it will be understood that such process may be modified and is not limited to *Pichia pastoris* and not limited to collagen purification.

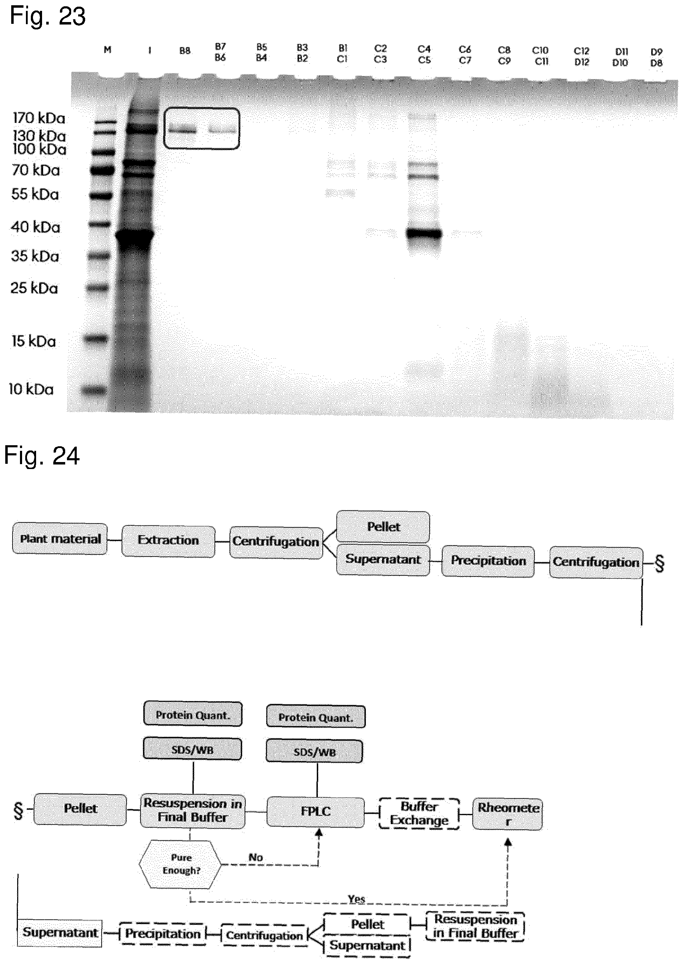

A still further exemplified, non-limiting example flow scheme is shown in Figure 24, where it will be understood that such process may be modified and is not limited to plants and not limited to collagen purification.

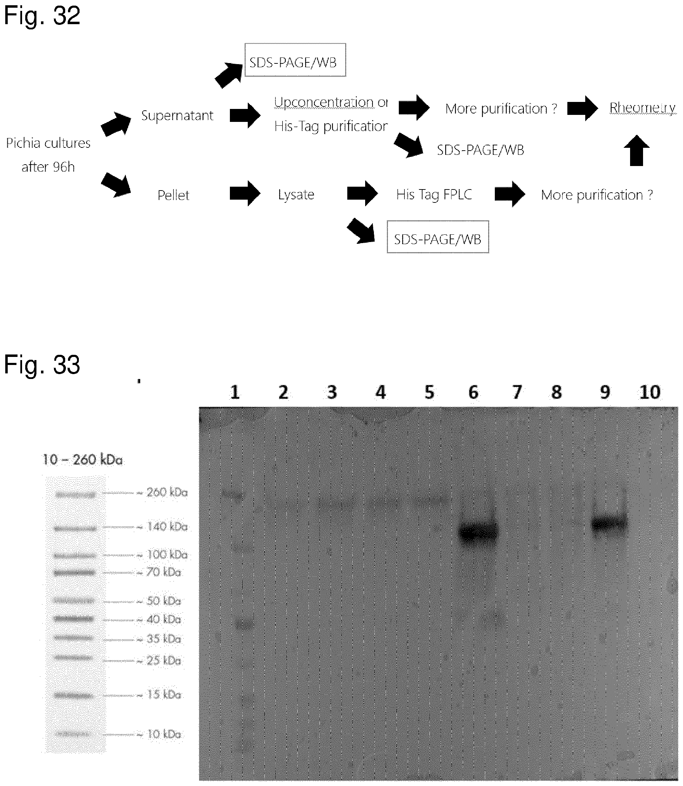

A still further exemplified, non-limiting example flow scheme is shown in Figure 32, where it will be understood that such process may be modified and is not limited to *Pichia pastoris* and not limited to collagen purification.

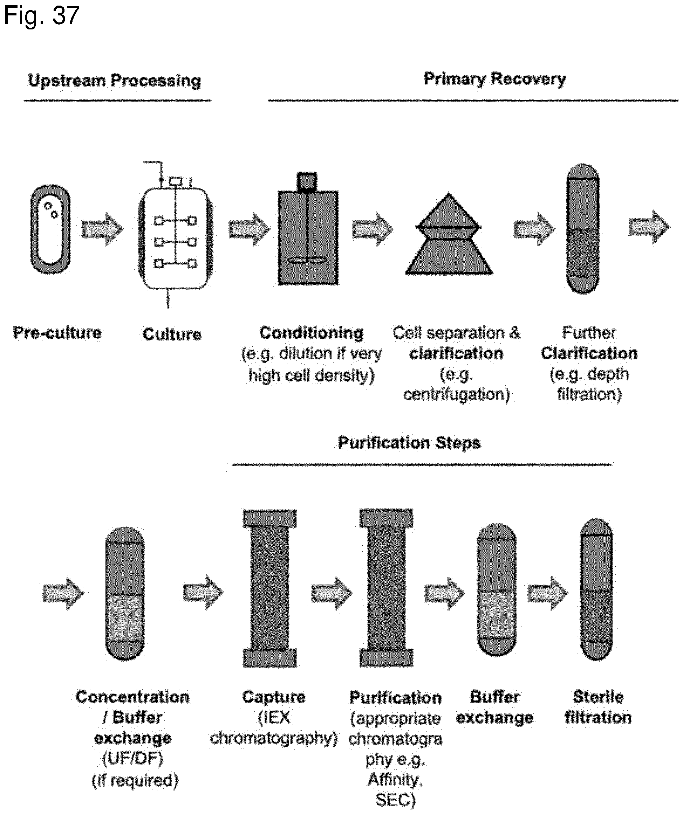

A still further exemplified, non-limiting example flow scheme for upscaling the process is shown in Figure 37.

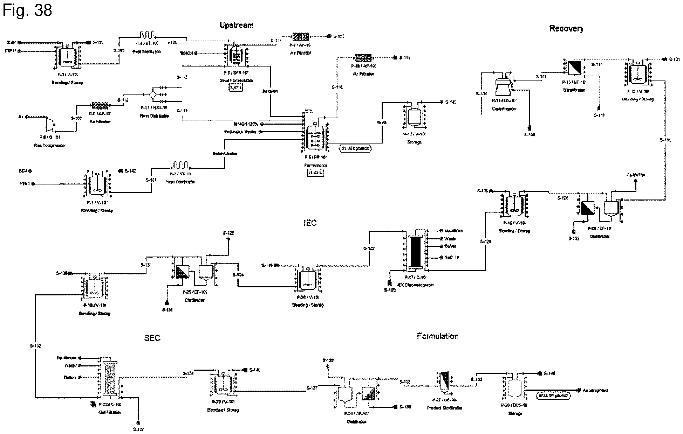

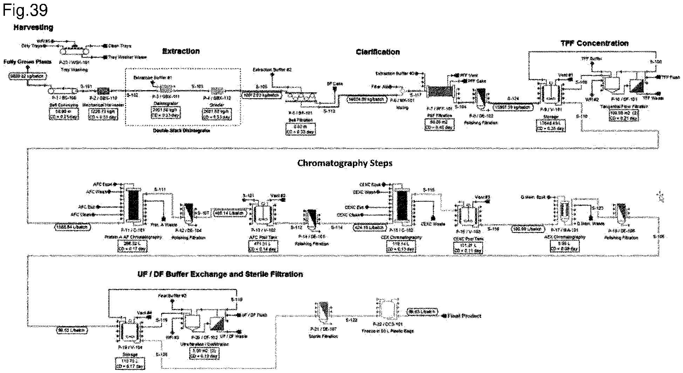

Further exemplified, non-limiting examples of detailed process schemes are shown in Figures 38 and 39.

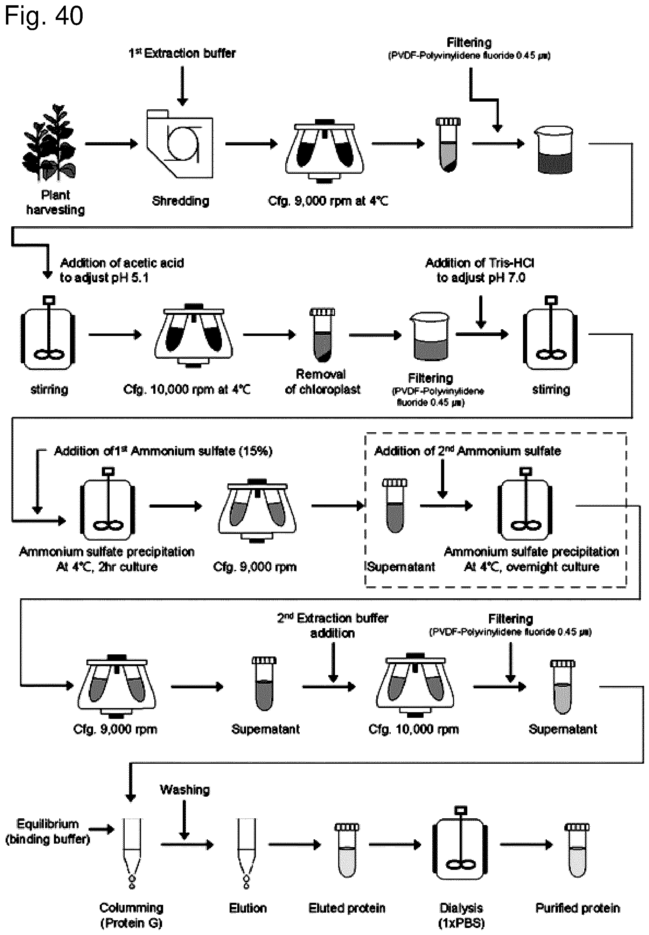

A still further exemplified, non-limiting example flow scheme for preparing collagen from plants is shown in Figure 40, where it will be understood that such process may be modified and is not limited to plants.

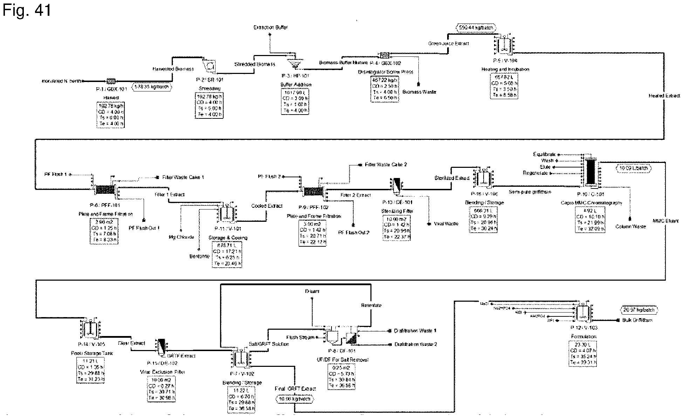

A still further exemplified, non-limiting examples of detailed process schemes is shown in Figure 41.

Optionally, the purity of the collagen may be further improved. In a preferred embodiment, the method comprises purification the collagen by affinity chromatography. For example, the content of the collagen may be at least 1% by weight, at least 5% by weight, at least 10% by weight, at least 25% by weight, at least 50% by weight, at least 70% by weight, at least 80% by weight, or at least 90% by weight, based on the total (poly)peptide/collagen content.

Affinity chromatography may be performed by any means. As indicated above, affinity chromatography may be based on a tag such as, e.g., a His-tag that is selectively bound by a binding partner, in case of a His-tag for example nickel ions. An affinity chromatography column may be loaded with the collagen, which may be eluted by using an elution agent such as, e.g., in case of a His-tag, a nickel-binding agent that may disturb the binding of the His-tag to the column.

In a preferred embodiment, the method further comprises:
(iii) preparing a solution of the collagen in dissolved form, optionally filtering and/or centrifugation of the solution to remove any remaining solid particles or debris and obtaining a clear collagen-containing solution, and optionally precipitating the collagen;
(iv) isolating the collagen of step (iii) via chromatography and collecting collagen-containing fractions;
(v) optionally subjecting the collagen of step (iv) to dialysis or buffer exchange vis a size exclusion chromatography column.

In a preferred embodiment, the method further comprises isolating the collagen by affinity chromatography, preferably by an antibody and/or by binding to a tag attached to the collagen, in particular a His-tag.

The obtained collagen may be stored at any conditions suitable for such storage. For instance, the collagen may be stored in solution or in dry state (e.g., dried or freeze-dried). It may be stored at any suitable temperature such as, e.g., in a range of -100 to -20°C, -20 to 0°C, 1 to 10°C, or 10 to 30°C, for example in liquid nitrogen, in a deep freezer (e.g., -90°C to -70°C), in a freezer (e.g., -25 to -5°C), in a fridge (e.g., 2 to 8°C) or at ambient temperature (e.g. 18 to 25°C).

As indicated above, it is of interest to prepare a comestible nutrient product. Thus, a further aspect of the present invention relates to a method for preparing a comestible nutrient product, in particular a vegetarian comestible nutrient product, comprising the step of adding the collagen that naturally occurs extracellularly in one or more animal species obtained from a method of preparing a collagen of the present invention to one or more further comestible nutrient ingredients.

It will be understood that the definitions and preferred embodiments laid out in the context of the non-animal organism and the method for preparing the collagen as laid out above *mutatis mutandis* apply to the method for preparing a comestible nutrient product.

As indicated above, such comestible nutrient product may be any comestible nutrient product known in the art such as, e.g., a beverage, a drink, food, etc.

As indicated above, the present invention also comprises a comestible nutrient product obtainable from the methods of the present invention. Thus, a further aspect refers to a comestible nutrient product, in particular a vegetarian comestible nutrient product, comprising at least one collagen that naturally occurs extracellularly in one or more animal species obtained from a method of the present invention.

It will be understood that the definitions and preferred embodiments laid out in the context of the non-animal organism, the method for preparing the collagen and the method for preparing a comestible nutrient product as laid out above *mutatis mutandis* apply to the comestible nutrient product.

The following Examples as well as the accompanying Figures are intended to provide illustrative embodiments of the present invention described and claimed herein. These Examples and Figures are not intended to provide any limitation on the scope of the invented subject-matter.

### Brief Description of the Figures

**Figure 1** shows His-tag purification of Collagen from *P. pastoris* (20ml), wherein the Y-axis shows conductivity in mS/cm, The UV detection at 280 nm (1), the conductivity (2) and the gradient of eluent (3) is depicted.
**Figure 2** shows mass spectrometry results of detected peptides aligned to the sequence of recombinant collagen. The black bars indicate hydroxylated amino acid residues. Besides proline hydroxylation, there is also a methionine hydroxylation that occurs during the preparation of samples. Methionine hydroxylation appears more frequently with much larger bars. His-Tag was not detected.
**Figure 3** shows an agarose gel analysis of the *Pichia pastoris* clones of Example 1. The lanes are as follows: 10 kb ladder (1), Clones A1-A8 (2-9), 100 bp ladder (10), empty (11 and 13), negative control (12), and positive control (14).

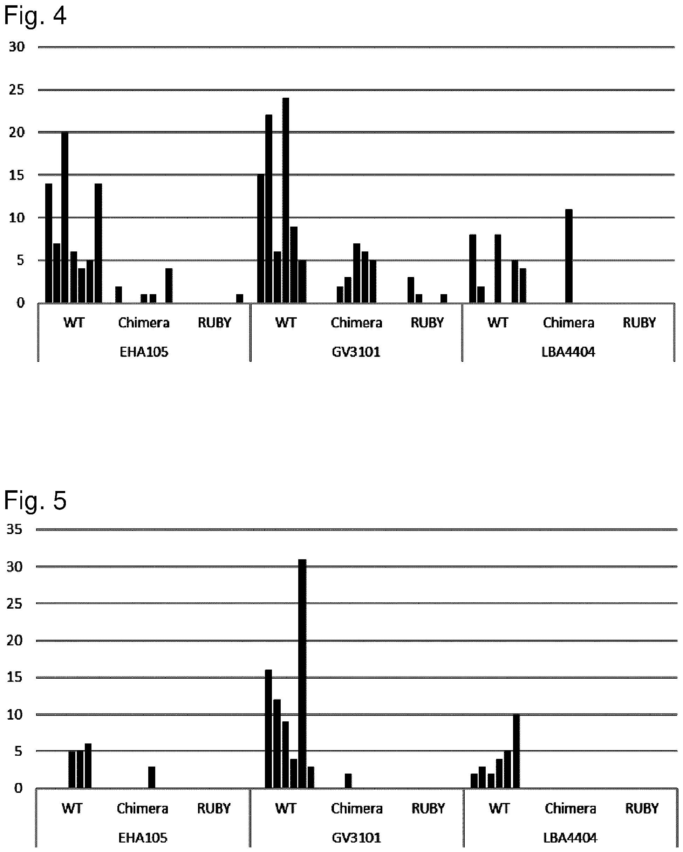

**Figure 4** shows the regeneration efficiency of Nicotiana benthamiana dependent on the concentration of selection agent (BASTA) and the Agrobacterium strain (EHA105, GV3101 and LBA4404) used for transformation. Regeneration Media (Musashiagei-Skoog media (MSII) inculiding Timentin, Terbinafin, Cefotaxime (MSII-TTC). From left to right, the results depict BASTA concentration of 0, 0.5, 1.0, 1.5, 2.0, 3.0 and 4.0 mg/ml. The Y-axis shows the number of regenerated explants. Regenerated explants have been scored wild-type (WT), if green, chimera, if not fully red and RUBY, if entire explant was red.
**Figure 5** shows the regeneration efficiency of Nicotiana benthamiana dependent on the concentration of selection agent (BASTA) and the Agrobacterium strain (EHA105, GV3101 and LBA4404) used for transformation. Regeneration Media (Musashiagei-Skoog media (MSII) including NAA and BAP (1-Naphthaleneacetic acid (NAA) 6-Benzylaminopurin (BAP) (MSII-NB). From left to right, the results depict BASTA concentration of 0, 0.5, 1.0, 1.5, 2.0, 3.0 and 4.0 mg/ml. The Y-axis shows the number of regenerated explants. Regenerated explants have been scored wild-type (WT), if green, chimera, if not fully red and RUBY, if entire explant was red.
**Figure 6** shows the scheme of rheology measurements. The top plate oscillates at a given stress or strain amplitude. In the present context, it preferably is set to a a present strain amplitude.
**Figure 7** shows the determination of rheology measurements. The measurements provide a gelation experiment with commercial PureCol 5 mg/mL at 37 °C (triplicate). The results are given in stirage modulus G' in Pa (shear strain y [%] = 3; angular frequency w [1/s] = 10).
**Figure 8** shows a native polyacrylate gel electrophoresis (PAGE) gel, testing Collagen expression in N. benthamiana. Herein: COL- =Col1a, COL + = Col1a + P19, COLa = Col1a + P4H-a, COLb = Col1a + P4H-β, and COLab = Col1a + P4Ha + P4H-β.
**Figure 9** shows immuno-blotted polyacrylate gel electrophoresis (PAGE) gels with collagen with/without P4H-a/P4H-b expression in *N*. *benthamiana* at different time points as depicted. Herein: COL- =Col1a, COL + = Colla + P19, COLa = Col1a + P4H-a, COLb = Col1a + P4H-β, and COLab = Col1a + P4H-a + P4H-β.
**Figure 10** shows a stainfree PAGE 4-20% gel with samples of different time points of Collagen expression in N. benthamiana.
**Figure 11** shows an immunoblot with x6His with samples of Collagen expression in N. benthamiana at different time points.
**Figure 12** shows an SDS-PAGE 7,5% Comassie staining of samples of Collagen expression in N. benthamiana at different time points.
**Figure 13** shows an immunoblot with samples of different time points corresponding to Figure 12.
**Figure 14** shows an SDS PAGE of a collagen purification process from P. pastoris. The lanes are peqGOLD protein marker IV (1), PureCol 3 mg/ml (2), lysate (3), fraction A3-B11 (FT) (4), fraction A3-B2(FT) (5), fractions/peaks D4-D5 (6), fractions/peaks D6-D7 (7), and fractions/peaks D8-D9 (8).
**Figure 15** shows the Western Blot of the collagen purification process of Figure 14. The lanes are peqGOLD protein marker IV (1), PureCol 3 mg/ml (2), lysate (3), fraction A3-B11 (FT) (4), fraction A3-B2(FT) (5), fractions/peaks D4-D5 (6), fractions/peaks D6-D7 (7), fractions/peaks D8-D9 (8), and negative control albumin (9).
**Figure 16** shows a gel electrophoresis of purified collagen from *Pichia pastoris.* The lanes are peqGOLD protein marker IV (1), PureCol 3 mg/ml (positive control) (2), PanCol 1 mg/ml (positive control) (3), fraction D7 purified with His-tag (4), fractions D8-D11 purified with His-tag (5), fraction D11 purified with His-tag (6), fraction D1 purified with His-tag (7), fraction D2 purified with His-tag (8), fraction D3 purified with His-tag (9), and pooled fractions D7-D10 and rebuffered. The box and arrow indicate the sample which was investigated by mass spectrometry.
**Figure 17** shows a Western Blot of a collagen purification process after P. pastoris culture optimisation steps. The lanes are peqGOLD protein marker IV (1), FFGF2 (2), albumin pellet (3), albumin SN (4), A8 pellet 20°C (5), A8 SN 20°C (6), A8 pellet 28°C (7), A8 SN 28°C (8), A11 pellet 20°C (9), A11 SN 20°C (10), A11 pellet 28°C (11), and A11 SN 28°C (12).
**Figure 18A** shows a general downstream processing flow scheme exemplified for collagen obtained from *Pichia pastoris.* "§" indicates that the process is pursued. Process steps are black boxes. Dashed boxes are optional steps and white solid boxes indicate analytical assays.
**Figure 18B** shows a general downstream processing flow scheme exemplified for collagen obtained from supernatant of *Pichia pastoris,* thus collecting secreted collagen. "§" indicates that the process is pursued. Frames indicate further analysed fractions.
**Figure 19** shows a general downstream processing flow scheme exemplified for collagen from plants. "§" indicates that the process is pursued.
**Figure 20A** shows an SDS PAGE of collagen obtained from the supernatant of *Pichia pastoris.* Captions 1, 2 and 3 indicate different batches of supernatant (22 mL, 22 mL, 300 mL).
**Figure 20B** shows a Western Blot of collagen obtained from the supernatant of *Pichia pastoris.* Captions 1, 2 and 3 indicate different batches of supernatant (22 mL, 22 mL, 300 mL); +- Pancol 0.2 mg/mL; Neg. - Pichia Albumin Supernatant (negative control).
**Figure 21A** shows an SDS PAGE of collagen obtained from *Pichia pastoris.*
**Figure 21B** shows a Western Blot of collagen obtained from *Pichia pastoris.*
**Figure 22** shows a size exclusion chromatography (SEC) chromatogram of collagen obtained from *Pichia pastoris,* including a UV peak (1) and a conductivity peak (3). The concentration of additional agents was kept low.
**Figure 23** shows an SDS PAGE of several fractions of the collagen peaks obtained from the SEC as shown in Figure 22.
**Figure 24** shows a general downstream processing flow scheme exemplified for collagen obtained from plants. "§" indicates that the process is pursued.
**Figure 25** shows a Western blot of purified collagen by using 6.8 g of plant material.
**Figure 26** shows an SDS PAGE of collagen using 6.8 g of plant material. The lanes are peqGOLD protein marker IV (1), ProColl 1.5 mg/ml (2), fraction V1-10 1:5 (3), fraction V1-10 40% ammonium sulfate (AS) precipitation (4), fraction V1-10 60% ammonium sulfate (AS) precipitation (5), fraction V19 1:5 (6), fraction V19 40% ammonium sulfate (AS) precipitation (7), and fraction V19 60% ammonium sulfate (AS) precipitation (8).
**Figure 27** shows a Western blot of purified collagen by using 1 g of plant material. The lanes are peqGOLD protein marker IV (1), ProColl (2), Tris 1:10 (3), Tris 40% ammonium sulfate (AS) precipitation (4), Tris 60% ammonium sulfate (AS) precipitation (5), Tris 1:10 negative (6), Tris 40% ammonium sulfate (AS) negative (7), Tris 60% ammonium sulfate (AS) negative (8), and acetic acid SN negative (9).
**Figure 28** shows an SDS PAGE of collagen using 6.8 g of plant material. The lanes are peqGOLD protein marker IV (1), ProColl (2), Tris 1:10 (3), Tris 40% ammonium sulfate (AS) precipitation (4), Tris 60% ammonium sulfate (AS) precipitation (5), Tris 1:10 negative (6), Tris 40% ammonium sulfate (AS) negative (7), Tris 60% ammonium sulfate (AS) negative (8), and acetic acid SN negative (9).
**Figure 29** shows a Western blot of purified collagen by using 5.8 g of plant material with polypeptide concentration of ca. 7 mg/ml on the gel. The lanes are peqGOLD protein marker IV (1), ProColl 0.25 mg/ml (2), Tris 16 (3), 40% ammonium sulfate (AS) precipitation 16 (4), 60% ammonium sulfate (AS) precipitation 16 (5), Tris 6 (6), 40% ammonium sulfate (AS) negative (7), 60% ammonium sulfate (AS) negative (8), sample buffer (9), and sample buffer (10).
**Figure 30** shows a Western blot of purified collagen by using 7.5 g of plant material. The lanes are peqGOLD protein marker IV (1), ProColl 1.5 mg/ml (2), V1-3 acetic acid (AA) 1:3 (3), V1-3 0.4 M (4), V1-3 0.9 M (5), V1-3 SN (6), 1-20 AA 1:3 (7), V1 - 20 0.4 M (8), V1-20 0.9 M (9), and V1-20 SN (10).
**Figure 31** shows an SDS PAGE of collagen using 6.8 g of plant material. The lanes are peqGOLD protein marker IV (1), ProColl 1.5 mg/ml (2), V1-3 acetic acid (AA) 1:3 (3), V1-3 0.4 M (4), V1-3 0.9 M (5), V1-3 SN (6), 1-20 AA 1:3 (7), V1-20 0.4 M (8), V1-20 0.9 M (9), and V1-20 SN (10).
**Figure 32** shows a general downstream processing flow scheme exemplified for proteins purified from *Pichia pastoris.*
**Figure 33** shows an SDS PAGE of collagen obtained from *Pichia pastoris.* The lanes are peqGOLD protein marker VI (1), Pichia A2, Lysate after 100 h (2), Pichia A3, Lysate after 100 h (3), Pichia A4, Lysate after 100 h (4), Pichia WT Negative Control, Lysate after 100 h (5), Pichia A8, Lysate after 72 h (6), Pichia A9, Lysate after 72 h (7), Pichia A10, Lysate after 72 h (8), Pichia A11, Lysate after 72 h (9), and PureColl 0.3 mg/ml (10)
**Figure 34** shows a, SDS PAGE and Western Blot (fraction) of collagen obtained from *Pichia pastoris.* The lanes are peqGOLD protein marker VI (1), PanCol 0.25 mg/ml (2), Tris 16 (3), 40% ammonium sulfate (AS) (4), AS60% 16, (5), Tris 6 (6), AS40% 6 (7), AS60% 6 (8), sample buffer (9), sample buffer (10), Pichia albumin control (11), Pichia A8 (12), and Pichia A11 (13). Lower part of the picture shows the bands after a longer exposure time.
**Figure 35** shows a gel electrophoresis of E. *coli* colonies. The lanes are 10 kB ladder (1), clones 1-6 (2-7), clone 8 (8), clone 9 (9), 100 bp ladder (10), negative control (11), and 10 kb ladder (12).
**Figure 36** shows SDS PAGE (above) and Western Blot (below) of collagen obtained from E. *coli.* The lanes are peqGOLD protein marker IV (1), PureCol positive control (2), PanCol positive control (3), Negative control spiked with 0.75mg/ml ProCol (4), extraction buffer (5 and 6), extraction buffer negative (7 and 8), E. coli (9 and 10).
**Figure 37** shows an upscaling flow scheme exemplified for preparing collagens. Reference: de Sá Magalhães, S.; Keshavarz-Moore, E. Pichia pastoris (Komagataella phaff ii) as a Cost-Effective Tool for Vaccine Production for Low- and Middle-Income Countries (LMICs). Bioengineering 2021, 8, 119. https://doi.org/10.3390/bioengineering8090119
**Figure 38** shows an example of a detailed process scheme for preparing collagens. Reference: da Silva Ferreira, Adamo Eduardo: Simulation and techno-economic analysis of production processes of novel generation L-Asparaginases using recombinant Escherichia coli and Pichia pastoris. Dissertação (Mestrado) - Escola Politécnica da Universidade de São Paulo. Departamento de Engenharia Química São Paulo, 2022. https://www.teses.usp.br/teses/disponiveis/3/3137/tde-23032023-071933/publico/ AdamoEduardodaSilvaF erreiraCorr22. pdf
**Figure 39** shows an example of a detailed process scheme for preparing collagens. Reference: Nandi, Sowen, Aaron T. Kwong, Barry R. Holtz, Robert L. Erwin, Sylvain Marcel & Karen A. McDonald (2016) Techno-economic analysis of a transient plant-based platform for monoclonal antibody production, mAbs, 8:8, 1456-1466, DOI: 10.1080/19420862.2016.1227901
**Figure 40** shows an example of a flow scheme for preparing collagens from plants. Reference: Park, Se-Ra & Lim, Chae-Yeon & Kim, Deuk-Su & Ko, Kisung. (2015). Optimization of Ammonium Sulfate Concentration for Purification of Colorectal Cancer Vaccine Candidate Recombinant Protein GA733-FcK Isolated from Plants. Frontiers in Plant Science. 6. 10.3389/fpls.2015.01040.
**Figure 41** shows an example of a detailed process scheme for preparing collagens from plants. Reference: Alam A, Jiang L, Kittleson GA, Steadman KD, Nandi S, Fuqua JL, Palmer KE, Tusé D, McDonald KA. Technoeconomic Modeling of Plant-Based Griffithsin Manufacturing. Front Bioeng Biotechnol. 2018 Jul 24;6:102. doi: 10.3389/fbioe.2018.00102. PMID: 30087892; PMCID: PMC6066545.

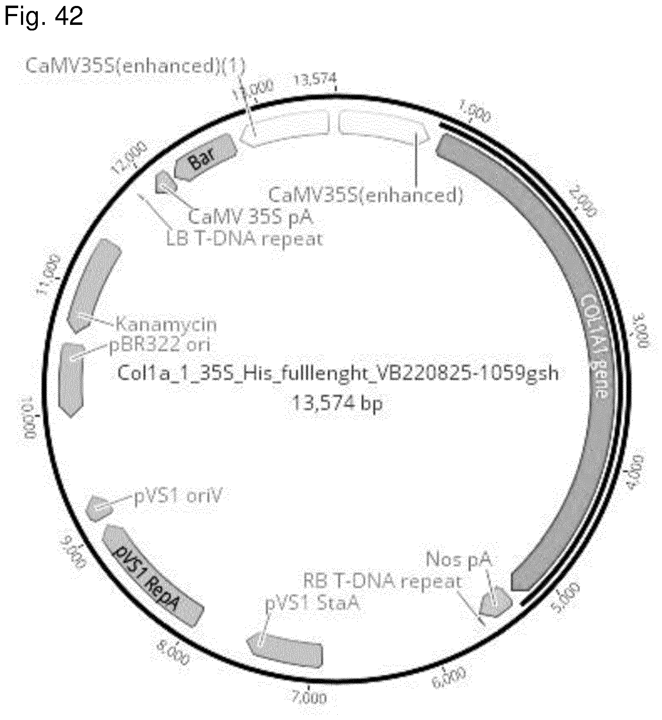

**Figure 42** shows vector map of vector Col1a_1 (35S_His_ full length).

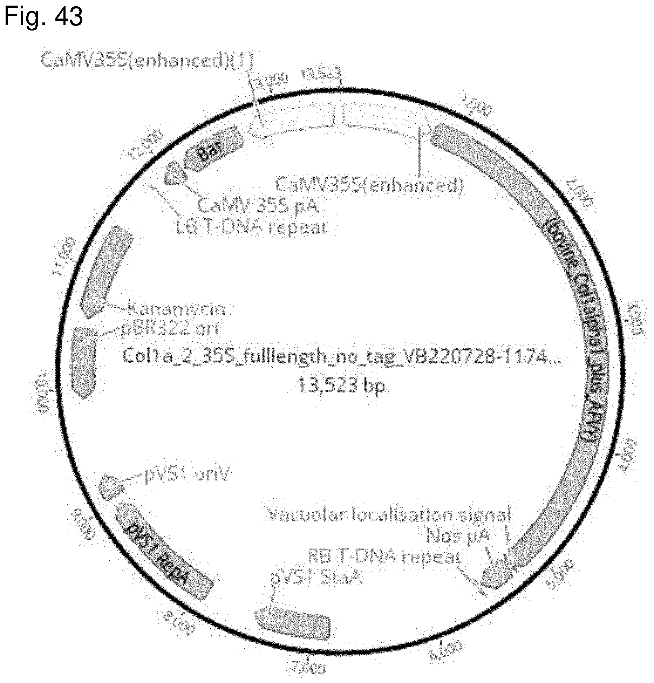

**Figure 43** shows vector map of vector Col1a_2 (35S_His_ full length_no tag).

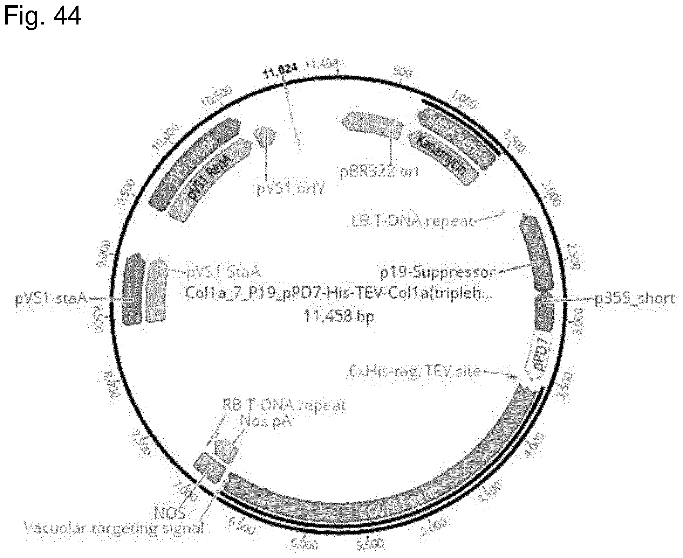

**Figure 44** shows vector map of vector Col1a_7 (P19_pPD7-His-TEV-Col1a(triple helical domain only)-AFVY-tNos).

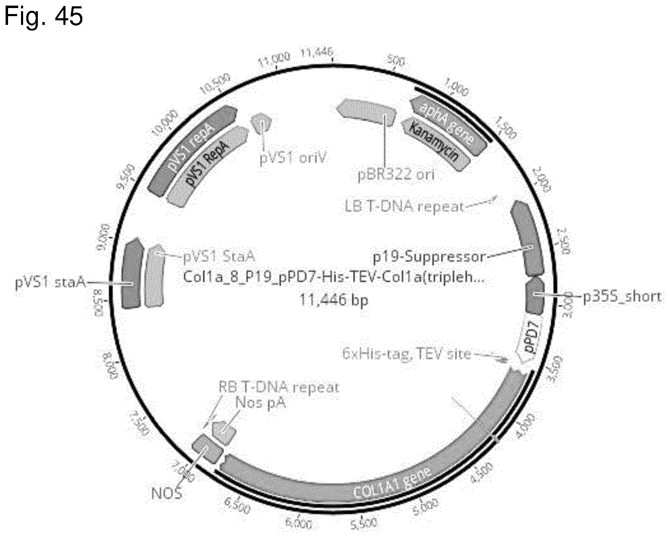

**Figure 45** shows vector map of vector Col1a_8 (P19_pPD7-His-TEV-Col1a(triple helical domain only)-AFVY-tNos).

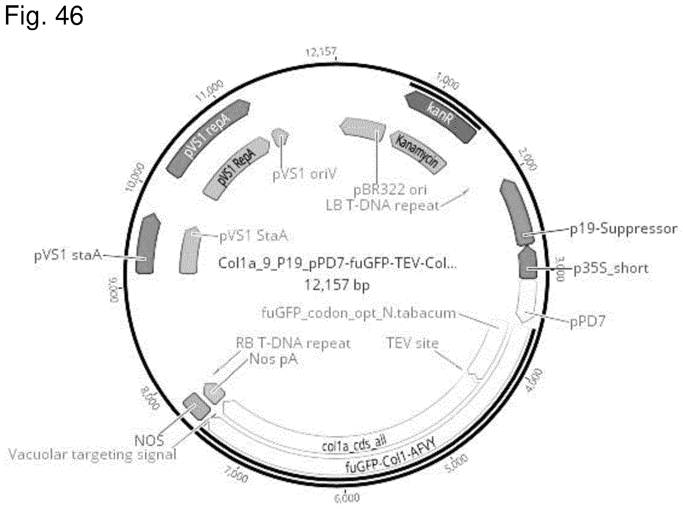

**Figure 46** shows vector map of vector Col1a_9 (P19_pPD7-fuGFP-TEV-Col1a(triple helical domain only)-AFVY-tNos).

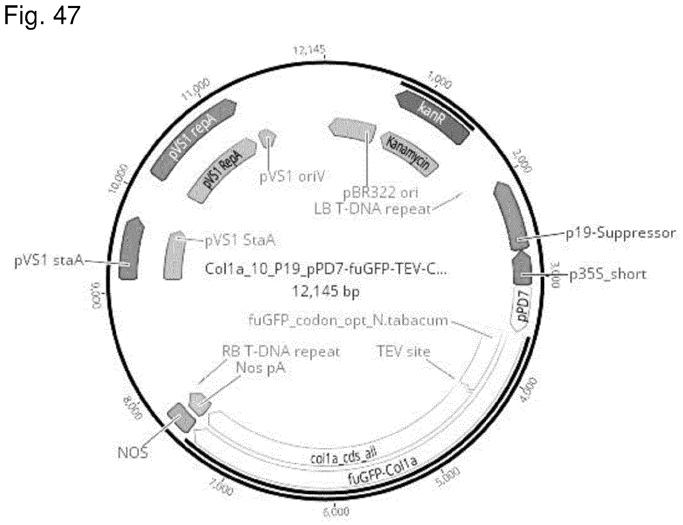

**Figure 47** shows vector map of vector Col1a_10 (P19_pPD7-fuGFP-TEV-Col1a(triple helical domain only)-tNos).

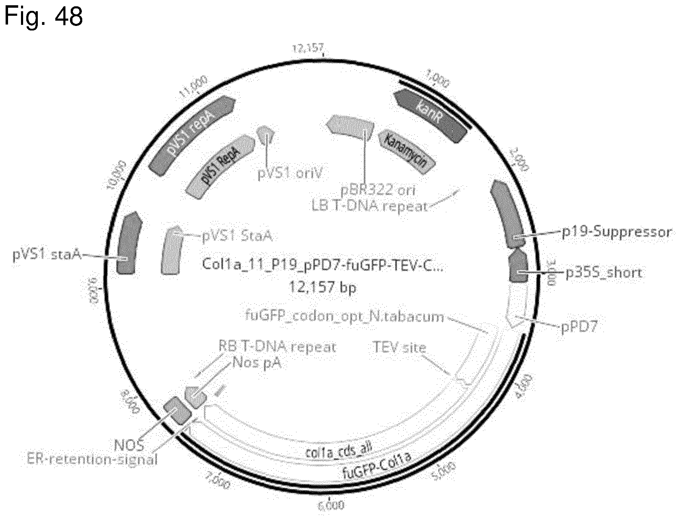

**Figure 48** shows vector map of vector Col1a_11 (P19_pPD7-fuGFP-TEV-Col1a(triple helical domain only)-KDEL-tNos).

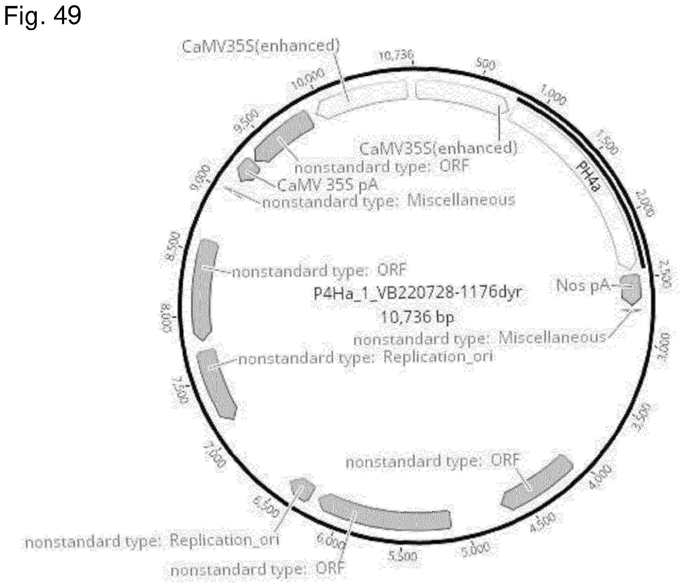

**Figure 49** shows vector map of vector P4Ha_1.

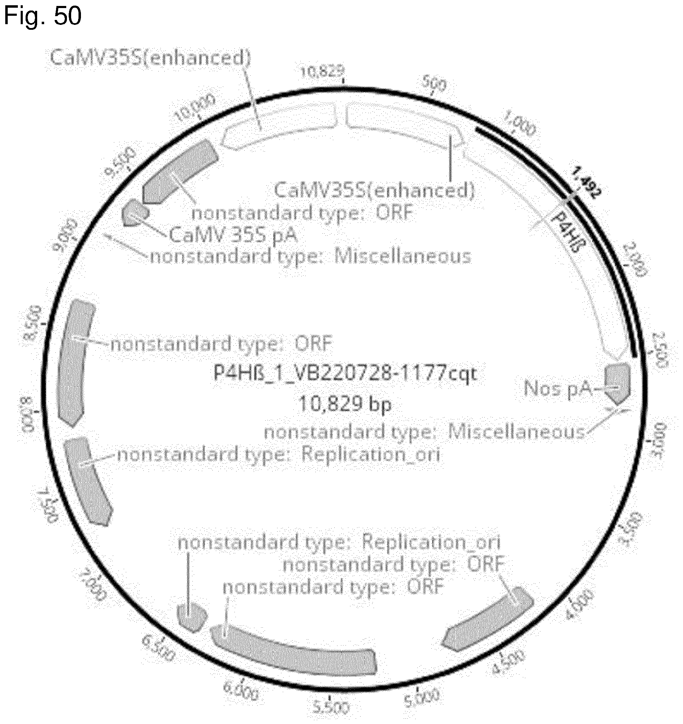

**Figure 50** shows vector map of vector P4Hbeta_1.

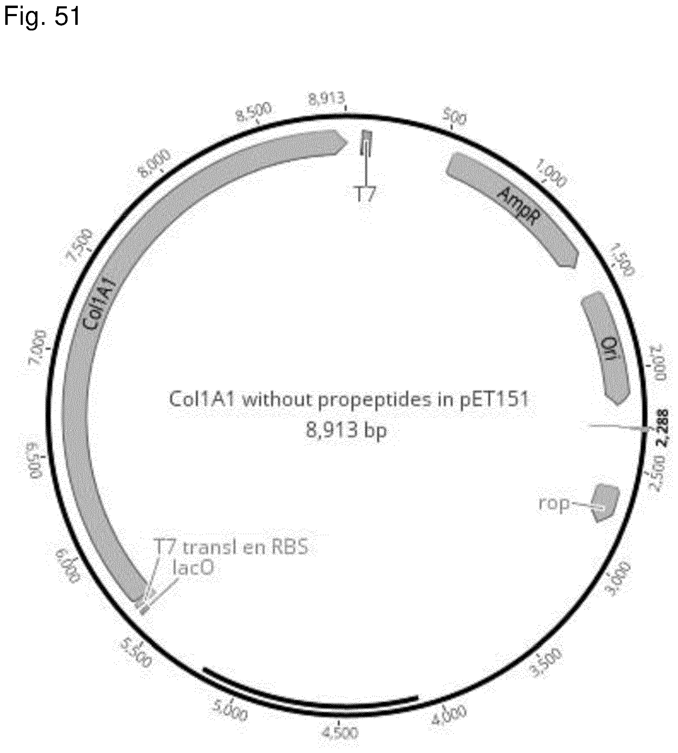

**Figure 51** shows vector map of Col1A1 expression vector for *E.coli.*

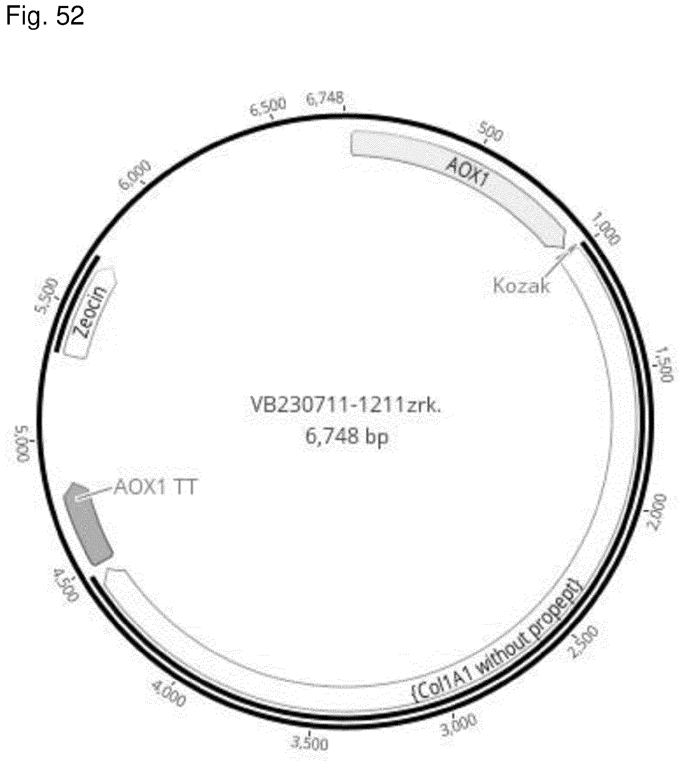

**Figure 52** shows vector map of Co!1A1 expression vector for *Pichia pastoris.*

### Examples

### Example 1 - Transformation of Pichia pastoris with Co11A1

The aim of the experiment is to transform *Pichia pastoris* with the *Co11A1* gene, without N- and C- terminal propeptides, in order to produce recombinant Collagen.

### Methods

### Transformation

Transformation of competent *Pichia pastoris* was achieved by electroporation with freshly made competent cells. Following plasmid has been used: pPP-Zeocin-AOX1>{Col1A1 without propept}

Plasmid has arrived in E. coli (Vectorbuilder), and before the transformation the plasmids were extracted with a MaxiPrep Kit (Qiagen).

The linearization of plasmid was carried out with a Pmel endonuclease (New England Biolabs) according to the manufacturer's manual. Linearization was verified by agarose gel electrophoresis. Ca. 2 µg of plasmid were used for transformation. The incubation time after the electroporation was 2h 20 min. The incubation on YPDS agar plates with 200 µl of 1 and 2 mg/ml Zeocin spread over agar took 3 days. As a negative control one aliquot was electroporated without addition of plasmids

### PCR Screening

For the colony PCR clones were grown in 3 ml YPD medium with 100 µg/ml Zeocin overnight, at room temperature and 220 rpm, in 15 ml tubes. After spinning down 1 ml in 1.5 ml tubes 500 µl of the lysis buffer (400 mM TRIS-HCl, pH 8; 60 mM EDTA, pH 8; 150 mM NaCl, 1% SDS) was added together with a spatula tip of 0.25 glass beads. The samples were disrupted for 5 min in the bead beater at 30Hz and incubated for 10 minutes. After that 150 µl of 3 M Potassium Acetate was added and the tubes were vortexed briefly before centrifugation (1 min 14000g) Supernatants were transferred in a fresh tube and mixed with same
amount of Isoporopanol. After another centrifugation for 5 min at 14000 g the pellets were washed with 500 µl 70% Ethanol and dried before the dissolution in 100 µl MilliQ H2O.
Primers: 73 GACTGGTTCCAATTGACAAGC (SEQ ID NO: 117)
74 GCAAATGGCATTCTGACATCC (SEQ ID NO: 118)
Expected band sizes for Col1A1 ca. 3200 bp

| | |
|---|---|
| PCR Programm: 33 Cycles 98°C | 30 sec |
| 98°C | 5 sec |
| 61°C | 5 sec |
| 72°C | 45 Sec |
| 72°C | 1 min |
| 4°C | - |

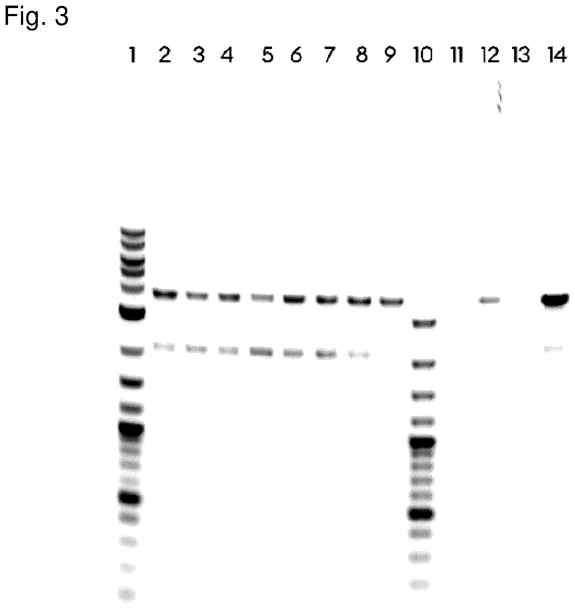

The Colony PCR results are shown in Figure 3. The lower bands show the native AOX1 from *Pichia.* The result is not conclusive due to a band in the negative control, possibly because of cross-contamination. Previous screenings have shown that most of the colonies that have grown on Zeocin contain the target gene.

### Conclusion

The transformations were successful. So many clones were generated, that it would make sense to dilute the cells before plating them out or increase the antibiotic concentration. PCR Screening can be repeated or the colonies can be screened directly for the protein expression

The clones are transferred on master plates and screened for production of collagen in liquid culture.

### Example 2 - Example of stable transformation of plants

**Stable transformation of plants** is a technique of interest in plant biotechnology. It allows scientists to introduce new genes into plants, thereby altering their characteristics or conferring resistance to pests, diseases, or environmental stressors.:
1. **Choice and Preparation of Explant Tissue:**
   ∘ The process begins by selecting an appropriate tissue sample (explant) from the plant. Common explants include leaf segments, stem sections, or immature embryos.
   ∘ These explants are then cultured in a nutrient-rich medium to encourage cell division and regeneration.
2. **DNA Delivery:**
   ∘ The next step involves introducing the desired DNA (transgene) into the plant cells. There are various methods for DNA delivery:
      ▪ **Agrobacterium-Mediated Transformation:** Agrobacterium tumefaciens, a soil bacterium, is used to transfer the transgene into the plant cells. This method is widely employed for many plant species.
      ▪ **Particle Bombardment (Gene Gun):** Tiny gold or tungsten particles coated with DNA are shot into the plant tissue using a gene gun. This method is particularly useful for species that are less amenable to Agrobacterium transformation.
      ▪ Other methods include electroporation and microinjection.
3. **Callus Induction and Regeneration:**
   ∘ After DNA delivery, the transformed cells are cultured on a special medium to form a mass of undifferentiated cells called callus.
   ∘ The callus is then induced to differentiate into shoots and roots, ultimately leading to the development of whole plants.
   ∘ Selection markers (such as antibiotic resistance genes) are often used to identify and propagate only those cells that have successfully incorporated the transgene.
4. **Recovery of Stably Transformed Plants:**
   ∘ Once the regenerated plants are established, they are transferred to soil and grown to maturity.
   ∘ These stably transformed plants carry the introduced transgene in their genome and can pass it on to their offspring.
5. **Applications:**
   ∘ Stable transformation is used for various purposes:
      ▪ **Crop Improvement:** Developing genetically modified (GM) crops with enhanced traits (e.g., pest resistance, drought tolerance, improved nutritional content).
      ▪ **Functional Genomics:** Studying gene function by overexpressing or silencing specific genes.
      ▪ **Biopharmaceutical Production:** Producing therapeutic proteins in plants.
      ▪ **Basic Research:** Investigating plant biology and development.

Remember, this process is essential for advancing our understanding of plant genetics and for developing sustainable agricultural solutions.

Detailed protocols are known, including insights into the steps involved

### Example 3 - Nicotiana benthamiana Tissue Culture RUBY expression

Determine the most efficient conditions for tobacco tissue culture as an estimate for stable transformation of POI.

### Method:

### Transformation:

*Agrobacterium tumefaciens* (A. tumefaciens; GV3101) strain carrying the transformation construct (35S-RUBY) inoculated in 50 ml culture induction medium (containing antibiotics), shake over night at 28 °C. Day of transformation: Pellet bacteria by centrifugation, resuspend bacteria in MMA medium (without antibiotics) to an OD600=1.5 (50 ml).

Harvest the 2-3 youngest, but fully expanded leaves of 4 weeks-old *Nicotiana benthamiana* plants.

Surface sterilize in 1.2 % NaOCI (+0.01 % Tween) and wash in H₂O. Cut surface-sterilized leaves into pieces and in A. tumefaciens suspension. Incubate at least 30 min. Transfer leaf cuts onto water wetted paper. Seal dishes and incubate for 2 days in the dark at 24 °C.

### Selection and shoot induction

Prepare petri dish with 50 ml water (containing Cefotaxime (250 mg/l)) and place leaf cuts in it for washing.

Gently shake the petri dish, and incubate for 10 min.

Dry leaf cuts and place on shoot induction medium (MS-II). Use standard round petri dishes to minimize the risk of contamination. Place 8-10 leaf cuts on each plate. Incubate in a light cabinet until shoots occur at 25 °C, 24 h.

### Root induction

Shoots start developing from calli on MS-II plates and need to be transferred to MS-III plates to induce rooting.

Cut well-developed shoots with a sterile blade. Stick shoots with the cut surface into MS-III medium, and incubate under the same conditions as before for further development of the shoot and rooting.

Shoots can further develop in MS-III media, and can eventually form roots. Transfer well-developed shoots to soil.

### Plant maturing and seed harvesting.

Wait until the pods are dry and seeds get exposed before harvesting. Make a BASTA seeding selection/germination test in 1/2 MS with 1mg/L Basta.

Quantify the germination ratio.

Select survival seedlings and transfer them to pods for further generation selection. Quantification of the total number of shoots and its classification showed that overall

*Agrobacterium* strain LBA4404 and *Agrobacterium* strain GV3101 were the most efficient strains for stable transformation as plants infected with both strains showed a higher number of chimeric shoots. However, GV3101 is clearly superior as it induced more chimeras per time interval.

### Results

Results of the transformation efficiency in Murashige-Skoog media II (MSII) Timentin, Terbinafin, Cefotaxime (TTC) are depicted in Figure 4. Results of the transformation efficiency in Murashige-Skoog media II NAA, BAP (1-Naphthaleneacetic acid (NAA)

6-Benzylaminopurin (BAP) (MSII-NB) are depicted in Figure 5.

The RUBY plant had red/purple flowers, while the wild-type had white flowers. The chimeric flowers were pink.

### Example 4 - Transient expression of Collagen in Nicotiana benthamiana

Transient expression of Collagen1a1 from *Bos taurus* in *N. benthamiana.* All steps from infiltration to WB results are depicted below.

### Method:

### Transient Transformation:

Grow *N. benthamiana* plants for 4-5 weeks on long day conditions as: 16H light (50% light and 60% humidity)/ 8H dark (0% light and 65% humidity) *GV3101 Agrobacterium tumefaciens* may be used for transient expression. Antibiotic selection for bacterial transformation is carried out according to the manufacturer.

Selected *Agrobacterium* strains carrying the genes Collagen 1 a, Hydroxylase P4Ha, Hydroxylase P4H-b were selected to further steps.

Grow *Agrobacterium* in 2mL of LB broth supplemented with the selection antibiotics in a test tube at 28 °C for 1 day.

Inoculate 500 µL of the preculture in 50 mL of LB with selection antibiotics and grow at 28 °C over night.

Next day, wash cells 1x: Pellet at low speed (max 3000g) and resuspend with 40ml MMA (10 mM MgCl2, 10 mM MES [pH 5.7], 100µM acetosyringone).

Pellet again at low speed (max 3000g) and resuspend with MMA (10 mM MgCl2, 10 mM MES [pH 5.7], 100 µM acetosyringone) media, adjust to final OD600 = 0.8. Infiltrate 100 µL approx. in the leave with a needleless syringe following the next co infiltration patterns:
1. Col1a
2. Col1a + P4H-a
3. Col1a + P4H-β
4. Col1a + P4H-a + P4H-β
Harvest 2 to 4 days after infiltration.

### Western blotting

Harvest plant material from the marked infiltrated areas in the tobacco leaves into conical tubes.

Freeze the tissue in liquid nitrogen and grind the leaves to a fine powder while the plant material is still frozen.

Add 400 µL of ice-cold extraction buffer:
Reagent Concentration
Tris/HCl (pH 8,5) 100 mM
Glycerol 10%
NaCl 0.5 M
Imidazole 20 mM
PVPP 5%
DTT 5 mM

Vortex vigorously to extract the soluble proteins Centrifuge for 30 minutes at 18000 RFC at 4°C Transfer the supernatant into a clean tube. Centrifuge once again for 20 minutes as in the previous step. Transfer the supernatant into a clean tube, keep sample in ice.

Total protein quantification via Bradford Assay according to manufacture.

Prepare samples to 15 µg, 30 µg or 50 µg (depending on the gel). Add H2O 4 x Loading Dye and DTT (1:10). Denature samples for 5 min at 95°C in Thermomixer at 300 rpm. Cool samples on ice on the bench. Store at -20°C or proceed to electrophoresis.

### Native PAGE gel

Dilute all the samples to the lower concentration and load the same amount of protein on each well. Using Bio-Rad stain free 15% polyacrylamide gel. Load 50 µL in the wells, flanking the samples with Chameleon duo color protein marker. Run the gel for 20 minutes at 80 V and then change the voltage up to 120 until the samples reach the bottom of the gel.

### Dry blotting

According to iBlot^{™} 2 Gel TransferDevice manufacturer protocoll. Choose program P0 (20V for 1min, 23V for 4min, 25V for 2min) or program of choice.

### Immunoblot

Prepare a 1:1000 solution of primary antibody (His) and pour it into a box. Place the membrane into the box assuring that the antibody solution covers completely the membrane. Incubate the membrane with the primary antibody overnight with rocking motion. Discard the primary antibody solution and proceed to wash the membrane with TBS-T four times

10 minutes, 5 minutes, 5 minutes, 10 minutes, Discard the TBS-T. Prepare a 1:10000 solution with the secondary antibody. Incubate for 2H at constant rocking motion. Discard the primary antibody solution and proceed to wash the membrane with TBS-T in four times

10 minutes, 5 minutes, 5 minutes, 10 minutes, Discard the TBS-T. Add 2 mL of reagents A and B on top of the membrane trying to cover all the surface. Use the transilluminator to record data.

### Results:

Estimated time from seed to plant ready to infiltrate: 3.5 weeks. Double/triple construct infiltration shows more severe necrosis. Six leaf disks were sampled, two leaves per construct.

**Table 3. Protein quantification. All data is presented in mg/mL.**

| | **Pure sample** | | | **1:10 Diluted** | | |
|---|---|---|---|---|---|---|
| **Sample** | Values | MeanValue | Std.Dev. | Values | MeanValue | Std. Dev. |
| **Col1** | 0.868 | 0.88 | 0.016 | 0.083 | 0.093 | 0.015 |
| | 0.891 | | | 0.104 | | |
| **Col2** | 0.489 | 0.46 | 0.041 | 0.028 | 0.046 | 0.026 |
| | 0.431 | | | 0.064 | | |
| **Colαβ1** | 0.657 | 0.558 | 0.141 | 0.045 | -0.016 | 0.086 |
| | 0.458 | | | -0.077 | | |
| **Colαβ2** | 0.613 | 0.646 | 0.047 | -0.003 | -0.058 | 0.077 |
| | 0.68 | | | -0.112 | | |
| **Colβ1** | 0.492 | 0.495 | 0.004 | 0.006 | 0.053 | 0.067 |
| | 0.498 | | | 0.1 | | |
| **Colβ2** | 0.679 | 0.734 | 0.078 | 0.054 | 0.06 | 0.008 |
| | 0.789 | | | 0.065 | | |
| **Buffer1** | 0.644 | 0.633 | 0.015 | 0.046 | 0.041 | 0.006 |
| | 0.623 | | | 0.037 | | |
| **Buffer2** | 0.459 | 0.468 | 0.012 | 0.042 | 0.041 | 0.002 |
| | 0.476 | | | 0.039 | | |

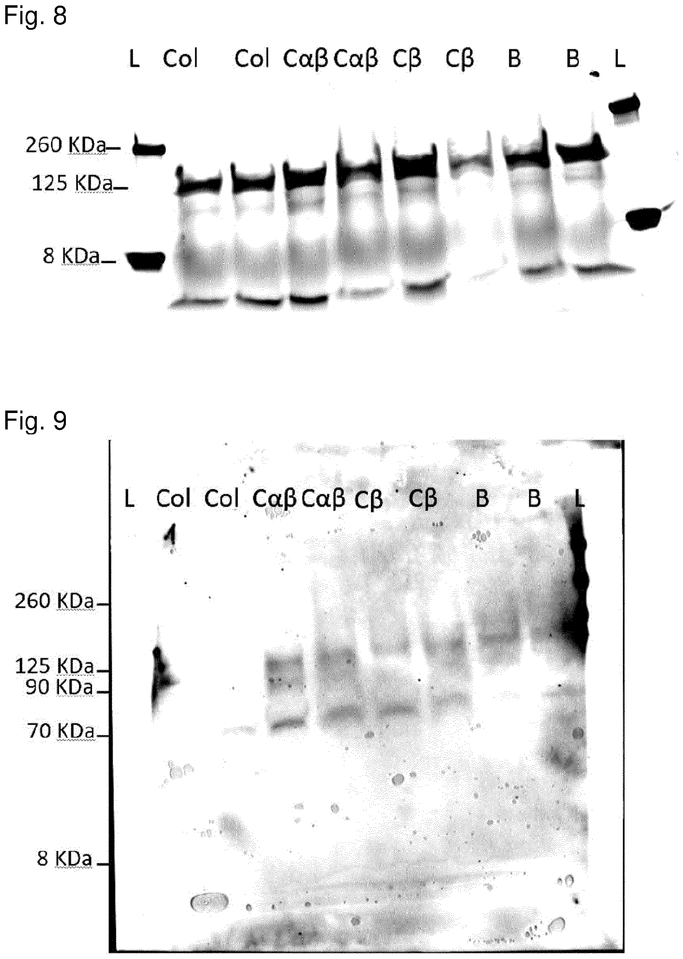

The gel electrophoresis results (native gel and immuno-stained gel) are depicted in Figures 8 and 9.

### Protein extraction modification:

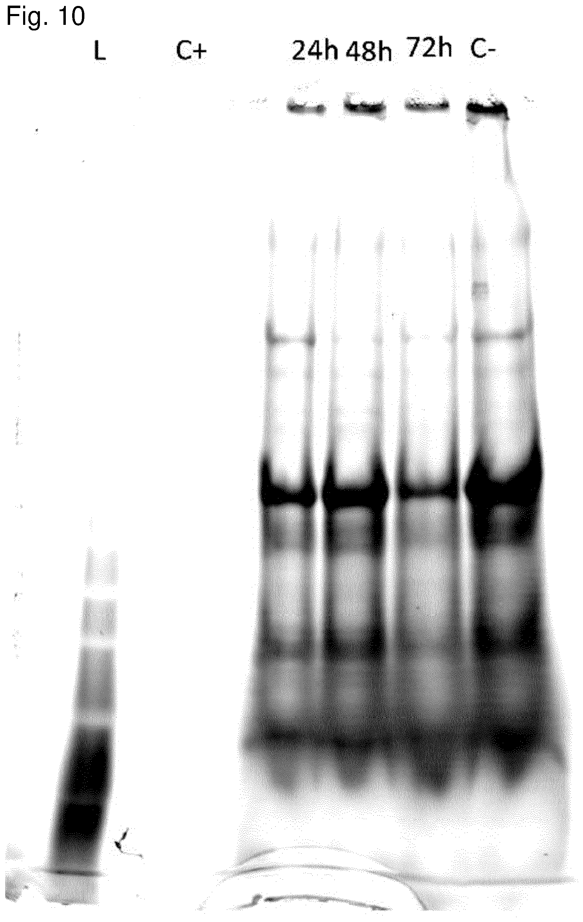

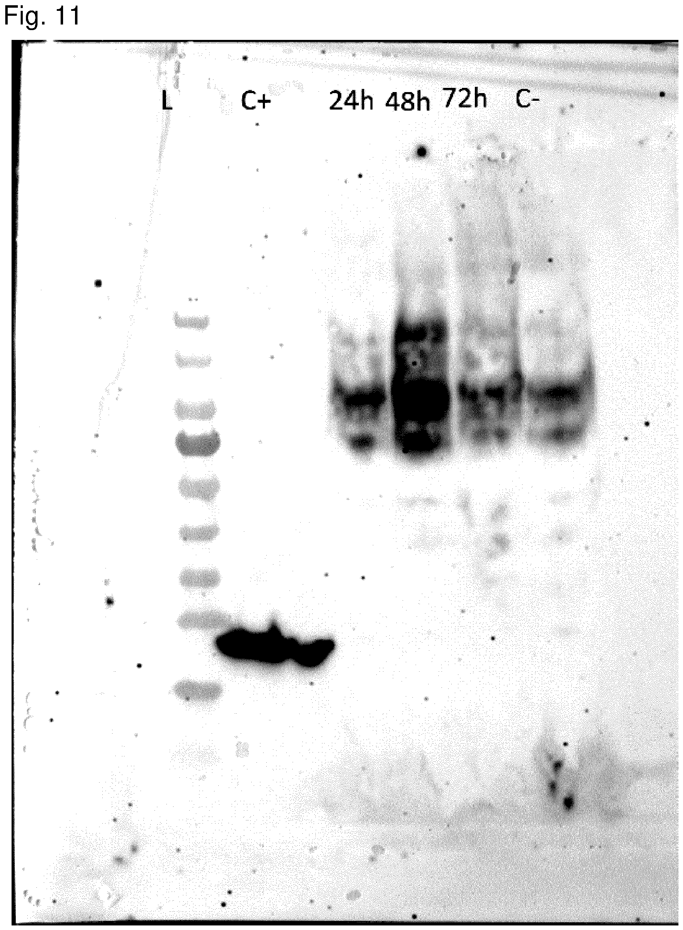

Extraction buffer replaced with loading dye reducing SDS. Samples were boiled for 5 minutes. Three time points of harvesting after infiltration, all samples contain Col1a+P19. Positive control (C+) is purified FgF2 9-point mutation (e.g. as described in EP 3380508). Negative control (C-) is protein extract from non-infiltrated leaves. SDS- PAGE stainfree 4-20%. The results are depicted in Figures 10 and 11.

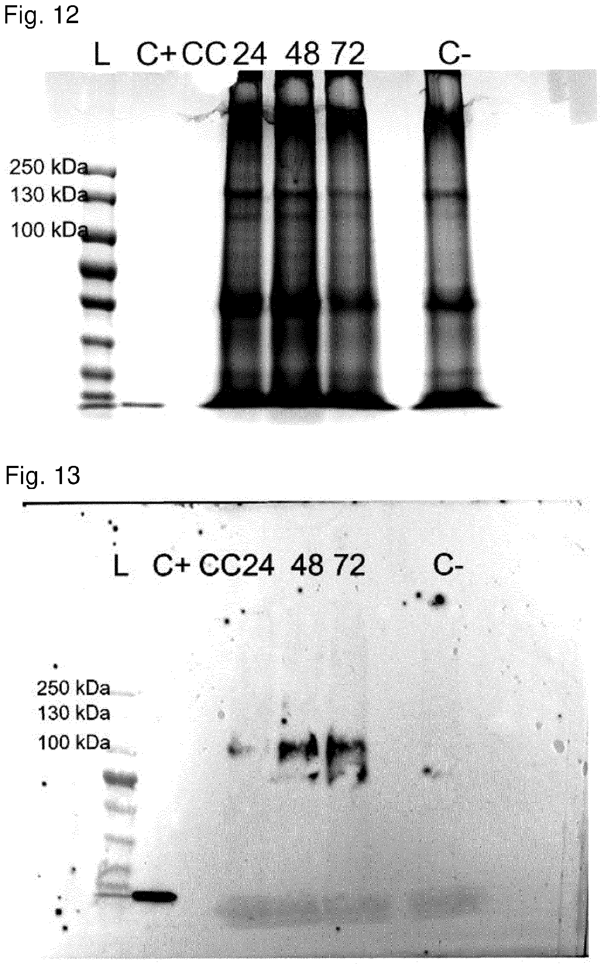

Three time points of harvesting after infiltration, all samples contain Col1a+P19. Positive control (C+) is purified FgF2 9 point mutation. Collagen coating solution (CC) is used a secondary positive control. Negative control (C-) is protein extract from non-infiltrated leaves. An SDS-PAGE 7,5% Comassie staining was performed as depicted in Figure 12 as well as an immune-stain of a gel as shown in Figure 13.

### Conclusion:

There are no visible signs of expression of Col1a as the expected theoretical band size (~150 KDa), however, there is a clear reaction to the His-tag at around 100 kDa, which corresponds to type I collagen.

Improvement of transfection in tobacco leaves may lead to better positive results in the expression and identification of collagen.

### Example 5 - Purification of Collagen

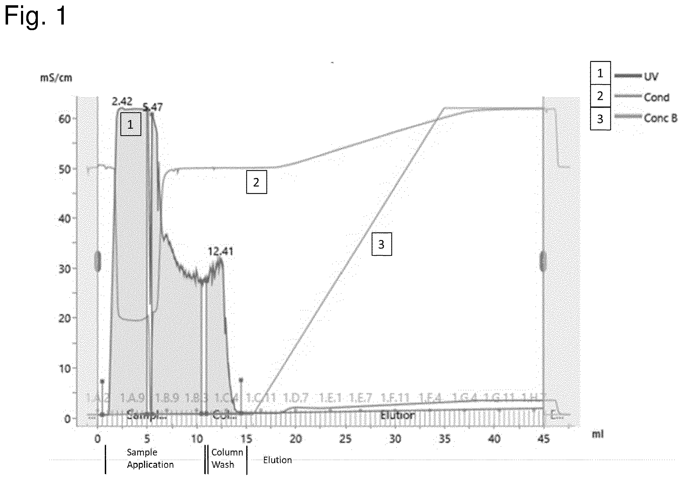

Collagen including a His-tag was prepared from *P. pastoris* as laid out above. This allows His-tag purification of Collagen from *P. pastoris* (20 ml) by means of affinity chromatography. The results are depicted in Figure 1. As expected, collagen was not visible at 280 nm.

A pellet from the produced batch of the *P. pastoris* (previously confirmed collagen expression by His WB) was lysated by sonication and used for His purification. Total volume was 5 mL.

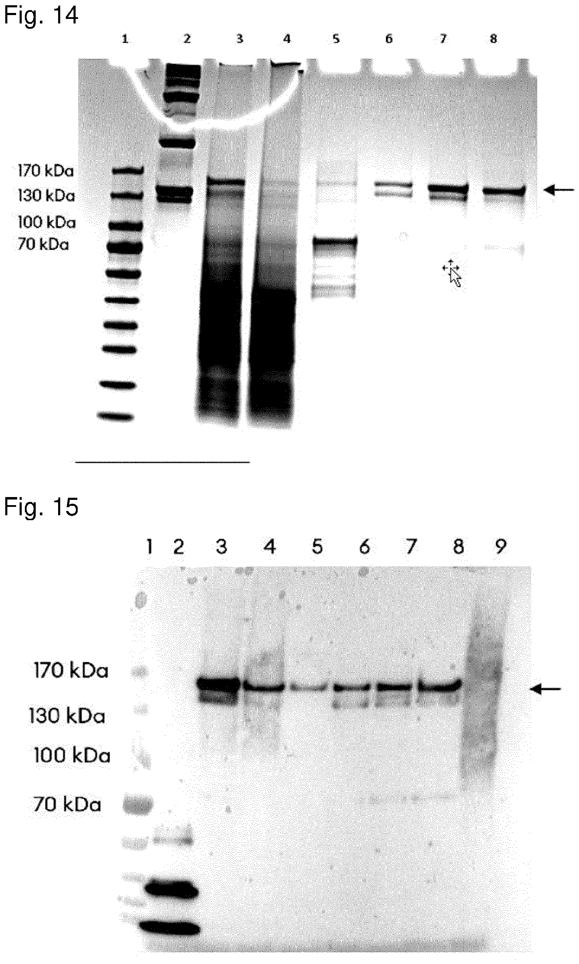

Gel electrophoretic results are shown in Figures 14 and 15. SDS PAGE showed a band ca. 130 kDa, and Western Blot using His antibody confirmed positive bands for *Pichia pastoris* samples, while no band was detected for albumin negative control. Clear Purification of initial Material (see SDS PAGE Lane 3) with the major impurities located in Lane 4+5 (Flowthrough) and Collagen in the Elution part (Lane 6 + 7 + 8). No A280nm signal was detected by the FPLC during elution phase, which made the choice of fractions hard. This is repeated for additional elution fractions (D10, D11, D12 ....) to check for presence of collagen.

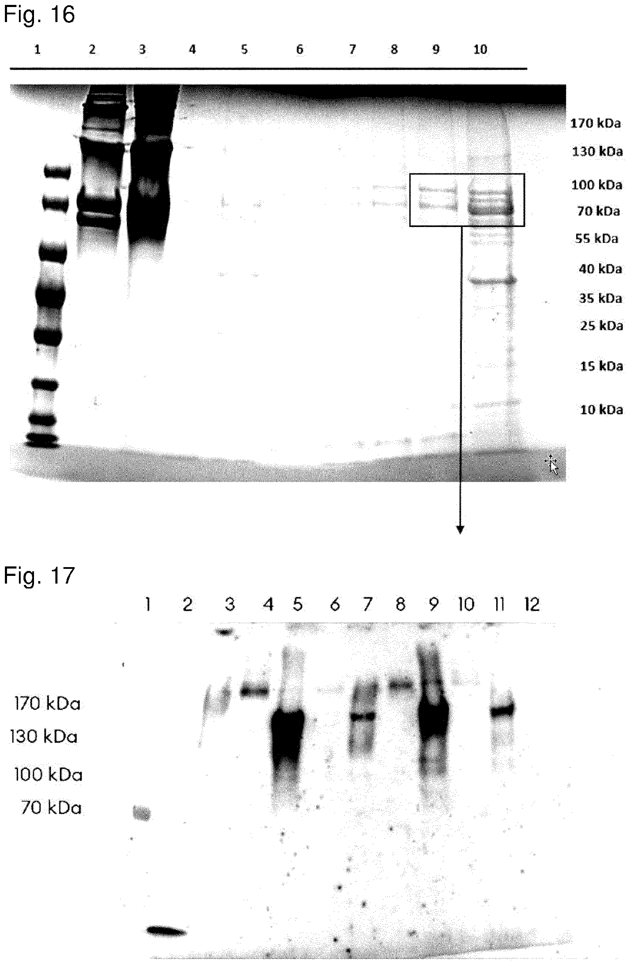

The purified collagen from *Pichia pastoris* was further investigated in an SDS PAGE with a gel of 7.5% Tris-glycine, a sample of 10 µl in 3.3 µl of LDS sample buffer and a Coomassie PageBlue stain. The results are depicted in Figure 16.

The sample was further investigated for sample/protein identity by mass spectrometry. The results are as follows:

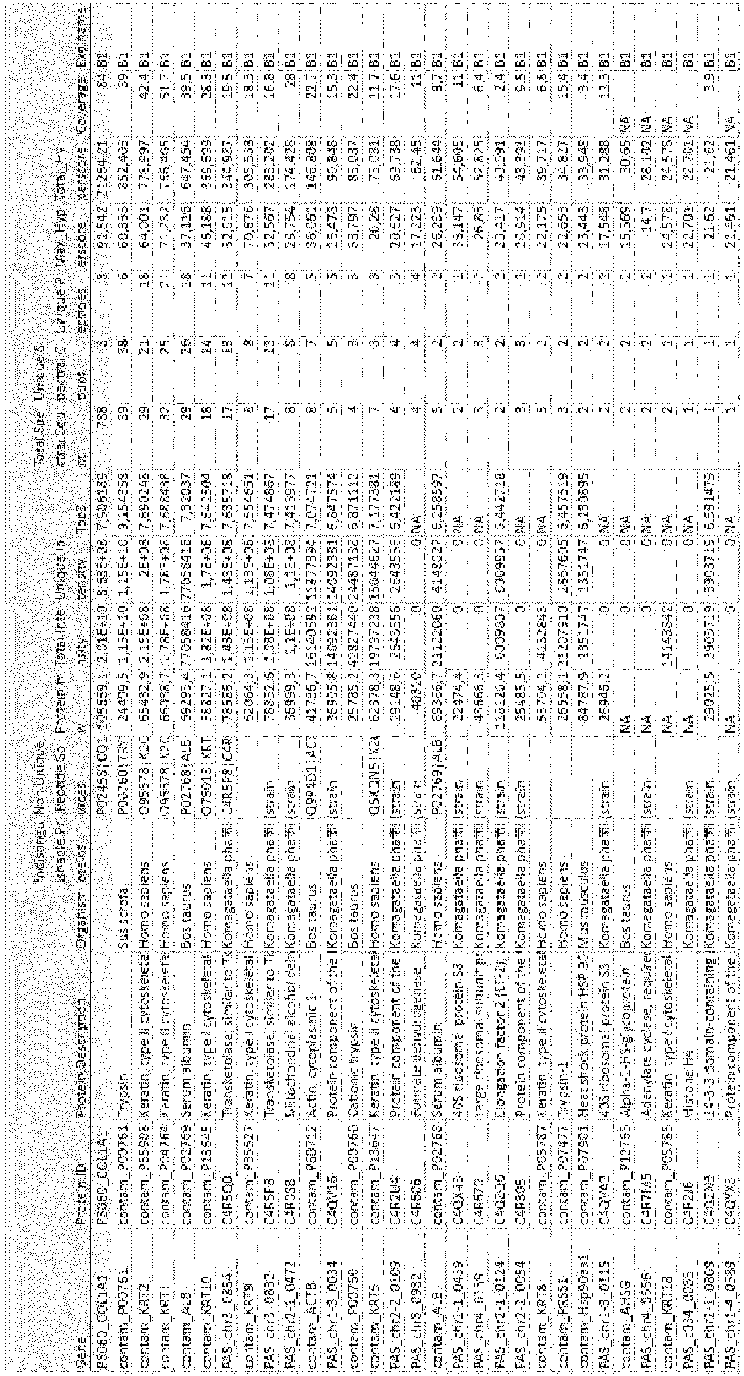

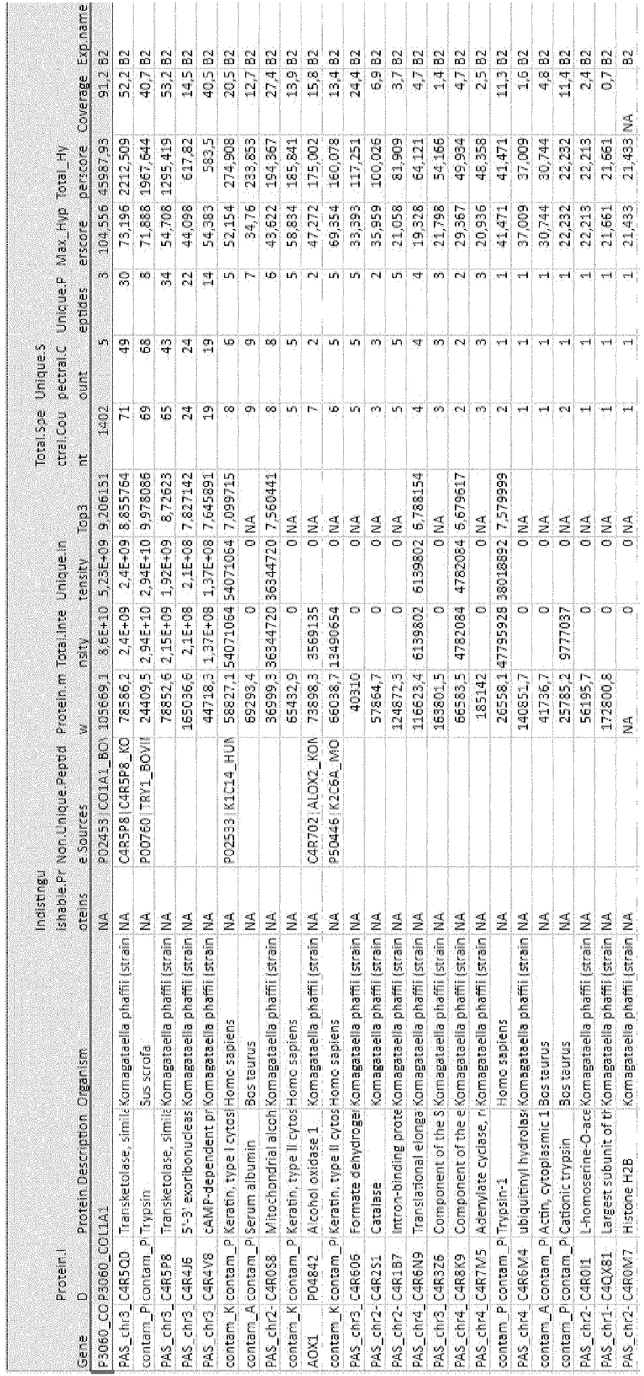

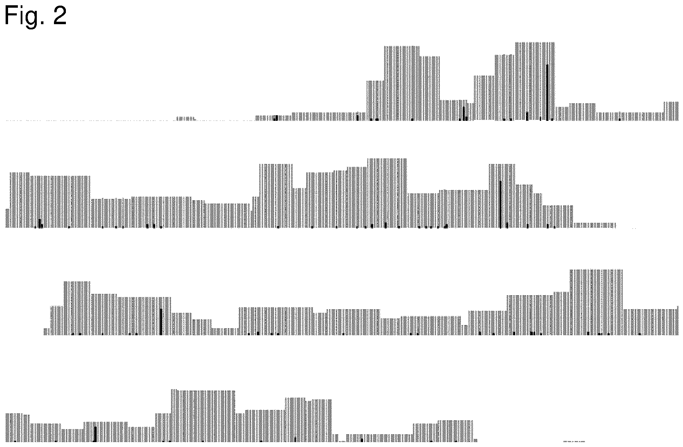

The hydroxylation pattern was investigated. The detected peptides were aligned to the sequence of recombinant collagen. Hydroxylated amino acid residues were detected. Besides proline hydroxylation, there is also a methionine hydroxylation that occurs during the preparation of samples. It was found that methionine hydroxylation is rather frequent. His-Tag was not detected. These results are shown in Figure 2.

The cultivating conditions of *Pichia pastoris* were optimized. It was found that cultivation at 20°C was better than cultivation at higher temperatures such as 28°C. The Western Blot analysis results (nitrocellulose (NC) membrane, 1000V, 55 min, blocking with Li-Cor TBS, + 5% milk for 1 hour, detection with His-antibody conjugated with horseradish peroxidase (HRP)) are depicted in Figure 17.

In a further development of the described purification of collagen, not only P. *pastoris* itself was used, but purification was also carried out from its supernatant. Thus, POI secreted by *P. pastoris,* here collagen, can also be obtained, increasing the overall yields. A procedure of the adapted process is depicted in Figure 18B and includes: collecting the supernatant from different batches; treatmeant with AS 40% (1h), precipitation. The obtained pellet is resuspended in extraction buffer (10-15% of the initial volume).

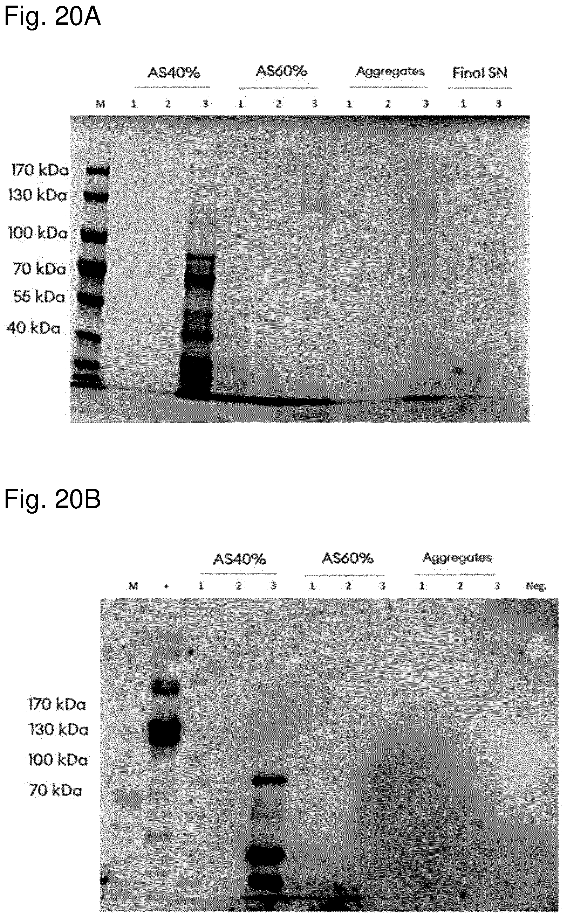

The supernatant is treated again with AS 60% (1h) and precipitated, the resulting pellet resuspended in extraction buffer (10-15% of the initial volume). Possible aggregates are removed from the supernatant and the final supernatant is obtained. All fractions were analyzed using SDS PAGE (Figure 20A) and Western blot (Figure 20B). AS 40% precipitation was able to concentrate most of the proteins and a collagen signal was detected in these AS 40% samples. Even though AS 60% and aggregates showed bands, no signal was clearly detected (Figure 20A). Collagen was analysed under denaturing conditions, resulting in a single Band at around 100 kDa in the western blot (Figure 20B).

### Conclusions:

Clones A8 and A11 express Collagen (intracellular) as stable clones. Purification with HisTrap column works efficiently, 20°C is the better cultivation temperature. Collagen was not only found intracellularly, but also in the supernatant and can be harvested to further increase yields.

### Example 6 - Comparison of different harvesting and purification method of collagen from plants

Different harvesting and purification methods were compared with each other.
(i) Method 1:
   Harvesting: Tobacco leaves frozen in liquid nitrogen, grounded and stored at -80°C. Extraction: 100mM Tris, pH 7.5 for 1h at 4°C. Sample-buffer ratio 1:3
   Purification:
      (1) AS 40% (1h) and AS 60% (1h) precipitation. Pellet resuspended in extraction buffer (10-15% initial volume
      (2) His purification. Collagen was detected in Flowthrough.
   Result: No collagen was detected when 50g batch was tested.
(ii) Method 2: Harvesting: Production of recombinant collagens by plants, in particular single-chain collagen type I α1 (I) and their use.
   Extraction: Acid Extraction 0.5M Acetic Acid
   Purification:
      (1) NaCl 0.7 M, NaCl 0.9 M Pellet resuspended in 0.5 M Acetic Acid.
      (2) Samples Insufficiently pure: dialysis in 0.5M Acetic acid + 2^{nd}
      (3) In case of further purification step: ion exchange (gradient elution 0-0.5M NaCl).
   Result: After precipitation with 0.7 M NaCl, then with 0.9 M NaCl the homotrimeric 80 to 90% pure collagen is present in the 0.9 M NaCl precipitate.
(iii) Method 3:
   Harvesting: 1 kg of tobacco leaves grounded with 2L chilled extraction buffer.
   Extraction: 100mM sodium phosphate buffer pH 7.5, 4.5 mM potassium Meta disulfite, 12.23 mM L-cystein and 7.5 mM EDTA.
   Purification:
      (1) 6.68 g charcoal and 16.67 g of PVPP were added to the extract and continuously stirred for 20 minutes
      (2) AS 15% (1h) and AS 25% (1h). Pellet resuspended in extraction buffer
      (3) Samples were digested with ficin (3h) and precipitated with two round of 3M NaCl (O.N.).
      (4) After precipitation, final pellet was resuspended in 10 mM HCl and dialysed against 10 mM HCl. Samples were filtered and concentrated
   Result: After digestion with ficin, the atelocollagen maintains its ability to form fibrils (at least 70 %).
(iv) Method 4:
   Harvesting: 450 g of tobacco leaves blended in 900 mL chilled extraction buffer.
   Extraction: 100 mM Tris-HCl, pH 7.5 containing 4.5 mM potassium meta bisulfite and 7.5 mM EDTA.
   Purification:
      (1) 3 g charcoal and 7.5 g of PVPP were added to the extract. Blending was performed in five intervals of 1 min each.
      (2) Extract was filtered through gauze pad, centrifuged and 10mM CaCl + 1g/L activated carbon was added.
      (3) Samples were digested with ficin and precipitated with 3.13 M NaCl (O.N.). Pellet was resuspended in 0.25 M Acid acid + 2 M NaCl for precipitation. Final pellet: 0.5 M Acetic acid.
      (4) Sample passed through 12 layers of gauze pad and precipitated with 3M NaCl. Final pellet was resuspended in 10 mM HCl and dialysed against 10 mM HCl. Samples were filtered + concentrated.
   Result: Human procollagen type I in plants by coexpression of human procollagen alpha 1 and alpha 2 chains together with human enzymes P4H alpha, P4H beta, and LH3.

### Example 7 - Downstream processing exemplified for collagen from Pichia pastoris

A general downstream processing flow schemes are depicted in Figures 18A and 19.

**Table 4. Exemplified process conditions**

| Harvesting | | | Cell Disruption | | | | Purificat ion | | | |
|---|---|---|---|---|---|---|---|---|---|---|
| Cultivation Parameters | Centrifugation | Lysis Buffer | Ratio | Disruption Parameters | Centrifugation | Filtration | FPLC Model | | | |
| 72h, 20 °C | 2000 g, 5 min | 50 mM Tris, 100 mM NaCl pH 8 + 10µL Protease Inhibitor/ mL buffer | ca. 1:3 but needs further investigation | Sonication: Probe MS 73, 60% A;plitude, 5 min 20s/on 20s/off | 18514 rcf, 30 min, -4°C | 0.45 µm syringe filter | Äkta Pure 25 | | | |

| | | | Buffer Exchange | | | | | SDS Page | | |
|---|---|---|---|---|---|---|---|---|---|---|
| Column | Elution Profile | Buffer | Device | MWCO | Centrifugation | Prot. Quant. mg/mL | Loading Conditions | Marker | SDS Gel Type | SDS Staining |
| His Trap HP | Linear Gradient | 1x PBS | Pall Micro/ Macrosep | 10 kDA | 4000 rcf, 4°c for at least 75 min /round | 0.5 was proven to work (WB) | Protocol dependent, NR conditions | Protein marker IV | Tris-Acetate 3-8%/ Tris-Glycine 7.5% | Coomassie (Page Blue) |

| | *Western Blot* | | | | | | | | | |
|---|---|---|---|---|---|---|---|---|---|---|
| WB Type and Time | WB Membrane | *WB Blocking* | *WB Detection* | | | | | | | |
| Wet Blot - 50 min 100V, Dry Blot - P0 (7min) | NC | 5% Milk (Filtered) in Intercept TBS | His 1:1000 + anti-mouse 1:10000 | | | | | | | |

In the general scheme as depicted in Figure 19, Tris (PVPP+EDTA) + NaCl precipitation of 1 g plan material and 1.1 mg collagen *Pichia* is used.

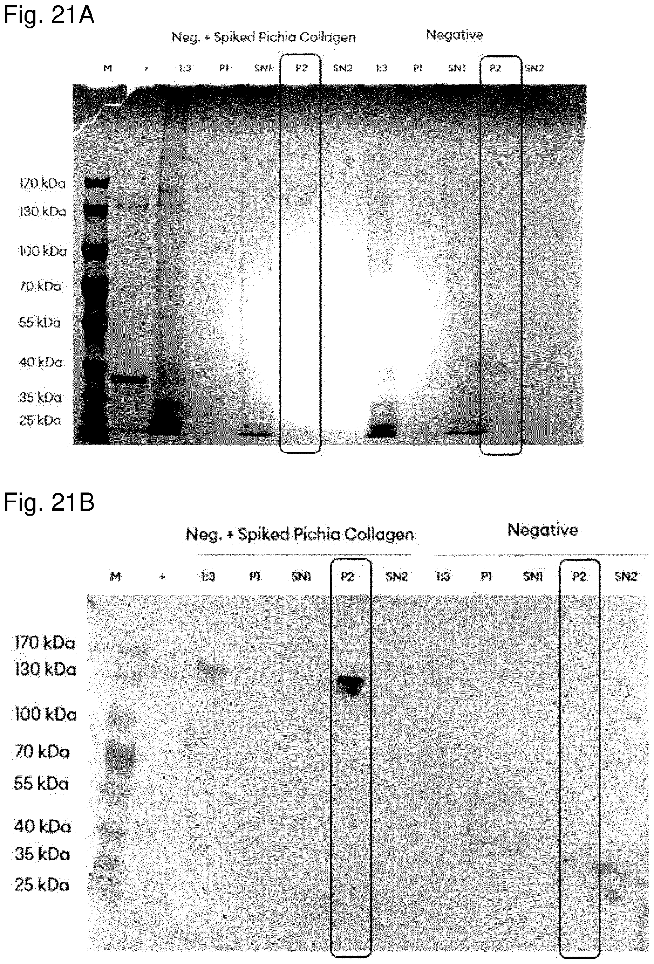

An SDS PAGE gel of the process as depicted in Figure 19 is shown in Figure 21A. Herein, a gel "Mini Protean TGX 7.5% Tris-glycine", a sample loading buffer of 10 µl sample in 3.3 µl LDS sample buffer and a Coomassie (Page blue) stain was used.

A Western Blot of the process as depicted in Figure 19 is shown in Figure 21B. A membrane 7.5% Tris-glycine gel, NC membrane was used. The method iBlot P0 was conducted for 7 min. LI-Cor TBS + 5% milk was used for blocking for 1 hour. Detection was performed by His antibody with horseradish peroxidase (HRP), 1:1000.

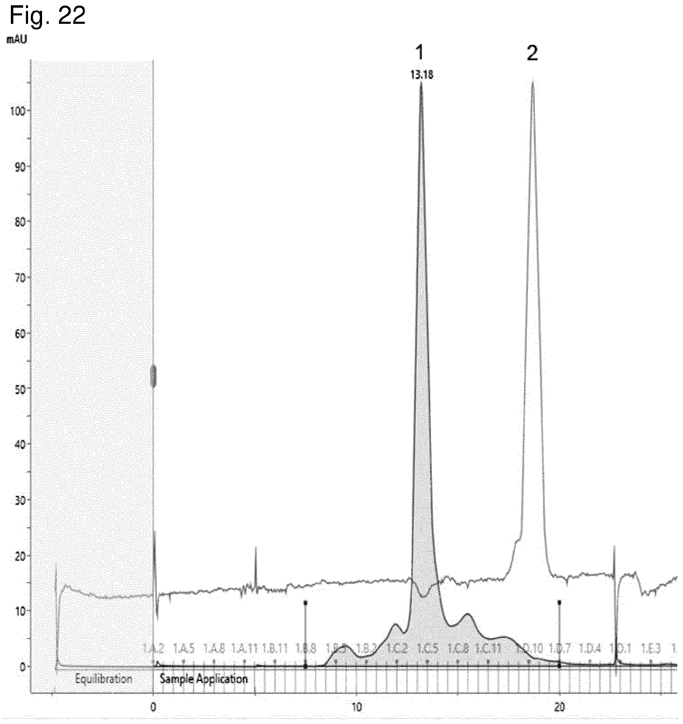

After previous large His-Tag purification from *Pichia pastoris,* the peak containing the collagen molecule showed additional bands (impurities). Size Exclusion Chromatography (SEC) and 3.13 M NaCl precipitation were tested to check protein profile separation. The results are depicted in Figures 22 and 23. It was found that SEC was successfully able to separate collagen from other proteins (Fractions B8-B6). Since collagen does not absorb at 280 nm, A6-B9 fractions were also tested but no band was detected in SDS PAGE. Protein quantification (A205 nm) and comparison to precipitation recovery may be conducted additionally. Purified Pichia Collagen was detected after spiking plant material. Gelation can be determined in rheological assays.

### Example 8 - Collagen purification from plants

A general process is shown in the flow scheme of Figure 24.

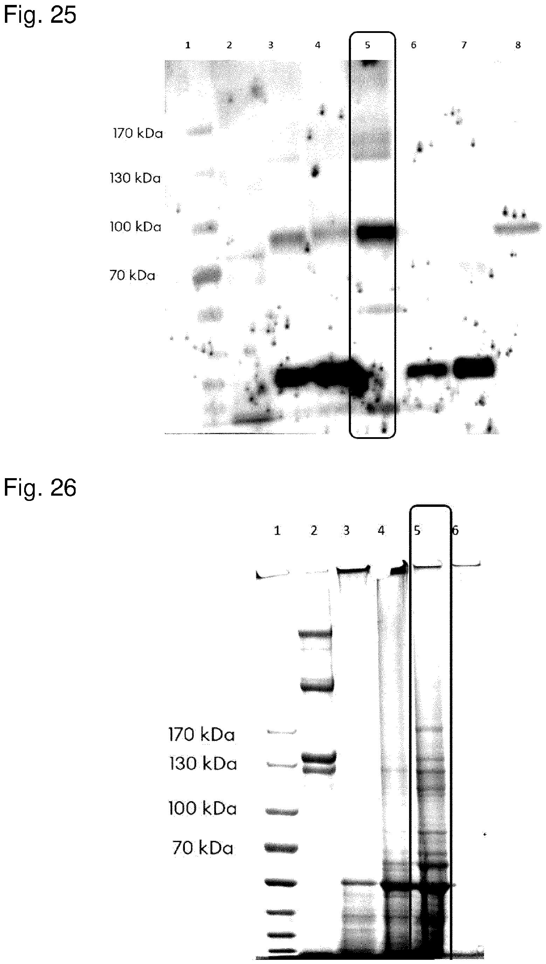

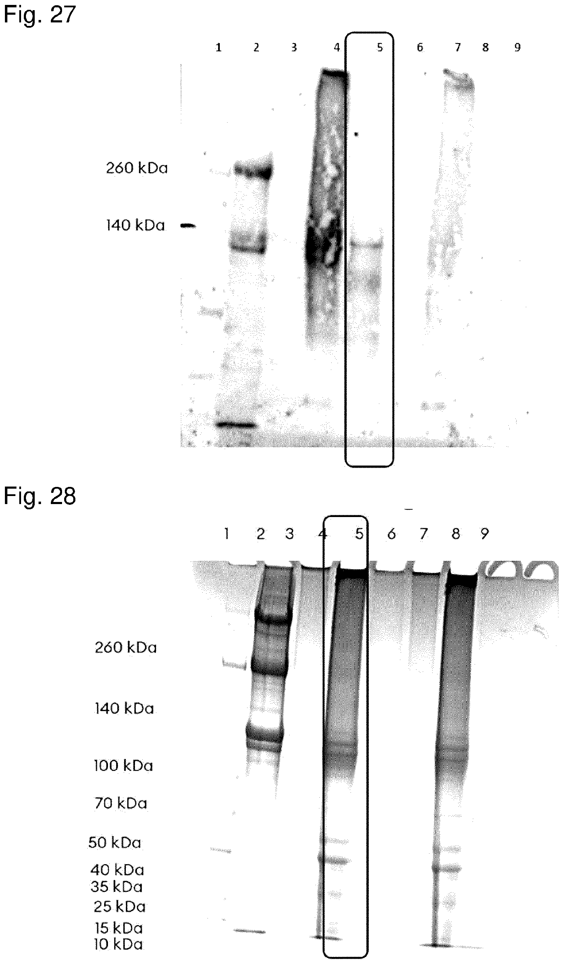

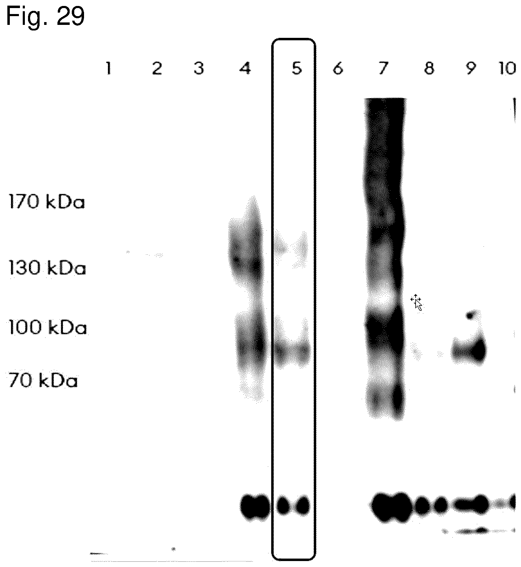

For analysis, different approaches were tested. In a first approach, plant material, more spefically plant leaves were collected, ground in liquid nitrogen and incubated in buffer, before freezing. Collagen extracted from the mixture using i) 100 mM Tris, pH 7.5 (1:3). Collagen precipitation was done using successively 40 and 60% ammonium sulfate. For analysis SDS PAGE and Western Blot were performed and a band of 130kDa (V1 construct, full length, including propeptides) was detected. For comparison see Figures 25 -29.

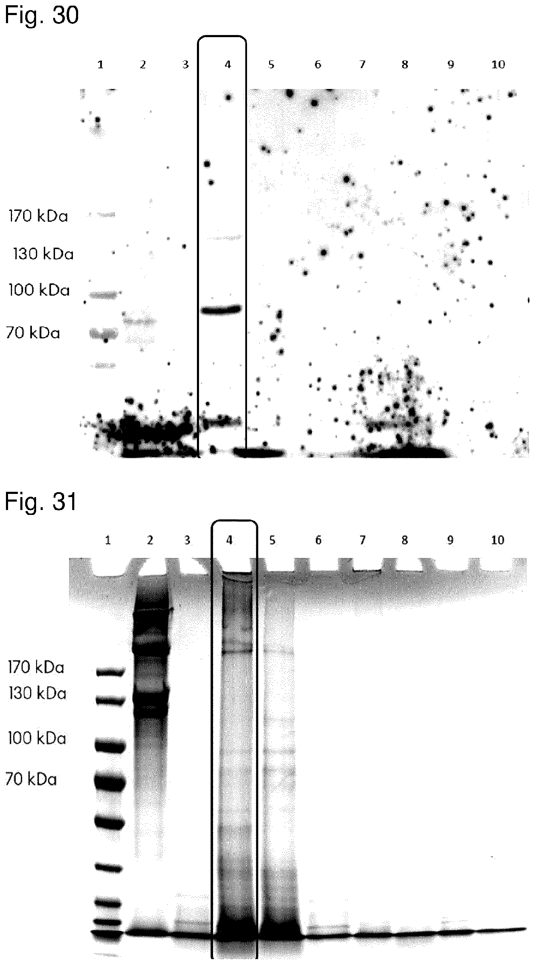

In a second approach plant material was extracted using 500 mM NaCl and collagen was precipitated using successively 400 and 900 mM NaCl. For analysis SDS PAGE and Western Blot were performed and a band of 130kDa (V1 construct, full length, including propeptides) was detected. For comparison see Figures 30, 31.

It was found that 100 mM Tris, pH 7.5 and AS 60% precipitation gave consistent results for collagen detection compared to Acid extraction and NaCl precipitation. Both 1:5 and 1:3 ratios showed positive bands on WB for AS Precipitation method. For scale-up, the less water to process, the better. Therefore, 1:3 ratio can be selected for further experiments. Positive controls were successfully detected when NC membrane was used. Therefore, this membrane can be selected for further experiments. Final protein purity still needs improvement. Chromatography techniques should increase protein purity. As alternative, investigation of different AS precipitation fractionations (10 - 60%) can be also performed.

General process conditions are at least 5 g pant material, extraction buffer: 1000 mM Tris, pH 7.5 in a dilution ratio of 1:3, precipitation in 60% ammonium sulfate, pellet (at least 10x), heating of the sample before conducting SDS PAGE.

### Example 9 - Optimization of Pichia pastoris purification method

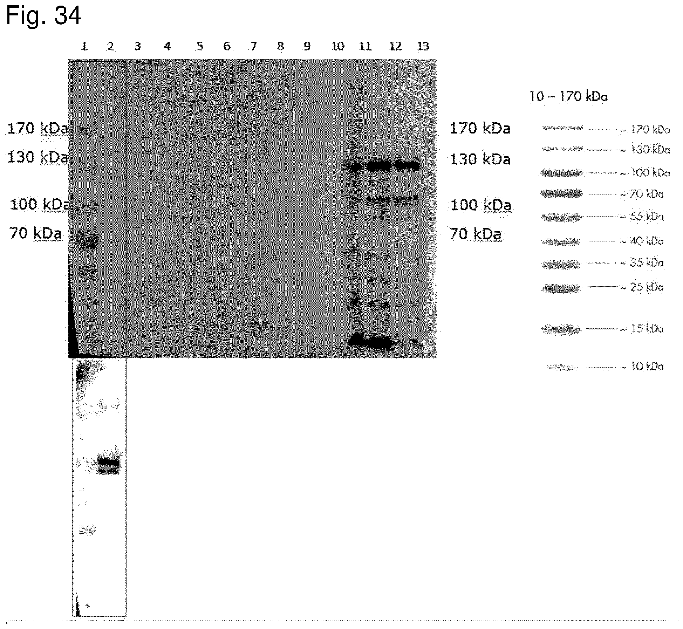

A scheme is shown in Figure 32. SDS PAGE (7.5 Tris-glycine, loading 21 µl sample +6-7 µl LDS buffer, 10 min, 70°C, 4-20% SDS PAGE) and Western Blot (0.45 µm nitrocellulose (NC), 1000 V for 65 min, blocking for 1 hour, detection via His-tag antibody 1:100, goat anti -mouse 1:10000) are performed. The results are depicted in Figures 33 and 34.

No bands were visible in the supernatant of the previous SDS-PAGE.

Expected MW for Collagen without propeptides in *P. pastoris:*
Without signal peptide 96.3 kDa
With signal peptide 105.6 kDa
Old Pichia Western Blots with His-Tag antibody have sometimes shown artifact bands in the upper part of the membrane, but they usually looked less specific.
Collagen usually appears larger on gels than its real MW
Collagen could be efficiently obtained from *Pichia Pastoris*

### Example 10 - Optimization of E. coli purification method

The method can be adapted to a Gibson assembly method:
Linearize pET151 by PCR without the FGF2 sequence. Amplify Col1A1 insert from plant plasmid
Dpnl digest of the original pET151 in the PCR mix
Run the Gibson assembly with the plasmid backbone and insert Transform E. coli Top10
Colony PCR or Sequencing for verification of obtained clones
Plasmid Mini-Prep
Transformation of E. coli BL21 or Shuffle T7

Performance conditions are 98°C - 30 sec, 98°C - 5 sec, 61°C - 5 sec, 72°C - 45 sec, 72°C - 1 min, and 4°C.

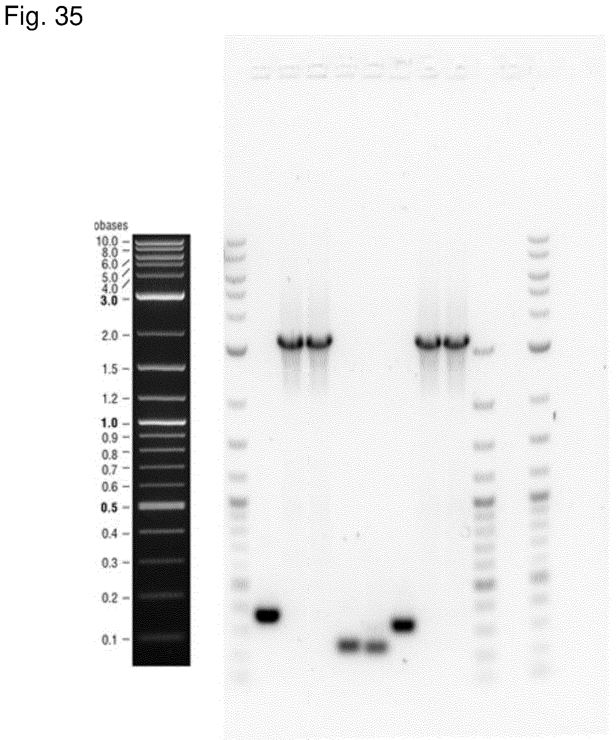

The results of an E. *coli* Top10 Colony-PCR after Gibson Assembly were investigated in a gel electrophoresis as depicted in Figure 35 with Primers 77/78, expected band sizes Col1A1 ca. 3200 bp.

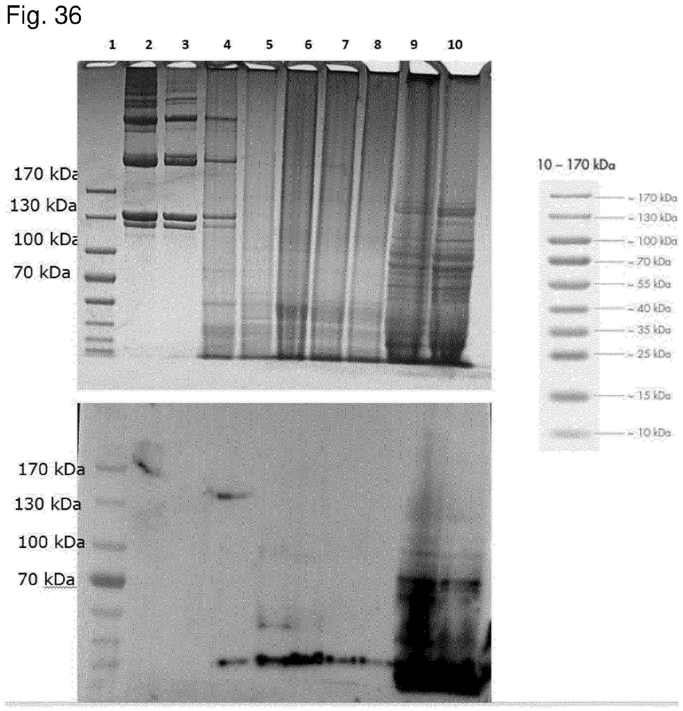

The results of SDS PAGE (3-8% Tris-acetate, sample loading 10 µl sample + 3.3. µl criterion SB, staining with Coomassie (Page Blue)) and Western Blot (0.45 µm PVDF membrane, 100 V for 55 min, blocking with Li-Cor TBS + 5% milk filtered for 1 hour, detection via Collagen detection kit) are depicted in Figure 36.

Collagen could be efficiently obtained from E. *coli.*

### Example 11 - Downstream processing and upscaling of the processes

It was considered to improve recombinant polypeptide such as collagen production in *P. pastoris* on a 100-1000 L scale followed by purification, and downstream processing of plant biomass on 100-1000 kg scale.

**Table 5. P. pastoris in comparison**

| Basic characteristics of different host systems for the expression of recombinant proteins | | | |
|---|---|---|---|
| **Characteristics** | ***Escherichia coli*** | ***Pichia pastoris*** | CHO **cell** |
| Doubling time | 30 min | 60-120 min | 24 hr |
| Cost of growth medium | Low | Low | High |
| Complexity of growth medium | Minimum | Minimum | Complex |
| Expression level | High | Low to high | Low to moderate |
| Extracellular expression | Secretion to periplasm | Secretion to medium | Secretion to medium |
| Protein folding | Refolding usually required | Refolding may be required | Proper folding |
| N-linked glycosylation | None | High mannose | Complex |
| O-linked glycosylation | No | Yes | Yes |
| Phosphorylation & acetylation | No | Yes | Yes |
| Drawback | Accumulation of LPS | Codon bias | Contamination with animal viruses |

| | | | |
|---|---|---|---|
| Abbreviations: CHO, Chinese hamster ovary; LPS, lipopolysaccharide. Reference: Mohsen Karbalaei, Seyed A. Rezaee, and Hadi Farsiani Pichia pastoris: A highly successful expression system for optimal synthesis of heterologous proteins. 2020 Sep; 235(9): 5867-5881. Link: ohttps://www.ncbi.nlm.nih.gov/pmc/articles/PMC7228273/ | | | |

**Table 6. SWOT analysis of P. pastoris platform technology**

| **Strengths** | **Weaknesses and Threats** | **Opportunities** |
|---|---|---|
| 1. Generally recognized as safe (GRAS _{*} status), robust organism | | |
| 2. Innate ability to secrete heterologous proteins | | |
| 3. A highly inducible promoter (alcohol oxidase) that can be easily exploited for recombinant protein production | 1. Low cellular productivity if not optimised | |
| 4., No Crabtree effects | 2. Protease release during fermentation | 1. Improved cellular productivity |
| 5. Ability to perform certain post-translational modifications | 3. Scale up requires large volume methanol handling and high oxygen input together with substantial heat generation | 2. Continuous culture with lower cell concentration and higher productivity, smaller footprint |
| 6. Suitable for platform manufacture | 4. Harvesting/dewatering of very high cell density results in instability in centrifugation | 3. Methanol-free systems |
| 7. Very high cell density achievable | | |
| 8. Low Cost of Goods compared to e.g., mammalian systems | | |
| 9. Relatively low secreted host cell protein (HCP) | | |
| 10. Absence of endotoxins/bacterio-phage contamination | | |

| | | |
|---|---|---|
| Reference: Salomé de Sá Magalhães andEli Keshavarz-Moore: Pichia pastoris (Komagataella phaffii) as a Cost-Effective Tool for Vaccine Production for Low- and Middle-Income Countries (LMICs). Bioengineering 2021, 8(9), 119. Link: https://www.mdpi.com/2306-5354/8/9/119. | | |

Cultivation and induction of protein expression:
- First cells a grown in a medium with glucose or glycerol to accumulate biomass, then switch to a medium with methanol
- Methanol is added for induction in "pulses" (typically 1-3% (v/v) per 24h)
- Good aeration is crucial during the methanol phase, dissolved oxygen should remain above 20%
- Pure O2 can be used in high density fermentations
- On a small-scale baffled shake-flasks should be used with high shaking speed

The number and type of unit operations may vary according to protein characteristics and final product purity required. Some columns can work directly with filtered supernatants. In case of intracellular protein production, a lysis step hast to be added.

For the proteins that are not secreted, the process would have to be complemented by a high-pressure homogenizer or similar equipment to lyse the cells. A detailed process scheme is depicted in Figure 38.

For instance 60 g plant or more may be used.

Scaling up from 10 to 100 liters for *P. pastoris* works. A flow scheme for preparing collagen from plants is shown in Figure 40.

**Table 7. Exemplified selection of collagen preparation in Nicotiana benthamiana**

| | | **Tusé et al.** | | | | | | |
|---|---|---|---|---|---|---|---|---|
| **Parameter** | **Unit** | | **(1)** | | **(2)** | **Walwyn et al.** | **Nandi et al.** | **Alam et al.** |
| Industry | - | Pharmaceutical | | Biofuel | | Reagent | Pharmaceutical | Pharmaceutical |
| Molecule | - | Butyrylcholin- esterase | | Cellulase enzyme | | Horseradish peroxidase enzyme | Monoclonal antibody | Antiviral Protein |
| Expression system | - | Transient; agroinfiltration | | Transgenic; inducible | | Transient; agroinfiltration | Transient; agroinfiltration | Transient; viral vector |
| Production | kg/year | 25 | | 3x10^6 | | 5 | 300 | 20 |
| Expression system | g/kg FW | 0.5 | | 4 | | 0.24 | 1 | 0.52 |
| Recovery | % | 20 | | - | | 54 | 65 | 70 |
| Purity | % | >95 | | - | | 250 kU/g | >95 | >99 |
| CAPEX | $ million | 92.4 (U/D 3:7) | | 11.5(U/D 10:0) | | - | 122 (U/D 4:6) | - |
| COGS | $/g | 1,180 (U/D 3:7) | | 6.9×10^-3 (U/D 10:0) | | 1,279 (U/D 2:8) | 90-121 (U/D 4:6) | 105.80 (U/D 6:4) |

| | | | | | | | | |
|---|---|---|---|---|---|---|---|---|
| Abbreviations: BChE, butyrylcholinesterase; CAPEX, capital expenditures; COGS, cost of goods sold; HRP, horseradish peroxidase; mAb, monoclonal antibody; U/D, ratio of upstream to downstream costs Reference: Matthew J. McNulty, Yuri Gleba, Daniel Tusé, Simone Hahn-Löbmann, Anatoli Giritch, Somen Nandi, Karen A. McDonald: Techno-economic analysis of a plant-based platform for manufacturing antimicrobial proteins for food safety. iotechnology Progress. 2020;36:e2896. https://doi.org/10.1002/btpr.2896. Link: https://aiche.onlinelibrary.wiley.com/doi/pdf/10.1002/btpr.2896. | | | | | | | | |

A detailed flow scheme for an example of upscaling is depicted in Figure 41.

In gerenal, the upscaled purification process for 1 kg plant mass would encompass the following steps:
1. 1 kg of transgenic tobacco leaves are ground with pre chilled 2 liters extraction buffer in a 4L reactor (ESCO model EL-3) for 20 minutes
2. Ammonium sulphate precipitation
3. Centrifugation
4. 3M NaCl precipitation
5. Filtration.

Harvesting and Collection can be done as described below and published in e.g. WO2009053985A1 and referenced in Fig. 37-41.

Collect a substantial amount of plant leaves from your source, ensuring that they are fresh and healthy.

### Cleaning and Preparation:

Remove any dirt, debris, or contaminants from the leaves.

Wash the leaves with water to eliminate surface impurities.

### Homogenization:

Grind or homogenize the cleaned leaves to create a plant leaf extract. You can use a large-scale blender, grinder, homogenizer or double stack disintegrator for this purpose.

### Extraction:

Transfer the homogenized leaf material into a suitable extraction buffer. The choice of buffer may depend on the type of proteins you are targeting.

Extract the proteins by shaking, stirring, or agitating the mixture. This step helps solubilize the proteins into the buffer.

### Filtration:

Exemplified process in detail:
Filter the plant extract to remove any remaining solid particles or debris, obtaining a clear protein-containing solution.

### (Optional Centrifugation:)

Centrifuge the protein extract to separate the soluble proteins from cell debris and other insoluble components. The supernatant, containing the soluble proteins, is collected.

### Precipitation or Salting Out:

Depending on the proteins of interest, you may need to precipitate them using techniques like ammonium sulfate precipitation or acetone precipitation. This step helps concentrate the proteins.

### (Optional) Chromatography:

Perform chromatography, such as size exclusion chromatography, ion exchange chromatography, or affinity chromatography, to further purify and separate the target proteins based on their properties.

### Elution and Collection:

Elute the purified proteins from the chromatography columns, collecting fractions containing the target proteins.

### Dialysis or Buffer Exchange:

Dialyze the protein fractions or perform buffer exchange to transfer the purified proteins into a suitable storage or assay buffer while removing unwanted substances.

### Concentration:

Concentrate the purified protein solutions to achieve the desired concentration, if necessary.

### Analysis and Characterization:

Analyze the purified proteins for purity, concentration, and integrity using techniques like SDS-PAGE, Western blotting, or mass spectrometry.

### Storage:

Store the purified proteins in aliquots at appropriate temperatures and conditions to maintain their stability and activity.

Processing greenhouse-propagated plants provides 25-75 g purified product at an expected yield of 250 mg/kg fresh weight of plant materials.

### Example 12 - Determining the rheological properties of collagen

It has been verified that collagen has significant thickening properties.

Method establishment for testing and analysing the gelation of collagen.

The measurements were conducted using an Anton Paar MCR 90 Rheometer with a CP25-1 (25 mm, 1°) probe attached. PureCol^{™} 5 mg/mL was used for a proof-of-concept method establishment to measure the gel strength of a heat induced gelation of collagen.

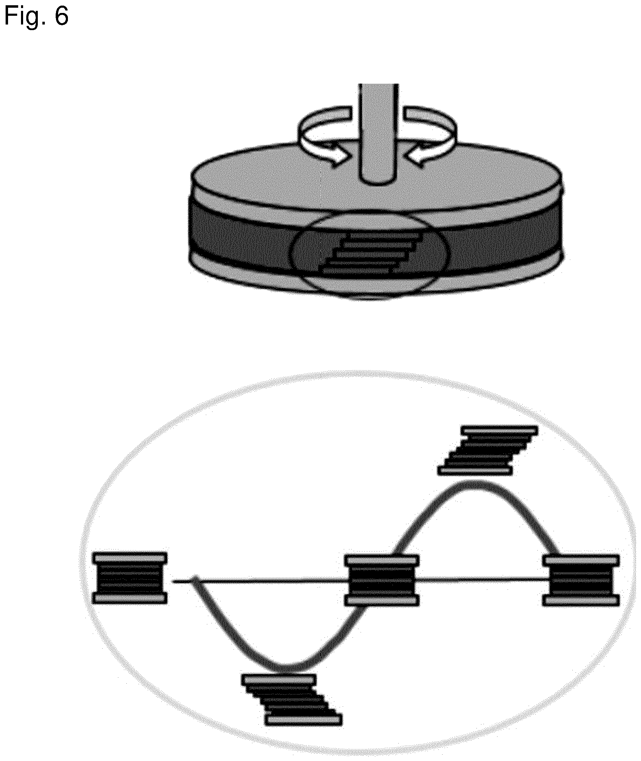

Prior to measurement, the sample needs be cooled at 4°C and a neutral pH 7 +/-0.5 must be ensured. The measuring plate was set to 4°C before putting 150 µL of the cooled collagen on the surface. The surrounding area was covered with water and a self-made solvent trap (yoghurt cup) was placed around the sample and the probe without touching it. The principle of measurement is depicted in Figure 6.

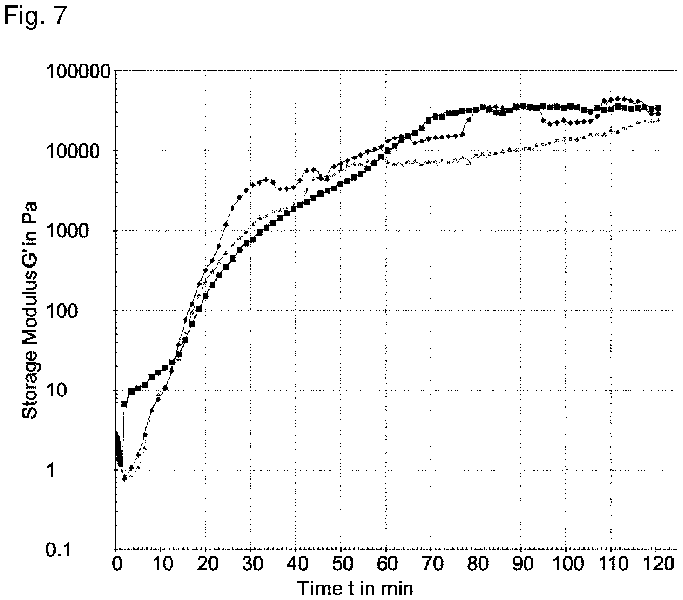

Within the method a pre-shear with a shear rate y* = 0.5 [1/s] was applied to ensure homogeneity. The shear test was conducted using two intervals. The first interval uses a shear strain (oscillating) y = 3 [%], an angular frequency w = 10 [1/s] and a linear temperature gradient from 4°C to 37°C in 60 s. The second interval uses a shear strain (oscillating) y = 3 [%], an angular frequency w = 10 [1/s] and a constant temperature of 37°C for 120 min. The storage modulus G' [Pa] over time was tracked as output function. The experiment was performed as triplicate. The results are shown in Figure 7.

## Claims

1. A non-animal organism expressing a collagen that naturally occurs extracellularly in one or more animal species.

2. The non-animal organism of claim 1, wherein the non-animal organism comprises the genetic material encoding for the collagen permanently, in particular wherein the genetic material is incorporated in the non-animal organism's genome.

3. The non-animal organism of any one of claims 1 or 2, wherein the non-animal organism is selected from the group consisting of:
(A) a plant, in particular a cultivated plant, in particular tobacco or soy;
(B) a fungus, preferably a yeast cell, in particular *Pichia pastoris;* or
(C) a bacterium, in particular an *Escherichia coli* bacterium.

4. The non-animal organism of any one of claims 1 to 3, wherein the collagen has at least 80%, at least 85%, at least 90%, at least 95%, at least 98%, at least 99%, or 100% homology (or identity) of an extracellular matrix polypeptide/ protein such as collagen, more preferably wherein the extracellular matrix collagen is selected from mammal extracellular matrix collagen and bird extracellular matrix collagen.

5. The non-animal organism of any one of claims 1 to 4, wherein the collagen is a protein that is comprised in vegetarian or vegan animal-based comestible nutrient, in particular a vegetarian animal-based comestible nutrient selected from the group consisting of candy, ice cream, a bakery good, a dip, a diary product, aspic, a vegetarian sausage, cream cheese, jam, spread, margarine and/or a sauce.

6. The non-animal organism of any one of claims 1 to 5, wherein the expressed collagen is located intracellularly in the non-animal organism.

7. The non-animal organism of any one of claims 1 to 6, wherein the expressed collagen is located extracellularly in the non-animal organism.

8. The non-animal organism of any one of claims 1 to 7, wherein the expressed collagen contains a tag, in particular a His-tag.

9. A method for preparing a collagen that naturally occurs extracellularly in one or more animal species, comprising the steps of:
(i) providing a non-animal organism comprising genetic information encoding for the collagen that naturally occurs extracellularly in one or more animal species;
(ii) expressing the collagen in the non-animal organism and optionally subjecting the collagen to one or more posttranslational modifications.

10. The method of claim 9, further comprising one or more of the following:
harvesting the non-animal organism that has expressed the collagen; isolating the collagen from the non-animal organism or cell-culture medium in which the non-animal organism is cultivated;
preserving the collagen or composition comprising such from spoiling; and/or freeze-drying or drying of the collagen or composition comprising such.

11. The method of any one of claims 9 or 10, wherein the method comprises purification the collagen by affinity chromatography.

12. The method of any one of claims 9 to 11, wherein the method further comprises:
(iii) preparing a solution of the collagen in dissolved form, optionally filtering and/or centrifugation of the solution to remove any remaining solid particles or debris and obtaining a clear collagen-containing solution, and optionally precipitating the collagen;
(iv) isolating the collagen of step (iii) via chromatography and collecting collagen-containing fractions;
(v) optionally subjecting the collagen of step (iv) to dialysis or buffer exchange vis a size exclusion chromatography column.

13. The method of any one of claims 9 to 12, wherein the method further comprises isolating the collagen by affinity chromatography, preferably by an antibody and/or by binding to a tag attached to the collagen, in particular a His-tag.

14. A method for preparing a comestible nutrient product, in particular a vegetarian comestible nutrient product, comprising the step of adding the collagen that naturally occurs extracellularly in one or more animal species obtained from a method of any one of claims 9 to 13 to one or more further comestible nutrient ingredients.

15. A comestible nutrient product, in particular a vegetarian comestible nutrient product, comprising at least one collagen that naturally occurs extracellularly in one or more animal species obtained from a method of any one of claims 9 to 13.
